# EUROPEAN PATENT APPLICATION

(11) **EP 1 902 733 A1**
(43) Date of publication of application: **26.03.2008**
(21) Application number: 06384014.4
(22) Date of filing: 19.09.2006
(51) Int. Cl.: A61K 45/06, A61P 3/04, A61P 3/10, A61P 1/00, A61P 9/00, A61P 25/16, A61P 25/28, A61P 37/02

(54) **Combination of a NMDA-receptor ligand and a compound with 5-HT6 receptor affinity**

(71) Applicant: LABORATORIOS DEL DR. ESTEVE, S.A., 08041 Barcelona (ES)
(72) Inventor: Buschmann, Helmut Heinrich, E-08960 San Just Desvern, Barcelona (ES); Codony-Soler, Xavier, E-08301 Mataro (ES)
(74) Representative: ABG Patentes, S.L.

(57) **Abstract**

The present invention relates to an active substance combination comprising at least one compound with 5-HT₆ receptor affinity, and at least NMDA-receptor ligand, a medicament comprising said active substance combination, and the use of said active substance combination for the manufacture of a medicament.

## Description

The present invention relates to an active substance combination comprising at least one compound with 5-HT₆ receptor affinity, and at least one N-methyl-D-aspartate-receptor ligand (NMDA-receptor ligand), a medicament comprising said active substance combination, and the use of said active substance combination for the manufacture of a medicament.

Cognitive and/or degenerative brain disorders are characterized clinically by progressive loss of memory, cognition, reasoning, judgement and emotional stability that gradually leads to profound mental deterioration and ultimately death. In an example of such disorders, Alzheimer's disease is a common cause of progressive mental failure (dementia) in aged humans and is believed to represent the fourth most common medical cause of death in the United States. In particular, Alzheimer's disease is associated with degeneration of cholinergic neurons in the basal forebrain that play a fundamental role in cognitive functions, including memory. Cognitive and/or degenerative brain disorders have been observed in varied races and ethnic groups world-wide and presents a major public health problem. These diseases are currently estimated to affect about two to three million individuals in the United States alone and the occurrence will increase world-wide as the human life span increases.

Cognitive and/or degenerative brain disorders are incurable with presently used medications, however, the symptoms of these disorders seem to be possibly alleviated by using compounds such as memantine.

Whereas known compounds which act as NMDA-receptor ligands are generally effective for treating disorders related to NMDA-receptors such as cognitive disorders, in particular for treating Alzheimer's disease, in some instances they show undesirable side effects. Specifically, many of these compounds that have been tested in humans can cause potentially serious side effects such as gastrointestinal complications including insomnia, restlessness, headache, akathisia, fatigue, nausea, emesis, ulcers, constipation, flatulence, diarrhea, hypertension, respiratory depression and psychological and physical dependence.

It was therefore an object of the present invention to provide a medicament suitable for the prophylaxis and/or treatment of disorders related to NMDA-receptors and to 5-HT₆ receptors, which preferably does not show the undesired side effects of the conventional compounds which act as NMDA-receptor ligands, or at least less frequent and/or less pronounced.

In particular, it was an object of the present invention to provide a medicament suitable for the prophylaxis and/or treatment of cognitive disorders, in particular for treating Alzheimer's disease, which preferably does not show the undesired side effects of the conventional medicaments for the prophylaxis and/or treatment of cognitive disorders, in particular for treating Alzheimer's disease, or at least less frequent and/or less pronounced.

Said object has been achieved by providing an active substance combination comprising
(A) at least one compound with 5-HT₆ receptor affinity,
   and
(B) at least one NMDA-receptor ligand.

It has surprisingly been found that the compounds with 5-HT₆ receptor affinity and the compounds which act as NMDA-receptor ligands show a synergistic effect in their pharmacological activities. Consequently, the dose of the corresponding compounds may be reduced in comparison to the dose necessary for an individual administration of said compounds.

According to the invention it has also been found that the action of a NMDA-receptor antagonist potentiates the action of the compound with 5-HT₆ receptor affinity, so the combination of a NMDA-receptor antagonist and a compound with 5-HT₆ receptor affinity for use in the treatment of disorders that are related to NMDA-receptors, and to 5-HT₆ receptors may result in a faster onset of action and an increased success rate. The invention therefore particularly resides in the combined action of a NMDA-receptor compound, particularly an antagonist, and a compound with 5-HT₆ receptor affinity, or the dual action of a substance possessing both NMDA-receptor antagonist activity and 5-HT₆ receptor affinity, for the treatment of disorders that are related to NMDA-receptors, and/or to 5-HT₆ receptors.

In the treatment of cognitive disorders, the effect on e.g. memory or novel object discrimination is significantly greater in the group that is treated with a combination of at least one NMDA-receptor antagonist and at least one compound with 5-HT₆ receptor affinity than in the group that is treated with at least one NMDA-receptor antagonist or at least one compound with 5-HT₆ receptor affinity exclusively.

Also there is an indication that in the treatment of depression, the effect on the symptoms - in an animal model - is more pronounced in the group that is treated with a combination of at least one NMDA-receptor antagonist and at least one compound with 5-HT₆ receptor affinity than in the group that is treated with at least one NMDA-receptor antagonist or at least one compound with 5-HT₆ receptor affinity exclusively.

In one embodiment of the present invention the binding of compounds present as component (A) to the 5-HT₆-receptor is determined by a Kᵢ value of less than 7000 nM, particularly preferably of less than 6500 nM, more particularly preferably of less than 200 nM, more particularly preferably of less than 100 nM.

In one embodiment of the present invention, the compounds present as component (B) act as NMDA-receptor antagonists. The NMDA-receptor antagonist of the invention may be any ligand that binds to and inhibits the NMDA-receptor, thereby resulting in a biological response. The potential of a given substance to act as a NMDA-receptor antagonist may be determined using standard in vitro binding assays and/or standard in vivo functionality tests.

In one embodiment of the present invention the binding of compounds present as component (B) to the NMDA-receptor is determined by an EC₅₀ or IC₅₀ value of less than 300 µM, preferably less than 100 µM, when determined in a standard functionality assay using a mouse, rat, or human NMDA-receptor ion channel.

In one embodiment the NMDA-receptor antagonist blocks the NMDA receptor at the PCP binding site

In another embodiment of the present invention as component (A) at least one compound is present, which is selected from the group consisting of the benzoxazinone-derived sulfonamide compounds of general formula (1a) 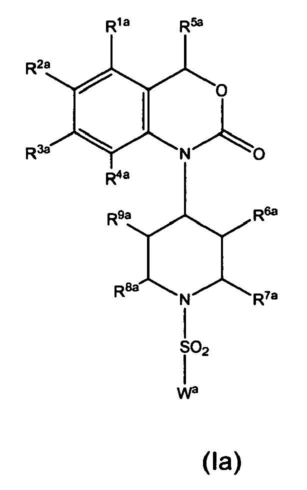 wherein
R^{1a}, R^{2a}, R^{3a} and R^{4a}, independently of one another, each represent a hydrogen atom; halogen; an unbranched or branched, saturated or unsaturated, unsubstituted or at least mono-substituted aliphatic radical; a saturated or unsaturated, unsubstituted or at least mono-substituted, optionally at least one heteroatom as ring member containing cycloaliphatic radical, which may be bonded via an unsubstituted or at least mono-substituted alkylene group and/or which may be condensed with an unsubstituted or at least mono-substituted mono- or bicyclic ring system; an unsubstituted or at least mono-substituted aryl- or heteroaryl radical, which may be bonded via an unsubstituted or at least mono-substituted alkylene group and/or which may be condensed with an unsubstituted or at least mono-substituted mono-or bicyclic ringsystem; nitro; cyano; -O-R^{10a}; -O-(C=O)-R^{11a}; -(C=O)-OR^{11a}; -SR^{12a};-SOR^{12a}; -SO₂R^{12a;}, -NH-SO₂R^{12a}; -SO₂NH₂ or -NR^{13a}R^{14a} ;
R^{5a} represents a hydrogen atom; an unbranched or branched, saturated or unsaturated, unsubstituted or at least mono-substituted aliphatic radical or a saturated or unsaturated, unsubstituted or at least mono-substituted, optionally at least one heteroatom as ring member containing cycloaliphatic radical;
R^{6a}, R^{7a}, R^{8a}, R^{9a}, independently of one another, each represent a hydrogen atom; an unbranched or branched, saturated or unsaturated, unsubstituted or at least mono-substituted aliphatic radical; a saturated or unsaturated, unsubstituted or at least mono-substituted, optionally at least one heteroatom as ring member containing cycloaliphatic radical; a cyano group or a -C(=O)-OR^{15a} moiety;
W^{a} represents an unbranched or branched, saturated or unsaturated, unsubstituted or at least mono-substituted aliphatic radical;
a saturated or unsaturated, unsubstituted or at least mono-substituted, optionally at least one heteroatom as ring member containing cycloaliphatic radical, which may be bonded via an optionally mono-substituted alkylene group and/or which may be condensed with an unsubstituted or at least mono-substituted mono- or bicyclic ring system;
an unsubstituted or at least mono-substituted aryl or heteroaryl radical, which may be bonded via an unsubstituted or at least mono-substituted alkylene or alkenylene group and/or which may be condensed with an unsubstituted or at least mono-substituted mono- or bicyclic ring system;
a -NR^{16a}R^{17a} moiety, or
a -C(=O)-R^{18a} moiety;
R^{10a} represents a hydrogen atom; an unbranched or branched, saturated or unsaturated, unsubstituted or at least mono-substituted aliphatic radical; a saturated or unsaturated, unsubstituted or at least mono-substituted, optionally at least one heteroatom as ring member containing cycloaliphatic radical, which may be bonded via an unsubstituted or at least mono-substituted alkylene group and/or which may be condensed with an unsubstituted or at least mono-substituted mono- or bicyclic ring system; or an unsubstituted or at least mono-substituted aryl or heteroaryl radical, which may be bonded via an unsubstituted or at least mono-substituted alkylene group and/or which may be condensed with an unsubstituted or at least mono-substituted mono- or bicyclic ring system;
R^{11a} represents a hydrogen atom; an unbranched or branched, saturated or unsaturated, unsubstituted or at least mono-substituted aliphatic radical; a saturated or unsaturated, unsubstituted or at least mono-substituted, optionally at least one heteroatom as ring member containing cycloaliphatic radical, which may be bonded via an unsubstituted or at least mono-substituted alkylene group and/or which may be condensed with an unsubstituted or at least mono-substituted mono- or bicyclic ring system; or an unsubstituted or at least mono-substituted aryl or heteroaryl radical, which may be bonded via an unsubstituted or at least mono-substituted alkylene group and/or which may be condensed with an unsubstituted or at least mono-substituted mono- or bicyclic ring system;
R^{12a} represents an unbranched or branched, saturated or unsaturated, unsubstituted or at least mono-substituted aliphatic radical; a saturated or unsaturated, unsubstituted or at least mono-substituted, optionally at least one heteroatom as ring member containing cycloaliphatic radical, which may be bonded via an unsubstituted or at least mono-substituted alkylene group and/or which may be condensed with an unsubstituted or at least mono-substituted mono- or bicyclic ring system; or an unsubstituted or at least mono-substituted aryl or heteroaryl radical, which may be bonded via an unsubstituted or at least mono-substituted alkylene group and/or which may be condensed with an unsubstituted or at least mono-substituted mono-or bicyclic ring system;
R^{13a} and R^{14a}, independently of one another, each represent a hydrogen atom; an unbranched or branched, saturated or unsaturated, unsubstituted or at least mono-substituted aliphatic radical; a saturated or unsaturated, unsubstituted or at least mono-substituted, optionally at least one heteroatom as ring member containing cycloaliphatic radical, which may be bonded via an unsubstituted or at least mono-substituted alkylene group and/or which may be condensed with an unsubstituted or at least mono-substituted mono- or bicyclic ring system; or an unsubstituted or at least mono-substituted aryl or heteroaryl radical, which may be bonded via an unsubstituted or at least mono-substituted alkylene group and/or which may be condensed with an unsubstituted or at least mono-substituted mono- or bicyclic ring system;
or R^{13a} and R^{14a} together with the bridging nitrogen atom form a saturated, unsaturated or aromatic heterocyclic ring, which is unsubstituted or at least mono-substituted and/or which may contain at least one further heteroatom as a ring member;
R^{15a} represents a hydrogen atom; an unbranched or branched, saturated or unsaturated, unsubstituted or at least mono-substituted aliphatic radical; a saturated or unsaturated, unsubstituted or at least mono-substituted, optionally at least one heteroatom as ring member containing cycloaliphatic radical or an unsubstituted or at least mono-substituted aryl or heteroaryl radical, which may be bonded via an unsubstituted or at least mono-substituted alkylene group and/or which may be condensed with an unsubstituted or at least mono-substituted mono- or bicyclic ring system;
R^{16a} represents an unbranched or branched, saturated or unsaturated, unsubstituted or at least mono-substituted aliphatic radical;
R^{17a} represents an unbranched or branched, saturated or unsaturated, unsubstituted or at least mono-substituted aliphatic radical, and
R^{18a} represents an unsubstituted or at least mono-substituted aryl radical;
optionally in form of one of its stereoisomers, preferably enantiomers or diastereomers, its racemate or in form of a mixture of at least two of its stereoisomers, preferably enantiomers or diastereomers, in any mixing ratio, or a physiologically acceptable salt thereof, or a solvate, respectively.

Preferred compounds of general formula (Ia) are those, wherein
R^{1a}, R^{2a}, R^{3a} and R^{4a}, independently of one another, each represent a hydrogen atom; a fluorine atom; a chlorine atom; a bromine atom; a methyl group or a methoxy group;
R^{5a} represents a hydrogen atom;
R^{6a}, R^{7a}, R^{8a} and R^{9a} each represent a hydrogen atom;
W^{a} represents
an alkyl radical selected from the group consisting of methyl; ethyl; n-propyl; isopropyl; n-butyl; sec-butyl; isobutyl and tert-butyl; vinyl (CH₂=CH-); -N(CH₃)₂; 1-naphthyl; benzyl; 2-naphtyl; phenyl; 2-methyl-phenyl; 3-methyl-phenyl; 4-methyl-phenyl; 2-ethyl-phenyl; 3-ethyl-phenyl; 4-ethyl-phenyl; 2-n-propyl-phenyl; 3-n-propyl-phenyl; 4-n-propyl-phenyl; 2-isopropyl-phenyl; 3-isopropyl-phenyl; 4-isopropyl-phenyl; 2-n-butyl-phenyl; 3-n-butyl-phenyl; 4-n-butyl-phenyl; 2-isobutyl-phenyl; 3-isobutyl-phenyl; 4-isobutyl-phenyl; 2-tert-butyl-phenyl; 3-tert-butyl-phenyl; 4-tert-butyl-phenyl; 1,1-dimethylpropyl-phenyl; 2-cyclopentyl-phenyl; 3-cyclopentyl-phenyl; 4-cyclopentyl-phenyl 2-cyclohexyl-phenyl; 3-cyclohexyl-phenyl; 4-cyclohexyl-phenyl; 2-methoxy-phenyl; 3-methoxy-phenyl; 4-methoxy-phenyl; 2-ethoxy-phenyl; 3-ethoxy-phenyl; 4-ethoxy-phenyl; 2-n-propoxy-phenyl; 3-n-propoxy-phenyl; 4-n-propoxy-phenyl; 2-iso-propoxy-phenyl; 3-iso-propoxy-phenyl; 4-isopropoxy-phenyl;2-fluoro-phenyl; 3-fluoro-phenyl; 4-fluoro-phenyl; 2-chloro-phenyl; 3-chloro-phenyl; 4-chloro-phenyl; 2-bromo-phenyl; 3-bromo-phenyl; 4-bromo-phenyl; 2-trifluoromethyl-phenyl; 3-trifluoromethyl-phenyl; 4-trifluoromethyl-phenyl; 2-trifluoromethoxy-phenyl; 3-trifluoromethoxy-phenyl; 4-trifluoromethoxy-phenyl; 2-carboxy-phenyl; 3-carboxy-phenyl; 4-carboxy-phenyl; 2-acetyl-phenyl; 3-acetyl-phenyl; 4-acetyl-phenyl; 2-(C=O)-O-CH₃-phenyl; 3-(C=O)-O-CH₃-phenyl; 4-(C=O)-O-CH₃-phenyl; 2-(CH₂)-(CH₂)-(C=O)-O-CH₃-phenyl; 3-(CH₂)-(CH₂)-(C=O)-O-CH₃-phenyl; 4-(CH₂)-(CH₂)-(C=O)-O-CH₃-phenyl; 2-cyano-phenyl; 3-cyano-phenyl; 4-cyano-phenyl; 2-nitro-phenyl; 3-nitro-phenyl; 4-nitro-phenyl; 4-(4-bromophenoxy)-phenyl; 2-methylsulfonyl-phenyl; 3-methylsulfonyl-phenyl; 4-methylsulfonyl-phenyl; 2-phenyl-phenyl (biphenyl-2-yl); 3-phenyl-phenyl (biphenyl-3-yl); 4-phenyl-phenyl (biphenyl-4-yl); 2-phenoxy-phenyl; 3-phenoxy-phenyl; 4-phenoxy-phenyl; 2,4-dimethyl-phenyl; 3,4-dimethyl-phenyl; 2,4,6-trimethylphenyl; 2,3,5,6-tetramethyl-phenyl; pentamethyl-phenyl; 2,5-dimethoxy-phenyl; 3,4-dimethoxy-phenyl; 2,3-dichloro-phenyl; 2,4-dichloro-phenyl; 2,5-dichloro-phenyl; 3,4-dichloro-phenyl; 3,5-dichloro-phenyl; 2,6-dichloro-phenyl; 2,4-difluoro-phenyl; 3,4-difluoro-phenyl; 2,5-difluoro-phenyl; 2,6-difluoro-phenyl; 3-chloro-2-fluoro-phenyl; 3-chloro-4-fluoro-phenyl; 5-chloro-2-fluoro-phenyl; 2,3,4-trichloro-phenyl; 2,4,5-trichloro-phenyl; 2,4,6-trichloro-phenyl; 2,4,5-trifluoro-phenyl; 2,3,4-trifluoro-phenyl-; 2-chloro-4,5-difluoro-phenyl; 2-bromo-4-fluoro-phenyl; 2-bromo-4,6-difluoro-phenyl; 4-chloro-2,5-difluoro-phenyl; 5-chloro-2,4-difluoro-phenyl; 4-bromo-2,5-difluoro-phenyl; 5-bromo-2,4-difluoro-phenyl; pentafluoro-phenyl; 2,4-dinitro-phenyl; 4-chloro-3-nitro-phenyl; 2-methyl-5-nitro-phenyl; 5-bromo-2-methoxy-phenyl; 3-chloro-2-methyl-phenyl; 4-bromo-3-methyl-phenyl; 4-chloro-2,5-dimethyl-phenyl; 4-fluoro-3-methyl-phenyl; 5-fluoro-2-methyl-phenyl; 2-nitro-4-trifluoromethyl-phenyl; 2-methoxy-4-methyl-phenyl; 3,5-dichloro-2-hydroxy-phenyl; 3,5-dichloro-4-hydroxy-phenyl; 5-chloro-2,4-difluoro-phenyl; 3-chloro-4-(NH)-(C=O)-CH₃-phenyl; 2-chloro-6-methyl-phenyl; 2-chloro-5-trifluoromethyl-phenyl; 2-chloro-5-trifluoromethoxy-phenyl; 4-bromo-2-trifluoromethoxy-phenyl; 4-bromo-2-trifluoromethyl-phenyl; 4-bromo-3-trifluoromethyl-phenyl; 3-carboxy-4-fluoro-phenyl; 3-carboxy-4-chloro-6-fluoro-phenyl; 4-methoxy-2,3,6-trimethyl-phenyl-; or one of the following groups: 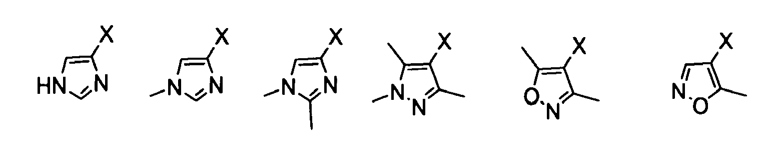 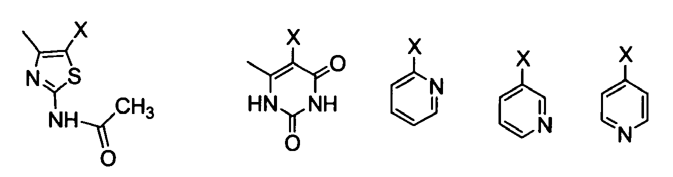 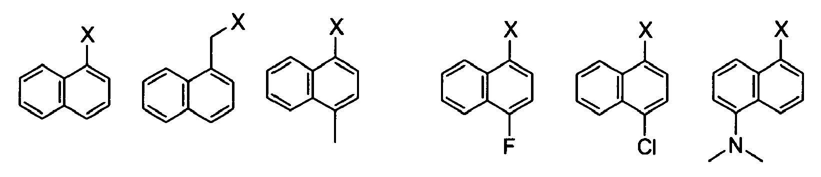 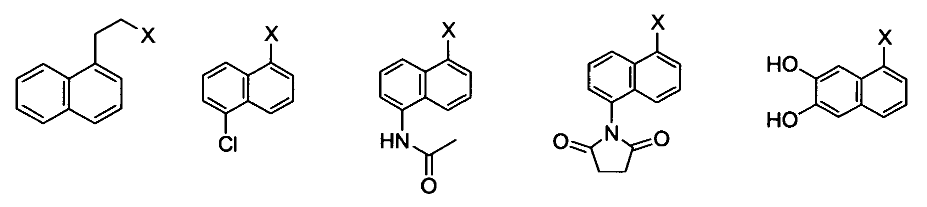 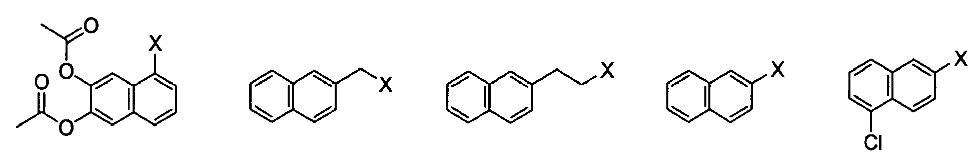 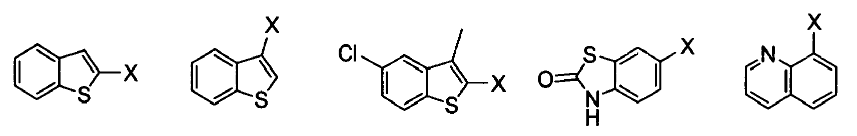 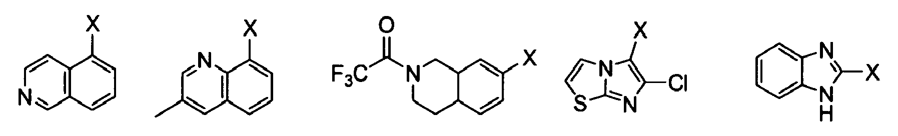 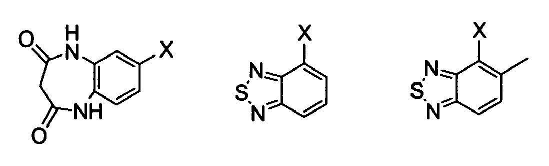 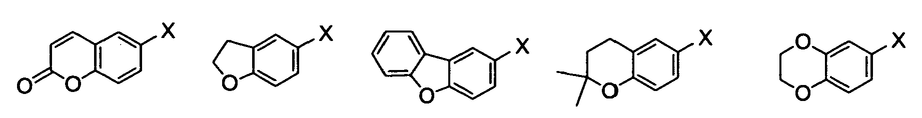 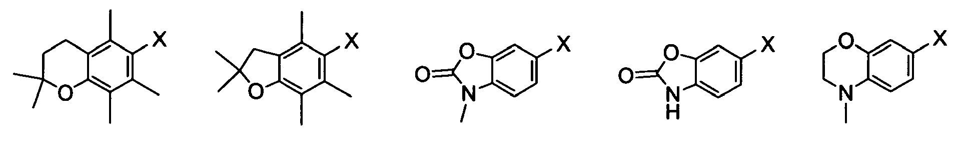 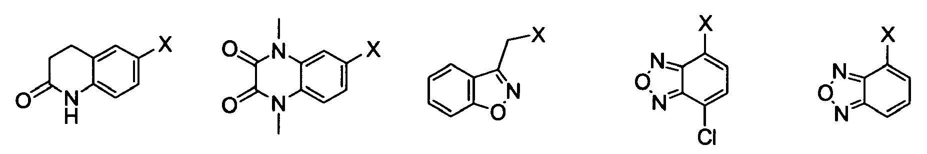 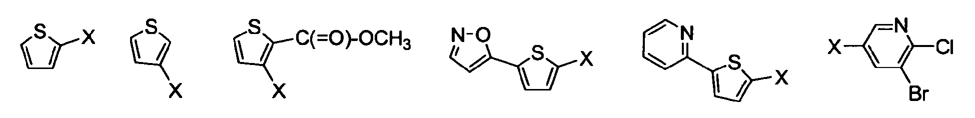 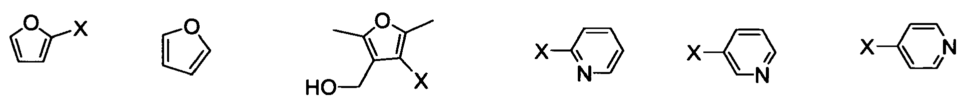 whereby in each case X denotes the position by which the respective substituent W^{a} is bonded to the -SO₂ group of formula (1a);
optionally in form of one of its stereoisomers, preferably enantiomers or diastereomers, its racemate or in form of a mixture of at least two of its stereoisomers, preferably enantiomers or diastereomers, in any mixing ratio, or a physiologically acceptable salt thereof, or a solvate, respectively.

Preferred compounds of general formula (Ia) are those selected from the group consisting of
1 1-[1-(Naphthalene-2-sulfonyl)-piperidin-4-yl]-1,4-dihydro-benzo[d][1,3]oxazin-2-one
2 1-[1-(Toluene-4-sulfonyl)-piperidin-4-yl]-1,4-dihydro-benzo[d][1,3]oxazin-2-one
3 1-(1-Phenylmethanesulfonyl-piperidin-4-yl)-1,4-dihydro-benzo[d][1,3]oxazin-2-one
4 1-(1-Benzenesulfonyl-piperidin-4-yl)-6-chloro-1,4-dihydro-benzo[d][1,3]oxazin-2-one
5 6-Chloro-1-[1-(toluene-4-sulfonyl)-piperidin-4-yl]-1,4-dihydro-benzo[d][1,3]oxazin-2-one
6 6-Chloro-1-(1-phenylmethanesulfonyl-piperidin-4-yl)-1,4-dihydro-benzo[d][1,3]oxazin-2-one
7 6-Chloro-1-[1-(naphthalene-1-sulfonyl)-piperidin-4-yl]-1,4-dihydro-benzo[d][1,3]oxazin-2-one
8 6-Chloro-1-[1-(naphthalene-2-sulfonyl)-piperidin-4-yl]-1,4-dihydro-benzo[d][1,3]oxazin-2-one
9 6-Chloro-1-[1-(5-chloro-3-methyl-benzo[b]thiophene-2-sulfonyl)-piperidin-4-yl]-1,4-dihydro-benzo[d][1,3]oxazin-2-one
10 1-[1-(Thiophene-2-sulfonyl)-piperidin-4-yl]-1,4-dihydro-benzo[d][1,3]oxazin-2-one
11 1-[1-(4-Acetyl-benzenesulfonyl)-piperidin-4-yl]-1,4-dihydro-benzo[d][1,3]oxazin-2-one 12 2-[4-(2-Oxo-4H-benzo[d][1,3]oxazin-1-yl)-piperidine-1-suffonyl]-benzonitrile
13 1-[1-(2,4-Dimethyl-benzenesulfonyl)-piperidin-4-yl]-1,4-dihydro-benzo[d][1,3]oxazin-2-one
14 1-[1-(4-Methoxy-benzenesulfonyl)-piperidin-4-yl]-1,4-dihydro-benzo[d][1,3]oxazin-2-one
15 1-[1-(2-Naphthalen-1-yl-ethanesu/fonyl)-piperidin-4-yl]-1,4-dihydro-benzo[d][1,3]oxazin-2-one
16 8-Methyl-1-[1-(thiophene-2-sulfonyl)-piperidin-4-yl]-1,4-dihydro-benzo[d][1,3]oxazin-2-one
17 1-[1-(4-Acetyl-benzenesulonyl)-piperidin-4-yl]-8-methyl-1,4-dihydro-benzo[d][1,3]oxazin-2-one
18 2-[4-(8-Methyl-2-oxo-4H-benzo[d][1,3]oxazin-1-yl)piperidine-1-sulfonyl]-benzonitrile
19 1-[1-(2,4-Dimethyl-benzenesulfonyl)-piperidin-4-yl]-8-methyl-1,4-dihydro-benzo[d][1,3]oxazin-2-one
20 1-[1-(4-Methoxy-benzenesulfonyl)-piperidin-4-yl]-8-methyl-1,4-dihydro-benzo[d][1,3]oxazin-2-one
21 8-Methyl-1-[1-(2-naphthalen-1-yl-ethanesulfonyl)-piperidin-4-yl]-1,4-dihydro-benzo[d][1,3]oxazin-2-one
22 4-(8-Methyl-2-oxo-4H-benzo[d][1,3]oxazin-1-yl)-piperidine-1-sulfonic acid dimethylamide
23 2-[4-(2-Oxo-4H-benzo[d][1,3]oxazin-1-yl)-piperidine-1-sulfonyl]-benzoic acid methyl ester
24 1-[1-(3-Trifluoromethyl-benzenesulfonyl)-piperidin-4-yl]-1,4-dihydro-benzo[d][1,3]oxazin-2-one
25 2-[4-(8-Methyl-2-oxo-4H-benzo[d][1,3]oxazin-1-yl)-piperidine-1-sulfonyl]-benzoic acid methyl ester
26 8-Methyl-1-[1-(3-trifluoromethyl-benzenesulfonyl)-piperidin-4-yl]-1,4-dihydro-benzo[d][1,3]oxazin-2-one
27 1-[1-(4-Acetyl-benzenesulfonyl)-piperidin-4-yl]-6-chloro-1,4-dihydro-benzo[d][1,3]oxazin-2-one
28 2-[4-(6-Chloro-2-oxo-4H-benzo[d][1,3]oxazin-1-yl)-piperidine-1-sulfonyl]-benzonitrile
29 6-Chloro-1-[1-(4-methoxy-benzenesulfonyl)-piperidin-4-yl]-1,4-dihydro-benzo[d][1,3]oxazin-2-one
30 2-[4-(6-Chloro-2-oxo-4H-benzo[d][1,3]oxazin-1-yl)-piperidine-1-sulfonyl]-benzoic acid methyl ester
31 6-Chloro-1-[1-(2,4-dimethyl-benzenesulfonyl)-piperidin-4-yl]-1,4-dihydro-benzo[d][1,3]oxazin-2-one
32 6-Chloro-1-[1-(3-trifluoromethyl-benzenesulfonyl)-piperidin-4-yl]-1,4-dihydro-benzo[d][1,3]oxazin-2-one
33 1-[1-(5-Chloro-3-methyl-benzo[b]thiophene-2-sulfonyl)-piperidin-4-yl]-8-methyl-1,4-dihydro-benzo[d][1,3]oxazin-2-one
34 1-{1-[4-(4-Bromo-phenoxy)-benzenesulfonyl]-piperidin-4-yl}-8-methyl-1,4-dihydro-benzo[d][1,3]oxazin-2-one
35 1-[1-(4-Fluoro-benzenesulfonyl)-piperidin-4-yl]-8-methyl-1,4-dihydro-benzo[d][1,3]oxazin-2-one
36 8-Methyl-1-[1-(naphthalene-2-sulfonyl)-piperidin-4-yl]-1,4-dihydro-benzo[d][1,3]oxazin-2-one
37 8-Methyl-1-(1-phenylmethanesulfonyl-piperidin-4-yl)-1,4-dihydro-benzo[d][1,3]oxazin-2-one
38 1-[1-(4-Bromo-benzenesulfonyl)-piperidin-4-yl]-6-chloro-1,4-dihydro-benzo[d][1,3]oxazin-2-one
39 6-Chloro-1-[1-(4-methanesulfonyl-benzenesulfonyl)-piperidin-4-yl]-1,4-dihydro-benzo[d][1,3]oxazin-2-one
40 1-[1-(Butane-1-sulfonyl)-piperidin-4-yl]-6-chloro-1,4-dihydro-benzo[d][1,3]oxazin-2-one
41 1-[1-(4-Bromo-benzenesulfonyl)-piperidin-4-yl]-8-methyl-1,4-dihydro-benzo[d][1,3]oxazin-2-one
42 1-[1-(4-Methanesulfonyl-benzenesulfonyl)-piperidin-4-yl]-8-methyl-1,4-dihydro-benzo[d][1,3]oxazin-2-one
43 1 -[1 -(Butane-1-sulfonyl)-piperidin-4-yl]-8-methyl-1,4-dihydro-benzo[d][1,3]oxazin-2-one
44 6-Chloro-1-[1-(2-nitro-benzenesulfonyl)-piperidin-4-yl]-1,4-dihydro-benzo[d][1,3]oxazin-2-one
45 6-Chloro-1-[1-(3-nitro-benzenesulfonyl)-piperidin-4-yl]-1,4-dihydro-benzo[d][1,3]oxazin-2-one
46 1-[1-(Biphenyl-4-sulfonyl)-piperidin-4-yl]-6-chloro-1,4-dihydro-benzo[d][1,3]oxazin-2-one
47 8-Methyl-1-[1-(2-nitro-benzenesulfonyl)-piperidin-4-yl]-1,4-dihydro-benzo[d][1,3]oxazin-2-one
48 8-Methyl-1-[1-(3-nitro-benzenesulfonyl)-piperidin-4-yl]-1,4-dihydro-benzo[d][1,3]oxazin-2-one
49 1-[1-(Biphenyl-4-sulfonyl)-piperidin-4-yl]-8-methyl-1,4-dihydro-benzo[d][1,3]oxazin-2-one

50 8-Methyl-1-[1-(4-nitro-benzenesulfonyl)-piperidin-4-yl]-1,4-dihydro-benzo[d][1,3]oxazin-2-one
51 6-Chloro-1-[1-(4-nitro-benzenesulfonyl)-piperidin-4-yl]-1,4-dihydro-benzo[d][1,3]oxazin-2-one
52 1-(1-Ethanesulfonyl-piperidin-4-yl)-1,4-dihydro-benzo[d][1,3]oxazin-2-one
53 1-[1-(Propane-1-sulfonyl)-piperidin-4-yl]-1,4-dihydro-benzo[d][1,3]oxazin-2-one
54 1-[1-(Propane-2-sulfonyl)-piperidin-4-yl]-1,4-dihydro-benzo[d][1,3]oxazin-2-one
55 6-Chloro-1-(1-ethanesulfonyl-piperidin-4-yl)-1,4-dihydro-benzo[d][1,3]oxazin-2-one
56 6-Chloro-1-[1-(propane-1-sulfonyl)-piperidin-4-yl]-1,4-dihydro-benzo[d][1,3]oxazin-2-one
57 6-Chloro-1-[1-(propane-2-sulfonyl)-piperidin-4-yl]-1,4-dihydro-benzo[d][1,3]oxazin-2-one
58 6-Chloro-1-[1-(quinoline-8-sulfonyl)-piperidin-4-yl]-1,4-dihydro-benzo[d][1,3]oxazin-2-one
59 1-[1-(4-Nitro-benzenesulfonyl)-piperidin-4-yl]-1,4-dihydro-benzo[d][1,3]oxazin-2-one
60 6-Methyl-1-[1-(quinoline-8-sulfonyl)-piperidin-4-yl]-1,4-dihydro-benzo[d][1,3]oxazin-2-one
61 6-Methyl-1-[1-(2-naphthalen-1-yl-ethanesulfonyl)-piperidin-4-yl]-1,4-dihydro-benzo[d][1,3]oxazin-2-one
62 6-Methyl-1-[1-(toluene-4-sulfonyl)-piperidin-4-yl]-1,4-dihydro-benzo[d][1,3]oxazin-2-one
63 1-[1-(4-Fluoro-benzenesulfonyl)-piperidin-4-yl]-6-methyl-1,4-dihydro-benzo[d][1,3]oxazin-2-one
64 6-Methyl-1-[1-(naphthalene-1-sulfonyl)-piperidin-4-yl]-1,4-dihydro-benzo[d][1,3]oxazin-2-one
65 6-Methyl-1-[1-(naphthalene-2-sulfonyl)-piperidin-4-yl]-1,4-dihydro-benzo[d][1,3]oxazin-2-one
66 1-[1-(5-Chloro-3-methyl-benzo[b]thiophene-2-sulfonyl)-piperidin-4-yl]-6-methyl-1,4-dihydro-benzo[d][1,3]oxazin-2-one
67 6-Methyl-1-[1-(4-nitro-benzenesulfonyl)-piperidin-4-yl]-1,4-dihydro-benzo[d][1,3]oxazin-2-one
68 1-(1-Benzenesulfonyl-piperidin-4-yl)-6-methyl-1,4-dihydro-benzo[d][1,3]oxazin-2-one
69 1-[1-(4-Chloro-3-nitro-benzenesulfonyl)-piperidin-4-yl]-6-methyl-1,4-dihydro-benzo[d][1,3]oxazin-2-one
70 1-[1-(5-Dimethylamino-naphthalene-1-sulfonyl)-piperidin-4-yl]-6-methyl-1,4-dihydro-benzo[d][1,3]oxazin-2-one
71 1-[1-(4-Chloro-3-nitro-benzenesulfonyl)-piperidin-4-yl]-8-methyl-1,4-dihydro-benzo[d][1,3]oxazin-2-one
72 1-[1-(4-Chloro-3-nitro-benzenesulfonyl)-piperidin-4-yl]-1,4-dihydro-benzo[d][1,3]oxazin-2-one
73 6-Chloro-1-[1-(4-chloro-3-nitro-benzenesulfonyl)-piperidin-4-yl]-1,4-dihydro-benzo[d][1,3]oxazin-2-one
74 6-Chloro-1-[1-(5-dimethylamino-naphthalene-1-sulfonyl)-piperidin-4-yl]-1,4-dihydro-benzo[d][1,3]oxazin-2-one
75 1-[1-(4-Methoxy-2,3,6-trimethyl-benzenesulfonyl)-piperidin-4-yl]-1,4-dihydro-benzo[d][1,3]oxazin-2-one
76 1-[1-(4-Methoxy-2,3,6-trimethyl-benzenesulfonyl)-piperidin-4-yl]-8-methyl-1,4-dihydro-benzo[d][1,3]oxazin-2-one
77 6-Chloro-1-[1-(4-methoxy-2,3,6-trimethyl-benzenesulfonyl)-piperidin-4-yl]-1,4-dihydro-benzo[d][1,3]oxazin-2-one
78 1-[1-(4-Methoxy-2,3,6-trimethyl-benzenesulfonyl)-piperidin-4-yl]-6-methyl-1,4-dihydro-benzo[d][1,3]oxazin-2-one
79 1-[1-(2-Bromo-benzenesulfonyl)-piperidin-4-yl]-1,4-dihydro-benzo[d][1,3]oxazin-2-one
80 1-[1-(2-Bromo-benzenesulfonyl)-piperidin-4-yl]-8-methyl-1,4-dihydro-benzo[d][1,3]oxazin-2-one
81 1-[1-(2-Bromo-benzenesulfonyl)-piperidin-4-yl]-6-chloro-1,4-dihydro-benzo[d][1,3]oxazin-2-one
82 1-[1-(2-Bromo-benzenesulfonyl)-piperidin-4-yl]-6-methyl-1,4-dihydro-benzo[d][1,3]oxazin-2-one
83 6-Chloro-1-[1-(2,3-dichloro-benzenesulfonyl)-piperidin-4-yl]-1,4-dihydro-benzo[d][1,3]oxazin-2-one
84 1-[1-(2,3-Dichloro-benzenesulfonyl)-piperidin-4-yl]-6-methyl-1,4-dihydro-benzo[d][1,3]oxazin-2-one
85 1-[1-(2,4,5-Trichloro-benzenesulfonyl)-piperidin-4-yl]-1,4-dihydro-benzo[d][1,3]oxazin-2-one
86 8-Methyl-1-[1-(2,4,5-trichloro-benzenesulfonyl)-piperidin-4-yl]-1,4-dihydro-benzo[d][1,3]oxazin-2-one
87 6-Chloro-1-[1-(2,4,5-trichloro-benzenesulfonyl)-piperidin-4-yl]-1,4-dihydro-benzo[d][1,3]oxazin-2-one
88 6-Methyl-1-[1-(2,4,5-trichloro-benzenesulfonyl)-piperidin-4-yl]-1,4-dihydro-benzo[d][1,3]oxazin-2-one
89 1-[1-(5-Bromo-2-methoxy-benzenesulfonyl)-piperidin-4-yl]-1,4-dihydro-benzo[d][1,3]oxazin-2-one
90 1-[1-(5-Bromo-2-methoxy-benzenesulfonyl)-piperidin-4-yl]-8-methyl-1,4-dihydro-benzo[d][1,3]oxazin-2-one
91 1-[1-(5-Bromo-2-methoxy-benzenesulfonyl)-1-piperidin-4-yl]-6-chloro-1,4-dihydro-benzo[d][1,3]oxazin-2-one
92 1-[1-(5-Bromo-2-methoxy-benzenesulfonyl)-piperidin-4-yl]-6-methyl-1,4-dihydro-benzo[d][1,3]oxazin-2-one
93 1-[1-(2,5-Dimethoxy-benzenesulfonyl)-piperidin-4-yl]-1,4-dihydro-benzo[d][1,3]oxazin-2-one
94 1-[1-(2,5-Dimethoxy-benzenesulfonyl)-piperidin-4-yl]-8-methyl-1,4-dihydro-benzo[d][1,3]oxazin-2-one
95 6-Chloro-1-[1-(2,5-dimethoxy-benzenesulfonyl)-piperidin-4-yl]-1,4-dihydro-benzo[d][1,3]oxazin-2-one
96 1-[1-(2,5-Dimethoxy-benzenesulfonyl)-piperidin-4-yl]-6-methyl-1,4-dihydro-benzo[d][1,3]oxazin-2-one
97 1-(1-Pentamethylbenzenesulfonyl-piperidin-4-yl)-1,4-dihydro-benzo[d][1,3]oxazin-2-one
98 8-Methyl-1-(1-pentamethylbenzenesulfonyl-piperidin-4-yl)-1,4-dihydro-benzo[d][1,3]oxazin-2-one
99 6-Chloro-1-(1-pentamethylbenzenesulfonyl-piperidin-4-yl)-1,4-dihydro-benzo[d][1,3]oxazin-2-one

100 6-Methyl-1-(1-pentamethylbenzenesulfonyl-piperidin-4-yl)-1,4-dihydro-benzo[d][1,3]oxazin-2-one
101 1-{1-[2-(2,2,2-Trifluoro-acetyl)-1,2,3,4-tetrahydro-isoquinoline-7-sulfonyl]-piperidin-4-yl}-1,4-dihydro-benzo[d][1,3]oxazin-2-ne
102 8-Methyl-1-{1-[2-(2,2,2-trifluoro-acetyl)-1,2,3,4-tetrahydro-isoquinoline-7-sulfonyl]-piperidin-4-yl}-1,4-dihydro-benzo[d][1,3]oxazin-2-one
103 6-Chloro-1-{1-[2-(2,2,2-trifluoro-acetyl)-1,2,3,4-tetrahydro-isoquinoline-7-sulfonyl]-piperidin-4-yl}-1,4-dihydro-benzo[d][1,3]oxazin-2-one
104 6-Methyl-1-{1-[2-(2,2,2-trifluoro-acetyl)-1,2,3,4-tetrahydro-isoquinoline-7-sulfonyl]-piperidin-4-yl}-1,4-dihydro-benzo[d][1,3]oxazin-2-one
105 1-[1-(2-Methyl-5-nitro-benzenesulfonyl)-piperidin-4-yl]-1,4-dihydro-benzo[d][1,3]oxazin-2-one
106 8-Methyl-1-[1-(2-methyl-5-nitro-benzenesulfonyl)-piperidin-4-yl]-1,4-dihydro-benzo[d][1,3]oxazin-2-one
107 6-Chloro-1-[1-(2-methyl-5-nitro-benzenesulfonyl)-piperidin-4-yl]-1,4-dihydro-benzo[d][1,3]oxazin-2-one
108 6-Methyl-1-[1-(2-methyl-5-nitro-benzenesulfonyl)-piperidin-4-yl]-1,4-dihydro-benzo[d][1,3]oxazin-2-one
109 1-[1-(4-Bromo-2,5-difluoro-benzenesulfonyl)-piperidin-4-yl]-1,4-dihydro-benzo[d][1,3]oxazin-2-one
110 1-[1-(4-Bromo-2,5-difluoro-benzenesulfonyl)-piperidin-4-yl]-8-methyl-1,4-dihydro-benzo[d][1,3]oxazin-2-one
111 1-[1-(4-Bromo-2,5-difluoro-benzenesulfonyl)-piperidin-4-yl]-6-chloro-1,4-dihydro-benzo[d][1,3]oxazin-2-one
112 1-[1-(4-Bromo-2,5-difluoro-benzenesulfonyl)-piperidin-4-yl]-6-methyl-1,4-dihydro-benzo[d][1,3]oxazin-2-one
113 1-[1-(4-Chloro-2,5-dimethyl-benzenesulfonyl)-piperidin-4-yl]-1,4-dihydro-benzo[d][1,3]oxazin-2-one
114 1-[1-(4-Chloro-2,5-dimethyl-benzenesulfonyl)-piperidin-4-yl]-8-methyl-1,4-dihydro-benzo[d][1,3]oxazin-2-one
115 6-Chloro-1-[1-(4-chloro-2,5-dimethyl-benzenesulfonyl)-piperidin-4-yl]-1,4-dihydro-benzo[d][1,3]oxazin-2-one
116 1-[1-(4-Chloro-2,5-dimethyl-benzenesulfonyl)-piperidin-4-yl]-6-methyl-1,4-dihydro-benzo[d][1,3]oxazin-2-one
117 1-[1-(4-Methoxy-benzenesulfonyl)-piperidin-4-yl]-6-methyl-1,4-dihydro-benzo[d][1,3]oxazin-2-one
118 1-[1-(4-lsopropyl-benzenesulfonyl)-piperidin-4-yl]-1,4-dihydro-benzo[d][1,3]oxazin-2-one
119 1-[1-(4-Isopropyl-benzenesulfonyl)-piperidin-4-yl]-8-methyl-1,4-dihydro-benzo[d][1,3]oxazin-2-one
120 6-Chloro-1-[1-(4-isopropyl-benzenesulfonyl)-piperidin-4-yl]-1,4-dihydro-benzo[d][1,3]oxazin-2-one
121 1-[1-(4-Isopropyl-benzenesulfonyl)piperidin-4-yl]-6-methyl-1,4-dihydro-benzo[d][1,3]oxazin-2-one
122 1-[1-(3-Chloro-4-fluoro-benzenesulfonyl)-piperidin-4-yl]-1,4-dihydro-benzo[d][1,3]oxazin-2-one
123 1-[1-(3-Chloro-4-fluoro-benzenesulfonyl)-piperidin-4-yl]-8-methyl-1,4-dihydro-benzo[d][1,3]oxazin-2-one
124 6-Chloro-1-[1-(3-chloro-4-fluoro-benzenesulfonyl)-piperidin-4-yl]-1,4-dihydro-benzo[d][1,3]oxazin-2-one
125 1-[1-(3-Chloro-4-fluoro-benzenesulfonyl)-piperidin-4-yl]-6-methyl-1,4-dihydro-benzo[d][1,3]oxazin-2-one
126 1-[1-(4-Bromo-benzenesulfonyl)-piperidin-4-yl]-6-methyl-1,4-dihydro-benzo[d][1,3]oxazin-2-one
127 6-Methyl-1-[1-(3-nitro-benzenesulfonyl)-piperidin-4-yl]-1,4-dihydro-benzo[d][1,3]oxazin-2-one
128 6-Methyl-1-[1-(3-trifluoromethyl-benzenesulfonyl)-piperidin-4-yl]-1,4-dihydro-benzo[d][1,3]oxazin-2-one
129 1-[1-(4-Trifluoromethoxy-benzenesulfonyl)-piperidin-4-yl]-1,4-dihydro-benzo[d][1,3]oxazin-2-one
130 1-[1-(2-Nitro-4-trifluoromethyl-benzenesulfonyl)-piperidin-4-yl]-1,4-dihydro-benzo[d][1,3]oxazin-2-one
131 1-[1-(3-Fluoro-benzenesulfonyl)-piperidin-4-yl]-1,4-dihydro-benzo[d][1,3]oxazin-2-one
132 1-[1-(2,4-Dichloro-benzenesulfonyl)-piperidin-4-yl]-1,4-dihydro-benzo[d][1,3]oxazin-2-one
133 1-[1-(2,4,6-Trimethyl-benzenesulfonyl)-piperidin-4-yl]-1,4-c1ihydro-benzo[d][1,3]oxazin-2-one
134 1-[1-(2-Trifluoromethyl-benzenesulfonyl)-piperidin-4-yl]-1,4-dihydro-benzo[d][1,3]oxazin-2-one
135 8-Methyl-1-[1-(4-trifluoromethoxy-benzenesulfonyl)-piperidin-4-yl]-1,4-dihydro-benzo[d][1,3]oxazin-2-one
136 8-Methyl-1-[1-(2-nitro-4-trifluoromethyl-benzenesulfonyl)-piperidin-4-yl]-1,4-dihydro-benzo[d][1,3]oxazin-2-one
137 1-[1-(3-Fluoro-benzenesulfonyl)-piperidin-4-yl]-8-methyl-1,4-dihydro-benzo[d][1,3]oxazin-2-one
138 1-[1-(2,4-Dichloro-benzenesulfonyl)-piperidin-4-yl]-8-methyl-1,4-dihydro-benzo[d][1,3]oxazin-2-one
139 8-Methyl-1-[1-(2,4,6-trimethyl-benzenesulfonyl)-piperidin-4-yl]-1,4-dihydro-benzo[d][1,3]oxazin-2-one
140 8-Methyl-1-[1-(2-trifluoromethyl-benzenesulfonyl)-piperidin-4-yl]-1,4-dihydro-benzo[d][1,3]oxazin-2-one
141 1-[1-(4-Fluoro-benzenesulfonyl)-piperidin-4-yl]-1,4-dihydro-benzo[d][1,3]oxazin-2-one
142 1-[1-(4-Bromo-benzenesulfonyl)-piperidin-4-yl]-1,4-dihydro-benzo[d][1,3]oxazin-2-one
143 1-[1-(3-Nitro-benzenesulfonyl)-piperidin-4-yl]-1,4-dihydro-benzo[d][1,3]oxazin-2-one
144 1-{1-[4-(4-Bromo-phenoxy)-benzenesulfony)]-piperidin-4-yl}-1,4-dihydro-benzo[d][1,3]oxazin-2-one
145 1-[1-(3-Methoxy-benzenesulfonyl)-piperidin-4-yl]-1,4-dihydro-benzo[d][1,3]oxazin-2-one
146 1-[1-(2-Nitro-benzenesulfonyl)-piperidin-4-yl]-1,4-dihydro-benzo[d][1,3]oxazin-2-one
147 8-Methyl-1-[1-(toluene-4-sulfonyl)-piperidin-4-yl]-1,4-dihydro-benzo[d][1,3]oxazin-2-one
148 1-(1-Benzenesulfonyl-piperidin-4-yl)-8-methyl-1,4-dihydro-benzo[d][1,3]oxazin-2-one
149 1-[1-(3-ethoxy-benzenesulfonyl)-piperidin-4-yl]-8-methyl-1,4-dihydro-benzo[d][1,3]oxazin-2-one

150 1-[1-(2,4-Dimethyl-benzenesulfonyl)-piperidin-4-yl]-6-methyl-1,4-dihydro-benzo[d][1,3]oxazin-2-one
151 1-{1-[4-(4-Bromo-phenoxy)-benzenesulfonyl]-piperidin-4-yl}-6-methyl-1,4-dihydro-benzo[d][1,3]oxazin-2-one
152 6-Methyl-1-[1-(thiophene-2-sulfonyl)-piperidin-4-yl]-1,4-dihydro-benzo[d][1,3]oxazin-2-one
153 1 -[1 -(Toluene-3-sulfonyl)-piperidin-4-yl]-1,4-dihydro-benzo[d][1,3]oxazin-2-one
154 1-[1-(5-Fluoro-2-methyl-benzenesulfonyl)-piperidin-4-yl]-1,4-dihydro-benzo[d][1,3]oxazin-2-one
155 1-[1-(4-)sopropoxy-benzenesulfonyl)-piperidin-4-yl]-1,4-dihydro-benzo[d][1,3]oxazin-2-one
156 1-[1-(3-Chloro-benzenesulfonyl)-piperidin-4-yl]-1,4-dihydro-benzo[d][1,3]oxazin-2-one
157 1-[1-(3,4-Dimethoxy-benzenesulfonyl)-piperidin-4-yl]-1,4-dihydro-benzo[d][1,3]oxazin-2-one
158 1-(1-Pentafluorobenzenesulfonyl-piperidin-4-yl)-1,4-dihydro-benzo[d][1,3]oxazin-2-one
159 8-Methyl-1-[1-(toluene-3-sulfonyl)-piperidin-4-yl]-1,4-dihydro-benzo[d][1,3]oxazin-2-one
160 1-[1-(5-Fluoro-2-methyl)-benzenesulfonyl)-piperidin-4-yl]-8-methyl-1,4-dihydrobenzo[d][1,3]oxazin-2-one
161 1-[1-(4-Isopropoxy-benzenesulfonyl)-piperidin-4-yl]-8-methyl-1,4-dihydro-benzo[d][1,3] oxazin-2-one
162 1-[1-(3-Chloro-benzenesulfonyl)-piperidin-4-yl]-8-methyl-1,4-dihydro-benzo[d][1,3]oxazin-2-one
163 1-[1-(3,4-Dimethoxy-benzenesulfonyl)-piperidin-4-yl]-8-methyl-1,4-dihydro-benzo[d][1,3] oxazin-2-one
164 8-Methyl-1-(1-pentafluorobenzenesulfonyl-piperidin-4-yl)-1,4-dihydro-benzo[d][1,3]oxazin-2-one
165 6-Methyl-1-[1-(toluene-3-sulfonyl)-piperidin-4-yl]-1,4-dihydro-benzo[d][1,3]oxazin-2-one
166 1-[1-(5-Fluoro-2-methyl-benzenesulfonyl)-piperidin-4-yl]-6-methyl-1,4-dihydro-benzo[d][1,3]oxazin-2-one
167 1-[1-(4-Isopropoxy-benzenesulfonyl)-piperidin-4-yl]-6-methyl-1,4-dihydro-benzo[d][1,3]oxazin-2-one
168 1-[1-(3-Chloro-benzenesulfonyl)-piperidin-4-yl]-6-methyl-1,4-dihydro-benzo[d][1,3]oxazin-2-one
169 1-[1-(3,4-Dimethoxy-benzenesulfonyl)-piperidin-4-yl]-6-methyl-1,4-dihydro-benzo[d][1,3]oxazin-2-one
170 6-Methyl-1-(1-pentafluorobenzenesulfonyl-piperidin-4-yl)-1,4-dihydro-benzo[d][1,3]oxazin-2-one
171 6-Methyl-1-[1-(4-trifluoromethoxy-benzenesulfonyl)-piperidin-4-yl]-1,4-dihydro-benzo[d][1,3]oxazin-2-one
172 6-Methyl-1-[1-(2-nitro-4-trifluoromethyl-benzenesulfonyl)-piperidin-4-yl]-1,4-dihydro-benzo[d][1,3]oxazin-2-one
173 1-[1-(3-Fluoro-benzenesulfonyl)-piperidin-4-yl]-6-methyl-1,4-dihydro-benzo[d][1,3]oxazin-2-one
174 1-[1-(2,4-Dichloro-benzenesulfonyl)-piperidin-4-yl]-6-methyl-1,4-dihydro-benzo[d][1,3]oxazin-2-one
175 6-Methyl-1-[1-(2,4,6-trimethyl-benzenesulfonyl)-piperidin-4-yl]-1,4-dihydro-benzo[d][1,3]oxazin-2-one
176 6-Methyl-1-[1-(2-trifluoromethyl-benzenesulfonyl)-piperidin-4-yl]-1,4-dihydro-benzo[d][1,3]oxazin-2-one
177 1-[1-(3-Methoxy-benzenesulfonyl)-piperidin-4-yl]-6-methyl-1,4-dihydro-benzo[d][1,3]oxazin-2-one
178 6-Methyl-1-[1-(2-nitro-benzenesulfonyl)-piperidin-4-yl]-1,4-dihydro-benzo[d][1,3]oxazin-2-one
179 1-[1-(4-Acetyl-benzenesulfonyl)-piperidin-4-yl]-6-methyl-1,4-dihydro-benzo[d][1,3]oxazin-2-one
180 1-[1-(4-Methanesulfonyl-benzenesulfonyl)-piperidin-4-yl]-6-methyl-1,4-dihydro-benzo[d][1,3]oxazin-2-one
181 6-Methyl-1-(1-phenylmethanesulfonyl-piperidin-4-yl)-1,4-dihydro-benzo[d][1,3]oxazin-2-one
182 2-[4-(6-Methyl-2-oxo-4H-benzo[d][1,3]oxazin-1-yl)-piperidine-1-sulfonyl]benzoic acid methyl ester
183 6-Methyl-1-[1-(2-oxo-2H-chromene-6-sulfonyl)-piperidin-4-yl]-1,4-dihydro-benzo[d][1,3]oxazin-2-one
184 6-Chloro-1-[1-(4-fluoro-benzenesulfonyl)-piperidin-4-yl]-1,4-dihydro-benzo[d][1,3]oxazin-2-one
185 6-Chloro-1-[1-(3,5-dichloro-benzenesulfonyl)-piperidin-4-yl]-1,4-dihydro-benzo[d][1,3]oxazin-2-one
186 1-{1-[4-(4-Bromo-phenoxy)-benzenesulfonyl]-piperidin-4-yl}-6-chloro-1,4-dihydro-benzo[d][1,3]oxazin-2-one
187 6-Chloro-1-[1-(thiophene-2-sulfonyl)-piperidin-4-yl]-1,4-dihydro-benzo[d][1,3]oxazin-2-one
188 6-Chloro-1-[1-(3-methoxy-benzenesulfonyl)-piperidin-4-yl]-1,4-dihydro-benzo[d][1,3]oxazin-2-one
189 6-Chloro-1-[1-(2-oxo-2H-chromene-6-sulfonyl)-piperidin-4-yl]-1,4-dihydro-benzo[d][1,3]oxazin-2-one
190 6-Chloro-1-[1-(toluene-3-sulfonyl)-piperidin-4-yl]-1,4-dihydro-benzo[d][1,3]oxazin-2-one
191 6-Chloro-1-[1-(5-fluoro-2-methyl-benzenesulfonyl)-piperidin-4-yl]-1,4-dihydro-benzo[d][1,3]oxazin-2-one
192 6-Chloro-1-[1-(4-isopropoxy-benzenesulfonyl)-piperidin-4-yl]-1,4-dihydro-benzo[d][1,3]oxazin-2-one
193 6-Chloro-1-[1-(3-chloro-benzenesulfonyl)-piperidin-4-yl]-1,4-dihydro-benzo[d][1,3]oxazin-2-one
194 6-Chloro-1-[1-(3,4-dimethoxy-benzenesulfonyl)-piperidin-4-yl]-1,4-dihydro-benzo[d][1,3]oxazin-2-one
195 6-Chloro-1-(1-pentafluorobenzenesulfonyl-piperidin-4-yl)-1,4-dihydro-benzo[d][1,3]oxazin-2-one
196 6-Chloro-1-[1-(4-trifluoromethoxy-benzenesulfonyl)-piperidin-4-yl]-1,4-dihydro-benzo[d][1,3]oxazin-2-one
197 6-Chloro-1 -[1-(2-nitro-4-trifluoromethyl-benzenesulfonyl)-piperidin-4-yl]-1,4-dihydro-benzo[d][1,3]oxazin-2-one
198 6-Chloro-1-[1 -(3-fluoro-benzenesulfonyl)-piperidin-4-yl]-1,4-dihydro-benzo[d][1,3]oxazin-2-one
199 6-Chloro-1-[1-(2,4-dichloro-benzenesulfonyl)-pipendin-4-yl]-1,4-dihydro-benzo[d][1,3]oxazin-2-one

200 6-Chloro-1-[1-(2,4,6-trimethyl-benzenesulfonyl)-piperidin-4-yl]-1,4-dihydro-benzo[d][1,3]oxazin-2-one
201 6-Chloro-1-[1-(2-trifluoromethyl-benzenesulfonyl)-piperidin-4-yl]-1,4-dihydro-benzo[d][1,3]oxazin-2-one
202 1-[1-(2-Oxo-2H-chromene-6-sulfonyl)-piperidin-4-yl]-1,4-dihydro-benzo[d][1,3]oxazin-2-one
203 1-[1-(3,5-Dichloro-benzenesulfonyl)-piperidin-4-yl]-1,4-dihydro-benzo[d][1,3]oxazin-2-one
204 1-[1-(2,5-Dichloro-benzenesulfonyl)-piperidin-4-yl]-1,4-dihydro-benzo[d][1,3]oxazin-2-one
205 1-[1-(5-Bromo-6-chloro-pyridine-3-sulfonyl)-piperidin-4-yl]-1,4-dihydro-benzo[d][1,3]oxazin-2-one
206 1-[1-(4-Chloro-benzenesulfonyl)-piperidin-4-yl]-1,4-dihydro-benzo[d][1,3]oxazin-2-one
207 1-[1-(2,6-Dichloro-benzenesulfonyl)-piperidin-4-yl]-1,4-dihydro-benzo[d][1,3]oxazin-2-one
208 8-Methyl-1-[1-(2-oxo-2H-chromene-6-sulfonyl)-piperidin-4-yl]-1,4-dihydro-benzo[d][1,3]oxazin-2-one
209 1-[1-(3,5-Dichloro-benzenesulfonyl)-piperidin-4-yl]-8-methyl-1,4-dihydro-benzo[d][1,3]oxazin-2-one
210 1-[1-(2,5-Dichloro-benzenesulfonyl)-piperidin-4-yl]-8-methyl-1,4-dihydro-benzo[d][1,3]oxazin-2-one
211 1-[1-(5-Bromo-6-chloro-pyridine-3-sulfonyl)-piperidin-4-yl]-8-methyl-1,4-dihydro-benzo[d][1,3]oxazin-2-one
212 1-[1-(4-Chloro-benzenesulfonyl)-piperidin-4-yl]-8-methyl-1,4-dihydro-benzo[d][1,3]oxazin-2-one
213 1-[1-(2,6-Dichloro-benzenesulfonyl)-piperidin-4-yl]-8-methyl-1,4-dihydro-benzo[d][1,3]oxazin-2-one
214 1-[1-(Biphenyl-4-sulfonyl)-piperidin-4-yl]-1,4-dihydro-benzo[d][1,3]oxazin-2-one
215 6-Chloro-1-[1-(2,5-dichloro-benzenesulfonyl)-piperidin-4-yl]-1,4-dihydro-benzo[d][1,3]oxazin-2-one
216 1-[1-(5-Bromo-6-chloro-pyridine-3-sulfonyl)-piperidin-4-yl]-6-chloro-1,4-dihydro-benzo[d][1,3]oxazin-2-one
217 6-Chloro-1-[1-(4-chloro-benzenesulfonyl)-piperidin-4-yl]-1,4-dihydro-benzo[d][1,3]oxazin-2-one
218 6-Chloro-1-[1-(2,6-dichloro-benzenesulfonyl)-piperidin-4-yl]-1,4-dihydro-benzo[d][1,3]oxazin-2-one
219 1-[1-(Biphenyl-4-sulfonyl)-piperidin-4-yl]-6-methyl-1,4-dihydro-benzo[d][1,3]oxazin-2-one
220 2-[4-(6-Methyl-2-oxo-4H-benzo[d][1,3]oxazin-1-yl)-piperidine-1-sulfonyl]-benzonitrile
221 1-[1-(2,5-Dichloro-benzenesulfonyl)-piperidin-4-yl]-6-methyl-1,4-dihydro-benzo[d][1,3]oxazin-2-one
222 1-[1-(5-Bromo-6-chloro-pyridine-3-sulfonyl)-piperidin-4-yl]-6-methyl-1,4-dihydro-benzo[d][1,3]oxazin-2-one
223 1-[1-(4-Chloro-benzenesulfonyl)-piperidin-4-yl]-6-methyl-1,4-dihydro-benzo[d][1,3]oxazin-2-one
224 1-[1-(2,6-Dichloro-benzenesulfonyl)-piperidin-4-yl]-6-methyl-1,4-dihydro-benzo[d][1,3]oxazin-2-one
225 1-[1-(3,5-Dichloro-benzenesulfonyl)-piperidin-4-yl]-6-methyl-1,4-dihydro-benzo[d][1,3]oxazin-2-one
226 6-Methyl-1-[1-(1-methyl-1H-imidazole-4-sulfonyl)-piperidin-4-yl]-1,4-dihydro-benzo[d][1,3]oxazin-2-one
227 1-[1-(5-Bromo-2,4-difluoro-benzenesulfonyl)-piperidin-4-yl]-6-methyl-1,4-dihydro-benzo[d][1,3]oxazin-2-one
228 1-[1-(4-Methanesulfonyl-benzenesulfonyl)-piperidin-4-yl]-1,4-dihydro-benzo[d][1,3]oxazin-2-one
229 1-[1-(1-Methyl-1H-imidazole-4-sulfonyl)-piperidin-4-yl]-1,4-dihydro-benzo[d][1,3]oxazin-2-one
230 1-[1-(5-Bromo-2,4-difluoro-benzenesulfonyl)-piperidin-4-yl]-1,4-dihydro-benzo[d][1,3]oxazin-2-one
231 1-[1-(6-Chloro-imidazo[2,1-b]thiazole-5-sulfonyl)-piperidin-4-yl]-1,4-dihydro-benzo[d][1,3]oxazin-2-one
232 1-[1 1-(4-Ethyl-benzenesulfonyl)-piperidin-4-yl]-1,4-dihydro-benzo[d][1,3]oxazin-2-one
233 1-[1-(Benzo[b]thiophene-3-sulfonyl)-piperidin-4-yl]-1,4-dihydro-benzo[d][1,3]oxazin-2-one
234 1-[1-(6-Chloro-imidazo[2,1-b]thiazole-5-sulfonyl)-piperidin-4-yl]-8-methyl-1,4-dihydro-benzo[d][1,3]oxazin-2-one
235 1-[1-(4-Ethyl-benzenesulfonyl)-piperidin-4-yl]-8-methyl-1,4-dihydro-benzo[d][1,3]oxazin-2-one
236 1-[1-(Benzo[b]thiophene-3-sulfonyl)-piperidin-4-yl]-8-methyl-1,4-dihydro-benzo[d][1,3]oxazin-2-one
237 6-Chloro-1-[1-(6-chloro-imidazo[2,1-b]thiazole-5-sulfonyl)-piperidin-4-yl]-1,4-dihydro-benzo[d][1,3]oxazin-2-one
238 6-Chloro-1-[1-(4-ethyl-benzenesulfonyl)-piperidin-4-yl]-1,4-dihydro-benzo[d][1,3]oxazin-2-one
239 1-[1-(Benzo[b]thiophene-3-sulfonyl)-piperidin-4-yl]-6-chloro-1,4-dihydro-benzo[d][1,3]oxazin-2-one
240 1-[1-(6-Chloro-imidazo[2,1-b]thiazole-5-sulfonyl)-piperidin-4-yl]-6-methyl-1,4-dihydro-benzo[d][1,3]oxazin-2-one
241 1-[1-(4-Ethyl-benzenesulfonyl)-piperidin-4-yl]-6-methyl-1,4-dihydro-benzo[d][1,3]oxazin-2-one
242 1-[1-(Benzo[b]thiophene-3-sulfonyl)-piperidin-4-yl]-6-methyl-1,4-dihydro-benzo[d][1,3]oxazin-2-one
243 1-[1-(7-Chloro-benzo[1,2,5]oxadiazole-4-sulfonyl)-piperidin-4-yl]-1,4-dihydro-benzo[d][1,3]oxazin-2-one
244 1-[1-(2-Methoxy-4-methyl-benzenesulfonyl)-piperidin-4-yl]-1,4-dihydro-benzo[d][1,3]oxazin-2-one
245 3-{4-[4-(2-Oxo-4H-benzo[d][1,3]oxazin-1-yl)-piperidine-1-sulfonyl]-phenyl}-propionic acid methyl ester
246 1-[1-(2,4-Dinitro-benzenesulfonyl)-piperidin-4-yl]-1,4-dihydro-benzo[d][1,3]oxazin-2-one
247 1-[1-(7-Chloro-benzo[1,2,5]oxadiazole-4-sulfonyl)-piperidin-4-yl]-8-methyl-1,4-dihydro-benzo[d][1,3]oxazin-2-one
248 1-[1-(2-Methoxy-4-methyl-benzenesulfonyl)-piperidin-4-yl]-8-methyl-1,4-dihydro-benzo[d][1,3]oxazin-2-one
249 3-{4-[4-(8-Methyl-2-oxo-4H-benzo[d][1,3]oxazin-1-yl)-piperidine-1-sulfonyl]-phenyl}-propionic acid methyl ester

250 1-[1-(2,4-Dinitro-benzenesulfonyl)-piperidin-4-yl]-8-methyl-1,4-dihydro-benzo[d][1,3]oxazin-2-one
251 1-[1-(7-Chloro-benzo[1,2,5]oxadiazole-4-sulfonyl)-piperidin-4-yl]-6-methyl-1,4-dihydro-benzo[d][1,3]oxazin-2-one
252 1-[1-(2-Methoxy-4-methyl-benzenesulfonyl)-piperidin-4-yl]-6-methyl-1,4-dihydro-benzo[d][1,3]oxazin-2-one
253 3-{4-[4-(6-Methyl-2-oxo-4H-benzo[d][1,3]oxazin-1-yl)-piperidine-1-sulfonyl]-phenyl}-propionic acid methyl ester
254 1-[1-(2,4-Dinitro-benzenesulfonyl)-piperidin-4-yl]-6-methy1-1,4-dihydro-benzo[d][1,3]oxazin-2-one
255 6-Chloro-1-[1-(7-chloro-benzo[1,2,5]oxadiazole-4-sulfonyl)-piperidin-4-yl]-1,4-dihydro-benzo[d][1,3]oxazin-2-one
256 6-Chloro-1-[1-(2-methoxy-4-methyl-benzenesulfonyl)-piperidin-4-yl]-1,4-dihydro-benzo[d][1,3]oxazin-2-one
257 3-{4-[4-(6-Chloro-2-oxo-4H-benzo[d][1,3]oxazin-1-yl)-piperidine-1-sulfonyl]-phenyl}-propionic acid methyl ester
258 6-Chloro-1-[1-(2,4-dinitro-benzenesulfonyl)-piperidin-4-yl]-1,4-dihydro-benzo[d][1,3]oxazin-2-one
259 6-Chloro-1-[1-(1-methyl-1H-imidazole-4-sulfonyl)-pipendin-4-yl]-1,4-dihydro-benzo[d][1,3]oxazin-2-one
260 1-[1-(5-Bromo-2,4-difluoro-benzenesulfonyl)-piperidin-4-yl]-6-chloro-1,4-dihydro-benzo[d][1,3]oxazin-2-one
261 8-Methyl-1-[1-(1-methyl-1H-imidazole-4-sulfonyl)-piperidin-4-yl]-1,4-dihydro-benzo[d][1,3]oxazin-2-one
262 1-[1-(5-Bromo-2,4-difluoro-benzenesulfonyl)-piperidin-4-yl]-8-methyl-1,4-dihydro-benzo[d][1,3]oxazin-2-one
263 1-[1-(Benzo[b]thiophene-2-sulfonyl)-piperidin-4-yl]-8-methyl-1,4-dihydro-benzo[d][1,3]oxazin-2-one
264 1-[1-(Benzo[b]thiophene-2-sulfonyl)-piperidin-4-yl]-1,4-dihydro-benzo[d][1,3]oxazin-2-one
265 1-[1-(Benzo[b]thiophene-2-sulfonyl)-piperidin-4-yl]-6-chloro-1,4-dihydro-benzo[d][1,3]oxazin-2-one
266 1-[1-(Benzo[b]thiophene-2-sulfonyl)-piperidin-4-yl]-6-methyl-1,4-dihydro-benzo[d][1,3]oxazin-2-one
267 1-[1-(2,5-Difluoro-benzenesulfonyl)-piperidin-4-yl]-1,4-dihydro-benzo[d][1,3]oxazin-2-one
268 1-[1-(2,5-Difluoro-benzenesulfonyl)-piperidin-4-yl]-8-methyl-1,4-dihydro-benzo[d][1,3]oxazin-2-one
269 6-Chloro-1-[1-(2,5-difluoro-benzenesulfonyl)-piperidin-4-yl]-1,4-dihydro-benzo[d][1,3]oxazin-2-one
270 1-[1-(2,5-Difluoro-benzenesulfonyl)-piperidin-4-yl]-6-methyl-1,4-dihydro-benzo[d][1,3]oxazin-2-one
271 1-[1-(4-Chloro-2,5-difluoro-benzenesulfonyl)-piperidin-4-yl]-1,4-dihydro-benzo[d][1,3]oxazin-2-one
272 1-[1-(4-Chloro-2,5-difluoro-benzenesulfonyl)-piperidin-4-yl]-8-methyl-1,4-dihydro-benzo[d][1,3]oxazin-2-one
273 6-Chloro-1-[1-(4-chloro-2,5-difluoro-benzenesulfonyl)-piperidin-4-yl]-1,4-dihydro-benzo[d][1,3]oxazin-2-one
274 1-[1-(4-Chloro-2,5-difluoro-benzenesulfonyl)-piperidin-4-yl]-6-methyl-1,4-dihydro-benzo[d][1,3]oxazin-2-one
275 1-[1-(2,4,5-Trifluoro-benzenesulfonyl)-piperidin-4-yl]-1,4-dihydro-benzo[d][1,3]oxazin-2-one
276 8-Methyl-1-[1-(2,4,5-trifluoro-benzenesulfonyl)-piperidin-4-yl]-1,4-dihydro-benzo[d][1,3]oxazin-2-one
277 6-Chloro-1-[1-(2,4,5-trifluoro-benzenesulfonyl)-piperidin-4-yl]-1,4-dihydro-benzo[d][1,3]oxazin-2-one
278 6-Methyl-1-[1-(2,4,5-trifluoro-benzenesulfonyl)-piperidin-4-yl]-1,4-dihydro-benzo[d][1,3]oxazin-2-one
279 1-[1-(3,5-Dichloro-2-hydroxy-benzenesulfonyl)-piperidin-4-y)]-1,4-dihydro-benzo[d][1,3]oxazin-2-one
280 1-[1-(2,6-Difluoro-benzenesulfonyl)-piperidin-4-yl]-1,4-dihydro-benzo[d][1,3]oxazin-2-one
281 1-[1-(2,6-Difluoro-benzenesulfonyl)-piperidin-4-yl]-8-methyl-1,4-dihydro-benzo[d][1,3]oxazin-2-one
282 6-Chloro-1-[1-(2,6-difluoro-benzenesulfonyl)-piperidin-4-yl]-1,4-dihydro-benzo[d][1,3]oxazin-2-one
283 1-[1-(2,6-Difluoro-benzenesulfonyl)-piperidin-4-yl]-6-methyl-1,4-dihydro-benzo[d][1,3]oxazin-2-one
284 1-[1-(5-Chloro-2,4-difluoro-benzenesulfonyl)-piperidin-4-yl]-1,4-dihydro-benzo[d][1,3]oxazin-2-one
285 1-[1-(5-Chloro-2,4-difluoro-benzenesulfonyl)-piperidin-4-yl]-8-methyl-1,4-dihydro-benzo[d][1,3]oxazin-2-one
286 6-Chloro-1-[1-(5-chloro-2,4-difluoro-benzenesulfonyl)-piperidin-4-yl]-1,4-dihydro-benzo[d][1,3]oxazin-2-one
287 1-[1-(5-Chloro-2,4-difluoro-benzenesulfonyl)-piperidin-4-yl]-6-methyl-1,4-dihydro-benzo[d][1,3]oxazin-2-one
288 1-[1-(2-Chloro-benzenesuffonyl)-piperidin-4-yl]-1,4-dihydro-benzo[d][1,3]oxazin-2-one
289 1-[1-(2-Chloro-benzenesulfonyl)-piperidin-4-yl]-8-methyl-1,4-dihydro-benzo[d][1,3]oxazin-2-one
290 6-Chloro-1-[1-(2-chloro-benzenesulfonyl)-piperidin-4-yl]-1,4-dihydro-benzo[d][1,3]oxazin-2-one
291 1-[1-(2-Chloro-benzenesulfonyl)-piperidin-4-yl]-6-methyl-1,4-dihydro-benzo[d][1,3]oxazin-2-one
292 6-Chloro-1-[1-(2-naphthalen-1-yl-ethanesulfonyl)-piperidin-4-yl]-1,4-dihydro-benzo[d][1,3]oxazin-2-one
293 6-Bromo-1-[1-(4-bromo-benzenesulfonyl)-piperidin-4-yl]-1,4-dihydro-benzo[d][1,3]oxazin-2-one
294 6-Bromo-1-[1-(toluene-4-sulfonyl)-piperidin-4-yl]-1 4-dihydro-benzo[d][1,3]oxazin-2-one
295 6-Bromo-1-[1-(2,4-dimethyl-benzenesulfonyl)-piperidin-4-yl]-1,4-dihydro-benzo[d][1,3]oxazin-2-one
296 6-Bromo-1-[1-(2-naphthalen-1-yl-ethanesulfonyl)-piperidin-4-yl]-1,4-dihydro-benzo[d][1,3]oxazin-2-one
297 6-Bromo-1-[1-(quinoline-8-sulfonyl)-piperidin-4-yl]-1,4-dihydro-benzo[d][1,3]oxazin-2-one
298 6-Bromo-1-[1-(5-chloro-3-methyl-benzo[b]thiophene-2-sulfonyl)-piperidin-4-yl]-1,4-dihydro-benzo[d][1,3]oxazin-2-one
299 6-Bromo-1-[1-(3-nitro-benzenesulfonyl)-piperidin-4-yl]-1,4-dihydro-benzo[d][1,3]oxazin-2-one

300 6-Bromo-1-[1-(naphthalene-1-sulfonyl)-piperidin-4-yl]-1,4-dihydro-benzo[d][1,3]oxazin-2-one
301 6-Bromo-1-[1-(naphthalene-2-sulfonyl)-piperidin-4-yl]-1,4-dihydro-benzo[d][1,3]oxazin-2-one
302 1-(1-Benzenesulfonyl-piperidin-4-yl)-6-bromo-1,4-dihydro-benzo[d][1,3]oxazin-2-one
303 6-Bromo-1-{1-[4-(4-bromo-phenoxy)-benzenesulfonyl]-piperidin-4-yl}-1,4-dihydro-benzo[d][1,3]oxazin-2-one
304 6-Bromo-1-[1-(thiophene-2-sulfonyl)-piperidin-4-yl]-1,4-dihydro-benzo[d][1,3]oxazin-2-one
305 6-Bromo-1-[1-(2-methyl-5-nitro-benzenesulfonyl)-piperidin-4-yl]-1,4-dihydro-benzo[d][1,3]oxazin-2-one
306 6-Bromo-1-[1-(4-bromo-2,5-difluoro-benzenesulfonyl)-piperidin-4-yl]-1,4-dihydro-benzo[d][1,3]oxazin-2-one
307 6-Bromo-1-[1 -(toluene-3-sulfonyl)-piperidin-4-yl]-1,4-dihydro-benzo[d][1,3]oxazin-2-one
308 6-Bromo-1-[1-(5-fluoro-2-methyl-benzenesulfonyl)-piperidin-4-yl]-1,4-dihydro-benzo[d][1,3]oxazin-2-one
309 6-Bromo-1-[1-(4-isopropoxy-benzenesulfonyl)-piperidin-4-yl]-1,4-dihydro-benzo[d][1,3]oxazin-2-one
310 6-Bromo-1-[1-(3-chloro-benzenesulfonyl)-piperidin-4-yl]-1,4-dihydro-benzo[d][1,3]oxazin-2-one
311 6-Bromo-1-[1-(3,4-dimethoxy-benzenesufonyl)-piperidin-4-yl]-1,4-dihydro-benzo[d][1,3]oxazin-2-one
312 6-Bromo-1-(1-pentafluorobenzenesulfonyl-piperidin-4-yl)-1,4-dihydro-benzo[d][1,3]oxazin-2-one
313 6-Bromo-1-[1-(4-chloro-2,5-dimethyl-benzenesulfonyl)-piperidin-4-yl]-1,4-dihydro-benzo[d][1,3]oxazin-2-one
314 6-Bromo-1-[1-(3-methoxy-benzenesulfonyl)-piperidin-4-yl]-1,4-dihydro-benzo[d][1,3]oxazin-2-one
315 6-Bromo-1-[1-(4-isopropyl-benzenesulfonyl)-piperidin-4-yl]-1,4-dihydro-benzo[d][1,3]oxazin-2-one
316 6-Bromo-1-[1-(4-fluoro-benzenesulfonyl)-piperidin-4-yl]-1,4-dihydro-benzo[d][1,3]oxazin-2-one
317 6-Bromo-1-[1-(3-chloro-4-fluoro-benzenesulfonyl)-piperidin-4-yl]-1,4-dihydro-benzo[d][1,3]oxazin-2-one
318 6-Bromo-1-(1-pentamethylbenzenesulfonyl-piperidin-4-yl)-1,4-dihydro-benzo[d][1,3]oxazin-2-one
319 6-Bromo-1-[1-(2-nitro-benzenesulfonyl)-piperidin-4-yl]-1,4-dihydro-benzo[d][1,3]oxazin-2-one
320 6-Bromo-1-[1-(4-chloro-3-nitro-benzenesulfonyl)-piperidin-4-yl]-1,4-dihydro-benzo[d][1,3]oxazin-2-one
321 6-Bromo-1-[1-(5-dimethylamino-naphthalene-1-sulfonyl)-piperidin-4-yl]-1,4-dihydro-benzo[d][1,3]oxazin-2-one
322 6-Bromo-1-[1-(4-nitro-benzenesulfonyl)-piperidin-4-yl]-1,4-dihydro-benzo[d][1,3]oxazin-2-one
323 1-[1-(4-Acetyl-benzenesulfonyl)-pipendin-4-yl]-6-bromo-1,4-dihydro-benzo[d][1,3]oxazin-2-one
324 6-Bromo-1-[1-(4-methanesulfonyl-benzenesulfonyl)-pipendin-4-yl]-1,4-dihydro-benzo[d][1,3]oxazin-2-one
325 1-[1-(Biphenyl-4-sulfonyl)-piperidin-4-yl]-6-bromo-1,4-dihydro-benzo[d][1,3]oxazin-2-one
326 6-Bromo-1-(1-phenylmethanesulfonyl-piperidin-4-yl)-1,4-dihydro-benzo[d][1,3]oxazin-2-one
327 6-Bromo-1-[1-(2,5-dimethoxy-benzenesulfonyl)-piperidin-4-yl]-1,4-dihydro-benzo[d][1,3]oxazin-2-one
328 6-Bromo-1-{1-[2-(2,2,2-trifluoro-acetyl)-1,2,3,4-tetrahydro-isoquinoline-7-sulfonyl]-piperidin-4-yl}-1,4-dihydro-benzo[d][1,3]oxazin-2-one
329 6-Bromo-1-[1-(2,3-dichloro-benzenesulfonyl)-piperidin-4-yl]-1,4-dihydro-benzo[d][1,3]oxazin-2-one
330 6-Bromo-1-[1-(2,4,5-trichloro-benzenesulfonyl)-piperidin-4-yl]-1,4-dihydro-benzo[d][1,3]oxazin-2-one
331 6-Bromo-1-[1-(5-bromo-2-methoxy-benzenesulfonyl)-piperidin-4-yl]-1,4-dihydro-benzo[d][1,3]oxazin-2-one
332 6-Bromo-1-[1-(4-trifluoromethoxy-benzenesulfonyl)-piperidin-4-yl]-1,4-dihydro-benzo[d][1,3]oxazin-2-one
333 6-Bromo-1-[1-(2-nitro-4-trifluoromethyl-benzenesulfonyl)-piperidin-4-yl]-1,4-dihydro-benzo[d][1,3]oxazin-2-one
334 6-Bromo-1-[1-(3-fluoro-benzenesulfonyl)-piperidin-4-yl]-1,4-dihydro-benzo[d][1,3]oxazin-2-one
335 6-Bromo-1-[1-(2,4-dichloro-benzenesulfonyl)-piperidin-4-yl]-1,4-dihydro-benzo[d][1,3]oxazin-2-one
336 6-Bromo-1-[1-(2,4,6-trimethyl-benzenesulfonyl)-piperidin-4-yl]-1,4-dihydro-benzo[d][1,3]oxazin-2-one
337 6-Bromo-1-[1-(2-trifluoromethyl-benzenesulfonyl)-piperidin-4-yl]-1,4-dihydro-benzo[d][1,3]oxazin-2-one
338 6-Bromo-1-[1-(2-bromo-benzenesulfonyl)-piperidin-4-yl]-1,4-dihydro-benzo[d][1,3]oxazin-2-one
339 6-Bromo-1-[1-(4-methoxy-2,3,6-trimethyl-benzenesulfonyl)-piperidin-4-yl]-1,4-dihydro-benzo[d][1,3]oxazin-2-one
340 1-[1-(3,5-Dichloro-4-hydroxy-benzenesulfonyl)-piperidin-4-yl]-1,4-dihydro-benzo[d][1,3]oxazin-2-one
341 1-[1-(3,5-Dichloro-4-hydroxy-benzenesulfonyl)-piperidin-4-yl]-8-methyl-1,4-dihydro-benzo[d][1,3]oxazin-2-one
342 6-Chloro-1-[1-(3,5-dichloro-4-hydroxy-benzenesulfonyl)-piperidin-4-yl]-1,4-dihydro-benzo[d][1,3]oxazin-2-one
343 1-[1-(3,5-Dichloro-4-hydroxy-benzenesulfonyl)-piperidin-4-yl]-6-methyl-1,4-dihydro-benzo[d][1,3]oxazin-2-one
344 6-Bromo-1-[1-(3,5-dichloro-4-hydroxy-benzenesulfonyl)-piperidin-4-yl]-1,4-dihydro-benzo[d][1,3]oxazin-2-one
345 6-Chloro-1-[1-(3,5-dichloro-2-hydroxy-benzenesulfonyl)-piperidin-4-yl]-1,4-dihydro-benzo[d][1,3]oxazin-2-one
346 6-Bromo-1-[1-(3,5-dichloro-2-hydroxy-benzenesulfonyl)-piperidin-4-yl]-1,4-dihydro-benzo[d][1,3]oxazin-2-one
347 2-[4-(6-Bromo-2-oxo-4H-benzo[d][1,3]oxazin-1-yl)-piperidine-1-sulfonyl]-benzonitrile
348 6-Bromo-1-[1-(4-methoxy-benzenesulfonyl)-piperidin-4-yl]-1,4-dihydro-benzo[d][1,3]oxazin-2-one
349 2-[4-(6-Bromo-2-oxo-4H-benzo[d][1,3]oxazin-1-yl)-piperidine-1-sulfonyl]-benzoic acid methyl ester

350 6-Bromo-1-[1-(3-trifluoromethyl-benzenesulfonyl)-piperidin-4-yl]-1,4-dihydro-benzo[d][1,3]oxazin-2-one
351 6-Bromo-1-[1-(2-oxo-2H-chromene-6-sulfonyl)-piperidin-4-yl]-1,4-dihydro-benzo[d][1,3]oxazin-2-one
352 6-Bromo-1-[1-(3,5-dichloro-benzenesulfonyl)-piperidin-4-yl]-1,4-dihydro-benzo[d][1,3]oxazin-2-one
353 6-Bromo-1-[1-(2,5-dichloro-benzenesuffonyl)-piperidin-4-yl]-1,4-dihydro-benzo[d][1,3joxazin-2-one
354 6-Bromo-1-[1-(5-bromo-6-chloro-pyridine-3-sulfonyl)-piperidin-4-yl]-1,4-dihydro-benzo[d][1,3]oxazin-2-one
355 6-Bromo-1-[1-(4-chloro-benzenesulfonyl)-piperidin-4-yl]-1,4-dihydro-benzo[d][1,3]oxazin-2-one
356 6-Bromo-1-[1-(2,6-dichloro-benzenesulfonyl)-piperidin-4-yl]-1,4-dihydro-benzo[d][1,3]oxazin-2-one
357 6-Bromo-1-[1-(1-methyl-1H-imidazole-4-sulfonyl)-piperidin-4-yl]-1,4-dihydro-benzo[d][1,3]oxazin-2-one
358 6-Bromo-1-[1-(5-bromo-2,4-difluoro-benzenesulfonyl)-piperidin-4-yl]-1,4-dihydro-benzo[d][1,3]oxazin-2-one
359 6-Bromo-1-[1-(4-ethyl-benzenesulfonyl)-piperidin-4-yl]-1,4-dihydro-benzo[d][1,3]oxazin-2-one
360 6-Bromo-1-[1-(6-chloro-imidazo[2,1-b]thiazole-5-sulfonyl)-piperidin-4-yl]-1,4-dihydro-benzo[d][1,3]oxazin-2-one
361 1-[1-(Benzo[b]thiophene-3-sulfonyl)-piperidin-4-yl]-6-bromo-1,4-dihydro-benzo[d][1,3]oxazin-2-one
362 6-Bromo-1-[1-(7-chloro-benzo[1,2,5]oxadiazole-4-sulfonyl)-piperidin-4-yl]-1,4-dihydro-benzo[d][1,3]oxazin-2-one
363 6-Bromo-1-[1-(2-methoxy-4-methyl-benzenesulfonyl)-piperidin-4-yl]-1,4-dihydro-benzo[d][1,3]oxazin-2-one
364 3-{4-[4-(6-Bromo-2-oxo-4H-benzo[d][1,3]oxazin-1-yl)-piperidine-1-sulfonyl]-phenyl}-propionic acid methyl ester
365 6-Bromo-1-[1-(2,4-dinitro-benzenesulfonyl)-piperidin-4-yl]-1,4-dihydro-benzo[d][1,3]oxazin-2-one
366 1-[1-(Benzo[b]thiophene-2-sulfonyl)-piperidin-4-yl]-6-bromo-1,4-dihydro-benzo[d][1,3]oxazin-2-one
367 6-Bromo-1-[1-(2,5-difluoro-benzenesulfonyl)-piperidin-4-yl]-1,4-dihydro-benzo[d][1,3]oxazin-2-one
368 6-Bromo-1-[1-(4-chloro-2,5-difluoro-benzenesulfonyl)-piperidin-4-yl]-1,4-dihydro-benzo[d][1,3]oxazin-2-one
369 6-Bromo-1-[1-(2,4,5-trifluoro-benzenesulfonyl)-piperidin-4-yl]-1,4-dihydro-benzo[d][1,3]oxazin-2-one
370 6-Bromo-1-[1-(2,6-difluoro-benzenesulfonyl)-piperidin-4-yl]-1,4-dihydro-benzo[d][1,3]oxazin-2-one
371 6-Bromo-1-[1-(5-chloro-2,4-difluoro-benzenesulfonyl)-piperidin-4-yl]-1,4-dihydro-benzo[d][1,3]oxazin-2-one
372 6-Bromo-1-[1-(2-chloro-benzenesulfonyl)-piperidin-4-yl]-1,4-dihydro-benzo[d][1,3]oxazin-2-one
373 6-Bromo-1-[1-(2,3,4-trifluoro-benzenesulfonyl)-piperidin-4-yl]-1,4-dihydro-benzo[d][1,3]oxazin-2-one
374 N-{4-[4-(6-Bromo-2-oxo-4H-benzo[d][1,3]oxazin-1-yl)-piperidine-1-sulfonyl]-2-chloro-phenyl}-acetamide
375 1-[1-(2,3,4-Trifluoro-benzenesulfonyl)-piperidin-4-yl]-1,4-dihydro-benzo[d][1,3]oxazin-2-one
376 8-Methyl-1-[1-(2,3,4-trifluoro-benzenesulfonyl)-piperidin-4-yl]-1,4-dihydro-benzo[d][1,3]oxazin-2-one
377 6-Chloro-1-[1-(2,3,4-trifluoro-benzenesulfonyl)-piperidin-4-yl]-1,4-dihydro-benzo[d][1,3]oxazin-2-one
378 6-Methyl-1-[1-(2,3,4-trifluoro-benzenesulfonyl)-piperidin-4-yl]-1,4-dihydro-benzo[d][1,3]oxazin-2-one
379 N-{2-Chloro-4-[4-(6-methyl-2-oxo-4H-benzo[d][1,3]oxazin-1-yl)-piperidine-1-sulfonyl]-phenyl}-acetamide
380 1-[1-(3,4-Difluoro-benzenesulfonyl)-piperidin-4-yl]-1,4-dihydro-benzo[d][1,3]oxazin-2-one
381 1-[1-(3,4-Difluoro-benzenesulfonyl)-piperidin-4-yl]-8-methyl-1,4-dihydro-benzo[d][1,3]oxazin-2-one
382 6-Chloro-1-[1-(3,4-difluoro-benzenesulfonyl)-piperidin-4-yl]-1,4-dihydro-benzo[d][1,3]oxazin-2-one
383 1-[1-(3,4-Difluoro-benzenesulfonyl)-piperidin-4-yl]-6-methyl-1,4-dihydro-benzo[d][1,3]oxazin-2-one
384 6-Bromo-1-[1-(3,4-difluoro-benzenesulfonyl)-piperidin-4-yl]-1,4-dihydro-benzo[d][1,3]oxazin-2-one
385 N-{2-Chloro-4-[4-(8-methyl-2-oxo-4H-benzo[d][1,3]oxazin-1-yl)-piperidine-1-sulfonyl]-phenyl}-aoetamide
386 1-[1-(2-Chloro-4,5-difluoro-benzenesulfonyl)-piperidin-4-yl]-1,4-dihydro-benzo[d][1,3]oxazin-2-one
387 1-[1-(2-Chloro-4,5-difluoro-benzenesulfonyl)-piperidin-4-yl]-8-methyl-1,4-dihydro-benzo[d][1,3]oxazin-2-one
388 6-Chloro-1-[1-(2-chloro-4,5-difluoro-benzenesulfonyl)-piperidin-4-yl]-1,4-dihydro-benzo[d][1,3]oxazin-2-one
389 1-[1-(2-Chloro-4,5-difluoro-benzenesulfonyl)-piperidin-4-yl]-6-methyl-1,4-dihydro-benzo[d][1,3]oxazin-2-one
390 6-Bromo-1-[1-(2-chloro-4,5-difluoro-benzenesulfonyl)-piperidin-4-yl]-1,4-dihydro-benzo[d][1,3]oxazin-2-one
391 N-{2-Chloro-4-[4-(2-oxo-4H-benzo[d][1,3]oxazin-1-yl)-piperidine-1-sulfonyl]-phenyl}-acetamide
392 1-[1-(Benzo[1,2,5]oxadiazole-4-sulfonyl)-piperidin-4-yl]-1,4-dihydro-benzo[d][1,3]oxazin-2-one
393 1-[1-(Benzo[1,2,5]oxadiazole-4-sulfonyl)-piperidin-4-yl]-8-methyl-1,4-dihydro-benzo[d][1,3]oxazin-2-one
394 1-[1-(Benzo[1,2,5]oxadiazole-4-sulfonyl)-piperidin-4-yl]-6-chloro-1,4-dihydro-benzo[d][1,3]oxazin-2-one
395 1-[1-(Benzo[1,2,5]oxadiazole-4-sulfonyl)-piperidin-4-yl]-6-methyl-1,4-dihydro-benzo[d][1,3]oxazin-2-one
396 1-[1-(Benzo[1,2,5]oxadiazole-4-sulfonyl)-piperidin-4-yl]-6-bromo-1,4-dihydro-benzo[d][1,3]oxazin-2-one
397 N-{2-Chloro-4-[4-(6-chloro-2-oxo-4H-benzo[d][1,3]oxazin-1-yl)-piperidine-1-sulfonyl]-phenyl}-acetamide
398 1-[1-(Benzo[1,2,5]thiadiazole-4-sulfonyl)-piperidin-4-yl]-1,4-dihydro-benzo[d][1,3]oxazin-2-one
399 1-[1-(Benzo[1,2,5]thiadiazole-4-sulfonyl)-piperidin-4-yl]-8-methyl-1,4-dihydro-benzo[d][1,3]oxazin-2-one

400 1-[1-(Benzo[1,2,5]thiadiazole-4-sulfonyl)-piperidin-4-yl]-6-chloro-1,4-dihydro-benzo[d][1,3]oxazin-2-one
401 1-[1-(Benzo[1,2,5]thiadiazole-4-sulfonyl)-piperidin-4-yl]-6-methyl-1,4-dihydro-benzo[d][1,3]oxazin-2-one
402 1-[1-(Benzo[1,2,5]thiadiazole-4-sulfonyl)-piperidin-4-yl]-6-bromo-1,4-dihydro-benzo[d][1,3]oxazin-2-one
403 1-(1-Ethanesulfonyl-piperidin-4-yl)-8-methyl-1,4-dihydro-benzo[d][1,3]oxazin-2-one
404 1-[1-(2,4-Difluoro-benzenesulfonyl)-piperidin-4-yl]-1,4-dihydro-benzo[d][1,3]oxazin-2-one
405 1-[1-(2,4-Difluoro-benzenesulfonyl)-piperidin-4-yl]-8-methyl-1,4-dihydro-benzo[d][1,3]oxazin-2-one
406 6-Chloro-1-[1-(2,4-difluoro-benzenesulfonyl)-piperidin-4-yl]-1,4-dihydro-benzo[d][1,3]oxazin-2-one
407 1-[1-(2,4-Difluoro-benzenesulfonyl)-piperidin-4-yl]-6-methyl-1,4-dihydro-benzo[d][1,3]oxazin-2-one
408 6-Bromo-1-[1-(2,4-difluoro-benzenesulfonyl)-piperidin-4-yl]-1,4-dihydro-benzo[d][1,3]oxazin-2-one
409 8-Methyl-1-[1-(propane-2-sulfonyl)-piperidin-4-yl]-1,4-dihydro-benzo[d][1,3]oxazin-2-one
410 1-[1-(3,4-Dichloro-benzenesulfonyl)-piperidin-4-yl]-1,4-dihydro-benzo[d][1,3]oxazin-2-one
411 1-[1-(3,4-Dichloro-benzenesulfonyl)-piperidin-4-yl]-8-methy1-1,4-dihydro-benzo[d][1,3]oxazin-2-one
412 6-Chloro-1-[1-(3,4-dichloro-benzenesulfonyl)-piperidin-4-yl]-1,4-dihydro-benzo[d][1,3]oxazin-2-one
413 1-[1-(3,4-Dichloro-benzenesulfonyl)-piperidin-4-yl]-6-methyl-1,4-dihydro-benzo[d][1,3]oxazin-2-one
414 6-Bromo-1-[1-(3,4-dichloro-benzenesulfonyl)-piperidin-4-yl]-1,4-dihydro-benzo[d][1,3]oxazin-2-one
415 8-Methyl-1-[1-(propane-1-sulfonyl)-piperidin-4-yl]-1,4-dihydro-benzo[d][1,3]oxazin-2-one
416 1-[1-(2-Chloro-6-methyl-benzenesulfonyl)-piperidin-4-yl]-1,4-dihydro-benzo[d][1,3]oxazin-2-one
417 1-[1-(2-Chloro-6-methyl-benzenesulfonyl)-piperidin-4-yl]-8-methyl-1,4-dihydro-benzo[d][1,3]oxazin-2-one
418 6-Chloro-1-[1-(2-chloro-6-methyl-benzenesulfonyl)-piperidin-4-yl]-1,4-dihydro-benzo[d][1,3]oxazin-2-one
419 1-[1-(2-Chloro-6-methyl-benzenesulfonyl)-piperidin-4-yl]-6-methyl-1,4-dihydro-benzo[d][1,3]oxazin-2-one
420 1-[1-(2-Chloro-6-methyl-benzenesulfonyl)-piperidin-4-yl]-6-methyl-1,4-dihydro-benzo[d][1,3]oxazin-2-one
421 8-Methyl-1-[1-(2,3,5,6-tetramethyl-benzenesulfonyl)-piperidin-4-yl]-1,4-dihydro-benzo[d][1,3]oxazin-2-one
422 1-[1-(2,3,4-Trichloro-benzenesulfonyl)-piperidin-4-yl]-1,4-dihydro-benzo[d][1,3]oxazin-2-one
423 8-Methyl-1-[1-(2,3,4-trichloro-benzenesulfonyl)-piperidin-4-yl]-1,4-dihydro-benzo[d][1,3]oxazin-2-one
424 6-Chloro-1-[1-(2,3,4-trichloro-benzenesulfonyl)-piperidin-4-yl]-1,4-dihydro-benzo[d][1,3]oxazin-2-one
425 6-Methyl-1-[1-(2,3,4-trichloro-benzenesulfonyl)-piperidin-4-yl]-1,4-dihydro-benzo[d][1,3]oxazin-2-one
426 6-Bromo-1-[1-(2,3,4-trichloro-benzenesulfonyl)-piperidin-4-yl]-1,4-dihydro-benzo[d][1,3]oxazin-2-one
427 1-[1-(2,3,5,6-Tetramethyl-benzenesulfonyl)-piperidin-4-yl]-1,4-dihydro-benzo[d][1,3]oxazin-2-one
428 1-[1-(Thiophene-3-sulfonyl)-piperidin-4-yl]-1,4-dihydro-benzo[d][1,3]oxazin-2-one
429 8-Methyl-1-[1-(thiophene-3-sulfonyl)-piperidin-4-yl]-1,4-dihydro-benzo[d][1,3]oxazin-2-one
430 6-Chloro-1-[1-(thiophene-3-sulfonyl)-piperidin-4-yl]-1,4-dihydro-benzo[d][1,3]oxazin-2-one
431 6-Methyl-1-[1-(thiophene-3-sulfonyl)-piperidin-4-yl]-1,4-dihydro-benzo[d][1,3]oxazin-2-one
432 6-Bromo-1-[1-(thiophene-3-sulfonyl)-piperidin-4-yl]-1,4-dihydro-benzo[d][1,3]oxazin-2-one
433 6-Chloro-1-[1-(2,3,5,6-tetramethyl-benzenesulfonyl)-piperidin-4-yl]-1.4-dihydro-benzo[d][1,3]oxazin-2-one
434 1-[1-(2,4,6-Trichloro-benzenesulfonyl)-piperidin-4-yl]-1,4-dihydro-benzo[d][1,3]oxazin-2-one
435 8-Methyl-1-[1-(2,4,6-trichloro-benzenesulfonyl)-piperidin-4-yl]-1,4-dihydro-benzo[d][1,3]oxazin-2-one
436 6-Chloro-1-[1-(2,4,6-trichloro-benzenesulfonyl)-piperidin-4-yl]-1,4-dihydro-benzo[d][1,3]oxazin-2-one
437 6-Methyl-1-[1-(2,4,6-trichloro-benzenesulfonyl)-piperidin-4-yl]-1,4-dihydro-benzo[d][1,3]oxazin-2-one
438 6-Bromo-1-[1-(2,4,6-trichloro-benzenesulfonyl)-piperidin-4-yl]-1,4-dihydro-benzo[d][1,3]oxazin-2-one
439 6-Methyl-1-[1-(2,3,5,6-tetramethyl-benzenesulfonyl)-piperidin-4-yl]-1,4-dihydro-benzo[d][1,3]oxazin-2-one
440 1-[1-(2-Bromo-4,6-difluoro-benzenesulfonyl)-piperidin-4-yl]-8-methyl-1,4-dihydro-benzo[d][1,3]oxazin-2-one
441 1 -[1 -(2-Bromo-4,6-difluoro-benzenesulfonyl)-piperidin-4-yl]-6-chloro-1,4-dihydro-benzo[d][1,3]oxazin-2-one
442 1 -[1 -(2-Bromo-4,6-difluoro-benzenesulfonyl)-pipendin-4-yl]-6-methyl-1,4-dihydro-benzo[d][1,3]oxazin-2-one
443 6-Bromo-1-[1-(2-bromo-4,6-difluoro-benzenesulfonyl)-piperidin-4-yl]-1,4-dihydro-benzo[d][1,3]oxazin-2-one
444 6-Bromo-1 -[1 -(2,3,5,6-tetramethyl-benzenesulfonyl)-piperidin-4-yl]-1,4-dihydro-benzo[d][1,3]oxazin-2-one
445 1-[1-(4-Bromo-2-trifluoromethoxy-benzenesulfonyl)-piperidin-4-yl]-1,4-dihydro-benzo[d][1,3]oxazin-2-one
446 1-[1-(4-Bromo-2-trifluoromethoxy-benzenesulfonyl)-piperidin-4-yl]-8-methyl-1,4-dihydro-benzo[d][1,3]oxazin-2-one
447 1-[1-(4-Bromo-2-trifluoromethoxy-benzenesulfonyl)-piperidin-4-yl]-6-chloro-1,4-dihydro-benzo[d][1,3]oxazin-2-one
448 1-[1-(4-Bromo-2-trifluoromethoxy-benzenesulfonyl)-piperidin-4-yl]-6-methyl-1,4-dihydro-benzo[d][1,3]oxazin-2-one
449 6-Bromo-1-[1-(4-bromo-2-trifluoromethoxy-benzenesulfonyl)-piperidin-4-yl]-1,4-dihydro-benzo[d][1,3]oxazin-2-one

450 1-[1-(4-Phenoxy-benzenesulfonyl)-piperidin-4-yl]-1,4-dihydro-benzo[d][1,3]oxazin-2-one
451 1-[1-(3-Bromo-benzenesulfonyl)-piperidin-4-yl]-1,4-dihydro-benzo[d][1,3]oxazin-2-one
452 1-[1-(3-Bromo-benzenesulfonyl)-piperidin-4-yl]-8-methyl-1,4-dihydro-benzo[d][1,3]oxazin-2-one
453 1-[1-(3-Bromo-benzenesulfonyl)-piperidin-4-yl]-6-chloro-1,4-dihydro-benzo[d][1,3]oxazin-2-one
454 1-[1-(3-Bromo-benzenesulfonyl)-piperidin-4-yl]-6-methyl-1,4-dihydro-benzo[d][1,3]oxazin-2-one
455 6-Bromo-1-[1-(3-bromo-benzenesulfonyl)-piperidin-4-yl]-1,4-dihydro-benzo[d][1,3]oxazin-2-one
456 8-Methyl-1-[1-(4-phenoxy-benzenesulfonyl)-piperidin-4-yl]-1,4-dihydro-benzo[d][1,3]oxazin-2-one
457 1-[1-(4-tert-Butyl-benzenesulfonyl)-piperidin-4-yl]-1,4-dihydro-benzo[d][1,3]oxazin-2-one
458 1 -[1 -(4-tert-Butyl-benzenesulfonyl)-piperidin-4-yl]-8-methyl-1,4-dihydro-benzo[d][1,3]oxazin-2-one
459 1-[1-(4-tert-Butyl-benzenesulfonyl)-piperidin-4-yl]-6-chloro-1,4-dihydro-benzo[d][1,3]oxazin-2-one
460 1-[1-(4-tert-Butyl-benzenesulfonyl)-piperidin-4-yl]-6-methyl-1,4-dihydro-benzo[d][1,3]oxazin-2-one
461 6-Bromo-1-[1-(4-tert-butyl-benzenesulfonyl)-piperidin-4-yl]-1,4-dihydro-benzo[d][1,3]oxazin-2-one
462 6-Chloro-1-[1-(4-phenoxy-benzenesulfonyl)-piperidin-4-yl]-1,4-dihydro-benzo[d][1,3]oxazin-2-one
463 1-[1-(2-Bromo-4,6-difluoro-benzenesulfonyl)-piperidin-4-yl]-1,4-dihydro-benzo[d][1,3]oxazin-2-one
464 1-[1-(2-Methanesulfonyl-benzenesulfonyl)-piperidin-4-yl]-8-methyl-1,4-dihydro-benzo[d][1,3]oxazin-2-one
465 6-Chloro-1-[1-(2-methanesulfonyl-benzenesulfonyl)-piperidin-4-yl]-1,4-dihydro-benzo[d][1,3]oxazin-2-one
466 1-[1-(2-Methanesulfonyl-benzenesulfonyl)-piperidin-4-yl]-6-methyl-1,4-dihydro-benzo[d][1,3]oxazin-2-one
467 6-Bromo-1-[1-(2-methanesulfonyl-benzenesulfonyl)-piperidin-4-yl]-1,4-dihydro-benzo[d][1,3]oxazin-2-one
468 8-Methyl-1-[1-(4-propyl-benzenesulfonyl)-piperidin-4-yl]-1,4-dihydro-benzo[d][1,3]oxazin-2-one
469 6-Chloro-1-[1-(4-propyl-benzenesulfonyl)-piperidin-4-yl]-1,4-dihydro-benzo[d][1,3]oxazin-2-one
470 6-Methyl-1-[1-(4-propyl-benzenesulfonyl)-piperidin-4-yl]-1,4-dihydro-benzo[d][1,3]oxazin-2-one
471 6-Bromo-1-[1-(4-propyl-benzenesulfonyl)-piperidin-4-yl]-1,4-dihydro-benzo[d][1,3]oxazin-2-one
472 1-[1-(3-Chloro-2-methyl-benzenesulfonyl)-piperidin-4-yl]-8-methyl-1,4-dihydro-benzo[d][1,3]oxazin-2-one
473 6-Chloro-1-[1-(3-chloro-2-methyl-benzenesulfonyl)-piperidin-4-yl]-1,4-dihydro-benzo[d][1,3]oxazin-2-one
474 1-[1-(3-Chloro-2-methyl-benzenesulfonyl)-piperidin-4-yl]-6-methyl-1,4-dihydro-benzo[d][1,3]oxazin-2-one
475 6-Bromo-1-[1-(3-chloro-2-methyl-benzenesulfonyl)-piperidin-4-yl]-1,4-dihydro-benzo[d][1,3]oxazin-2-one
476 1-[1-(4-Butyl-benzenesulfonyl)-piperidin-4-yl]-8-methyl-1,4-dihydro-benzo[d][1,3]oxazin-2-one
477 1-[1-(4-Butyl-benzenesulfonyl)-piperidin-4-yl]-6-chloro-1,4-dihydro-benzo[d][1,3]oxazin-2-one
478 1-[1-(4-Butyl-benzenesulfonyl)-piperidin-4-yl]-6-methyl-1,4-dihydro-benzo[d][1,3]oxazin-2-one
479 6-Bromo-1-[1-(4-butyl-benzenesulfonyl)-piperidin-4-yl]-1,4-dihydro-benzo[d][1,3]oxazin-2-one
480 1-[1-(4-Bromo-3-methyl-benzenesulfonyl)-piperidin-4-yl]-8-methyl-1,4-dihydro-benzo[d][1,3]oxazin-2-one
481 1-[1-(4-Bromo-3-methyl-benzenesulfonyl)-piperidin-4-yl]-6-chloro-1,4-dihydro-benzo[d][1,3]oxazin-2-one
482 1-[1-(4-Bromo-3-methyl-benzenesulfonyl)-piperidin-4-yl]-6-methyl-1,4-dihydro-benzo[d][1,3]oxazin-2-one
483 6-Bromo-1-[1-(4-bromo-3-methyl-benzenesulfonyl)-piperidin-4-yl]-1,4-dihydro-benzo[d][1,3]oxazin-2-one
484 1-{1-[4-(1,1-Dimethyl-propyl)-benzenesulfonyl]-piperidin-4-yl}-8-methyl-1,4-dihydro-benzo[d][1,3]oxazin-2-one
485 6-Chloro-1-{1-[4-(1,1-dimethyl-propyl)-benzenesulfonyl]-piperidin-4-yl}-1,4-dihydro-benzo[d][1,3]oxazin-2-one
486 1-{1-[4-(1,1-Dimethyl-propyl)-benzenesulfonyl]-piperidin-4-yl}-6-methyl-1,4-dihydro-benzo[d][1,3]oxazin-2-one
487 6-Bromo-1-{1-[4-(1,1-dimethyl-propyl)-benzenesulfonyl]-piperidin-4-yl}-1,4-dihydro-benzo[d][1,3]oxazin-2-one
488 1-(1-Ethenesulfonyl-pipendin-4-yl)-8-methyl-1,4-dihydro-benzo[d][1,3]oxazin-2-one
489 3-[4-(8-Methyl-2-oxo-4H-benzo[d][1,3]oxazin-1-yl)-piperidine-1-sulfonyl]-benzoic acid
490 3-[4-(6-Methyl-2-oxo-4H-benzo[d][1,3]oxazin-1-yl)-piperidine-1-sulfonyl]-benzoic acid
491 3-[4-(6-Bromo-2-oxo-4H-benzo[d][1,3]oxazin-1-yl)-piperidine-1-sulfonyl]-benzoic acid
492 1-[1-(3-Chloro-2-fluoro-benzenesulfonyl)-piperidin-4-yl]-8-methyl-1,4-dihydro-benzo[d][1,3]oxazin-2-one
493 6-Chloro-1-[1-(3-chloro-2-fluoro-benzenesulfonyl)-piperidin-4-yl]-1,4-dihydro-benzo[d][1,3]oxazin-2-one
494 1-[1-(3-Chloro-2-fluoro-benzenesulfonyl)-piperidin-4-yl]-6-methyl-1,4-dihydro-benzo[d][1,3]oxazin-2-one
495 6-Bromo-1-[1-(3-chloro-2-fluoro-benzenesulfonyl)-piperidin-4-yl]-1,4-dihydro-benzo[d][1,3]oxazin-2-one
496 N-{4-Methyl-5-[4-(8-methyl-2-oxo-4H-benzo[d][1,3]oxazin-1-yl)-piperidine-1-sulfonyl]-thiazol-2-yl}-acetamide
497 N-{5-[4-(6-Chloro-2-oxo-4H-benzo[d][1,3]oxazin-1-yl)-piperidine-1-sulfonyl]-4-methyl-thiazol-2-yl}-acetamide
498 N-{4-Methyl-5-[4-(6-methyl-2-oxo-4H-benzo[d][1,3]oxazin-1-yl)-piperidine-1-sulfonyl]-thiazol-2-yl}-acetamide
499 N-{5-[4-(6-Bromo-2-oxo-4H-benzo[d][1,3]oxazin-1-yl)-piperidine-1-sulfonyl]-4-methyl-thiazol-2-yl}-acetamide

500 1-[1-(2-Bromo-4-fluoro-benzenesulfonyl)-piperidin-4-yl]-8-methyl-1,4-dihydro-benzo[d][1,3]oxazin-2-one
501 1-[1-(2-Bromo-4-fluoro-benzenesulfonyl)-piperidin-4-yl]-6-chloro-1,4-dihydro-benzo[d][1,3]oxazin-2-one
502 1-[1-(2-Bromo-4-fluoro-benzenesulfonyl)-piperidin-4-yl]-6-methyl-1,4-dihydro-benzo[d][1,3]oxazin-2-one
503 6-Bromo-1-[1-(2-bromo-4-fluoro-benzenesulfonyl)-piperidin-4-yl]-1,4-dihydro-benzo[d][1,3]oxazin-2-one
504 1-[1-(5-Chloro-2-fluoro-benzenesulfonyl)-piperidin-4-yl]-8-methyl-1,4-dihydro-benzo[d][1,3]oxazin-2-one
505 6-Chloro-1-[1-(5-chloro-2-fluoro-benzenesulfonyl)-piperidin-4-yl]-1,4-dihydro-benzo[d][1,3]oxazin-2-one
506 1-[1-(5-Chloro-2-fluoro-benzenesulfonyl)-piperidin-4-yl]-6-methyl-1,4-dihydro-benzo[d][1,3]oxazin-2-one
507 6-Bromo-1-[1-(5-chloro-2-fluoro-benzenesulfonyl)-piperidin-4-yl]-1,4-dihydro-benzo[d][1,3]oxazin-2-one
508 1-[1-(4-Bromo-3-trifluoromethyl-benzenesulfonyl)-piperidin-4-yl]-8-methyl-1,4-dihydro-benzo[d][1,3]oxazin-2-one
509 1-[1-(4-Bromo-3-trifluoromethyl-benzenesulfonyl)-piperidin-4-yl]-6-chloro-1,4-dihydro-benzo[d][1,3]oxazin-2-one
510 1-[1-(4-Bromo-3-trifluoromethyl-benzenesulfonyl)-piperidin-4-yl]-6-methyl-1,4-dihydro-benzo[d][1,3]oxazin-2-one
511 6-Bromo-1-[1-(4-bromo-3-trifluoromethyl-benzenesulfonyl)-piperidin-4-yl]-1,4-dihydro-benzo[d][1,3]oxazin-2-one
512 1-[1-(2-Methanesulfonyl-benzenesulfonyl)-piperidin-4-yl]-1,4-dihydro-benzo[d][1,3]oxazin-2-one
513 1-[1-(4-Propyl-benzenesulfonyl)-piperidin-4-yl]-1,4-dihydro-benzo[d][1,3]oxazin-2-one
514 1-[1-(3-Chloro-2-methyl-benzenesulfonyl)-piperidin-4-yl]-1,4-dihydro-benzo[d][1,3]oxazin-2-one
515 1-[1-(4-Butyl-benzenesulfonyl)-piperidin-4-yl]-1,4-dihydro-benzo[d][1,3]oxazin-2-one
516 1-[1-(4-Bromo-3-methyl-benzenesulfonyl)-piperidin-4-yl]-1,4-dihydro-benzo[d][1,3]oxazin-2-one
517 1-{1-[4-(1,1-Dimethyl-propyl)-benzenesulfonyl]-piperidin-4-yl}-1,4-dihydro-benzo[d][1,3]oxazin-2-one
518 N-{4-Methyl-5-[4-(2-oxo-4H-benzo[d][1,3]oxazin-1-yl)-piperidine-1-sulfonyl]-thiazol-2-yl}-acetamide
519 1-[1-(3-Chloro-2-fluoro-benzenesulfonyl)-piperidin-4-yl]-1,4-dihydro-benzo[d][1,3]oxazin-2-one
520 1-[1-(2-Bromo-4-fluoro-benzenesulfonyl)-piperidin-4-yl]-1,4-dihydro-benzo[d][1,3]oxazin-2-one
521 1-[1-(4-Bromo-3-trifluoromethyl-benzenesulfonyl)-piperidin-4-yl]-1,4-dihydro-benzo[d][1,3]oxazin-2-one
522 1-[1-(5-Chloro-2-fluoro-benzenesulfonyl)-piperidin-4-yl]-1,4-dihydro-benzo[d][1,3]oxazin-2-one
523 1-[1-(Isoquinoline-5-sulfonyl)-piperidin-4-yl]-1,4-dihydro-benzo[d][1,3]oxazin-2-one
524 6-Fluoro-1-[1-(2-methanesulfonyl-benzenesulfonyl)-piperidin-4-yl]-1,4-dihydro-benzo[d][1,3]oxazin-2-one
525 6-Fluoro-1-[1-(4-propyl-benzenesulfonyl)-piperidin-4-yl]-1,4-dihydro-benzo[d][1,3]oxazin-2-one
526 1-[1-(3-Chloro-2-methyl-benzenesulfonyl)-piperidin-4-yl]-6-fluoro-1,4-dihydro-benzo[d][1,3]oxazin-2-one
527 1-[1-(4-Butyl-benzenesulfonyl)-piperidin-4-yl]-6-fluoro-1,4-dihydro-benzo[d][1,3]oxazin-2-one
528 1-[1-(4-Bromo-3-methyl-benzenesulfonyl)-piperidin-4-yl]-6-fluoro-1,4-dihydro-benzo[d][1,3]oxazin-2-one
529 1-{1-[4-(1,1-Dimethyl-propyl)-benzenesulfonyl]-piperidin-4-yl}-6-fluoro-1,4-dihydro-benzo[d][1,3]oxazin-2-one
530 N-{5-[4-(6-Fluoro-2-oxo-4H-benzo[d][1,3]oxazin-1-yl)-pipendine-1-sulfonyl]-4-methyl-thiazol-2-yl}-acetamide
531 1-[1-(3-Chloro-2-fluoro-benzenesulfonyl)-piperidin-4-yl]-6-fluoro-1,4-dihydro-benzo[d][1,3]oxazin-2-one
532 1-[1-(2-Bromo-4-fluoro-benzenesulfonyl)-piperidin-4-yl]-6-fluoro-1,4-dihydro-benzo[d][1,3]oxazin-2-one
533 1-[1-(4-Bromo-3-trifluoromethyl-benzenesulfonyl)-piperidin-4-yl]-6-fluoro-1,4-dihydro-benzo[d][1,3]oxazin-2-one
534 1-[1-(5-Chloro-2-fluoro-benzenesulfonyl)-piperidin-4-yl]-6-fluoro-1,4-dihydro-benzo[d][1,3]oxazin-2-one
535 6-Fluoro-1-[1-(isoquinoline-5-sulfonyl)-piperidin-4-yl]-1,4-dihydro-benzo[d][1,3]oxazin-2-one
536 6-Fluoro-1-[1-(quinoline-8-sulfonyl)-piperidin-4-yl]-1,4-dihydro-benzo[d][1,3]oxazin-2-one
537 1-[1-(5-Chloro-3-methyl-benzo[b]thiophene-2-sulfonyl)-piperidin-4-yl]-6-fluoro-1,4-dihydro-benzo[d][1,3]oxazin-2-one
538 6-Fluoro-1-[1-(naphthalene-1-sulfonyl)-piperidin-4-yl]-1,4-dihydro-benzo[d][1,3]oxazin-2-one
539 6-Fluoro-1-[1-(naphthalene-2-sulfonyl)-piperidin-4-yl]-1,4-dihydro-benzo[d][1,3]oxazin-2-one
540 1-[1-(Benzo[b]thiophene-2-sulfonyl)-piperidin-4-yl]-6-fluoro-1,4-dihydro-benzo[d][1,3]oxazin-2-one
541 1-[1-(Benzo[b]thiophene-3-sulfonyl)-piperidin-4-yl]-6-fluoro-1,4-dihydro-benzo[d][1,3]oxazin-2-one
542 8-Methoxy-1-[1-(quinoline-8-sulfonyl)-piperidin-4-yl]-1,4-dihydro-benzo[d][1,3]oxazin-2-one
543 1-[1-(5-Chloro-3-methyl-benzo[b]thiophene-2-sulfonyl)-piperidin-4-yl]-8-methoxy-1,4-dihydro-benzo[d][1,3]oxazin-2-one
544 8-Methoxy-1-[1-(naphthalene-1-sulfonyl)-piperidin-4-yl]-1,4-dihydro-benzo[d][1,3]oxazin-2-one
545 8-Methoxy-1-[1-(naphthalene-2-sulfonyl)-piperidin-4-yl]-1,4-dihydro-benzo[d][1,3]oxazin-2-one
546 1-[1-(Benzo[b]thiophene-2-sulfonyl)-piperidin-4-yl]-8-methoxy-1,4-dihydro-benzo[d][1,3]oxazin-2-one
547 1-[1-(Benzo[b]thiophene-3-sulfonyl)-piperidin-4-yl]-8-methoxy-1,4-dihydro-benzo[d][1,3]oxazin-2-one
548 5-Chloro-1-[1-(2-methanesulfonyl-benzenesulfonyl)-piperidin-4-yl]-1,4-dihydro-benzo[d][1,3]oxazin-2-one
549 5-Chloro-1-[1-(4-propyl-benzenesulfonyl)-piperidin-4-yl]-1,4-dihydro-benzo[d][1,3]oxazin-2-one

550 5-Chloro-1-[1-(3-chloro-2-methyl-benzenesulfonyl)-piperidin-4-yl]-1,4-dihydro-benzo[d][1,3]oxazin-2-one
551 1-[1-(4-Butyl-benzenesulfonyl)-piperidin-4-yl]-5-chloro-1,4-dihydro-benzo[d][1,3]oxazin-2-one
552 1-[1-(4-Bromo-3-methyl-benzenesulfonyl)-piperidin-4-yl]-5-chloro-1,4-dihydro-benzo[d][1,3]oxazin-2-one
553 5-Chloro-1-{1-[4-(1,1-dimethyl-propyl)-benzenesulfonyl]-piperidin-4-yl}-1,4-dihydro-benzo[d][1,3]oxazin-2-one
554 N-{5-[4-(5-Chloro-2-oxo-4H-benzo[d][1,3]oxazin-1-yl)-piperidine-1-sulfonyl]-4-methyl-thiazol-2-yl}-acetamide
555 5-Chloro-1-[1-(3-chloro-2-fluoro-benzenesulfonyl)-piperidin-4-yl]-1,4-dihydro-benzo[d][1,3]oxazin-2-one
556 1-[1-(2-Bromo-4-fluoro-benzenesulfonyl)-piperidin-4-yl]-5-chloro-1,4-dihydro-benzo[d][1,3]oxazin-2-one
557 1-[1-(4-Bromo-3-trifluoromethyl-benzenesulfonyl)-piperidin-4-yl]-5-chloro-1,4-dihydro-benzo[d][1,3]oxazin-2-one
558 5-Chloro-1-[1-(5-chloro-2-fluoro-benzenesulfonyl)-piperidin-4-yl]-1,4-dihydro-benzo[d][1,3]oxazin-2-one
559 5-Chloro-1-[1-(isoquinoline-5-sulfonyl)-piperidin-4-yl]-1,4-dihydro-benzo[d][1,3]oxazin-2-one
560 1-[1-(2-Methanesulfonyl-benzenesulfonyl)-piperidin-4-yl]-8-methoxy-1,4-dihydro-benzo[d][1,3]oxazin-2-one
561 1-[1-(2-Methanesulfonyl-benzenesulfonyl)-piperidin-4-yl]-8-methoxy-1,4-dihydro-benzo[d][1,3]oxazin-2-one
562 1-[1-(3-Chloro-2-methyl-benzenesulfonyl)-piperidin-4-yl]-8-methoxy-1,4-dihydro-benzo[d][1,3]oxazin-2-one
563 1-[1-(4-Butyl-benzenesulfonyl)-piperidin-4-yl]-8-methoxy-1,4-dihydro-benzo[d][1,3]oxazin-2-one
564 1-[1-(4-Bromo-3-methyl-benzenesulfonyl)-piperidin-4-yl]-8-methoxy-1,4-dihydro-benzo[d][1,3]oxazin-2-one
565 1-{1-[4-(1,1-Dimethyl-propyl)-benzenesulfonyl]-piperidin-4-yl}-8-methoxy-1,4-dihydro-benzo[d][1,3]oxazin-2-one
566 N-{5-[4-(8-Methoxy-2-oxo-4H-benzo[d][1,3]oxazin-1-yl)-piperidine-1-sulfonyl]-4-methyl-thiazol-2-yl}-acetamide
567 1-[1-(3-Chloro-2-fluoro-benzenesulfonyl)-piperidin-4-yl]-8-methoxy-1,4-dihydro-benzo[d][1,3]oxazin-2-one
568 1-[1-(2-Bromo-4-fluoro-benzenesulfonyl)-piperidin-4-yl]-8-methoxy-1,4-dihydro-benzo[d][1,3]oxazin-2-one
569 1-[1-(4-Bromo-3-trifluoromethyl-benzenesulfonyl)-piperidin-4-yl]-8-methoxy-1,4-dihydro-benzo[d][1,3]oxazin-2-one
570 1-[1-(5-Chloro-2-fluoro-benzenesulfonyl)-piperidin-4-yl]-8-methoxy-1,4-dihydro-benzo[d][1,3]oxazin-2-one
571 1-[1-(Isoquinoline-5-sulfonyl)-piperidin-4-yl]-8-methoxy-1,4-dihydro-benzo[d][1,3]oxazin-2-one; hydrochloride
572 1-[1-(4-Methyl-naphthalene-1-sulfonyl)-piperidin-4-yl]-1,4-dihydro-benzo[d][1,3]oxazin-2-one
573 6-Chloro-1-[1-(4-methyl-naphthalene-1-sulfonyl)-piperidin-4-yl]-1,4-dihydro-benzo[d][1,3]oxazin-2-one
574 6-Methyl-1-[1-(4-methyl-naphthalene-1-sulfonyl)-piperidin-4-yl]-1,4-dihydro-benzo[d][1,3]oxazin-2-one
575 8-Methyl-1-[1-(4-methyl-naphthalene-1-sulfonyl)-piperidin-4-yl]-1,4-dihydro-benzo[d][1,3]oxazin-2-one
576 6-Fluoro-1-[1-(4-methyl-naphthalene-1-sulfonyl)-piperidin-4-yl]-1,4-dihydro-benzo[d][1,3]oxazin-2-one
577 8-Methoxy-1-[1-(4-methyl-naphthalene-1-sulfonyl)-piperidin-4-yl]-1,4-dihydro-benzo[d][1,3]oxazin-2-one
578 5-Chloro-1-[1-(4-methyl-naphthalene-1-sulfonyl)-piperidin-4-yl]-1,4-dihydro-benzo[d][1,3]oxazin-2-one
579 5-Chloro-1-[1-(naphthalene-1-sulfonyl)-piperidin-4-yl]-1,4-dihydro-benzo[d][1,3]oxazin-2-one
580 5-Chloro-1-[1-(naphthalene-2-sulfonyl)-piperidin-4-yl]-1,4-dihydro-benzo[d][1,3]oxazin-2-one
581 5-Chloro-1-[1-(quinoline-8-sulfonyl)-piperidin-4-yl]-1,4-dihydro-benzo[d][1,3]oxazin-2-one
582 5-Chloro-1-[1-(5-chloro-3-methyl-benzo[b]thiophene-2-sulfonyl)-piperidin-4-yl]-1,4-dihydro-benzo[d][1,3]oxazin-2-one
583 1-[1-(Benzo[b]thiophene-2-sulfonyl)-piperidin-4-yl]-5-chloro-1,4-dihydro-benzo[d][1,3]oxazin-2-one
584 1-[1-(Benzo[b]thiophene-3-sulfonyl)-piperidin-4-yl]-5-chloro-1,4-dihydro-benzo[d][1,3]oxazin-2-one
585 6-Bromo-1-[1 -(4-methyl-naphthalene-1-sulfonyl)-piperidin-4-yl]-1,4-dihydro-benzo[d][1,3]oxazin-2-one
586 2-Chloro-4-fluoro-5-[4-(8-methyl-2-oxo-4H-benzo[d][1,3]oxazin-1-yl)-piperidine-1-sulfonyl]-benzoic acid
587 2-Chloro-5-[4-(6-chloro-2-oxo-4H-benzo[d][1,3]oxazin-1-yl)-piperidine-1-sulfonyl]-4-fluoro-benzoic acid
588 2-Chloro-4-fluoro-5-[4-(6-methyl-2-oxo-4H-benzo[d][1,3]oxazin-1-yl)-piperidine-1-sulfonyl]-benzoic acid
589 2-Chloro-4-fluoro-5-[4-(2-oxo-4H-benzo[d][1,3]oxazin-1-yl)-piperidine-1-sulfonyl]-benzoic acid
590 2-Chloro-4-fluoro-5-[4-(8-methoxy-2-oxo-4H-benzo[d][1,3]oxazin-1-yl)-piperidine-1-sulfonyl]-benzoic acid
591 2-Chloro-5-[4-(5-chloro-2-oxo-4H-benzo[d][1,3]oxazin-1-yl)-piperidine-1-sulfonyl]-4-fluoro-benzoic acid
592 3-[4-(2-Oxo-4H-benzo[d][1,3]oxazin-1-yl)-piperidine-1-sulfonyl]-benzoic acid
593 3-[4-(8-Methoxy-2-oxo-4H-benzo[d][1,3]oxazin-1-yl)-piperidine-1-sulfonyl]-benzoic acid
594 3-[4-(5-Chloro-2-oxo-4H-benzo[d][1,3]oxazin-1-yl)-piperidine-1-sulfonyl]-benzoic acid
595 1-[1-(Isoquinoline-5-sulfonyl)-piperidin-4-yl]-8-methyl-1,4-dihydro-benzo[d][1,3]oxazin-2-one; hydrochloride
596 6-Chloro-1-[1-(isoquinoline-5-sulfonyl)-piperidin-4-yl]-1,4-dihydro-benzo[d][1,3]oxazin-2-one; hydrochloride
597 -[1-(Isoquinoline-5-sulfonyl)-piperidin-4-yl]-6-methyl-1,4-dihydro-benzo[d][1,3]oxazin-2-one; hydrochloride
598 6,7-Difluoro-1-[1-(quinoline-8-sulfonyl)-piperidin-4-yl]-1,4-dihydro-benzo[d][1,3]oxazin-2-one
599 1-[1-(5-Chloro-3-methyl-benzo[b]thiophene-2-sulfonyl)-piperidin-4-yl]-6,7-difluoro-1,4-dihydro-benzo[d][1,3]oxazin-2-one

600 6,7-Difluoro-1-[1-(naphthalene-1-sulfonyl)-piperidin-4-yl]-1,4-dihydro-benzo[d][1,3]oxazin-2-one
601 6,7-Difluoro-1-[1-(naphthalene-2-sulfonyl)-piperidin-4-yl]-1,4-dihydro-benzo[d][1,3]oxazin-2-one
602 1-[1-(Benzo[b]thiophene-2-sulfonyl)piperidin-4-yl]-6,7-difluoro-1,4-dihydro-benzo[d][1,3]oxazin-2-one
603 1-[1-(Benzo[b]thiophene-3-sulfonyl)-piperidin-4-yl]-6,7-difluoro-1,4-dihydro-benzo[d][1,3]oxazin-2-one
604 1-[1-(5-Dimethylamino-naphthalene-1-sulfonyl)-piperidin-4-yl]-6,7-difluoro-1,4-dihydro-benzo[d][1,3]oxazin-2-one
605 1-[1-(Biphenyl-4-sulfonyl)-piperidin-4-yl]-6,7-difluoro-1,4-dihydro-benzo[d][1,3]oxazin-2-one
606 1-[1-(Benzo[1,2,5]thiadiazole-4-sulfonyl)-piperidin-4-yl]-6,7-difluoro-1,4-dihydro-benzo[d][1,3]oxazin-2-one
607 1-[1-(Benzo[1,2,5]oxadiazole-4-sulfonyl)-piperidin-4-yl]-6,7-difluoro-1,4-dihydro-benzo[d][1,3]oxazin-2-one
608 1-[1-(7-Chloro-benzo[1,2,5]oxadiazole-4-sulfonyl)-piperidin-4-yl]-6,7-difluoro-1,4-dihydro-benzo[d][1,3]oxazin-2-one
609 6,7-Difluoro-1-[1-(4-methyl-naphthalene-1-sulfonyl)-piperidin-4-yl]-1,4-dihydro-benzo[d][1,3]oxazin-2-one
610 1-[1-(4-Chloro-naphthalene-1-sulfonyl)-piperidin-4-yl]-1,4-dihydro-benzo[d][1,3]oxazin-2-one
611 1-[1-(4-Fluoro-naphthalene-1-sulfonyl)-piperidin-4-yl]-1,4-dihydro-benzo[d][1,3]oxazin-2-one
612 1-[1-(Dibenzofuran-2-sulfonyl)-piperidin-4-yl]-1,4-dihydro-benzo[d][1,3]oxazin-2-one
613 1-[1-(2,3-Dihydro-benzofuran-5-sulfonyl)-piperidin-4-yl]-1,4-dihydro-benzo[d][1,3]oxazin-2-one
614 1-[1-(Biphenyl-2-sulfonyl)-piperidin-4-yl]-1,4-dihydro-benzo[d][1,3]oxazin-2-one
615 1-[1-(5-Isoxazol-5-yl-thiophene-2-sulfonyl)-piperidin-4-yl]-1,4-dihydro-benzo[d][1,3]oxazin-2-one
616 1-[1-(4-Chloro-naphthalene-1-sulfonyl)-piperidin-4-yl]-8-methyl-1,4-dihydro-benzo[d][1,3]oxazin-2-one
617 1-[1-(4-Fluoro-naphthalene-1-sulfonyl)-piperidin-4-yl]-8-methyl-1,4-dihydro-benzo[d][1,3]oxazin-2-one
618 1-[1-(Dibenzofuran-2-sulfonyl)-piperidin-4-yl]-8-methyl-1,4-dihydro-benzo[d][1,3]oxazin-2-one
619 1-[1-(2,3-Dihydro-benzofuran-5-sulfonyl)piperidin-4-yl]-8-methyl-1,4-dihydro-benzo[d][1,3]oxazin-2-one
620 1-[1-(Biphenyl-2-sulfonyl)-piperidin-4-yl]-8-methyl-1,4-dihydro-benzo[d][1,3]oxazin-2-one
621 1-[1-(5-Isoxazol-5-yl-thiophene-2-sulfonyl)-piperidin-4-yl]-8-methyl-1,4-dihydro-benzo[d][1,3]oxazin-2-one
622 5-Chloro-1-[1-(4-chloro-naphthalene-1-sulfonyl)-piperidin-4-yl]-1,4-dihydro-benzo[d][1,3]oxazin-2-one
623 5-Chloro-1-[1-(4-fluoro-naphthalene-1-sulfonyl)-piperidin-4-yl]-1,4-dihydro-benzo[d][1,3]oxazin-2-one
624 5-Chloro-1-[1-(dibenzofuran-2-sulfonyl)-piperidin-4-yl]-1,4-dihydro-benzo[d][1,3]oxazin-2-one
625 5-Chloro-1-[1-(2,3-dihydro-benzofuran-5-sulfonyl)-piperidin-4-yl]-1,4-dihydro-benzo[d][1,3]oxazin-2-one
626 1-[1-(Biphenyl-2-sulfonyl)-piperidin-4-yl]-5-chloro-1,4-dihydro-benzo[d][1,3]oxazin-2-one
627 5-Chloro-1-[1-(5-isoxazol-5-yl-thiophene-2-sulfonyl)-piperidin-4-yl]-1,4-dihydro-benzo[d][1,3]oxazin-2-one
628 1-[1-(4-Chloro-naphthalene-1-sulfonyl)-piperidin-4-yl]-8-methoxy-1,4-dihydro-benzo[d][1,3]oxazin-2-one
629 1-[1-(4-Fluoro-naphthalene-1-sulfonyl)-piperidin-4-yl]-8-methoxy-1,4-dihydro-benzo[d][1,3]oxazin-2-one
630 1-[1-(Dibenzofuran-2-sulfonyl)-piperidin-4-yl]-8-methoxy-1,4-dihydro-benzo[d][1,3]oxazin-2-one
631 1-[1-(2,3-Dihydro-benzofuran-5-sulfonyl)-piperidin-4-yl]-8-methoxy-1,4-dihydro-benzo[d][1,3]oxazin-2-one
632 1-[1-(Biphenyl-2-sulfonyl)-piperidin-4-yl]-8-methoxy-1,4-dihydro-benzo[d][1,3]oxazin-2-one
633 1-[1-(5-Isoxazol-5-yl-thiophene-2-sulfonyl)-piperidin-4-yl]-8-methoxy-1,4-dihydro-benzo[d][1,3]oxazin-2-one
634 6-Chloro-1-[1-(4-chloro-naphthalene-1-sulfonyl)-piperidin-4-yl]-1,4-dihydro-benzo[d][1,3]oxazin-2-one
635 6-Chloro-1-[1-(4-fluoro-naphthalene-1-suffonyl)-piperidin-4-yl]-1,4-dihydro-benzo[d][1,3]oxazin-2-one
636 6-Chloro-1-[1-(dibenzofuran-2-sulfonyl)-piperidin-4-yl]-1,4-dihydro-benzo[d][1,3]oxazin-2-one
637 6-Chloro-1-[1-(2,3-dihydro-benzofuran-5-sulfonyl)-piperidin-4-yl]-1,4-dihydro-benzo[d][1,3]oxazin-2-one
638 1-[1-(Biphenyl-2-sutfonyl)-piperidin-4-yl]-6-chloro-1,4-dihydro-benzo[d][1,3]oxazin-2-one
639 6-Chloro-1-[1-(5-isoxazol-5-yl-thiophene-2-sulfonyl)-piperidin-4-yl]-1,4-dihydro-benzo[d][1,3]oxazin-2-one
640 1-[1-(4-Chloro-naphthalene-1-sulfonyl)-piperidin-4-yl]-6-methyl-1,4-dihydro-benzo[d][1,3]oxazin-2-one
641 1-[1-(4-Fluoro-naphthalene-1-sulfonyl)-piperidin-4-yl]-6-methyl-1,4-dihydro-benzo[d][1,3]oxazin-2-one
642 1-[1-(Dibenzofuran-2-sulfonyl)-piperidin-4-yl]-6-methyl-1,4-dihydro-benzo[d][1,3]oxazin-2-one
643 1-[1-(2,3-Dihydro-benzofuran-5-sulfonyl)-piperidin-4-yl]-6-methyl-1,4-dihydro-benzo[d][1,3]oxazin-2-one
644 1-[1-(Biphenyl-2-sulfonyl)-piperidin-4-yl]-6-methyl-1,4-dihydro-benzo[d][1,3]oxazin-2-one
645 1-[1-(5-Isoxazol-5-yl-thiophene-2-sulfonyl)-piperidin-4-yl]-6-methyl-1,4-dihydro-benzo[d][1,3]oxazin-2-one
646 1-[1-(4-Chloro-naphthalene-1-sulfonyl)-piperidin-4-yl]-6,7-difluoro-1,4-dihydro-benzo[d][1,3]oxazin-2-one
647 6,7 -Difluoro-1-[1-(4-fluoro-naphthalene-1-sulfonyl)-piperidin-4-yl]-1,4-dihydro-benzo[d][1,3]oxazin-2-one
648 1-[1-(Dibenzofuran-2-sulfonyl)-piperidin-4-yl]-6,7-difluoro-1,4-dihydro-benzo[d][1,3]oxazin-2-one
649 1-[1-(2,3-Dihydro-benzofuran-5-sulfonyl)-piperidin-4-yl]-6.7-difluoro-1,4-dihydro-benzo[d][1,3]oxazin-2-one

650 1-[1-(Biphenyl-2-sulfonyl)-piperidin-4-y)]-6,7-difluoro-1,4-dihydro-benzo[d][1,3]oxazin-2-one
651 6,7-Difluoro-1-[1-(5-isoxazol-5-yl-thiophene-2-sulfonyl)-piperidin-4-yl]-1,4-dihydro-benzo[d][1,3]oxazin-2-one
652 1-[1-(1,2-Dimethyl-1H-imidazole-4-sulfonyl)-piperidin-4-yl]-1,4-dihydro-benzo[d][1,3]oxazin-2-one
653 1-[1-(5-Methyl-benzo[1,2,5]thiadiazole-4-sulfonyl)-piperidin-4-yl]-1,4-dihydro-benzo[d][1,3]oxazin-2-one
654 1-[1-(3,5-Dimethyl-isoxazole-4-sulfonyl)-piperidin-4-yl]-1,4-dihydro-benzo[d][1,3]oxazin-2-one
655 1-[1-(1,2-Dimethyl-1H-imidazole-4-sulfonyl)-piperidin-4-yl]-8-methyl-1,4-dihydro-benzo[d][1,3]oxazin-2-one
656 8-Methyl-1-[1-(5-methyl-benzo[1,2,5]thiadiazole-4-sulfonyl)-piperidin-4-yl]-1,4-dihydro-benzo[d][1,3]oxazin-2-one
657 1-[1-(3,5-Dimethyl-isoxazole-4-sulfonyl)-piperidin-4-yl]-8-methyl-1,4-dihydro-benzo[d][1,3]oxazin-2-one
658 6-Chloro-1-[1-(1,2-dimethyl-1H-imidazole-4-sulfonyl)-piperidin-4-yl]-1,4-dihydro-benzo[d][1,3]oxazin-2-one
659 6-Chloro-1-[1-(5-methyl-benzo[1,2,5]thiadiazole-4-sulfonyl)-piperidin-4-yl]-1,4-dihydro-benzo[d][1,3]oxazin-2-one
660 6-Chloro-1-[1-(3,5-dimethyl-isoxazole-4-sulfonyl)-piperidin-4-yl]-1,4-dihydro-benzo[d][1,3]oxazin-2-one
661 1-[1-(1,2-Dimethyl-1H-imidazole-4-sulfonyl)-piperidin-4-yl]-8-methoxy-1,4-dihydro-benzo[d][1,3]oxazin-2-one
662 8-Methoxy-1-[1-(5-methyl-benzo[1,2,5]thiadiazole-4-sulfonyl)-piperidin-4-yl]-1,4-dihydro-benzo[d][1,3]oxazin-2-one
663 1-[1-(3,5-Dimethyl-isoxazole-4-sulfonyl)-piperidin-4-yl]-8-methoxy-1,4-dihydro-benzo[d][1,3]oxazin-2-one
664 5-Chloro-1-[1-(1,2-dimethyl-1H-imidazole-4-sulfonyl)-piperidin-4-yl]-1,4-dihydro-benzo[d][1,3]oxazin-2-one
665 5-Chloro-1-[1-(5-methyl-benzo[1,2,5]thiadiazole-4-sulfonyl)-piperidin-4-yl]-1,4-dihydro-benzo[d][1,3]oxazin-2-one
666 5-Chloro-1-[1-(3,5-dimethyl-isoxazole-4-sulfonyl)-piperidin-4-yl]-1,4-dihydro-benzo[d][1,3]oxazin-2-one
667 1-[1-(1,2-Dimethyl-1H-imidazole-4-sulfonyl)-piperidin-4-yl]-6-methyl-1,4-dihydro-benzo[d][1,3]oxazin-2-one
668 6-Methyl-1-[1-(5-methyl-benzo[1,2,5]thiadiazole-4-sulfonyl)-piperidin-4-yl]-1,4-dihydro-benzo[d][1,3]oxazin-2-one
669 1-[1-(3,5-Dimethyl-isoxazole-4-sulfonyl)-piperidin-4-yl]-6-methyl-1,4-dihydro-benzo[d][1,3]oxazin-2-one
670 1-[1-(1,2-Dimethyl-1H-imidazole-4-sulfonyl)-piperidin-4-yl]-6-fluoro-1,4-dihydro-benzo[d][1,3]oxazin-2-one
671 6-Fluoro-1-[1-(5-methyl-benzo[1,2,5]thiadiazole-4-sulfonyl)-piperidin-4-yl]-1,4-dihydro-benzo[d][1,3]oxazin-2-one
672 1-[1-(3,5-Dimethyl-isoxazole-4-sulfonyl)-piperidin-4-yl]-6-fluoro-1,4-dihydro-benzo[d][1,3]oxazin-2-one
673 1-[1-(1,2-Dimethyl-1H-imidazole-4-sulfonyl)-piperidin-4-yl]-6,7-difluoro-1,4-dihydro-benzo[d][1,3]oxazin-2-one
674 6,7-Difluoro-1-[1-(5-methyl-benzo[1,2,5]thiadiazole-4-sulfonyl)-piperidin-4-yl]-1,4-dihydro-benzo[d][1,3]oxazin-2-one
675 1-[1-(3,5-Dimethyl-isoxazole-4-sulfonyl)-piperidin-4-yl]-6,7-difluoro-1,4-dihydro-benzo[d][1,3]oxazin-2-one
676 1-[1-(5-Chloro-naphthalene-1-sulfonyl)-piperidin-4-yl]-1,4-dihydro-benzo[d][1,3]oxazin-2-one
677 1-[1-(5-Chloro-naphthalene-2-sulfonyl)-piperidin-4-yl]-1,4-dihydro-benzo[d][1,3]oxazin-2-one
678 N-{5-[4-(2-oxo-4H-benzo[d][1,3]oxazin-1-yl)-piperidine-1-sulfonyl]-naphthalen-1-yl}-acetamide
679 1-[1-(5-Chloro-naphthalene-1-sulfonyl)-piperidin-4-yl]-8-methyl-1,4-dihydro-benzo[d][1,3]oxazin-2-one
680 1-[1-(5-Chloro-naphthalene-2-sulfonyl)-piperidin-4-yl]-8-methyl-1,4-dihydro-benzo[d][1,3]oxazin-2-one
681 N-{5-[4-(8-Methyl-2-oxo-4H-benzo[d][1,3]oxazin-1-yl)-piperidine-1-sulfonyl]-naphthalen-1-yl}-acetamide
682 5-Chloro-1-[1-(5-chloro-naphthalene-1-sulfonyl)-piperidin-4-yl]-1,4-dihydro-benzo[d][1,3]oxazin-2-one
683 5-Chloro-1-[1 -(5-chloro-naphthaene-2-sulonyl)-piperidin-4-yl]-1,4-dihydro-benzo[d][1,3]oxazin-2-one
684 N-{5-[4-(5-Chloro-2-oxo-4H-benzo[d][1.3]oxazin-1-yl)-piperidine-1-sulfonyl]-naphthalen-1-yl}-acetamide
685 1-[1-(5-Chloro-naphthalene-1-sulfonyl)-piperidin-4-y]-8-methoxy-1,4-dihydro-benzo[d][1,3]oxazin-2-one
686 1-[1-(5-Chloro-naphthalene-2-sulfonyl)-piperidin-4-yl]-8-methoxy-1,4-dihydro-benzo[d][1,3]oxazin-2-one
687 N-{5-[4-(8-Methoxy-2-oxo-4H-benzo[d][1,3]oxazin-1-yl)-piperidine-1-sulfonyl]-naphthalen-1-yl}-acetamide
688 2,5-Dimethyl-4-[4-(8-methyl-2-oxo-4H-benzo[d][1,3]oxazin-1-yl)-piperidine-1-sulfonyl]-furan-3-carboxylic acid methyl ester
689 8-Methyl-1-[1-(2-oxo-2,3-dihydro-benzothiazole-6-sulfonyl)-piperidin-4-yl]-1,4-dihydro-benzo[d][1,3]oxazin-2-one
690 1-[1-(4-Fluoro-3-methyl-benzenesulfonyl)-piperidin-4-yl]-8-methyl-1,4-dihydro-benzo[d][1,3]oxazin-2-one
691 8-Methyl-1-[1-(2-oxo-2,3-dihydro-benzooxazole-6-sulfonyl)-piperidin-4-yl]-1,4-dihydro-benzo[d][1,3]oxazin-2-one
692 1-[1-(4-Cyclohexyl-benzenesulfonyl)-piperidin-4-yl]-8-methyl-1,4-dihydro-benzo[d][1,3]oxazin-2-one
693 2,5-Dimethyl-4-[4-(2-oxo-4H-benzo[d][1,3]oxazin-1-yl)-piperidine-1-sulfonyl]-furan-3-carboxylic acid methyl ester
694 1-[1-(4-Fluoro-3-methyl-benzenesulfonyl)-piperidin-4-yl]-1,4-dihydro-benzo[d][1,3]oxazin-2-one
695 1-[1-(2-Oxo-2,3-dihydro-benzooxazole-6-sulfonyl)-piperidin-4-yl]-1,4-dihydro-benzo[d][1,3]oxazin-2-one
696 1-[1-(4-Cyclohexyl-benzenesulfonyl)-piperidin-4-yl]-1,4-dihydro-benzo[d][1,3]oxazin-2-one
697 2-Fluoro-5-[4-(8-methyl-2-oxo-4H-benzo[d][1,3]oxazin-1-yl)-pipendine-1-sulfonyl]-benzoic acid
698 2-Fluoro-5-[4-(2-oxo-4H-benzo[d][1,3]oxazin-1-yl)-piperidine-1-sulfonyl]-benzoic acid
699 1-[1-(2-Oxo-2,3-dihydro-benzothiazole-6-suffonyl)-piperidin-4-yl]-1,4-dihydro-benzo[d][1,3]oxazin-2-one

700 1-[1-(5-Pyridin-2-yl-thiophene-2-sulfonyl)-piperidin-4-yl]-1,4-dihydro -benzo[d][1,3]oxazin-2-one
701 3-[4-(2-Oxo-4H-benzo[d][1,3]oxazin-1-yl)-piperidine-1-sulfonyl]-benzonitrile
702 3-[4-(2-Oxo-4H-benzo[d][1,3]oxazin-1-yl)-piperidine-1-sulfonyl]-thiophene-2-carboxylic acid methyl ester
703 1-{5-[4-(2-Oxo-4H-benzo[d][1,3]oxazin-1-yl)-piperidine-1-sulfonyl]-naphthalen-1-yl}-pyrrolidine-2,5-dione
704 1-[1-(2-Chloro-5-trifluoromethyl-benzenesulfonyl)-piperidin-4-yl]-1,4-dihydro-benzo[d][1,3]oxazin-2-one
705 1-[1-(3,4-Dimethyl-benzenesulfonyl)-piperidin-4-yl]-1,4-dihydro-benzo[d][1,3]oxazin-2-one
706 8-Methyl-1-[1-(5-pyridin-2-yl-thiophene-2-sulfonyl)-piperidin-4-yl]-1,4-dihydro-benzo[d][1,3]oxazin-2-one
707 3-[4-(8-Methyl-2-oxo-4H-benzo[d][1,3]oxazin-1-yl)-piperidine-1-sulfonyl]-benzonitrile
708 3-[4-(8-Methyl-2-oxo-4H-benzo[d][1,3]oxazin-1-yl)-piperidine-1-sulfonyl]-thiophene-2-carboxylic acid methyl ester
709 1-{5-[4-(8-Methyl-2-oxo-4H-benzo[d][1,3]oxazin-1-yl)-piperidine-1-sulfonyl]-naphthalen-1-yl}-pyrrolidine-2,5-dione
710 1-[1-(2-Chloro-5-trifluoromethyl-benzenesulfonyl)-piperidin-4-yl]-8-methyl-1,4-dihydro-benzo[d][1,3]oxazin-2-one
711 1-[1-(3,4-Dimethyl-benzenesulfonyl)-piperidin-4-yl]-8-methyl-1,4-dihydro-benzo[d][1,3]oxazin-2-one
712 5-Chloro-1-[1-(5-pyridin-2-yl-thiophene-2-sulfonyl)-piperidin-4-yl]-1,4-dihydro-benzo[d][1,3]oxazin-2-one
713 3-[4-(5-Chloro-2-oxo-4H-benzo[d][1,3]oxazin-1-yl)-piperidine-1-sulfonyl]-benzonitrile
714 3-[4-(5-Chloro-2-oxo-4H-benzo[d][1,3]oxazin-1-yl)-piperidine-1-sulfonyl]-thiophene-2-carboxylic acid methyl ester
715 1-{5-[4-(5-Chloro-2-oxo-4H-benzo[d][1,3]oxazin-1-yl)-piperidine-1-sulfonyl]-naphthalen-1-yl}-pyrrolidine-2,5-dione
716 5-Chloro-1-[1-(2-chloro-5-trifluoromethyl-benzenesulfonyl)-piperidin-4-yl]-1,4-dihydro-benzo[d][1,3]oxazin-2-one
717 5-Chloro-1-[1-(3,4-dimethyl-benzenesulfonyl)-piperidin-4-yl]-1,4-dihydro-benzo[d][1,3]oxazin-2-one
718 6-Methyl-1-[1-(5-pyridin-2-yl-thiophene-2-sulfonyl)-piperidin-4-yl]-1,4-dihydro-benzo[d][1,3]oxazin-2-one
719 3-[4-(6-Methyl-2-oxo-4H-benzo[d][1,3]oxazin-1-yl)-piperidine-1-sulfonyl]-benzonitrile
720 3-[4-(6-Methyl-2-oxo-4H-benzo[d][1,3]oxazin-1-yl)-piperidine-1-sulfonyl]-thiophene-2-carboxylic acid methyl ester
721 1-{5-[4-(6-Methyl-2-oxo-4H-benzo[d][1,3]oxazin-1-yl)-piperidine-1-sulfonyl]-naphthalen-1-yl}-pyrrolidine-2.5-dione
722 1-[1-(2-Chloro-5-trifluoromethyl-benzenesulfonyl)-piperidin-4-yl]-6-methyl-1,4-dihydro-benzo[d][1,3]oxazin-2-one
723 1-[1-(3,4-Dimethyl-benzenesulfonyl)-piperidin-4-yl]-6-methyl-1,4-dihydro-benzo[d][1,3]oxazin-2-one
724 6-Chloro-1-[1-(5-pyridin-2-yl-thiophene-2-sulfonyl)-pipendin-4-yl]-1,4-dihydro-benzo[d][1,3]oxazin-2-one
725 3-[4-(6-Chloro-2-oxo-4H-benzo[d][1,3]oxazin-1-yl)-piperidine-1-sulfonyl]-benzonitrile
726 3-[4-(6-Chloro-2-oxo-4H-benzo[d][1,3]oxazin-1-yl)-piperidine-1-sulfonyl]-thiophene-2-carboxylic acid methyl ester
727 1-{5-[4-(6-Chloro-2-oxo-4H-benzo[d][1,3]oxazin-1-yl)-piperidine-1-sulfonyl]-naphthalen-1-yl}-pyrrolidine-2,5-dione
728 6-Chloro-1-[1-(2-chloro-5-trifluoromethyl-benzenesulfonyl)-piperidin-4-yl]-1,4-dihydro-benzo[d][1,3]oxazin-2-one
729 6-Chloro-1-[1-(3,4-dimethyl-benzenesulfonyl)-piperidin-4-yl]-1,4-dihydro-benzo[d][1,3]oxazin-2-one
730 1-[1-(5-Methyl-isoxazole-4-sulfonyl)-piperidin-4-yl]-1,4-dihydro-benzo[d][1,3]oxazin-2-one
731 1-[1-(2,2-Dimethyl-chroman-6-sulfonyl)-piperidin-4-yl]-1,4-dihydro-benzo[d][1,3]oxazin-2-one
732 1-[1-(4-Methyl-3,4-dihydro-2H-benzo[1,4]oxazine-7-sulfonyl)-piperidin-4-yl]-1,4-dihyd ro-benzo[d][1,3]oxazin-2-one
733 1-[1-(2,3-Dihydro-benzo[1,4]d ioxine-6-sulfonyl)-piperidin-4-yl]-1,4-dihydro-benzo[d][1,3]oxazin-2-one
734 1-[1-(1,3,5-Trimethyl-1H-pyrazole-4-sulfonyl)-piperidin-4-yl]-1,4-dihydro-benzo[d][1,3]oxazin-2-one
735 1-[1-(3-Methyl-2-oxo-2,3-dihydro-benzooxazole-6-sulfonyl)-piperidin-4-yl]-1,4-dihydro-benzo[d][1,3]oxazin-2-one
736 8-Methyl-1-[1 (5-methyl-isoxazole-4-sulfonyl)-piperidin-4-yl]-1,4-dihydro-benzo[d][1,3]oxazin-2-one
737 1-[1-(2,2-Dimethyl-chroman-6-sulfonyl)-piperidin-4-yl]-8-methyl-1,4-dihydro-benzo[d][1,3]oxazin-2-one
738 8-Methyl-1-[1-(4-methyl-3,4-dihydro-2H-benzo[1,4]oxazine-7-sulfonyl)-piperidin-4-yl]-1,4-dihydro-benzo[d][1,3]oxazin-2-one
739 1-[1-(2,3-Dihydro-benzo[1,4]dioxine-6-sulfonyl)-piperidin-4-yl]-8-methyl-1,4-dihydro-benzo[d][1,3]oxazin-2-one
740 8-Methyl-1-[1-(1,3,5-trimethyl-1H-pyrazole-4-sulfonyl)-piperidin-4-yl]-1,4-dihydro-benzo[d][1,3]oxazin-2-one
741 8-Methyl-1-[1-(3-methyl-2-oxo-2,3-dihydro-benzooxazole-6-sulfonyl)-piperidin-4-yl]-1,4-dihydro-benzo[d][1,3]oxazin-2-one
742 8-Methoxy-1-[1-(1,3,5-trimethyl-1H-pyrazole-4-sulfonyl)-piperidin-4-yl]-1,4-dihydro-benzo[d][1,3]oxazin-2-one
743 8-Methoxy-1-[1-(3-methyl-2-oxo-2,3-dihydro-benzooxazole-6-sulfonyl)-piperidin-4-yl]-1,4-dihydro-benzo[d][1,3]oxazin-2-one
744 1-[1-(Benzo[d]isoxazol-3-ylmethanesulfonyl)-piperidin-4-yl]-1,4-dihydro-benzo[d][1,3]oxazin-2-one
745 1-[1-(2,2,4,6,7-Pentamethyl-2,3-dihydro-benzofuran-5-sulfonyl)-piperidin-4-yl]-1,4-dihydro-benzo[d][1,3]oxazin-2-one
746 6-Methyl-5-[4-(2-oxo-4H-benzo[d][1,3]oxazin-1-yl)-piperidine-1-sulfonyl]-1H-pyrimidine-2,4-dione
747 1-[1-(3-Methyl-quinoline-8-sulfonyl)-piperidin-4-yl]-1,4-dihydro-benzo[d][1,3]oxazin-2-one
748 1-[1-(2,2,5,7,8-Pentamethyl-chroman-6-sulfonyl)-piperidin-4-yl]-1,4-dihydro-benzo[d][1,3]oxazin-2-one
749 1,4-Dimethyl-6-[4-(2-oxo-4H-benzo[d][1,3]oxazin-1-yl)-piperidine-1-sulfonyl]-1,4-dihydro-quinoxaline-2,3-dione

750 1-[1-(1H-Imidazole-4-sulfonyl)-piperidin-4-yl]-1,4-dihydro-benzo[d][1,3]oxazin-2-one
751 1-[1-(2-Oxo-1,2,3,4-tetrahydro-quinoline-6-sulfonyl)-piperidin-4-yl]-1,4-dihydro-benzo[d][1,3]oxazin-2-one
752 7-[4-(2-Oxo-4H-benzo[d][1,3]oxazin-1-yl)-piperidine-1-sulfonyl]-1,5-dihydro-benzo[b][1,4]diazepine-2,4-dione
753 8-Methyl-1-[1-(3-methyl-quinoline-8-sulfonyl)-piperidin-4-yl]-1,4-dihydro-benzo[d][1,3]oxazin-2-one
754 6-Chloro-1-[1-(3-methyl-quinoline-8-sulfonyl)-piperidin-4-yl]-1,4-dihydro-benzo[d][1,3]oxazin-2-one
755 5-Chloro-1-[1-(3-methyl-quinoline-8-sulfonyl)-piperidin-4-yl]-1,4-dihydro-benzo[d][1,3]oxazin-2-one
756 8-Methoxy-1-[1-(3-methyl-quinoline-8-sulfonyl)-piperidin-4-yl]-1,4-dihydro-benzo[d][1,3]oxazin-2-one
757 1-[1-(Pyridine-2-sulfonyl)-piperidin-4-yl]-1,4-dihydro-benzo[d][1,3]oxazin-2-one
758 1-[1-(6,7-Dihydroxy-naphthalene-1-sulfonyl)-piperidin-4-yl]-1,4-dihydro-benzo[d][1,3]oxazin-2-one
759 Acetic acid 3-acetoxy-5-[4-(2-oxo-4H-benzo[d][1,3]oxazin-1-yl)-piperidine-1-sulfonyl]-naphthalen-2-yl ester
760 1-[1-(1H-Benzoimidazole-2-sulfonyl)-piperidin-4-yl]-1,4-dihydro-benzo[d][1,3]oxazin-2-one
761 1-[1-(1H-Benzoimidazole-2-sulfonyl)-piperidin-4-yl]-8-methoxy-1,4-dihydro-benzo[d][1,3]oxazin-2-one
762 1-[1-(1H-Benzoimidazole-2-sulfonyl)-piperidin-4-yl]-5-chloro-1,4-dihydro-benzo[d][1,3]oxazin-2-one
763 1-[1-(2,5-Dimethoxy-benzenesulfonyl)-piperidin-4-yl]-6-fluoro-1,4-dihydro-benzo[d][1,3]oxazin-2-one
764 1-[1-(2,5-Dimethoxy-benzenesulfonyl)-piperidin-4-yl]-8-methoxy-1,4-dihydro-benzo[d][1,3]oxazin-2-one
765 1-[1-(2,5-Dimethoxy-benzenesulfonyl)-piperidin-4-yl]-6,7-difluoro-1,4-dihydro-benzo[d][1,3]oxazin-2-one
766 5-Chloro-1-[1-(2,5-dimethoxy-benzenesulfonyl)-piperidin-4-yl]-1,4-dihydro-benzo[d][1,3]oxazin-2-one
767 1-[1-(5-Dimethylamino-naphthalene-1-sulfonyl)-piperidin-4-yl]-8-methoxy-1,4-dihydro-benzo[d][1,3]oxazin-2-one
768 5-Chloro-1-[1-(5-dimethylamino-naphthalene-1-sulfonyl)-piperidin-4-yl]-1,4-dihydro-benzo[d][1,3]oxazin-2-one
769 6-Chloro-1-[1-(5-chloro-naphthalene-1-sulfonyl)-piperidin-4-yl]-1,4-dihydro-benzo[d][1,3]oxazin-2-one
770 1-[1-(5-Chloro-naphthalene-1-sulfonyl)-piperidin-4-yl]-6-methyl-1,4-dihydro-benzo[d][1,3]oxazin-2-one
771 1-[1-(5-Chloro-naphthalene-1-sulfonyl)-piperidin-4-yl]-6-fluoro-1,4-dihydro-benzo[d][1,3]oxazin-2-one
772 6-Chloro-1-[1-(5-chloro-naphthalene-2-sulfonyl)-piperidin-4-yl]-1,4-dihydro-benzo[d][1,3]oxazin-2-one
773 1-[1-(5-Chloro-naphthalene-2-sulfonyl)-piperidin-4-yl]-6-methyl-1,4-dihydro-benzo[d][1,3]oxazin-2-one
774 1-[1-(5-Chloro-naphthalene-2-sulfonyl)-piperidin-4-yl]-6-fluoro-1,4-dihydro-benzo[d][1,3]oxazin-2-one
775 6-Methyl-1-[1-(3-methyl-quinoline-8-sulfonyl)-piperidin-4-yl]-1,4-dihydro-benzo[d][1,3]oxazin-2-one
776 6-Fluoro-1-[1-(3-methyl-quinoline-8-sulfonyl)-pipendin-4-yl]-1,4-dihydro-benzo[d][1,3]oxazin-2-one
777 6,7-Difluoro-1-[1-(3-methyl-quinoline-8-suffonyl)-piperidin-4-yl]-1,4-dihydro-benzo[d][1,3]oxazin-2-one
778 6-Chloro-1-[1-(3-methyl-2-oxo-2,3-dihydro-benzooxazole-6-sulfonyl)-piperidin-4-yl]-1,4-dihydro-benzo[d][1,3]oxazin-2-0ne
779 6-Methyl-1-[1-(3-methyl-2-oxo-2,3-dihydro-benzooxazole-6-sulfonyl)-piperidin-4-yl]-1,4-dihydro-benzo[d][1,3]oxazin-2-one
780 6-Fluoro-1-[1-(3-methyl-2-oxo-2,3-dihydro-benzooxazole-6-sulfonyl)-piperidin-4-yl]-1,4-dihydro-benzo[d][1,3]oxazin-2-one
781 6, 7 -Difluoro-1-[1-(3-methyl-2-oxo-2,3-dihydro-benzooxazole-6-sulfonyl)-piperidin-4-yl]-1,4-dihydro-benzo[d][1,3]oxazin-2-one
782 5-Chloro-1-[1-(3-methyl-2-oxo-2,3-dihydro-benzooxazole-6-sulfonyl)-piperidin-4-yl]-1,4-dihydro-benzo[d][1,3]oxazin-2-one
783 6-Chloro-1-[1-(4-methyl-3,4-dihydro-2H-benzo[1,4]oxazine-7-sulfonyl)-piperidin-4-yl]-1,4-dihydro-benzo[d][1,3]oxazin-2-one
784 6-Methyl-1-[1-(4-methyl-3,4-dihydro-2H-benzo[1,4]oxazine-7-sulfonyl)-piperidin-4-yl]-1,4-dihydro-benzo[d][1,3]oxazin-2-one
785 6-Fluoro-1-[1-(4-methyl-3,4-dihydro-2H-benzo[1,4]oxazine-7-sulfonyl)-piperidin-4-yl]-1,4-dihydro-benzo[d][1,3]oxazin-2-one
786 8-Methoxy-1-[1-(4-methyl-3,4-dihydro-2H-benzo[1,4]oxazine-7-sulfonyl)-piperidin-4-yl]-1,4-dihydro-benzo[d][1,3]oxazin-2-one
787 5-Chloro-1-[1-(4-methyl-3,4-dihydro-2H-benzo[1,4]oxazine-7-sulfonyl)-piperidin-4-yl]-1,4-dihydro-benzo[d][1,3]oxazin-2-on;
optionally in form of one of its stereoisomers, preferably enantiomers or diastereomers, its racemate or in form of a mixture of at least two of its stereoisomers, preferably enantiomers or diastereomers, in any mixing ratio, or a physiologically acceptable salt thereof, or a solvate, respectively.

The compounds of general formula (Ia) may be prepared according to the disclosure of WO 2005/014045.

In another embodiment as component (A) at least one compound is present, which is selected from the group consisting of indole-derived sulfonamide compounds of general formula (Ib) 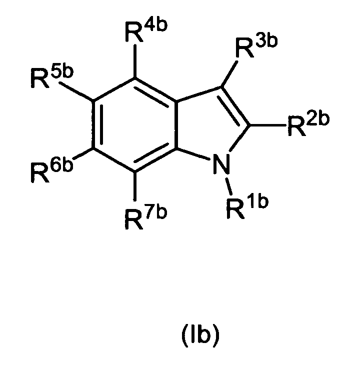 wherein
R^{1b} represents a hydrogen atom; a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical,
which may be bonded via a linear or branched alkylene group and/or which may be condensed with an unsubstituted or at least mono-substituted mono- or bicyclic ring system; an unsubstituted or at least mono-substituted aryl or heteroaryl radical, which may be bonded via a linear or branched alkylene group and/or which may be condensed with an unsubstituted or at least mono-substituted mono- or bicyclic ring system; a -(CH₂)_{mb}-NR^{13b}R^{14b} moiety with mb = 0, 1, 2, 3, 4 or 5; a -C(=O)-R^{8b} moiety; a -S(=O)₂-R^{9b} moiety; or a -S(=O)₂-C(H)A^{b}B^{b} moiety;
R^{2b} represents a hydrogen atom; -F; -Cl; -Br; -I; -NO₂; -NH₂; -SH; -OH; -CN; -C(=O)-OH; -O-R^{10b}; -S-R^{11b}; -C(=O)-OR^{12b}; a linear or branched, saturated or unsaturated, unsubstituted or at least mono-substituted, optionally at least one heteroatom as a chain member containing aliphatic radical; a saturated or unsaturated, unsubstituted or at least mono-substituted, optionally at least one heteroatom as a ring member containing cycloaliphatic radical, which may be bonded via a linear or branched alkylene group and/or which may be condensed with an unsubstituted or at least mono-substituted mono- or bicyclic ring system; or an unsubstituted or at least mono-substituted aryl or heteroaryl radical, which may be bonded via a linear or branched alkylene group and/or which may be condensed with an unsubstituted or at least mono-substituted mono- or bicyclic ring system;
R^{3b} represents a hydrogen atom; -F; -Cl; -Br; -I; -NO₂; -CN; -O-R^{10b}; -S-R^{11b}; a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via an unsubstituted or at least mono-substituted alkylene group and/or which may be condensed with an unsubstituted or at least mono-substituted mono- or bicyclic ring system; an unsubstituted or at least mono-substituted aryl or heteroaryl radical, which may be bonded via an unsubstituted or at least mono-substituted alkylene group and/or which may be condensed with an unsubstituted or at least mono-substituted mono- or bicyclic ring system; a -CH(OC₂H₅)-CH₂NR^{13b}R^{14b} moiety or a -(CH₂)_{nb}-NR^{13b}R^{14b} moiety with nb = 0, 1, 2, 3, 4 or 5; a -S(=O)₂-R^{9b} moiety; a -S(=O)₂-C(H)A^{b}B^{b} moiety; or a -C(=O)-(CH₂)p_{b}-C(=O)-N-D^{b}E^{b} moiety with pb = 0, 1, 2, 3, 4 or 5;
R^{4b}, R^{5b}, R^{6b} and R^{7b}, independently of one another, each represent a hydrogen atom; -NO₂; -NH₂; -SH; -OH; -CN; -C(=O)-OH; -C(=O)-H; -S(=O)₂OH; -C(=O)-NH₂;-S(=O)₂-NH₂; -C(=O)-R^{8b}; -S(=O)₂-R^{9b}; -O-R^{10b}; -S-R^{11b}; -C(=O)-OR^{12b}; -N(R^{15b})-S(=O)₂-R^{16b}; -NH-R^{17b}; -NR^{18b}R^{19b}; -C(=O)-NHR^{20b}, -C(=O)-NR^{21b}R^{22b}; -S(=O)₂-NHR^{23b}; -S(=O)₂-NR^{24b}R^{25b}; -O-C(=O)-R^{26b}; -NH-C(=O)-R^{27b}; -NR^{28b}-C(=O)-R^{29b}; NH-C(=O)-O-R^{30b}; NR^{31b}-C(=O)-O-R^{32b}; -S(=O)₂-O-R^{33b}; a halogen atom; a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via a linear or branched alkylene group; or an unsubstituted or at least mono-substituted aryl or heteroaryl radical, which may be bonded via a linear or branched alkylene group;
with the proviso that at least one of the substituents R^{4b}, R^{5b}, R^{6b} and R^{7b} represents a -N(R^{15b})-S(=O)₂-R^{16b} moiety;
R^{8b}, R^{12b}, R^{17b}, R^{18b}, R^{19b}, R²⁰b, R^{21b}, R^{22b}, R^{23b}, R^{24b}, R^{25b}, R^{26b}, R^{27b}, R^{28b}, R^{29b}, R^{30b}, R^{31b}, R^{32b} and R^{33b}, independently of one another, each represent a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via a linear or branched alkylene group; or an unsubstituted or at least mono-substituted aryl or heteroaryl radical, which may be bonded via a linear or branched alkylene, alkenylene or alkinylene group;
R^{9b} represents a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via a linear or branched alkylene group and/or which may be condensed with an unsubstituted or at least mono-substituted mono- or bicyclic ring system; or an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
R^{10b} and R^{11b}, independently of one another, each represent a linear or branched, saturated or unsaturated, unsubstituted or at least mono-substituted aliphatic radical; or an unsubstituted or at least mono-substituted aryl or heteroaryl radical, which may be bonded via a linear or branched alkylene group;
R^{13b} and R^{14b}, independently of one another, each represent a hydrogen atom; or a linear or branched, saturated or unsaturated, unsubstituted or at least mono-substituted aliphatic radical;
or
R^{13b} and R^{14b} together with the bridging nitrogen form an unsubstituted or at least mono-substituted, saturated, unsaturated or aromatic heterocyclic ring which may contain at least one further heteroatom as a ring member and/or which may be condensed with an unsubstituted or at least mono-substituted mono- or bicyclic ring system;
R^{15b} represents a hydrogen atom; a linear or branched, saturated or unsaturated, unsubstituted or at least mono-substituted aliphatic radical or a -S(=O)₂-R^{16b} moiety;
R^{16b} represents a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via a linear or branched, unsubstituted or at least mono-substituted alkylene, alkenylene or alkinylene group and/or which may be condensed with an unsubstituted or at least mono-substituted mono- or bicyclic ring system; or an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
A^{b} and B^{b} together with the bridging carbon form an unsubstituted or at least mono-substituted, saturated or unsaturated cycloaliphatic ring which may contain at least one further heteroatom as a ring member and/or which may be condensed with an unsubstituted or at least mono-substituted mono- or bicyclic ring system;
D^{b} and E^{b} together with the bridging nitrogen form an unsubstituted or at least mono-substituted, saturated, unsaturated or aromatic heterocyclic ring which may contain at least one further heteroatom as a ring member and/or which may be condensed with an unsubstituted or at least mono-substituted mono- or bicyclic ring system;
or
D^{b} and E^{b}, independently of one another, each represent a hydrogen atom; a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via a linear or branched alkylene group and/or which may be condensed with an unsubstituted or at least mono-substituted mono- or bicyclic ring system; or an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof, respectively.

In another embodiment of the present invention compounds of general formula (Ib) are selected from the group consisting of compounds of general formula (Ih) 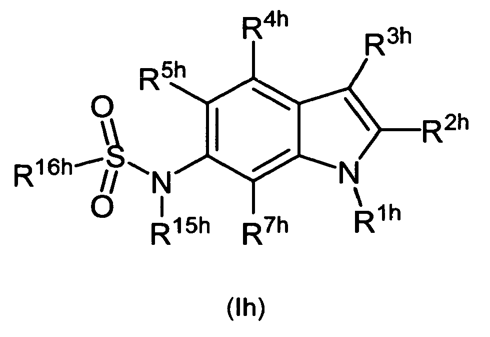 wherein
R^{1h} represents a saturated or unsaturated, unsubstituted or at least mono-substituted, optionally at least one heteroatom as a ring member containing cycloaliphatic radical, which may be bonded via a linear or branched alkylene group and/or which may be condensed with an unsubstituted or at least mono-substituted mono- or bicyclic ring system; or a -(CH₂)ₘₕ-NR^{13h}R^{14h} moiety with mh = 0, 1, 2, 3, 4 or 5;
R^{2h} represents a hydrogen atom; -F; -Cl; -Br; -I; -NO₂; -CN; -O-R^{10h}; a linear or branched, saturated or unsaturated, unsubstituted or at least mono-substituted aliphatic radical; or an unsubstituted or at least mono-substituted aryl or heteroaryl radical, which may be bonded via a linear or branched alkylene group and/or which may be condensed with an unsubstituted or at least mono-substituted mono- or bicyclic ring system;
R^{3h} represents a hydrogen atom; -F; -Cl; -Br; -I; -NO₂; -CN; -O-R^{10h}; a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via an unsubstituted or at least mono-substituted alkylene group and/or which may be condensed with an unsubstituted or at least mono-substituted mono- or bicyclic ring system; an unsubstituted or at least mono-substituted aryl or heteroaryl radical, which may be bonded via an unsubstituted or at least mono-substituted alkylene group and/or which may be condensed with an unsubstituted or at least mono-substituted mono- or bicyclic ring system; or a -(CH₂)ₙₕ-NR^{13h}R^{14h} moiety with nh = 0,1, 2, 3, 4 or 5;
R^{4h}, R^{5h} and R^{7h}, independently of one another, each represent a hydrogen atom;-NO₂; -CN; -O-R^{10h}; -C(=O)-OR^{12h} ; a halogen atom; a linear or branched, saturated or unsaturated, unsubstituted or at least mono-substituted aliphatic radical; or an unsubstituted or at least mono-substituted aryl or heteroaryl radical, which may be bonded via a linear or branched alkylene group;
R^{10h} represents a linear or branched, saturated or unsaturated, unsubstituted or at least mono-substituted aliphatic radical; or an unsubstituted or at least mono-substituted aryl or heteroaryl radical, which may be bonded via a linear or branched alkylene group;
R^{13h} and R^{14h}, independently of one another, each represent a hydrogen atom; or a linear or branched, saturated or unsaturated, unsubstituted or at least mono-substituted aliphatic radical;
or
R^{13h} and R^{14h} together with the bridging nitrogen form an unsubstituted or at least mono-substituted, saturated, unsaturated or aromatic heterocyclic ring which may contain at least one further heteroatom as a ring member and/or which may be condensed with an unsubstituted or at least mono-substituted mono- or bicyclic ring system;
R^{15h} represents a hydrogen atom; a linear or branched, saturated or unsaturated, unsubstituted or at least mono-substituted aliphatic radical or a -S(=O)₂-R^{16h} moiety;
and R^{16h} represents an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof, respectively.

Particularly preferred compounds of general formula (Ih) are those, wherein
R^{1h} represents a hydrogen atom or a -(CH₂)ₘₕ-NR^{13h}R^{14h} radical,
R^{2h}, and R^{7h} each represent hydrogen,
R^{3h} represents a hydrogen atom, 1-methyl-piperidin-4-yl or a -(CH₂)ₙₕ-NR^{13h}R^{14h} moiety with nh = 0, 1 or 2,
R^{4h} represents chlorine, bromine or a hydrogen atom,
R^{4h} represents -C(=O)-O-C₂H₅ or a hydrogen atom,
R^{15h} represents hydrogen or a -S(=O)₂-R^{16h} moiety,
R^{13h} and R^{14h}, identical or different, each represent methyl, ethyl, isopropyl or n-propyl, more preferably methyl,
or
R^{13h} and R^{14h}, together with the bridging nitrogen atom form a 5- or 6-membered heterocyclic ring, more preferably form a pyrrolidine ring or a piperidine ring
and
R^{16h} represents an aryl or heteroaryl radical selected from the group consisting of phenyl, naphthyl, thiophenyl, benzo[b]furanyl, benzo[b]thiophenyl and imidazo[2,1-b]thiazolyl which may be substituted by 1, 2 or 3 substituents selected from the group consisting of chlorine, methyl, phenyl and -O-phenyl and/or which may be bonded via a C₁₋₂ alkylene group,
and mh is 0, 1 or 2,
optionally in form of one of its stereoisomers, preferably enantiomers or diastereomers, a racemate or in form of a mixture of at least two of its stereoisomers, preferably enantiomers and/or diastereomers, in any mixing ratio, or a salt thereof, preferably a corresponding, physiologically acceptable salt thereof, or a corresponding solvate thereof.

More particularly preferred compounds of general formula (1h) are those selected from the group consisting of
[788] N-[1-(2-Dimethylaminoethyl)-1H-indol-6-yl]-5-chloro-3-methylbenzo[b]thiophene-2-sulfonamide,
[789] N-[1-(2-Dimethylaminoethyl)-1H-indol-6-yl]-naphthalene-2-sulfonamide,
[790] N-[1-(2-Dimethylaminoethyl)-1H-indol-6-yl]-naphthalene-1-sulfonamide,
[791] N-[1-(2-Dimethylaminoethyl)-1H-indol-6-yl]-6-chloroimidazo[2,1-b]thiazole-5-sulfonamide,
[792] N-[1-(2-Dimethylaminoethyl)-1H-indol-6-yl]-4-phenylbenzenesulfonamide,
[793] N-[1-(2-Dimethylaminoethyl)-1H-indol-6-yl]-2-(naphthalene-1-yl)-ethanesulfonamide,
[794] N-[1-(2-Dimethylaminoethyl)-1H-indol-6-yl]-4-phenoxybenzenesulfonamide,
[795] N-[1-(2-Dimethylaminoethyl)-1H-indol-6-yl]-3,5-dichlorobenzenesulfonamide,
[796] 5-Chloro-3-methyl-N-[1-[2-(pyrrolidin-1-yl)ethyl-1H-indol-6-yl]-benzo[b]thiophene-2-sulfonamide,
[797] N-(1-[2-(Pyrrolidin-1-yl)ethyl]-1H-indol-6-yl]-napthyl-2-sulfonamide,
[798] N-[1-[2-Pyrrolidin-1-yl]ethyl]-1H-indol-6-yl]-naphthalene-1-sulfonamide,
[799] 6-Chloro-N-[1-[2-(pyrrolidin-1-yl)ethyl]-1H-indol-6-yl]-imidazo[2,1-b]thiazole-5-sulfonamide,
[800] 4-Phenyl-N-(1-(2-(pyrrolidin-1-yl)ethyl)-1H-indol-6-yl)-benzenesulfonamide
[801] 2-(Naphth-1-yl)-N-(1-(2-(pyrrolidin-1-yl)ethyl)-1H-indol-6-yl)-ethansulfonamide,
[802] 4-Phenoxy-N-(1-(2-(pyrrolidin-1-yl)ethyl)-1H-indol-6-yl)-benzenesulfonamide
[803] 3,5-Dichloro-N-(1-(2-(pyrrolidin-1-yl)-1H-indol-6-yl)-benzenesulfonamide,
[804] 5-chloro-N-(3-(2-(diethylamino)ethyl)-1H-indol-6-yl)-3-methylbenzo[b]thiophene-2-sulfonamide,
[805] N-(3-(2-(diethylamino)ethyl)-1H-indol-6-yl)naphthalene-2-sulfonamide,
[806] N-(3-(2-(diethylamino)ethyl)-1H-indol-6-yl)naphthalene-1-sulfonamide,
[807] 6-chloro-N-(3-(2-(diethylamino)ethyl)-1H-indol-6-yl)imidazo[2, 1-b]thiazole-5-sulfonamide,
[808] N-(3-(2-(diethylamino)ethyl)-1H-indol-6-yl)-4-phenylbenzenesulfonamide,
[809] N-(3-(2-(diethylamino)ethyl)-1H-indol-6-yl)-4-phenoxybenzenesulfonamide,
[810] 3,5-dichloro-N-(3-(2-(diethylamino)ethyl)-1H-indol-6-yl)benzenesulfonamide,
[811] 4,5-dichloro-N-(3-(2-(diethylamino)ethyl)-1H-indol-6-yl)thiophene-2-sulfonamide,
[812] 5-chloro-N-(3-(2-(diethylamino)ethyl)-1H-indol-6-yl)naphthalene-1-sulfonamide,
[813] 5-chloro-N-(3-(2-(dimethylamino)ethyl)-1H-indol-6-yl)-3-methylbenzo[b]thiophene-2-sulfonamide,
[814] N-(3-(2-(dimethylamino)ethyl)-1H-indol-6-yl)naphthalene-2-sulfonamide,
[815] N-(3-(2-(dimethylamino)ethyl)-1H-indol-6-yl)naphthalene-1-sulfonamide,
[816] 6-chloro-N-(3-(2-(dimethylamino)ethyl)-1H-indol-6-yl)imidazo[2,1-b]thiazole-5-sulfonamide,
[817] N-(3-(2-(dimethylamino)ethy)-1H-indol-6-yl)-4-phenylbenzenesulfonamide,
[818] N-(3-(2-(dimethylamino)ethyl)-1H-indol-6-yl)-2-(naphthalen-1-yl)ethanesulfonamide,
[819] N-(3-(2-(dimethylamino)ethyl)-1H-indol-6-yl)-4-phenoxybenzenesulfonamide,
[820] 3,5-dichloro-N-(3-(2-(dimethylamino)ethyl)-1H-indol-6-yl)benzenesulfonamide,
[821] 4,5-dichloro-N-(3-(2-(dimethylamino)ethyl)-1H-indol-6-yl)thiophene-2-sulfonamide,
[822] 5-chloro-N-(3-(2-(dimethylamino)ethyl)-1H-indol-6-yl)naphthalene-1-sulfonamide,
[823] 6-bis(6-chloroimidazo[2,1-b]thiazol-5-ylsulfonyl)amino-3-(2-(dimethylamino)ethyl)-1H-indole,
[824] 6-bis(3,5-dichlorobenzenesulfonyl)amino-3-(2-(dimethylamino)ethyl)-1H-indole,
[825] 6-bis(4,5-dichlorothiophene-2-sulfonyl)amino-3-(2-(dimethylamino)ethyl)-1H-indole,
[826] 6-bis(5-chloro-3-methylbenzo[b]thiophene-2-sulfonyl)amino-3-(2-(dimethylamino)-1-ethoxyethyl)-1H-indole
[827] Ethyl 6-(5-chloro-3-methylbenzo[b]thiophene-2-sulfonamido)-3-(1-methylpiperidin-4-yl)-1H-indole-5-carboxylate,
[828] N-(4-bromo-3-(1-methylpiperidin-4-yl)-1H-indol-6-yl)naphthalene-1-sulfonamide,
[829] N-(7-bromo-3-(2-(dimethylamino)ethyl)-1H-indol-5-yl)benzofuran-2-sulfonamide,
[830] N-(7-methoxy-3-(2-(pyrrolidin-1-yl)ethyl)-1H-indol-5-yl)benzo[c][1,2,5]thiadiazole-4-sulfonamide,
[831] N-(7-methoxy-3-(2-(pyrrolidin-1-yl)ethyl)-1H-indol-5-yl)naphthalene-2-sulfonamide and
[832] 6-chloro-N-(7-methoxy-3-(2-(pyrrolidin-1-yl)ethyl)-1H-indol-5-yl)imidazo[2,1-b]thiazole-5-sulfonamide;
and their corresponding salts and solvates.

The compounds of general formula (Ih) may be prepared according to the disclosure of WO 2005/013976 and WO 2006/024535.

In another embodiment of the present invention compounds of general formula (Ib) are selected from the group consisting of compounds of general formula (Ik) 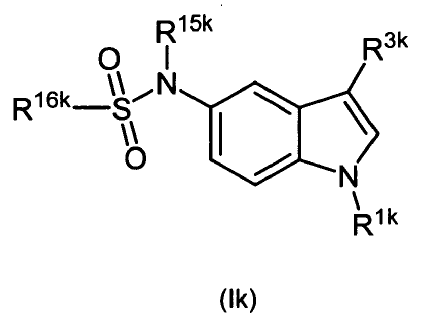 wherein
R^{1k} represents a hydrogen atom; a linear or branched, saturated or unsaturated, unsubstituted or at least mono-substituted aliphatic radical; an unsubstituted or at least mono-substituted phenyl radical or an unsubstituted or at least mono-substituted benzyl radical;
R^{3k} represents a saturated or unsaturated, unsubstituted or at least mono-substituted, optionally at least one heteroatom as a ring member containing cycloaliphatic radical, which may be bonded via an unsubstituted or at least mono-substituted alkylene group and/or which may be condensed with an unsubstituted or at least mono-substituted mono- or bicyclic ring system; or a -(CH₂)ₙₖ-NR^{13k}R^{14k} moiety with nk = 0, 1, 2, 3, 4 or 5;
R^{13k} and R^{14k}, independently of one another, each represent a hydrogen atom; or a linear or branched, saturated or unsaturated, unsubstituted or at least mono-substituted aliphatic radical;
or
R^{13k} and R^{14k} together with the bridging nitrogen form an unsubstituted or at least mono-substituted, saturated, unsaturated or aromatic heterocyclic ring which may contain at least one further heteroatom as a ring member and/or which may be condensed with an unsubstituted or at least mono-substituted mono- or bicyclic ring system;
R^{15k} represents a hydrogen atom; or a linear or branched, saturated or unsaturated, unsubstituted or at least mono-substituted aliphatic radical;
and R^{16k} represents an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system; optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof, respectively.

Preferred compounds of general formula (Ik) are those, wherein
nk represents 0, 1, 2, 3 or 4;
R^{1k} represents hydrogen,
R^{3k} represents a -NR^{13k}R^{14k} moiety or a moiety selected from the group consisting of 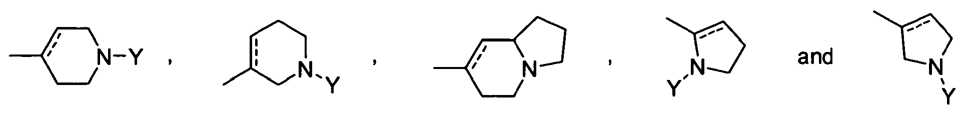 wherein, if present, the dotted line represents an optional chemical bond and Y represents hydrogen, a methyl group or an ethyl group,
R^{15k} represents hydrogen, a methyl group or an ethyl group,
R^{13k} and R^{14k}, identical or different, represent a methyl group, an ethyl group, an n-propyl group, an isopropyl group, an n-butyl group, an isobutyl group, a sec-butyl group, or a tert-butyl group, or
R^{13k} and R^{14k} together with the bridging nitrogen atom form a moiety selected from the group consisting of 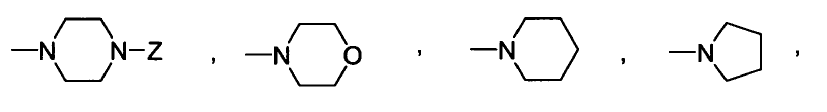 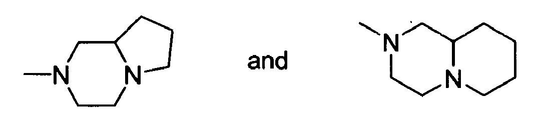 wherein Z represents hydrogen, a methyl group or an ethyl group,
R^{16k} represents a moiety selected from the group consisting of 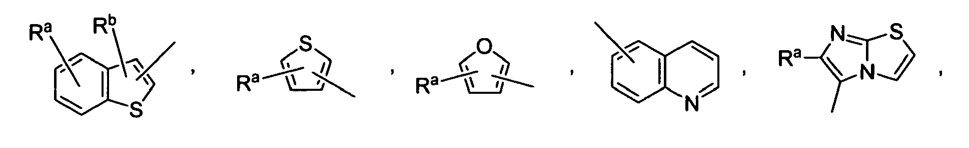 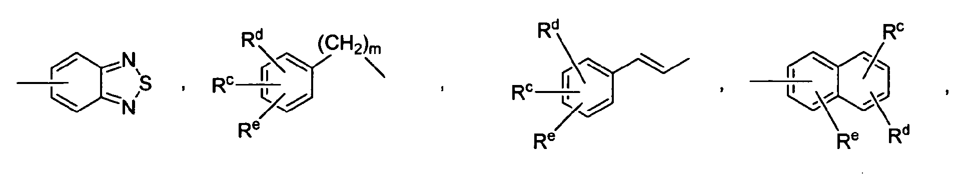 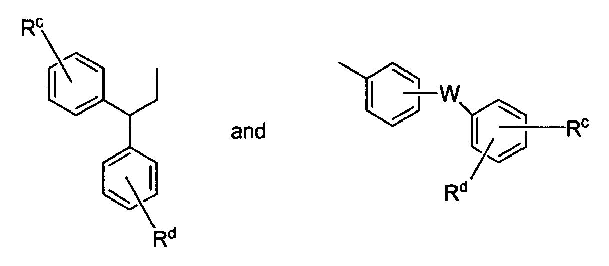 wherein
R^{a} and R^{b} are each independently selected from the group consisting of hydrogen, fluorine, chlorine, bromine, methyl, ethyl, pyridinyl, thiophenyl and furyl,
R^{c}, R^{d} and R^{e} are each independently selected from the group consisting of hydrogen, fluorine, chlorine, bromine, methyl, ethyl, methoxy, ethoxy and -CF₃,
W represents a single chemical bond between the two rings, a CH₂group, O, S or a NR^{f}-moiety, wherein R^{f} is hydrogen, methyl or ethyl,
m is 0, 1, 2, 3 or 4;
optionally in form of one of its stereoisomers, preferably enantiomers or diastereomers, a racemate or in form of a mixture of at least two of its stereoisomers, preferably enantiomers and/or diastereomers, in any mixing ratio, or a salt thereof, preferably a corresponding, physiologically acceptable salt thereof, or a corresponding solvate thereof.

Particularly preferred compounds of general formula (1k) are those selected from the group consisting of
[833] N-[3-(2-diethylaminoethyl)-1H-indol-5-yl]-5-chloro-3-methylbenzo[b]thiophene-2-sulphonamide,
[834] N-[3-(2-diethylaminoethyl)-1H-indol-5-yl]naphthalene-1-sulphonamide,
[835] Hydrochloride N-[3-(2-diethylaminoethyl)-1*H*-indol-5-yl]naphthalene-1-sulphonamide,
[836] N-[3-(2-diethylaminoethyl)-1*H*-indol-5-yl]-3,5-dichlorobenzenesulphonamide,
[837] N-[3-(2-diethylaminoethyl)-1*H*-indol-5-yl]-4-phenylbenzenesulphonamide,
[838] N-[3-(2-diethylaminoethyl)-1*H*-indol-5-yl]-5-chlorothiophene-2-sulphonamide,
[839] N-[3-(2-dimethylaminoethyl)-1*H*-indol-5-yl]-5-chloro-3-methylbenzo[b]thiophene-2-sulphonamide,
[840] N-[3-(2-dimethylaminoethyl)-1*H*-indol-5-yl]naphthalene-1-sulphonamide,
[841] N-[3-(2-dimethylamino-ethyl)-1H-indol-5-yl]-6-chloroimidazo[2,1-b]thiazol-5-sulphonamide,
[842] N-[3-(1-methylpiperidin-4-yl)-1H-indol-5-yl]-5-chloro-3-methylbenzo[b]thiophene-2-sulphonamide,
[843] N-[3-(1-methylpiperidin-4-yl)-1H-indol-5-yl]-5-chloro-3-methylbenzo[b]thiophene-2-sulphonamide hydrochloride,
[844] N-[3-(1-methylpiperidin-4-yl)-1H indol-5-yl]naphthalene-1-sulphonamide,
[845] N-[3-(1-methylpiperidin-4-yl)-1H-indol-5-yl]naphthalene-1-sulphonamide hydrochloride,
[846] N-[3-(1-methylpiperidin-4-yl)-1*H* indol-5-yl]-5-chlorothiophene-2-sulphonamide,
[847] N-[3-(1-methylpiperidin-4-yl)-1H-indol-5-yl]-4-phenylbenzenesulphonamide,
[848] N-[3-(1-methylpiperidin-4-yl)-1H-indol-5-yl]quinoline-8-sulphonamide,
[849] N-[3-(2-diethylaminoethyl)-1H indol-5-yl]naphthalene-2-sulphonamide,
[850] N-[3-(1-methyl-1,2,3,6-tetrahydropyddin-4-yl)1H-indol-5-yl]naphthalene-1 - sulphonamide,
[851] N-[3-(4-methylpiperazin-1-yl)methyl-1H-indol-5-yl]-5-chloro-3-methylbenzo[b]thiophene-2-sulphonamide,
[852] N-[3-(2-dimethylaminoethyl)-1H-indol-5-yl]-5-(2-pyridil)thiophene-2-sulphonamide,
[853] N-[3-(2-dimethylaminoethyl)-1*H*-indol-5-yl]-2,1,3-benzothiadiazol-4-sulphonamide,
[854] N-[3-(2-dimethylaminoethyly)-1*H*-indol-5-yl]quinoline-8-sulphonamide,
[855] N-[3-(2-dimethylaminoethyl)-1*H*-indol-5-yl]-5-chloronaphthalene-2-sulphonamide,
[856] N-[3-(2-dimethylaminoethyl)-1*H*-indol-5-yl]-4-phenoxybenzenesulphonamide,
[857] N-[3-(2-dimethylaminoethyl)-1*H*-indol-5-yl]-4-phenylbenzenesulphonamide,
[858] N-[3-(2-diethylaminoethyl)-1*H*-indol-5-yl]-N-ethyl-naphthalene-2-sulphonamide,
[859] N-{3-[2-(morpholin-4-yl)ethyl]-1H-indol-5-yl}-5-chloro-3-methylbenzo[b]thiophene-2-sulphonamide,
[860] N-{3-[2-(morpholin-4-yl)ethyl]-1H-indol-5-yl}naphthalene-1-sulphonamide,
[861] N-[3-(2-diethylaminoethyl)-1H-indol-5-yl]naphthalene-2-sulphonamide,
[862] N-[3-dimethylaminomethyl-1H-indol-5-yl]-5-chloro-3-methylbenzo[b]thiophene-2-sulphonamide,
[863] N-[3-(2-dipropylaminoethyl)-1H-indol-5-yl]naphthalene-1-sulphonamide,
[864] N-[3-(2-dipropylaminoethyl)-1H-indol-5-yl]-5-chloro-3-methylbenzo[b]thiophene-2-sulphonamide,
[865] N-[3-(2-dibutylaminoethyl)-1H-indol-5-yl]-5-chloro-3-methylbenzo[b]thiophene-2-sulphonamide,
[866] N-[3-(2-dibutylaminoethyl)-1H-indol-5-yl]naphthalene-1-sulphonamide,
[867] N-[3-(2-diethylaminoethyl)-1H-indol-5-yl]-5-chloronaphthalene-1-sulphonamide,
[868] N-[3-(2-diethylaminoethyl)-1H-indol-5-yl]-trans-β-styrenesulphonamide,
[869] N-[3-(4-methylpiperazin-1-yl)methyl-1*H*-indol-5-yl]-trans-β-styrenesulphonamide,
[870] N-[3-(octahydroindolizin-7-yl)-1H-indol-5-yl]-5-chloro-3-methylbenzo[b]thiophene-2-sulphonamide,
[871] N-[3-(2-diethylaminoethyl)-1*H*-indol-5-yl]-6-chloroimidazo[2,1-b]thiazol-5-sulphonamide,
[872] N-{3-[2-(morpholin-4-yl)ethyl]-1H-indol-5-yl}naphthalene-2-sulphonamide,
[873] N-[3-(4-methylpiperazin-1-yl)methyl-1H-indol-5-yl]-a-toluenesulphonamide,
[874] N-[3-(3-diethylaminopropyl)-1H-indol-5-yl]naphthalene-2-sulphonamide,
[875] N-[3-(3-diethylaminopropyl)-1H-indol-5-yl]-5-chloro-3-methylbenzo[b]thiophene-2-sulphonamide,
[876] N-{3-[2-(pyrrolidin-1-yl)ethyl]-1H-indol-5-yl}-5-chloro-3-methylbenzo[b]thiophene-2-sulphonamide,
[877] N-{3-[2-(pyrrolidin-1-yl)ethyl]-1H-indol-5-yl}naphthalene-1-sulphonamide,
[878] N-{3-[2-(pyrrolidin-1-yl)ethyl]-1H-indol-5-yl}naphthalene-2-sulphonamide,
[879] N-[3-(2-dipropylaminoethyl)-1H-indol-5-yl]naphthalene-2-sulphonamide,
[880] N-[3-(2-dimethylaminoethyl)-1H-indol-5-yl]-5-chloronaphthalene-1-sulphonamide,
[881] N-[3-(2-dimethylaminoethyl)-1H-indol-5-yl]naphthalene-2-sulphonamide,
[882] N-{3-[2-(morpholin-4-yl)ethyl]-1H-indol-5-yl}quinoline-8-sulphonamide,
[883] N-{3-[2-(morpholin-4-yl)ethyl]-1H-indol-5-yl}-4-phenylbenzenesulphonamide,
[884] N-[3-(4-methylpiperazin-1-yl)ethyl-1*H*-indol-5-yl]naphthalene-2-sulphonamide and
[885] N-[3-(4-methylpiperazin-1-yl)ethyl-1*H*-indol-5-yl]-5-chloronaphthalene-1-sulphonamide;
and their corresponding salts and solvates.

The compounds of general formula (Ik) may be prepared according to the disclosure of WO 2004/098588.

In another embodiment of the present invention compounds of general formula (Ib) are selected from the group consisting of compounds of general formula (Im) 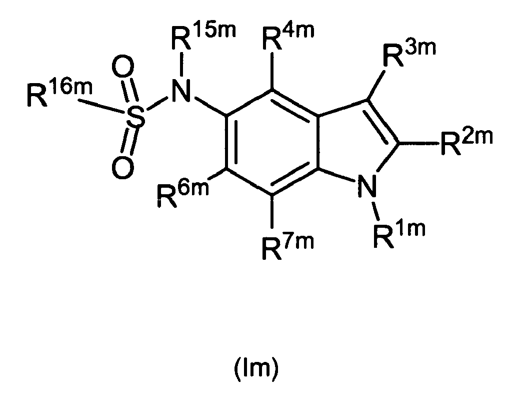 wherein
R^{1m} represents a saturated or unsaturated, unsubstituted or at least mono-substituted, optionally at least one heteroatom as a ring member containing cycloaliphatic radical, which may be bonded via a linear or branched alkylene group and/or which may be condensed with an unsubstituted or at least mono-substituted mono- or bicyclic ring system; or a -(CH₂)ₘₘ-NR^{13m}R^{14m} moiety with mm = 0, 1, 2, 3, 4 or 5;
R^{2m} represents a hydrogen atom; -F; -Cl; -Br; -l; -NO₂; -CN; -O-R^{10m}; a linear or branched, saturated or unsaturated, unsubstituted or at least mono-substituted aliphatic radical; or an unsubstituted or at least mono-substituted aryl or heteroaryl radical, which may be bonded via a linear or branched alkylene group and/or which may be condensed with an unsubstituted or at least mono-substituted mono- or bicyclic ring system;
R^{3m} represents a hydrogen atom; -F; -Cl; -Br; -l; -NO₂; -CN; -O-R^{10m}; a linear or branched, saturated or unsaturated, unsubstituted or at least mono-substituted aliphatic radical; or an unsubstituted or at least mono-substituted aryl or heteroaryl radical, which may be bonded via an unsubstituted or at least mono-substituted alkylene group and/or which may be condensed with an unsubstituted or at least mono-substituted mono- or bicyclic ring system;
R^{4m}, R^{6m} and R^{7m}, independently of one another, each represent a hydrogen atom;-NO₂; -CN; -O-R^{10m}; a halogen atom; a linear or branched, saturated or unsaturated, unsubstituted or at least mono-substituted aliphatic radical; or an unsubstituted or at least mono-substituted aryl or heteroaryl radical, which may be bonded via a linear or branched alkylene group;
R^{10m} represents a linear or branched, saturated or unsaturated, unsubstituted or at least mono-substituted aliphatic radical; or an unsubstituted or at least mono-substituted aryl or heteroaryl radical, which may be bonded via a linear or branched alkylene group;
R^{13m} and R^{14m}, independently of one another, each represent a hydrogen atom; or a linear or branched, saturated or unsaturated, unsubstituted or at least mono-substituted aliphatic radical;
or
R^{13m} and R^{14m} together with the bridging nitrogen form an unsubstituted or at least mono-substituted, saturated, unsaturated or aromatic heterocyclic ring which may contain at least one further heteroatom as a ring member and/or which may be condensed with an unsubstituted or at least mono-substituted mono- or bicyclic ring system;
R^{15m} represents a hydrogen atom; a linear or branched, saturated or unsaturated, unsubstituted or at least mono-substituted aliphatic radical or a -S(=O)₂-R^{16m} moiety;
and R^{16m} represents an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof, respectively.

Preferred compounds of general formula (Im) are those, wherein
R^{1m} represents a -(CH₂)ₘₘ-NR¹³mR¹⁴m radical,
R^{2m} represents hydrogen or an alkyl radical selected from the group consisting of methyl, ethyl, n-propyl and isopropyl, more preferably hydrogen or methyl,
R^{3m}, R^{4m} and R^{6m} each represent hydrogen,
R^{7m} represents a hydrogen atom, a chlorine atom, a bromine atom or -O-CH₃,
R^{15m} represents hydrogen,
R^{13m} and R^{14m}, identical or different, each represent methyl, ethyl, n-propyl or isopropyl, more preferably methyl or ethyl,
or
R^{13m} and R^{14m} together with the bridging nitrogen form a 5- or 6-membered heterocyclic ring, more preferably form pyrrolidine or piperidine,
R^{16m} represents an aryl or heteroaryl radical selected from the group consisting of phenyl, naphthyl, quinolinyl, benzo[b]furanyl, benzo[b]thiophenyl, benzo[1,2,5]thiadiazolyl, thiophenyl and imidazo[2,1-b]thiazolyl which may be substituted by 1, 2 or 3 substituents selected from the group consisting of fluorine, bromine, chlorine, methyl, phenyl, nitro, -C(=O)-CH₃, -O-CH₃ and -O-phenyl and/or which may be bonded via a C₁₋₂ alkylene group or a C₂ alkenylene group,
and
mm is 2 or 3,
optionally in form of one of its stereoisomers, preferably enantiomers or diastereomers, its racemate or in form of a mixture of at least two of its stereoisomers, preferably enantiomers or diastereomers, in any mixing ratio, or a salt thereof, preferably a corresponding, physiologically acceptable salt thereof, or a corresponding solvate thereof.

Particularly preferred compounds of general formula (Im) are those selected from the group consisting of
[886] N-[1-(2-dimethyaminoethyl)-1H-indole-5-yl]-5-chloro-3-methylbenzo[b]thiophene-2-sulfonamide,
[887] N-[1-(2-dimethylaminoethyl)-1H-indole-5-yl]-naphthalene-2-sulfonamide,
[888] N-[1-(2-dimethylaminoethyl)-1H-indole-5-yl]-naphthalene-1-sulfonamide,
[889] N-[1-(2-dimethylaminoethyl)-1H-indole-5-yl]-5-chloronaphthalene-1-sulfonamide,
[890] N-[1-(2-dimethylaminoethyl)-1H-indole-5-yl]-benzenesulfonamide,
[891] N-[1-(2-dimethylaminoethyl)-1H-indole-5-yl]-quinoline-8-sulfonamide,
[892] N-[1-(2-dimethylaminoethyl)-1H-indole-5-yl]-4-phenoxybenzenesulfonamide,
[893] N-[1-(2-dimethylaminoethyl)-1H-indole-5-yl]-4-methylbenzenesulfonamide,
[894] N-[1-(2-dimethylaminoethyl)-1H-indole-5-yl]-5-chlorothiophene-2-sulfonamide,
[895] N-[1-(2-dimethylaminoethylyl)-1H-indole-5-yl]-benzo[1,2,5]thiadiazole-4-sulfonamide,
[896] N-[1-(2-dimethylaminoethyl)-1H-indole-5-yl]-6-chloroimidazo[2,1-b]thiazole-5-sulfonamide,
[897] N-[1-(2-dimethylaminoethyl)-1H-indole-5-yl]-3,5-dichlorobenzenesulfonamide,
[898] N-[1-(2-dimethylaminoethyl)-1H-indole-5-yl]-3-bromobenzenesulfonamide,
[899] N-[1-(2-dimethylaminoethyl)-1H-indole-5-yl]-3-nitrobenzenesulfonamide,
[900] N-[1-(2-dimethylaminoethyl)-1H-indole-5-yl]-1-phenylmethanesulfonamide,
[901] N-[1-(2-pyrrolidine-1-yl-ethyl)-1H-indole-5-yl]-naphthalene-2-sulfonamide,
[902] N-[1-(2-pyrrolidine-1-yl-ethyl)-1H-indole-5-yl]-naphthalene-1-sulfonamide,
[903] N-[1-(2-pyrrolidine-1-yl-ethyl)-1H-indole-5-yl]- 5-chloro-3-methylbenzo[b]thiophene-2-sulfonamide,
[904] *trans*-N-[1-(2-dimethylaminoethyl)-1H-indole-5-yl]-2-phenylethenesulfonamide,
[905] N-[1-(2-dimethylaminoethyl)-1H-indole-5-yl]-4,5-dichlorothiophene-2-sulfonamide,
[906] N-[1-(2-dimethylaminoethyl)-1H-indole-5-yl]-4-acetybenzenesulfonamide,
[907] N-[1-(2-dimethylaminoethyl)-1H-indole-5-yl]-4-bromobenzenesulfonamide,
[908] N-[1-(2-dimethylaminoethyl)-1H-indole-5-yl]-4-methoxybenzenesulfonamide,
[909] N-[3-(2-diethylaminoethyl)-1H-indole-5-yl]-5-chloro-3-methylbenzo[b]thiophene-2-sulfonamide,
[910] N-[1-(2-dimethylaminoethyl)-1H-indole-5-yl]-4-nitrobenzenesulfonamide,
[911] N-[1-(2-dimethylaminoethyl)-1H-indole-5-yl]-4-fluorobenzenesulfonamide,
[912] N-[1-(2-diethylaminoethyl)-1H-indole-5-yl]-6-chloroimidazo[2,1-b]thiazole-5-sulfonamide,
[913] N-[1-(2-pyrrolidine-1-yl-ethyl)-1H-indole-5-yl]-]-6-chloroimidazo[2,1-b]thiazole-5-sulfonamide,
[914] N-(1-(2-(diethylamino)ethyl)-1H-indol-5-yl)-naphthalene-2-sulfonamide,
[915] N-(1-(2-(diethylamino)ethyl)-1H-indol-5-yl)-naphthalene-1-sulfonamide,
[916] N-(1-(2-(diethylamino)ethyl)-1H-indol-5-yl)-4-phenylbenzenesulfonamide,
[917] 5-chloro-N-(1-(2-(dimethylamino)ethyl)-2-methyl-1H-indol-5-yl)-3-methylbenzo[b]thiophene-2-sulfonamide,
[918] N-(1-(2-(dimethylamino)ethyl)-2-methyl-1H-indol-5-yl)-naphthalene-2-sulfonamide,
[919] N-(1-(2-(dimethylamino)ethyl)-2-methyl-1H-indol-5-yl)-naphthalene-1-sulfonamide,
[920] 6-chloro-N-(1-(2-(dimethylamino)ethyl)-2-methyl-1H-indol-5-yl)imidazo[2,1-b]thiazole-5-sulfonamide,
[921] N-(1-(2-(dimethylamino)ethyl)-2-methyl-1H-indol-5-yl)-4-phenylbenzenesulfonamide,
[922] N-(1-(2-dimethylamino)ethyl)-2-methyl-1H-indol-5-yl)-2-(naphth-1-yl)-ethanesulfonamide,
[923] N-(1-(2-(dimethylamino)ethyl)-2-methyl-1H-indol-5-yl)-4-phenoxy-benzenesulfonamide,
[924] 3,5-dichloro-N-(1-(2-(dimethylamino)ethyl)-2-methyl-1H-indol-5-yl)-benzenesulfonamide,
[925] N-(1-(2-(dimethylamino)ethyl)-2-methyl-1H-indol-5-yl)benzo[b]thiophene-3-sulfonamide,
[926] N-(1-(2-(diethylamino)ethyl)-1H-indol-5-yl)benzo[b]thiophene-3-sulfonamide
[927] N-(1-(2-(dimethylamino)ethyl)-1H-indol-5-yl)benzo[b]thiophene-3-sulfonamide,
[928] 5-chloro-3-methyl-N-(1-(3-(piperidin-1-yl)propyl)-1H-indol-5-yl)benzo[b]thiophene-2-sulfonamide,
[929] N-(1-(3-(piperidin-1-yl)propyl)-1H-indol-5-yl)naphthalene-2-sulfonamide,
[930] N-(1-(3-(piperidin-1-yl)propyl)-1H-indol-5-yl)naphthalene-1-sulfonamide,
[931] 6-chloro-N-(1-(3-piperidin-1-yl)propyl)-1H-indol-5-yl)imidazo[2,1-b]thiazole-5-sulfonamide,
[932] 4-phenyl-N-(1-(3-(piperidin-1-yl)propyl)-1H-indol-5-yl)benzenesulfonamide,
[933] 2-(naphth-1-yl)-N-(1-(3-(piperidin-1-yl)propyl)-1H-indol-5-yl)ethanesulfonamide,
[934] 4-phenoxy-N-(1-(3-(piperidin-1-yl)propyl)-1H-indol-5-yl)benzenesulfonamide,
[935] 3,5-dichloro-N-(1-(3-(piperidin-1-yl)propyl)-1H-indol-5-yl)benzenesulfonylamide,
[936] 4,5-dichloro-N-(1-(3-(piperidin-1-yl)propyl)-1H-indol-5-yl)thiophene-2-sulfonamide and
[937] 5-chloro-N-(1-(3-(piperidin-1-yl)propyl)-1H-indol-5-yl)naphthalene-1-sulfonamide,
[938] N-(3-(2-(diethylamino)ethyl)-7-methoxy-1H-indol-5-yl)naphthalene-2-sulfonamide,
[939] N-(3-(2-(diethylamino)ethyl)-7-methoxy-1H-indol-5-yl)benzo[c][1,2,5]thiadiazole-4-sulfonamide,
[940] 6-chloro-N-(3-(2-(diethylamino)ethyl)-7-methoxy-1H-indol-5-yl)imidazo[2,1-b]thiazole-5-sulfonamide,
and their corresponding salts and solvates.

The compounds of general formula (Im) may be prepared according to the disclosure of WO 2005/013977 and WO 2006/024535.

In another embodiment of the present invention compounds of general formula (Ib) are selected from the group consisting of compounds of general formula (In) 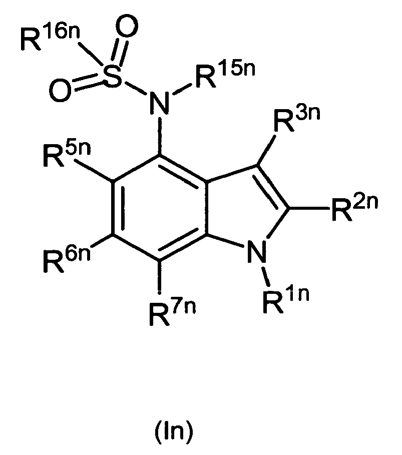 wherein
R¹ⁿ represents a saturated or unsaturated, unsubstituted or at least mono-substituted, optionally at least one heteroatom as a ring member containing cycloaliphatic radical, which may be bonded via a linear or branched alkylene group and/or which may be condensed with an unsubstituted or at least mono-substituted mono- or bicyclic ring system; or a -(CH₂)ₘₙ-NR¹³ⁿR¹⁴ⁿ moiety with mn = 0, 1, 2, 3, 4 or 5;
R²ⁿ represents a hydrogen atom; -F; -Cl; -Br; -I; -NO₂; -CN; -O-R¹⁰ⁿ; a linear or branched, saturated or unsaturated, unsubstituted or at least mono-substituted aliphatic radical; or an unsubstituted or at least mono-substituted aryl or heteroaryl radical, which may be bonded via a linear or branched alkylene group and/or which may be condensed with an unsubstituted or at least mono-substituted mono- or bicyclic ring system;
R³ⁿ represents a hydrogen atom; -F; -Cl; -Br; -1; -NO₂; -CN; -O-R¹⁰ⁿ; a linear or branched, saturated or unsaturated, unsubstituted or at least mono-substituted aliphatic radical; an unsubstituted or at least mono-substituted aryl or heteroaryl radical, which may be bonded via an unsubstituted or at least mono-substituted alkylene group and/or which may be condensed with an unsubstituted or at least mono-substituted mono- or bicyclic ring system; or a -(CH₂)ₙₙ-NR¹³ⁿR¹⁴ⁿ moiety with nn = 0, 1, 2, 3, 4 or 5;
R⁵ⁿ, R⁶ⁿ and R⁷ⁿ, independently of one another, each represent a hydrogen atom;-NO₂; -CN; -O-R¹⁰ⁿ; a halogen atom; a linear or branched, saturated or unsaturated, unsubstituted or at least mono-substituted aliphatic radical; or an unsubstituted or at least mono-substituted aryl or heteroaryl radical, which may be bonded via a linear or branched alkylene group;
R¹⁰ⁿ represents a linear or branched, saturated or unsaturated, unsubstituted or at least mono-substituted aliphatic radical; or an unsubstituted or at least mono-substituted aryl or heteroaryl radical, which may be bonded via a linear or branched alkylene group;
R¹³ⁿ and R¹⁴ⁿ, independently of one another, each represent a hydrogen atom; or a linear or branched, saturated or unsaturated, unsubstituted or at least mono-substituted aliphatic radical;
or
R¹³ⁿ and R¹⁴ⁿ together with the bridging nitrogen form an unsubstituted or at least mono-substituted, saturated, unsaturated or aromatic heterocyclic ring which may contain at least one further heteroatom as a ring member and/or which may be condensed with an unsubstituted or at least mono-substituted mono- or bicyclic ring system;
R¹⁵ⁿ represents a hydrogen atom; a linear or branched, saturated or unsaturated, unsubstituted or at least mono-substituted aliphatic radical or a -S(=O)₂₋R¹⁶ⁿ moiety;
R¹⁶ⁿ represents an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof, respectively.

Preferred compounds of general formula (In) are those, wherein
R¹ⁿ represents a hydrogen atom or a -(CH₂)ₘₙ-NR¹³ⁿR¹⁴ⁿ radical,
R²ⁿ, R⁵ⁿ, R⁶ⁿ and R⁷ⁿ each represent hydrogen,
R³ⁿ represents a hydrogen atom or or a -(CH₂)ₙₙ-NR¹³ⁿR¹⁴ⁿ moiety with nn = 0, 1 or 2;
R¹⁵ⁿ represents hydrogen,
R¹³ⁿ and R¹⁴ⁿ, identical or different, each represent methyl, ethyl, n-propyl, isopropyl, more preferably methyl,
or
R¹³ⁿ and R¹⁴ⁿ together with the bridging nitrogen form a 5- or 6-membered heterocyclic ring, more preferably form pyrrolidine or piperidine,
and
R¹⁶ⁿ represents an aryl or heteroaryl radical selected from the group consisting of phenyl, naphthyl, benzo[b]thiophenyl and imidazo[2,1-b]thiazolyl which may be substituted by 1, 2 or 3 substituents selected from the group consisting of chlorine, methyl, phenyl and -O-phenyl and/or which may be bonded via a C₁₋₂ alkylene group, and
mn is 1 or 2;
optionally in form of one of its stereoisomers, preferably enantiomers or diastereomers, its racemate or in form of a mixture of at least two of its stereoisomers, preferably enantiomers or diastereomers, in any mixing ratio, or a salt thereof, preferably a corresponding, physiologically acceptable salt thereof, or a corresponding solvate thereof.

Particularly preferred compounds of general formula (In) are those selected from the group consisting of
[941] N-[1-(2-dimethylaminoethyl)-1H-indole-4-yl]-5-chloro-3-methylbenzo[b]thiophene-2-sulfonamide,
[942] N-[1-(2-dimethylaminoethyl)-1H-indole-4-yl]-naphtalene-2-sulfonamide,
[943] N-[1-(2-dimethylaminoethyl)-1H-indole-4-yl]-naphtalene-1-sulfonamide,
[944] N-[1-(2-dimethylaminoethyl)-1H-indole-4-yl]-4-phenylbenzenesulfonamide,
[945] N-[1-(2-dimethylaminoethyl)-1H-indole-4-yl]-2-(naphtalene-1-yl)-ethanesulfonamide,
[946] N-[1-(2-dimethylaminoethyl)-1H-indole-4-yl]-4-phenoxybenzenesulfonamide,
[947] N-[1-(2-dimethylaminoethyl)-1H-indole-4-yl]-3,5-dichlorobenzenesulfonamide and
[948] 6-chloro-N-[1-(2-dimethylaminoethyl)-1H-indol-4-yl]-imidazo[2,1-b]thiazole-5-sulfonamide
[949] N-(3-(2-(dimethylamino)ethyl)-1H-indol-4-yl)-4-biphenylsulfonamide,
[950] N-(3-(2-(dimethylamino)ethyl)-1H-indol-4-yl)-4-phenoxybenzenesulfonamide,
[951] 3,5-dichloro-N-(3-(2-(dimethylamino)ethyl)-1H-indol-4-yl)benzenesulfonamide,
[952] 5-chloro-N-(3-(2-(dimethylamino)ethyl)-1H-indol-4-yl)-3-methylbenzo[b]thiophene-2-sulfonamide,
[953] N-(3-(2-(dimethylamino)ethyl)-1H-indol-4-yl)naphthalene-1-sulfonamide,
[954] 5-chloro-N-(3-(2-(dimethylamino)ethyl)-1H-indol-4-yl)naphthalene-2-sulfonamide,
[955] N-(3-(2-(dimethylamino)ethyl)-1H-indol-4-yl)naphthalene-2-sulfonamide,
[956] 6-chloro-N-(3-(2-(dimethylamino)ethyl)-1H-indol-4-yl)imidazo[2,1-b]thiazole-5-sulfonamide,
[957] N-(3-(2-(dimethylamino)ethyl)-1H-indol-4-yl)-2-(naphthalen-1-yl)ethanesulfonamide,
and their corresponding salts and solvates.

The compounds of general formula (In) may be prepared according to the disclosure of WO 2005/13978 and WO 2006/024535.

In another embodiment of the present invention compounds of general formula (Ib) are selected from the group consisting of compounds of general formula (Io) 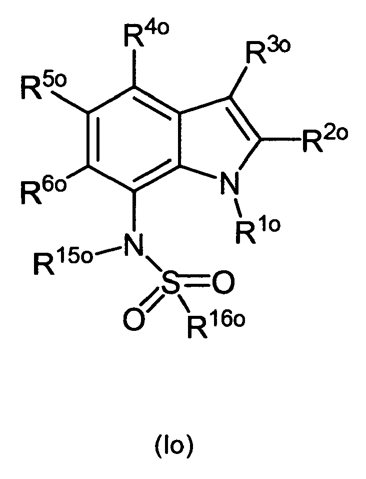 wherein
R^{1o} represents a saturated or unsaturated, unsubstituted or at least mono-substituted, optionally at least one heteroatom as a ring member containing cycloaliphatic radical, which may be bonded via a linear or branched alkylene group and/or which may be condensed with an unsubstituted or at least mono-substituted mono- or bicyclic ring system; or a -(CH2)ₘₒ-NR^{13o}R^{14o} moiety with mo = 0, 1, 2, 3, 4 or 5;
R^{2o} represents a hydrogen atom; -F; -Cl; -Br; -I; -NO₂; -CN; -O-R^{10o}; a linear or branched, saturated or unsaturated, unsubstituted or at least mono-substituted aliphatic radical; or an unsubstituted or at least mono-substituted aryl or heteroaryl radical, which may be bonded via a linear or branched alkylene group and/or which may be condensed with an unsubstituted or at least mono-substituted mono- or bicyclic ring system;
R^{3o} represents a hydrogen atom; -F; -Cl; -Br; -I; -NO₂; -CN; -O-R^{10o}; a linear or branched, saturated or unsaturated, unsubstituted or at least mono-substituted aliphatic radical; an unsubstituted or at least mono-substituted aryl or heteroaryl radical, which may be bonded via an unsubstituted or at least mono-substituted alkylene group and/or which may be condensed with an unsubstituted or at least mono-substituted mono- or bicyclic ring system; a -CH(OC₂H₅)-CH₂NR^{13o}R^{14o} moiety or a -(CH₂)ₙₒ-NR^{13o}R^{14o} moiety with no = 0, 1, 2, 3, 4 or 5;
R^{4o}, R^{5o} and R^{6o}, independently of one another, each represent a hydrogen atom;-NO₂; -CN; -O-R^{10o}; a halogen atom; a linear or branched, saturated or unsaturated, unsubstituted or at least mono-substituted aliphatic radical; or an unsubstituted or at least mono-substituted aryl or heteroaryl radical, which may be bonded via a linear or branched alkylene group;
R^{10o} represents a linear or branched, saturated or unsaturated, unsubstituted or at least mono-substituted aliphatic radical; or an unsubstituted or at least mono-substituted aryl or heteroaryl radical, which may be bonded via a linear or branched alkylene group;
R^{13o} and R^{14o}, independently of one another, each represent a hydrogen atom; or a linear or branched, saturated or unsaturated, unsubstituted or at least mono-substituted aliphatic radical;
or
R^{13o} and R^{14o} together with the bridging nitrogen form an unsubstituted or at least mono-substituted, saturated, unsaturated or aromatic heterocyclic ring which may contain at least one further heteroatom as a ring member and/or which may be condensed with an unsubstituted or at least mono-substituted mono- or bicyclic ring system;
R^{15o} represents a hydrogen atom; a linear or branched, saturated or unsaturated, unsubstituted or at least mono-substituted aliphatic radical or a -S(=O)₂-R^{16o} moiety;
and R^{16o} represents an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof, respectively.

Preferred compounds of general formula (Io) are those, wherein
R¹ is -(CH₂)ₘₒ-NR^{13o}R^{14o} radical,
R^{2o}, R^{4o} and R^{6o} each represent hydrogen,
R^{3o} represents a hydrogen atom, a -CH(OC₂H₅)-CH₂-NR^{13o}R^{14o} moiety or a -(CH₂)ₙₒ-NR^{13o}R^{14o} moiety with no = 0, 1 or 2,
R^{5o} represents a hydrogen atom, chlorine or bromine,
R^{15o} represents hydrogen or a -S(=O)₂-R1^{6o} moiety,
R^{13o} and R^{14o}, identical or different, each represent methyl, ethyl, n-propyl or isopropyl, more preferably methyl,
or
R^{13o} and R^{14o} together with the bridging nitrogen atom form a 5- or 6-membered heterocyclic ring, more preferably form a pyrrolidine or piperidine ring,
R^{16o} represents an aryl or heteroaryl radical selected from the group consisting of phenyl, naphthyl, benzo[b]thiophenyl and imidazo[2,1-b]thiazolyl which may be substituted by 1, 2 or 3 substituents selected from the group consisting of chlorine, methyl and phenyl and/or which may be bonded via a C₁₋₂ alkylene group,
and
no is 1 or 2;
optionally in form of one of its stereoisomers, preferably enantiomers or diastereomers, its racemate or in form of a mixture of at least two of its stereoisomers, preferably enantiomers or diastereomers, in any mixing ratio, or a salt thereof, preferably a corresponding, physiologically acceptable salt thereof, or a corresponding solvate thereof.

Particularly preferred compounds of general formula (Io) are those selected from the group consisting of
[958] N-[l-(2-dimethylaminoethylyl)-1H-indole-7-yl]-naphtalene-1-sulfonamide,
[959] N-[1-(2-dimethylaminoethyl)-1H-indole-7-yl]-5-chloro-3-methylbenzo[b]thiophene-2-sulfonamide,
[960] N-[1-(2-dimethylaminoethyl)-1H-indole-7-yl]-4-phenylbenzenesulfonamide and
[961] N-[1-(2-dimethylaminoethyl)-1H-indole-7-yl]-6-chloroimidazo[2,1-b]thiazole-5-sulfonamide
[962] 5-chloro-3-methyl-N-(1-(2-(pyrrolidin-1-yl)ethyl)-1H-indol-7-yl)-benzo[b]thiophen-2-sulfonamide,
[963] N-(1-(2-(pyrrolidin-1-yl)ethyl)-1H-indol-7-yl)naphthalene-1-sulfonamide,
[964] 6-chloro-N-(1-(2-(pyrroldin-1-yl)ethyl)-1H-indol-7-yl)imidazo[2,1-b]thiazole-5-sulfonamide and
[965] 2-(naphth-1-yl)-N-(1-(2-(pyn-olidin-1-yl)ethyl)-1H-indol-7-yl)ethansulfonamide
[966] 5-chloro-N-(3-(2-(dimethylamino)-1-ethoxyethyl)-1H-indol-7-yl)-3-methylbenzo[b]thiophene-2-sulfonamide,
[967] 5-chloro-N-(3-(2-(dimethylamino)ethyl)-1H-indol-7-yl)-3-methylbenzo[b]thiophene-2-sulfonamide,
[968] 7-bis(5-chloro-3-methylbenzo[b]thiophene-2-sulfonyl)amino-3-(2-(diethylamino)-1-ethoxyethyl)-1H-indole,
[969] 5-chloro-N-(3-(2-(diethylamino)-1-ethoxyethyl)-1H-indol-7-yl)-3-methylbenzo[b]thiophene-2-sulfonamide,
[970] 7-bis(5-chloro-3-methylbenzo[b]thiophene-2-sulfonyl)amino-3-(2-(dimethylamino)ethyl)-1H-indole,
[971] 7-bis(5-chloro-3-methylbenzo[b]thiophene-2-sulfonyl)amino-3-(2-(diethylamino)ethyl)-1H-indole,
[972] 5-chloro-N-(3-(2-(diethylamino)ethyl)-1H-indol-7-yl)-3-methylbenzo[b]thiophene-2-sulfonamide,
[973] 7-bis(6-chloroimidazo[2,1-b]thiazol-5-ylsulfonyl)amino-3-(2-(dimethylamino)ethyl)-1H-indole,
[974] N-(5-bromo-3-(2-(pyrrolidin-1-yl)ethyl)-1H-indol-7-yl)-6-chloroimidazo[2,1-b]thiazole-5-sulfonamide,
and their corresponding salts and solvates.

The compounds of general formula (Io) may be prepared according to the disclosure of WO 2005/13979 and WO 2006/024535.

In another embodiment of the present invention compounds of general formula (Ib) are selected from the group consisting of compounds of general formula (1p) 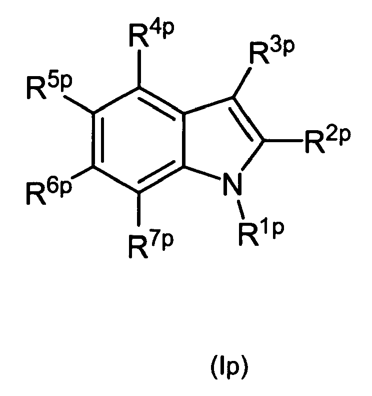 wherein
R^{1p} represents a -S(=O)₂-R^{9p} moiety or a -S(=O)₂-C(H)A^{p}B^{p} moiety;
R^{2p} represents a hydrogen atom; -F; -Cl; -Br; -l; -NO₂; -OH; -CN; -O-R^{10p}; -S-R^{11p}; a linear or branched, saturated or unsaturated, unsubstituted or at least mono-substituted aliphatic radical; or a saturated or unsaturated, unsubstituted or at least mono-substituted, optionally at least one heteroatom as a ring member containing cycloaliphatic radical, which may be bonded via a linear or branched alkylene group and/or which may be condensed with an unsubstituted or at least mono-substituted mono- or bicyclic ring system;
R^{3p} represents a saturated or unsaturated, unsubstituted or at least mono-substituted, optionally at least one heteroatom as a ring member containing cycloaliphatic radical, which may be bonded via an unsubstituted or at least mono-substituted alkylene group and/or which may be condensed with an unsubstituted or at least mono-substituted mono- or bicyclic ring system; or a -(CH₂)ₙₚ-NR^{13P}R^{14p} moiety with np = 0, 1, 2, 3, 4 or 5;
R^{4p}, R^{5p}, R^{6p} and R^{7p}, independently of one another, each represent a hydrogen atom; -NO₂; -NH₂; -OH; -CN; -C(=O)-R^{8p}; -O-R^{10p}; -S-R^{11p}; -NH-R^{17p}; -NR^{18p}R^{19p}; a halogen atom; a linear or branched, saturated or unsaturated, unsubstituted or at least mono-substituted aliphatic radical; or a saturated or unsaturated, unsubstituted or at least mono-substituted, optionally at least one heteroatom as a ring member containing cycloaliphatic radical, which may be bonded via a linear or branched alkylene group;
R^{8p} represents a hydrogen atom or a linear or branched, saturated or unsaturated, unsubstituted or at least mono-substituted aliphatic radical;
R^{8p}, R^{17p}, R^{18p} and R^{19p}, independently of one another, each represent a linear or branched, saturated or unsaturated, unsubstituted or at least mono-substituted aliphatic radical; or an unsubstituted or at least mono-substituted aryl or heteroaryl radical, which may be bonded via a linear or branched alkylene, alkenylene or alkinylene group;
R^{9p} represents a linear or branched, saturated or unsaturated, unsubstituted or at least mono-substituted aliphatic radical;
R^{10p} and R^{11p}, independently of one another, each represent a linear or branched, saturated or unsaturated, unsubstituted or at least mono-substituted aliphatic radical; or an unsubstituted or at least mono-substituted aryl or heteroaryl radical, which may be bonded via a linear or branched alkylene group;
R^{13p} and R^{14p}, independently of one another, each represent a hydrogen atom; or a linear or branched, saturated or unsaturated, unsubstituted or at least mono-substituted aliphatic radical;
or
R^{13p} and R^{14p} together with the bridging nitrogen form an unsubstituted or at least mono-substituted, saturated, unsaturated or aromatic heterocyclic ring which may contain at least one further heteroatom as a ring member and/or which may be condensed with an unsubstituted or at least mono-substituted mono- or bicyclic ring system;
A^{p} and B^{p} together with the bridging carbon form an unsubstituted or at least mono-substituted, saturated or unsaturated cycloaliphatic ring which may contain at least one further heteroatom as a ring member and/or which may be condensed with an unsubstituted or at least mono-substituted mono- or bicyclic ring system;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof, respectively.

Preferred compounds of general formula (Ip) are those, wherein
R^{1p} represents a -S(=O)₂-C(H)A^{p}B^{p} moiety;
R^{2p}, R^{3p}, R^{4p} and R^{6p} each represent hydrogen,
R^{3p} represents a -(CH₂)ₙp-NR^{13p}R^{14p} moiety or an unsaturated, optionally at least one nitrogen atom as a ring member containing 5- or 6-membered cycloaliphatic radical, which may be substituted by a methyl group and/or which may be condensed with a 5-membered cycloaliphatic ring,
more preferably R^{3p} represents a -(CH₂)ₙₚNR^{13p}R^{14p} moiety or a moiety selected from the group consisting of 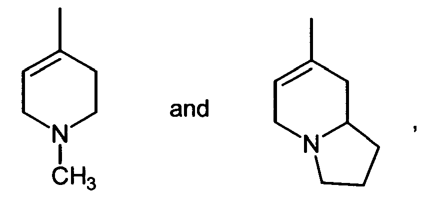 R^{5p} represents H, fluorine, chlorine, nitro or a -NH₂ group,
R^{13p} and R^{14p}, identical or different, each represent methyl, ethyl, n-propyl or isopropyl, more preferably methyl,
or
R^{13p} and R^{14p} together with the bridging nitrogen atom form a 5- or 6-membered heterocyclic ring, more preferably form a pyrrolidine or piperidine ring,
A^{p} and B^{p} together with the carbon atom to which they are bonded form a saturated or unsaturated C₃-C₈ cycloaliphatic ring, more preferably form a cyclohexyl ring,
and
np is 0, 1 or 2;
optionally in form of one of their stereoisomers, preferably enantiomers or diastereomers, their racemate or in form of a mixture of at least two of their stereoisomers, preferably enantiomers or diastereomers, in any mixing ratio, or a salt thereof, preferably a corresponding physiologically acceptable salt thereof or a corresponding solvate thereof.

Particularly preferred compounds of general formula (Ip) are those selected from the group consisting of
[975] 1-Cyclohexanesulfonyl-3-(1-methyl-1,2,3,6-tetrahydropyridine-4-yl)-5-nitro-1H-indole,
[976] 5-Chloro-1-cyclohexanesulfonyl-3-(1-methyl-1,2,3,6-tetrahydropyridine-4-yl)-1H-indole,
[977] 5-Amino-1-cyclohexanesulfonyl-3-(1-methyl-1,2,3,6-tetrahydropyridine-4-yl)-1H-indole and
[978] 1-Cydohexanesulfonyl-5-fluoro-3-(1,2,3,5,8,8a-hexahydro-indolizine-7-yl)-1H-indole hydrochloride
and their corresponding salts and solvates.

The compounds of general formula (Ip) may be prepared according to the disclosure of WO 2005/013974.

In another embodiment of the present invention compounds of general formula (Ib) are selected from the group consisting of compounds of general formula (Iq) 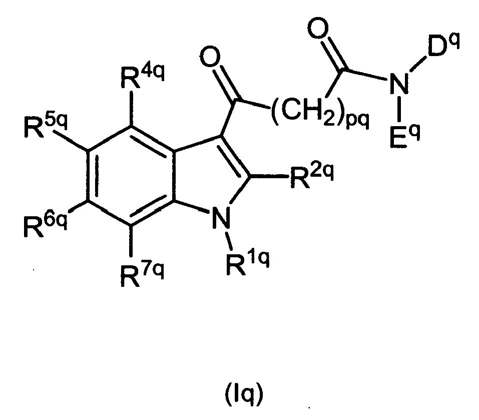 wherein
R^{1q} represents a hydrogen atom; a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical,
which may be bonded via a linear or branched alkylene group and/or which may be condensed with an unsubstituted or at least mono-substituted mono- or bicyclic ring system; an unsubstituted or at least mono-substituted aryl or heteroaryl radical, which may be bonded via a linear or branched alkylene group and/or which may be condensed with an unsubstituted or at least mono-substituted mono- or bicyclic ring system; a -C(=O)-R^{8q} moiety; a -S(=O)₂-R^{9q} moiety;
R^{2q} represents a hydrogen atom; -F; -Cl; -Br; -I; -NO₂; -NH₂; -SH; -OH; -CN; a linear or branched, saturated or unsaturated, unsubstituted or at least mono-substituted, optionally at least one heteroatom as a chain member containing aliphatic radical; a saturated or unsaturated, unsubstituted or at least mono-substituted, optionally at least one heteroatom as a ring member containing cycloaliphatic radical, which may be bonded via a linear or branched alkylene group and/or which may be condensed with an unsubstituted or at least mono-substituted mono- or bicyclic ring system; or an unsubstituted or at least mono-substituted aryl or heteroaryl radical, which may be bonded via a linear or branched alkylene group and/or which may be condensed with an unsubstituted or at least mono-substituted mono- or bicyclic ring system;
R^{4q}, R^{5q}, R^{6q} and R^{7q}, independently of one another, each represent a hydrogen atom; -NO₂; -NH₂; -SH; -OH; -CN; -C(=O)-OH; -C(=O)-H; -S(=O)₂OH; -C(=O)-NH₂;-S(=O)₂-NH₂; -C(=O)-R^{8q}; -S(=O)₂-R^{9q}; -O-R^{10q} ; -S-R^{11q}; -C(=O)-OR^{12q}; -N(R^{15q})-S(=O)₂-R^{16q}; -NH-R^{17q}; -NR^{18q}R^{19q}; -C(=O)-NHR^{20q}, -C(=O)-NR^{21q}R^{22q}; -S(=O)₂-NHR^{23q}; -S(=O)₂-NR^{24q}R^{25q}; -O-C(=O)-R^{26q}; -NH-C(=O)-R^{27q}; -NR^{28q}-C(=O)-R^{29q}; NH-C(=O)-O-R^{30q}; NR^{31q}-C(=O)-O-R^{32q}; -S(=O)₂-O-R^{33q} ; a halogen atom; a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via a linear or branched alkylene group; or an unsubstituted or at least mono-substituted aryl or heteroaryl radical, which may be bonded via a linear or branched alkylene group;
with the proviso that at least one of the substituents R^{4q}, R^{5q}, R^{6q} and R^{7q} represents a -N(R^{15q})-S(=O)₂-R^{16q} moiety;
R^{8q} R^{12q},R^{17q} R^{18q}, R^{19q}, R^{20q}, R^{21q}, R^{22q}, R^{23q}, R^{24q}, R^{25q}, R^{26q}, R^{27q}, R^{28q}, R^{29q}, R^{30q}, R^{31q}, R^{32q} and R^{33q}, independently of one another, each represent a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via a linear or branched alkylene group; or an unsubstituted or at least mono-substituted aryl or heteroaryl radical, which may be bonded via a linear or branched alkylene, alkenylene or alkinylene group;
R^{9q} represents a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via a linear or branched alkylene group and/or which may be condensed with an unsubstituted or at least mono-substituted mono- or bicyclic ring system; or an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
R^{10q} and R^{11q}, independently of one another, each represent a linear or branched, saturated or unsaturated, unsubstituted or at least mono-substituted aliphatic radical; or an unsubstituted or at least mono-substituted aryl or heteroaryl radical, which may be bonded via a linear or branched alkylene group;
R^{15q} represents a hydrogen atom; a linear or branched, saturated or unsaturated, unsubstituted or at least mono-substituted aliphatic radical or a -S(=O)₂-R^{16q} moiety;
R^{16q} represents a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via a linear or branched, unsubstituted or at least mono-substituted alkylene, alkenylene or alkinylene group and/or which may be condensed with an unsubstituted or at least mono-substituted mono- or bicyclic ring system; or an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
D^{q} and E^{q} together with the bridging nitrogen form an unsubstituted or at least mono-substituted, saturated, unsaturated or aromatic heterocyclic ring which may contain at least one further heteroatom as a ring member and/or which may be condensed with an unsubstituted or at least mono-substituted mono- or bicyclic ring system;
or
D^{q} and E^{q}, independently of one another, each represent a hydrogen atom; a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via a linear or branched alkylene group and/or which may be condensed with an unsubstituted or at least mono-substituted mono- or bicyclic ring system; or an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof, respectively.

Preferred compounds of general formula (Iq) are those, wherein
pq is 0,
R^{1q} represents a hydrogen atom,
R^{2q} represents a hydrogen atom,
D^{q} and E^{q}, identical or different, represent an alkyl radical selected from the group consisting of methyl, ethyl, n-propyl, isopropyl, n-butyl, sec-butyl, isobutyl and tert-butyl,
one of the substituents R^{4q}, R^{5q}, R^{6q} and R^{7q} represents an -N(R^{15q})-S(=O)-R^{16q}- moiety while the other three of these substituents each represent a hydrogen atom, R^{15q} represents a hydrogen atom,
R^{16q} represents an aryl or heteroaryl radical selected from the group consisting of phenyl, 1-naphthyl, 2-naphthyl, pyrazolyl, thiophenyl (thiophenyl), benzo[b]-thiophenyl, benzo[b]furanyl, quinolinyl, isoquinolinyl, imidazo[2,1-b]thiazolyl, 2-oxo-2,3-dihydro-benzooxazolyl and 2-oxo-2,3-dihydrobenzo[d]thiazolyl, whereby said aryl or heteroaryl radical may be bonded via a -(CH₂)_{1,2 or 3}- group and/or may be substituted by 1, 2, 3, 4 or 5 substituents independently selected from the group consisting of methyl, ethyl, n-propyl, isopropyl, n-butyl, sec-butyl, isobutyl, tert-butyl, F, Cl, Br, l, -CN, -CF₃, -CF₂H, CFH₂, -C(=O)-O-CH₃, C(=O)-O-CH₂-CH₃, cyclopropyl, cyclobutyl, cyclopentyl, cyclohexyl, phenyl, phenoxy and benzyl;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof, respectively.

Particularly preferred compounds of general formula (Iq) are those selected from the group consisting of
[979] 2-[5-(5-Chloro-3-methyl-benzo[b]thiophene-2-sulfonylamino)-1H-indol-3-yl]-N,N-diethyl-2-oxoacetamide,
[980] N,N-Diethyl-2-[5-(naphthalene-2-sulfonylamino)-1H-indol-3-yl]-2-oxo-acetamide,
[981] N,N-Diethyl-2-[5-(naphthalene-1-sulfonylamino)-1H-indol-3-yl]-2-oxo-acetamide,
[982] 2-[5-(Biphenyl-4-sulfonylaminoyl)-1H-indol-3-yl]-N,N-diethyl-2-oxo-acetamide,
[983] N,N-Diethyl-2-oxo-2-[5-(quinoline-8-sulfonylamino)-1H-indol-3-yl]-acetamide,
[984] N,N-Dimethyl-2-[5-(naphthalene-2-sulfonylamino)-1H-indol-3-yl]-2-oxo-acetamide,
[985] N,N-Dimethyl-2-[5-(naphthalene-1-sulfonylamino)-1H-indol-3-yl]-2-oxo-acetamide,
[986] 2-[5-(5-Chloro-3-methyl-benzo[b]thiophene-2-sulfonylamino)-1H-indol-3-yl]-N,N-dimethyl-2-oxo-acetamide,
[987] 2-[5-(6-Chloro-imidazo[2,1-b]thiazole-5-sulfonylamino)-1H-indol-3-yl]-N,N-diethyl-2-oxo-acetamide,
[988] 2-[5-(6-Chloro-imidazo[2,1-b]thiazole-5-sulfonylamino)-1H-indol-3-yl]-N,N-dimethyl-2-oxo-acetamide,
[989] N,N-Dimethyl-2-[4-(naphthalene-1-sulfonylamino)-1H-indol-3-yl]-2-oxo-acetamide,
[990] 2-[4-(5-Chloro-3-methyl-benzo[b]thiophene-2-sulfonylamino)-1H-indole-3-yl]-N, N-dimethyl-2-oxo-acetamide,
[991] 2-[4-(6-Chloro-imidazo[2,1-b]thiazole-5-sulfonylamino)-1H-indol-3-yl]-N,N-dimethyl-2-oxo-acetamide,
[992] N,N-Dimethyl-2-[5-[(4-fluoro-3-methyl-phenyl)-1-sulfonylamino]-1H-indol-3-yl]-2-oxo-acetamide,
[993] 5-(3-Dimethylaminooxalyl-1H-indol-5-ylsulfamoyl)-3-methyl-benzofuran-2-carboxylic acid ethyl ester,
[994] 2-[5-(Biphenyl-4-sulfonylamino)-1H-indol-3-yl]-N,N-dimethyl-2-oxo-acetamide,
[995] N,N-Dimethyl-2-oxo-2-[5-(2-oxo-2,3-dihydro-benzoxazole-6-sulfonylamino)-1H-indol-3-yl]-acetamide,
[996] N,N-Dimethyl-2-oxo-2-[5-(2-oxo-2,3-dihydrobenzo[d]thiazole-6-sulfonamido)-1H-indol-3-yl]acetamide,
[997] 2-[5-[(4-Cyclohexyl-phenyl)-1-sulfonylamino]-1H-indol-3-yl]-N,N-dimethyl-2-oxo-acetamide,
[998] N,N-Dimethyl-2-[5-[(4-phenoxy-phenyl)-1-sulfonylamino]-1H-indol-3-yl]-2-oxo-acetamide,
[999] 2-(5-(5-chloro-3-methylbenzo[b]thiophene-2-sulfonamido)-2-methyl-1H-indol-3-yl)-N,N-dimethyl-2-oxoacetamide,
[1000] 2-(5-(6-chloroimidazo[2,1-b]thiazole-5-sulfonamido)-2-methyl-1H-indol-3-yl)-N,N-dimethyl-2-oxoacetamide,
[1001] 2-(6-(5-chloro-3-methylbenzo[b]thiophene-2-sulfonamido)-1H-indol-3-yl)-N,N-dimethyl-2-oxoacetamide,
[1002] N,N-dimethyl-2-(6-(naphthalene-3-sulfonamido)-1H-indol-3-yl)-2-oxoacetamide
[1003] 2-(6-(biphenyl-4-sulfonamido)-1H-indol-3-yl)-N,N-dimethyl-2-oxoacetamide,
[1004] N,N-dimethyl-2-(6-(naphthalene-1-sulfonamido)-1H-indol-3-yl)-2-oxoacetamide,
[10055] N,N-dimethyl-2-(6-(2-(naphthalen-1-yl)ethylsulfonamido)-1H-indol-3-yl)-2-oxoacetamide,
[1006] N,N-dimethyl-2-oxo-2-(6-(4-phenoxyphenylsulfonamido)-1H-indol-3-yl)acetamide,
[1007] 2-(6-(3,4-dichlorothiophene-2-sulfonamido)-1H-indol-3-yl)-N,N-dimethyl-2-oxoacetamide,
[1008] 2-(6-(3,5-dichlorophenylsulfonamido)-1H-indol-3-yl)-N,N-dimethyl-2-oxoacetamide,
[1009] 2-(6-(1-chloronaphthalene-6-sulfonamido)-1H-indol-3-yl)-N,N-dimethyl-2-oxoacetamide,
[1010] 2-(6-(6-chloroimidazo[2,1-b]thiazole-5-sulfonamido)-1H-indol-3-yl)-N,N-dimethyl-2-oxoacetamide,
[1011] N,N-diethyl-2-(2-methyl-5-(5-methyl-1-phenyl-1H-pyrazole-4-sulfonamido)-1H-indol-3-yl)-2-oxoacetamide and
[1012] N,N-diethyl-2-(2-methyl-5-(1,3,5-trimethyl-1H-pyrazole-4-sulfonamido)-1H-indol-3-yl)-2-oxoacetamide;
and their corresponding salts and solvates.

The compounds of general formula (Iq) may be prepared according to the disclosure of WO 2006/015867.

In another embodiment of the present invention as component (A) at least one compound is present which is selected from the group consisting of indazolyl- and (2,3)-dihydro-indolyl-derived sulfonamide compounds of general formula (Ic) 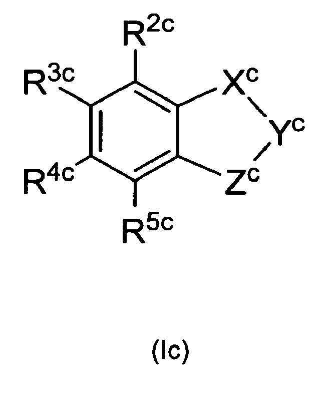 wherein
X^{c}-Y^{c} from left to right represents CR^{1c}=N and Z^{c} is N[(CH_{2c})_{nc}R^{6c}]
or
X^{c}-Y^{c} from left to right represents CR^{7c}=N, Z^{c} is NH, R^{7c} represents the following moiety 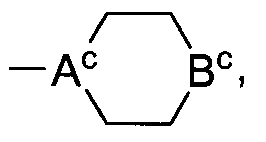 A^{c} represents CH or N and B^{c} represents NR^{8c}, O or S;
X^{c}-Y^{c} from left to right represents C[(CH_{2c})_{nc}R^{9c}]=N and Z^{c} is NR^{10c}
or
X^{c}-Y^{c} represents CH₂-CH₂ and Z^{c} is N[(CH_{2c})_{nc}R^{11c}];
nc is 0, 1, 2, 3 or 4;
R^{1c} represents a hydrogen atom; NO₂; -NH₂; -SH; -OH; -CN; -C(=O)-R^{12c}; -OR^{13c};-SR^{14c}; -F; -Cl, -Br; -l; a linear or branched, saturated or unsaturated, unsubstituted or at least mono-substituted aliphatic radical; or an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
R^{2c}, R^{3c}, R^{4c} and R^{5c}, independently of one another, each represent a hydrogen atom; -NO₂; -NH₂; -SH; -OH; -CN; -C(=O)-H; -C(=O)-R^{12c}; -OR^{13c}; -SR^{14c} -N(R^{15c})-S(=O)₂-R^{16c}; -NH-R^{17c}; -NR^{18c}R^{19c}; -F; -Cl, -Br; -l; a linear or branched, saturated or unsaturated, unsubstituted or at least mono-substituted aliphatic radical; or an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
with the proviso that at least one of the substituents R^{2c}, R^{3c}, R^{4c} and R^{5c} represents a -N(R^{15c})-S(=O)₂-R^{16c} moiety;
R^{6c}, R^{9c} and R^{11c}, independently of one another, each represent a -NR^{20c}R^{21c} radical
or
a saturated or unsaturated, unsubstituted or at least mono-substituted, optionally at least one heteroatom as a ring member containing cycloaliphatic radical, which may be bonded via a linear or branched, unsubstituted or at least mono-substituted alkylene, alkenylene or alkinylene group and/or which may be condensed with an unsubstituted or at least mono-substituted mono- or bicyclic ring system;
R^{8c} represents -C(=O)-R^{22c}; a linear or branched, saturated or unsaturated, unsubstituted or at least mono-substituted aliphatic radical; or an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
R^{10c} represents a linear or branched, saturated or unsaturated, unsubstituted or at least mono-substituted aliphatic radical-; or a -S(=O)₂R^{23c} moiety;
R^{12c}, R^{13c}, R^{14c}, R^{17c}, R^{18c} and R^{19c}, independently of one another, each represent a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via a linear or branched, unsubstituted or at least mono-substituted alkylene, alkenylene or alkinylene group and/or which may be condensed with an unsubstituted or at least mono-substituted mono- or bicyclic ring system; or an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
R^{15c} represents a hydrogen atom; a linear or branched, saturated or unsaturated, unsubstituted or at least mono-substituted aliphatic radical; or a -S(=O)₂-R^{24c} moiety;
R^{16c} and R^{24c}, independently of one another, each represent an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
R^{20c} and R^{21c}, independently of one another, each represent a hydrogen atom; or a linear or branched, saturated or unsaturated, unsubstituted or at least mono-substituted aliphatic radical;
or R^{20c} and R^{21c} together with the bridging nitrogen form an unsubstituted or at least mono-substituted, saturated, unsaturated or aromatic heterocyclic ring which may contain at least one further heteroatom as a ring member and/or which may be condensed with an unsubstituted or at least mono-substituted mono- or bicyclic ring system;
R^{22c} represents a linear or branched, saturated or unsaturated, unsubstituted or at least mono-substituted aliphatic radical; or an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
and
R^{23c} represents a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via a linear or branched, unsubstituted or at least mono-substituted alkylene, alkenylene or alkinylene group and/or which may be condensed with an unsubstituted or at least mono-substituted mono- or bicyclic ring system; or an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof, respectively.

Preferred compounds of general formula (Ic) are selected from the group consisting of compounds of general formula (Ir) 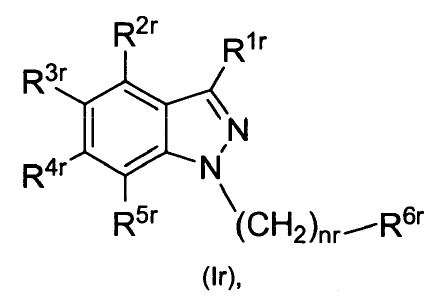 wherein
nr is 0, 1 or 2;
R^{1r} represents a hydrogen atom or an alkyl radical selected from the group consisting of methyl, ethyl, n-propyl, isopropyl, n-butyl, sec-butyl, isobutyl and tert-butyl;
R^{2r}, R^{3r}, R^{4r} and R^{5r}, independent from one another, each represent a hydrogen
atom; -NO₂; -NH₂; -SH; -OH; -CN; -N(R^{15r})-S(=O)₂-R^{16r}; F; Cl; Br; I; or an alkyl radical selected from the group consisting of methyl, ethyl, n-propyl, isopropyl, n-butyl, sec-butyl, isobutyl and tert-butyl;
with the proviso that at least one of the substituents R^{2r}, R^{3r}, R^{4r} and R^{5r} represents a -N(R^{15r})-S(=O)₂-R^{16r} moiety;
R^{6r} represents a -NR^{20r}R^{21r} radical;
R^{15r} represents a hydrogen atom or an alkyl radical selected from the group consisting of methyl, ethyl, n-propyl, isopropyl, n-butyl, sec-butyl, isobutyl and tert-butyl
R^{16r} represents an aryl or heteroaryl radical selected from the group consisting of phenyl, naphthyl, benzo[b]furanyl, benzo[b]thiophenyl and imidazo[2,1-b]thiazolyl, which may be substituted with 1, 2 or 3 substituent(s) independently selected from the group consisting of methyl, ethyl, n-propyl, isopropyl, n-butyl, sec-butyl, isobutyl, tert-butyl, F, Cl, Br, I, -CN, phenyl, phenoxy and benzyl
and
R^{20r} and R^{21r}, independent from one another, each represent an alkyl radical selected from the group consisting of methyl, ethyl, n-propyl, isopropyl, n-butyl, sec-butyl, isobutyl and tert-butyl;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof, respectively.

Particularly preferred compounds of general formula (Ir) are those selected from the group consisting of
[1013] N-(1-(2-(Dimethylamino)ethyl)-1H-indazol-6-yl)napthalene-2-sulfonamide and
[1014] 5-Chloro-N-(1-(2-(dimethylamino)ethyl)-1H-indazol-6-yl)-3-methylbenzo[b]thiophene-2-sulfonamide;
optionally in form of a physiologically acceptable salt thereof, or a corresponding solvate thereof.

Preferred compounds of general formula (Ic) are selected from the group consisting of compounds of general formula (Is) 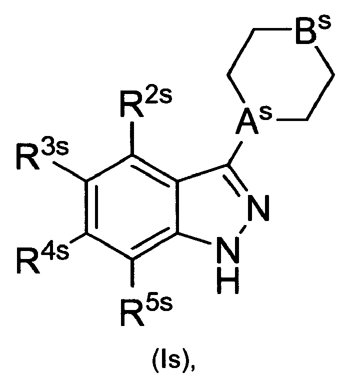 wherein
A^{s} represents CH and B^{s} represents NR^{8s}
or
A^{s} represents N and B^{s} represents NR^{8s}
or
A^{s} represents N and B^{s} represents O
or
A^{s} represents N and B^{s} represents S;
R^{2s}, R^{3s}, R^{4s} and R^{5s}, independent from one another, each represent a hydrogen atom; -NO₂; -NH₂; -SH; -OH; -CN; -N(R^{15s})-S(=O)₂-R^{16s}; F; Cl; Br; I; or an alkyl radical selected from the group consisting of methyl, ethyl, n-propyl, isopropyl, n-butyl, sec-butyl, isobutyl and tert-butyl;
with the proviso that at least one of the substituents R^{2s}, R^{3s}, R^{4s} and R^{5s} represents a -N(R^{15s})-S(=O)₂-R^{16s} moiety;
R^{8s} represents an alkyl radical selected from the group consisting of methyl, ethyl, n-propyl, isopropyl, n-butyl, sec-butyl, isobutyl and tert-butyl;
R^{15s} represents a hydrogen atom or an alkyl radical selected from the group consisting of methyl, ethyl, n-propyl, isopropyl, n-butyl, sec-butyl, isobutyl and tert-butyl
and
R^{16s} represents an aryl or heteroaryl radical selected from the group consisting of phenyl, naphthyl, benzo[b]furanyl, benzo[b]thiophenyl and imidazo[2,1-b]thiazolyl, which may be substituted with 1, 2 or 3 substituent(s) independently selected from the group consisting of methyl, ethyl, n-propyl, isopropyl, n-butyl, sec-butyl, isobutyl, tert-butyl, F, Cl, Br,l, -CN, phenyl, phenoxy and benzyl;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof, respectively.

Particularly preferred compounds of general formula (Is) are those selected from the group consisting of
[1015] Naphthalene-2-sulfonic acid [3-(1-methyl-piperidin-4-yl)-1H-indazol-5-yl]-amide,
[1016] 5-Chloro-3-methyl-benzo[b]thiophene-2-sulfonic acid [3-(1-methyl-piperidin-4-yl)-1H-indazol-5-yl]-amide,
[1017] Naphthalene-1-sulfonic acid [3-(1-methyl-piperidin-4-yl)-1H-indazol-5-yl]-amide,
[1018] 4-Phenylbenzene-4-sulfonic acid [3-(1-methyl-piperidin-4-yl)-1H-indazol-5-yi]-amide,
[1019] N-[3-(1-Methyl-piperidin-4-yl)-1H-indazol-5-yl]-4-phenoxy-benzenesulfonamide
   and
[1020] N-[3-(1-Methyl-piperidin-4-yl)-1H-indazol-5-yl]-benzenesulfonamide;
optionally in form of a physiologically acceptable salt thereof, or a corresponding solvate thereof.

Preferred compounds of general formula (Ic) are selected from the group consisting of compounds of general formula (It) 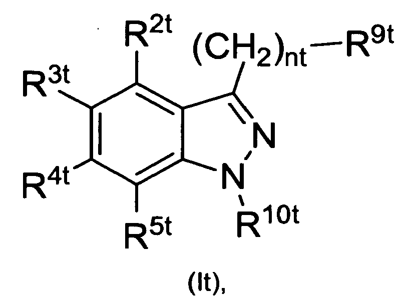 wherein
nt is 0, 1 or 2;
R^{2t}, R^{3t}, R^{4t} and R^{5t}, independent from one another, each represent a hydrogen atom; -NO₂; -NH₂; -SH; -OH; -CN; -N(R^{15t})-S(=O)₂-R^{16t}; F; Cl; Br; I; or an alkyl radical selected from the group consisting of methyl, ethyl, n-propyl, isopropyl, n-butyl, sec-butyl, isobutyl and tert-butyl;
with the proviso that at least one of the substituents R^{2t}, R^{3t}, R^{4t} and R^{5t} represents a -N(R^{15t})-S(=O)₂-R^{16t} moiety;
R^{9t} represents a -NR^{20t}R^{21t} radical;
R^{10t} represents an alkyl radical selected from the group consisting of methyl, ethyl, n-propyl, isopropyl, n-butyl, sec-butyl, isobutyl and tert-butyl or a -S(=O)-R^{23t} moiety;
R^{15t} represents a hydrogen atom or an alkyl radical selected from the group consisting of methyl, ethyl, n-propyl, isopropyl, n-butyl, sec-butyl, isobutyl and tert-butyl
R^{16t} represents an aryl or heteroaryl radical selected from the group consisting of phenyl, naphthyl, benzo[b]furanyl, benzo[b]thiophenyl and imidazo[2,1-b]thiazolyl, which may be substituted with 1, 2 or 3 substituent(s) independently selected from the group consisting of methyl, ethyl, n-propyl, isopropyl, n-butyl, sec-butyl, isobutyl, tert-butyl, F, Cl, Br, I, -CN, phenyl, phenoxy and benzyl.
R^{20t} and R^{21t}, independent from one another, each represent an alkyl radical selected from the group consisting of methyl, ethyl, n-propyl, isopropyl, n-butyl, sec-butyl, isobutyl and tert-butyl;
or
R^{20t} and R^{21t} together with the bridging nitrogen atom form an unsubstituted moiety selected from the group consisting of 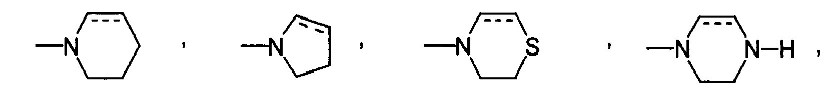 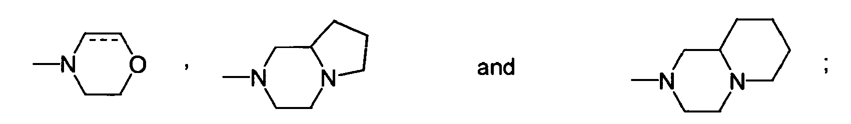 wherein, if present, the dotted line represents an optional chemical bond;
and
R^{23t} represents an alkyl radical selected from the group consisting of methyl, ethyl, n-propyl, isopropyl, n-butyl, sec-butyl, isobutyl and tert-butyl
or an aryl or heteroaryl radical selected from the group consisting of phenyl, naphthyl, benzo[b]furanyl, benzo[b]thiophenyl and imidazo[2,1-b]thiazolyl, which may be substituted with 1, 2 or 3 substituent(s) independently selected from the group
consisting of methyl, ethyl, n-propyl, isopropyl, n-butyl, sec-butyl, isobutyl, tert-butyl, F, Cl, Br, I, -CN, phenyl, phenoxy and benzyl; optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof, respectively.

Preferred compounds of general formula (1c) are selected from the group consisting of compounds of general formula (1u) 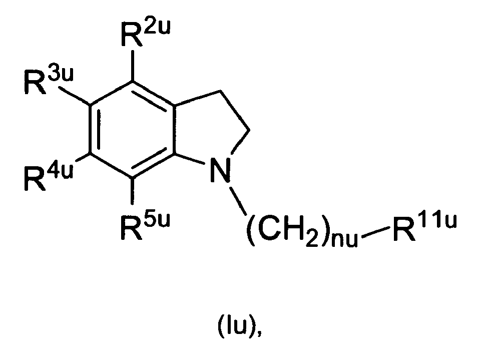 wherein
nu is 0, 1 or 2;
R^{2u}, R^{3u}, R^{4u} and R^{5u}, independent from one another, each represent a hydrogen atom; -NO₂; -NH₂; -SH; -OH; -CN; -C(=O)-H; -C(=O)-R^{12u}; -OR^{13u}; -SR^{14u}; -N(R^{15u})-S(=O)₂-R^{16u}; -NH-R^{17u}; -NR^{18u}R^{19u}; F; Cl; Br; I; or an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂. -SH, -O-CH₃, -O-C₂H₅,-NO₂, -CN and -S-CH₃;
with the proviso that at least one of the substituents R^{2u}, R^{3u}, R^{4u} and R^{5u} represents a -N(R^{15u})-S(=O)₂-R^{16u} moiety;
R^{11u} represents a -NR^{20u}R^{21u} radical
or
a (hetero)cycloaliphatic radical selected from the group consisting of 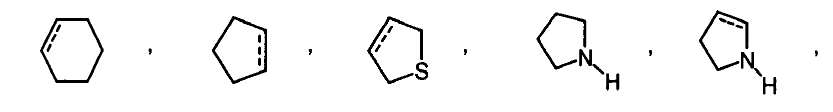 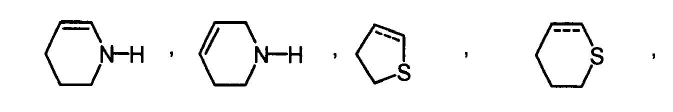 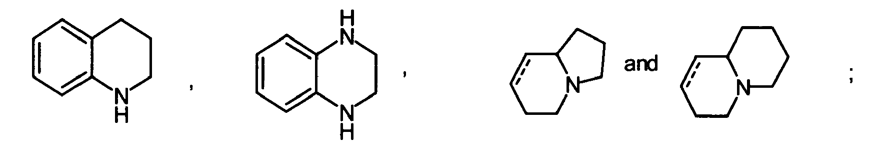 whereby each of these afore mentioned cyclic moieties may be substituted with 1, 2 or 3 substituent(s) independently selected from the group consisting of oxo (=O), thioxo (=S), methyl, ethyl, n-propyl, isopropyl, n-butyl, sec-butyl, isobutyl, tert-butyl,-O-CH₃, -O-C₂H₅, -S-CH₃, -S-C₂H₅, -C(=O)-OH, -C(=O)-O-CH₃, F, Cl, Br, I, -CN,-OCF₃, -SCF₃, -OH, -SH, -NH₂, -NH-CH₃, -NH-C₂H₅, -N(CH₃)₂, -N(C₂H₅)₂, -NO₂,-CHO, -CF₂H and -CFH₂ in any position including the -NH groups and is not bonded via a nitrogen atom and, if present, the dotted line represents an optional chemical bond;
R^{12u}, R^{13u}, R^{14u}, R^{17u}, R^{18u} and R^{19u}, independent from one another, each represent an alkyl radical selected from the group consisting of methyl, ethyl, n-propyl, isopropyl, n-butyl, sec-butyl, isobutyl and tert-butyl; a (hetero)cycloaliphatic radical selected from the group consisting of cyclopentyl, cyclohexyl, pyrrolidinyl, piperidinyl, morpholinyl, thiomorpholinyl and piperazinyl, which may be bonded via a -(CH₂)_{1,2} or ₃- group and which may be substituted with 1, 2 or 3 substituent(s) independently selected from the group consisting of oxo (=O), thioxo (=S), methyl, ethyl, -O-CH₃,-O-C₂H₅, -S-CH₃, -C(=O)-OH, -C(=O)-O-CH₃, -F, Cl, Br, I, -CN, -OCF₃, -SCF₃, -OH,-SH, -NH₂, -NH-CH₃, -NH-C₂H₅, -N(CH₃)₂ and -N(C₂H₅)₂; or an aryl or heteroaryl radical selected from the group consisting of phenyl, naphthyl, pyridinyl, furyl (furanyl), thiophenyl (thiophenyl) and pyrrolyl, which may be bonded via a -(CH₂)₁, 2 or ₃- group and which may be substituted with 1, 2 or 3 substituent(s) independently selected from the group consisting of -CF₃, methyl, ethyl, -O-CH₃, -O-C₂H₅, -O-CH₂-CH₂-CH₃, -S-CH₃, -S-C₂H₅, -C(=O)-OH, -C(=O)-O-CH₃, -C(=O)-O-CH₂-CH₃, F, Cl, Br, I, -CN, -OCF₃, -SCF₃, -OH, -SH, -NH₂, -NH-CH₃, -NH-C₂H₅, -N(CH₃)₂ and -N(C₂H₅)₂;
R^{15u} represents a hydrogen atom; or an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, -NO₂, -CN and -S-CH₃;
R^{16u} represents an aryl or heteroaryl radical selected from the group consisting of phenyl, naphthyl, pyridinyl, furyl (furanyl), thiophenyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, which may be bonded via a -(CH₂)_{1, 2 or 3}- group and which may be substituted with 1, 2 or 3 substituent(s) independently selected from the group consisting of -CF₃, methyl, ethyl, n-propyl, isopropyl, n-butyl, sec-butyl, isobutyl, tert-butyl, -O-CH₃, -O-C₂H₅, -S-CH₃, -S-C₂H₅, F, Cl, Br, I, -CN, -OCF₃, -SCF₃, -OH, -SH,-NH₂, -NH-CH₃, -NH-C₂H₅, -N(CH₃)₂, -N(C₂H₅)₂, -NO₂, phenyl, phenoxy and benzyl;
and
R^{20u} and R^{21u}, independent from one another, each represent a hydrogen atom; or an alkyl radical selected from the group consisting of methyl, ethyl, n-propyl, isopropyl, n-butyl, sec-butyl, isobutyl and tert-butyl;
or
R^{20u} and R^{21u} together with the bridging nitrogen atom form a moiety selected from the group consisting of 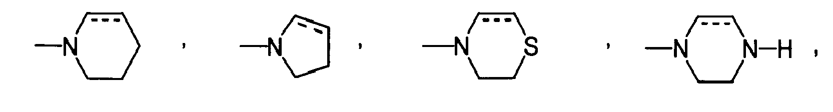 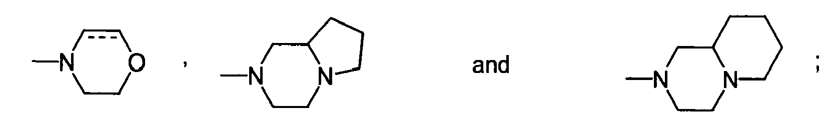 whereby each of these afore mentioned cyclic moieties may be substituted with 1, 2 or 3 substituent(s) independently selected from the group consisting of oxo (=O), thioxo (=S), methyl, ethyl, -O-CH₃, -O-C₂H₅, -S-CH₃, -S-C₂H₅, -C(=O)-OH, -C(=O)-O-CH₃, -C(=O)-O-CH₂-CH₃, F, Cl, Br, I, -CN, -OCF₃, -SCF₃, -OH, -SH, -NH₂, -NH-CH₃,-NH-C₂H₅, -N(CH₃)₂ and -N(C₂H₅)₂ in any position including the -NH groups; and, if present, the dotted line represents an optional chemical bond;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Particularly preferred compounds of general formula (Iu) are those selected from the group consisting of
[1021] N-[1-(2-Dimethylamino)ethyl)-2,3-dihydro-1H-indol-6-yl]-6-chloro-imidazo[2,1-b]thiazol-5-sulfonamide;
optionally in form of a physiologically acceptable salt thereof, or a corresponding solvate thereof.

The compounds of general formulae Ic, Ir, Is, It or Iu given above are prepared by a process, wherein at least one compound of general formula II, 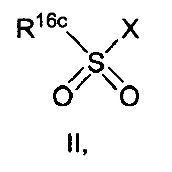 wherein R^{16c} has the meaning given above and X represents a leaving group, preferably a halogen atom, more preferably a chlorine atom, is reacted with at least one compound of general formula III, 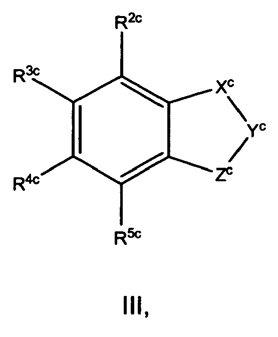 wherein X^{c}, Y^{c}, Z^{c} and R^{2c} to R^{5c} have the meaning given above with the proviso that at least one of the substituents R^{2c}, R^{3c}, R^{4c} and R^{5c}, represents a -NH₂ group, in a suitable reaction medium, preferably in the presence of at least one base, to yield a compound of general formula I, wherein X^{c}, Y^{c}, Z^{c} and R^{2c} to R^{5c} have the meaning given above with the proviso that at least one of the substituents R^{2c}, R^{3c}, R^{4c} and R^{5c} represents a -N(H)-S(=O)₂-R^{16c} group and R^{16c} has the meaning given above, which is optionally purified and/or isolated,
and optionally said compound of general formula I, wherein X^{c}, Y^{c}, Z^{c} and R^{2c} to R^{5c} have the meaning given above with the proviso that at least one of the substituents R^{2c}, R^{3c}, R^{4c} and R^{5c} represents a -N(H)-S(=O)₂-R^{16c} group and R^{16c} has the meaning given above, is reacted with at least one compound of general formula R^{15c}-X, wherein R^{15c} has the meaning given above and X represents a halogen atom, preferably a chlorine atom, in a suitable reaction medium, in the presence of at least one base, preferably at least one base selected from the group consisting of metal hydroxides, metal carbonates, metal alcoxides, preferably sodium methoxide or potassium tert-butoxid, metal hydrides and organometallic compounds, preferably n-butyllithium and tert-butyllithium,
or with at least one compound of general formula X-S(=O)₂-R^{24c}, wherein R^{24c} has the meaning given above and X represents a leaving group, preferably a halogen atom, more preferably a chlorine atom, in a suitable reaction medium, preferably in the presence of at least one base,
to yield a compound of general formula I, wherein X^{c}, Y^{c}, Z^{c} and R^{2c} to R^{5c} have the meaning given above with the proviso that at least one of the substituents R^{2c}, R^{3c}, R^{4c} and R_{C}⁵ represents a -N(R^{15c})-S(=O)₂-R^{16c} group and R^{15c} and R^{16c} have the meaning given above, which is optionally purified and/or isolated.

Suitable reaction media for the reaction between compounds of general formulae II and III include organic solvents, such as dialkyl ether, preferably diethyl ether, or a cyclic ether, preferably tetrahydrofuran or dioxane; or a halogenated hydrocarbon, preferably dichloromethane or chloroform; an alcohol, preferably methanol or ethanol; a dipolar aprotic solvent, preferably acetonitrile, pyridine or dimethylformamide, or any other suitable reaction medium. Of course, mixtures of at least two classes of solvents or of at least two solvents of one class may also be used.

The reaction between compounds of general formulae II and III is preferably carried out in the presence of at least one suitable base, for example, an inorganic base such as a hydroxide or a carbonate of an alkali metal and/or an organic base, preferably triethylamine or pyridine.

The reaction between compounds of general formulae II and III is preferably carried out at a temperature between -10°C and ambient temperature, i.e. approximately 25 °C and the reaction time is preferably between 5 minutes and 24 hours.

Suitable reaction media for the reaction between compounds of general formula I, wherein X^{c}, Y^{c}, Z^{c} and R^{2c} to R^{5c} have the meaning given above with the proviso that at least one of the substituents R^{2c}, R^{3c}, R^{4c} and R^{5c} represents a -N(H)-S(=O)₂R^{16c} group and R^{16c} has the meaning given above and compounds of general formula R^{15c}-X are dialkyl ether, preferably diethyl ether, or a cyclic ether, preferably tetrahydrofuran or dioxane, a hydrocarbon, preferably toluene, an alcohol, preferably methanol or ethanol, a dipolar aprotic solvent, preferably acetonitrile, pyridine or dimethylformamide, or any other suitable reaction medium. Of course, mixtures of at least two classes of solvents or of at least two solvents of one class may also be used.

The afore mentioned reaction is preferably carried out at a temperature between - 10 °C and ambient temperature, i.e. approximately 25 °C and the reaction time is preferably 1 and 24 hours.

Suitable reaction media for the reaction between compounds of general formula I, wherein X^{c}, Y^{c}, Z^{c} and R^{2c} to R^{5c} have the meaning given above with the proviso that at least one of the substituents R^{2c}, R^{3c}, R^{4c} and R^{5c} represents a -N(H)-S(=O)₂-R^{16c} group and R^{16c} has the meaning given above, and compounds of general formula X-S(=O)₂-R^{24c} include organic solvents, such as dialkyl ether, preferably diethyl ether, or a cyclic ether, preferably tetrahydrofuran or dioxane; or a halogenated hydrocarbon, preferably dichloromethane or chloroform; an alcohol, preferably methanol or ethanol; a dipolar aprotic solvent, preferably acetonitrile, pyridine or dimethylformamide, or any other suitable reaction medium. Of course, mixtures of at least two classes of solvents or of at least two solvents of one class may also be used.

The afore mentioned reaction is preferably carried out in the presence of at least one suitable base, for example, an inorganic base such as a hydroxide or a carbonate of an alkali metal and/or an organic base, preferably triethylamine or pyridine.

The afore mentioned reaction is preferably carried out at a temperature between - 10 °C and ambient temperature, i.e. approximately 25 °C and the reaction time is preferably between 5 minutes and 24 hours.

Those skilled in the art understand that the process described above can also be applied to the synthesis of compounds of general formula Ir, Is, It and lu given above.

The compounds of general formula lc, lr, Is, It or lu given above may be purified and/or isolated according to methods well known to those skilled in the art. Preferably, the compounds of general formula Ic, Ir, Is, It or lu may be isolated by evaporating the reaction medium, addition of water and adjusting the pH value to obtain the compound in form of a solid that can be isolated by filtration, or by extraction with a solvent that is not miscible with water such as chloroform and purification by chromatography or recrystallisation from a suitable solvent.

The compounds of general formula II are commercially available or may be prepared according to methods well known in the art, for example, analogous to the methods described in the bibliography of E.E. Gilbert, Synthesis, 1969, 1, 3. The respective part of the literature description cited above is hereby incorporated by reference and forms part of the disclosure.

The compounds of general formula III are commercially available or may also be prepared according to standard methods known in the prior art, for example by methods similar to those described in the literature: Savitskaya, N. V. et al. Synthesis of 5-amino-3-(b-aminoethyl)indazole. Zhumal Obshchei Khimii (1961), 31 1924-1926; Zhang, Han-Cheng et al. Discovery and Optimization of a Novel Series of Thrombin Receptor (PAR-1) Antagonists: Potent, Selective Peptide Mimetics Based on Indole and Indazole Templates. Journal of Medicinal Chemistry (2001), 44(7), 1021-1024; Ono, Shinichiro et al. Preparation of piperidine derivatives as muscarinic receptors stimulator for treatment of schizophrenia. WO 2004069828 A1; Wrzeciono, U. et al. Synthesis and antiinflammatory activity of some indazole derivatives. Part 36. Azoles. Pharmazie (1993), 48(8); 582-584.; Filla, S. A. et al. Preparation o 3-(1-methylpiperidin-4-yl)-1H-indoles and 3-(1-methylpiperidin-4-yl)4-aza-1H-indoles as 5-HT1F agonist. WO 20001487; Dumas, J. et al. Preparation of bicyclic (hetero)aryl- and pyridine-containing diaryl ureas as Raf kinase and angiogenesis inhibitors useful in the treatment of cancer and other disorders. WO 200478748; Mueller, S. G. et al. Preparation of ethynylpyridines and related compounds as melanin-concentrating hormone receptor (MCH-1) antagonist for the treatment of metabolic disorders. WO 200439780; Maeno, K. Preparation of aminoalkylindazole derivatives as 5-HT2c receptor agonists. WO 98/30548; Zhao, E.-C. et al. Synthesis of dialkylaminoalkyl derivatives of indazole. Zhurnal Obshchei Khimii (1959), 29, 1012-1020. Ham, P. et al. Preparation of N-heteroaryl-4'-oxadiazolylbiphenylcarboxamides as 5HT1 D antagonists. WO 9532967A1; Stenkamp, D. et al. Preparation of arylamides as melanin concentrating hormone (MCH) receptor antagonists. WO 200403974.

The compounds of general formula Ic, Ir, Is, It or lu given above may be purified and/or isolated according to methods well known to those skilled in the art. Preferably, the compounds of general formula Ic, Ir, Is, It or lu may be isolated by evaporating the reaction medium, addition of water and then adjusting the pH value to obtain the compound in form of a solid that can be isolated by filtration, or by extraction with a solvent that is not miscible with water such as chloroform and purified by chromatography or recrystallisation from a suitable solvent.

During some synthetic reactions described above or while preparing the compounds of general formulae Ic, Ir, Is, It, lu, Il and Il the protection of sensitive or reactive groups may be necessary and/or desirable. This can be performed by using conventional protective groups like those described in Protective groups in Organic Chemistry, ed. J. F. W. McOmie, Plenum Press, 1973; T.W. Greene & P.G.M. Wuts and Protective Groups in Organic Chemistry, John Wiley & sons, 1991. The respective parts of the description is hereby incorporated by reference and forms part of the disclosure. The protective groups may be eliminated when convenient by means well-known to those skilled in the art.

If the substituted indazolyl sulfonamide or 2,3-dihydro-indolyl sulfonamide compounds of general formula Ic are obtained in form of a mixture of stereoisomers, particularly enantiomers or diastereomers, said mixtures may be separated by standard procedures known to those skilled in the art, e.g. chromatographic methods or crystallization with chiral reagents.

The substituted indazolyl sulfonamide or 2,3-dihydro-indolyl sulfonamide compounds of general formula Ic and in each case stereoisomers thereof may be obtained in form of a corresponding salt according to methods well known to those skilled in the art, e.g. by reacting said compound with at least one inorganic and/or organic acid, preferably in a suitable reaction medium. Suitable reaction media include, for example, any of the ones given above. Suitable inorganic acids include but are not limited to hydrochloric acid, hydrobromic acid, phosphoric acid, sulfuric acid, nitric acid, suitable organic acids include but are not limited to citric acid, maleic acid, fumaric acid, tartaric acid, or derivatives thereof, p-toluenesulfonic acid, methanesulfonic acid or camphersulfonic acid.

In another embodiment of the present invention as component (A) at least one compound is present which is selected from the group consisting of phenyl-piperazine-derived compounds of general formula (Id) 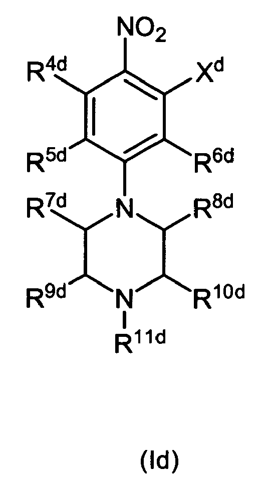 wherein
X^{d} represents a -NR^{1d}R^{2d} moiety or a -OR^{3d} moiety;
R^{1d} represents a linear or branched, saturated or unsaturated, unsubstituted or at least mono-substituted aliphatic radical;
an unsubstituted or at least mono-substituted radical selected from the group consisting of adamantyl, bicyclo[2.2.1]heptyl and bicyclo[3.1.1]heptyl, which may be bonded via a linear or branched, unsubstituted or at least mono-substituted alkylene, alkenylene or alkinylene group which may contain 1, 2 or 3 heteroatom(s) independently selected from the group consisting of nitrogen, oxygen and sulfur as chain member(s);
a saturated or unsaturated, unsubstituted or at least mono-substituted, optionally at least one heteroatom as a ring member containing cycloaliphatic radical, which may be bonded via a linear or branched, unsubstituted or at least mono-substituted alkylene, alkenylene or alkinylene group and/or which may be condensed with an unsubstituted or at least mono-substituted mono- or bicyclic ring system;
or an unsubstituted or at least mono-substituted aryl or heteroaryl radical, which may be condensed with an unsubstituted or at least mono-substituted mono- or bicyclic ring system and/or which may be bonded via a linear or branched, unsubstituted or at least mono-substituted alkylene, alkenylene or alkinylene group which may contain 1, 2 or 3 heteroatom(s) independently selected from the group consisting of nitrogen, oxygen and sulfur as chain member(s);
or a -C(=O)-R^{12d} moiety;
R^{2d} represents a hydrogen atom or a linear or branched, saturated or unsaturated, unsubstituted or at least mono-substituted aliphatic radical;
or
R^{1d} and R^{2d} together with the bridging nitrogen form an optionally at
least mono-substituted, saturated, unsaturated or aromatic heterocyclic ring which may contain at least one further heteroatom as a ring member and/or which may be condensed with an unsubstituted or at least mono-substituted mono- or bicyclic ring system;
R^{3d} represents or an unsubstituted or at least mono-substituted aryl or heteroaryl radical, which may be condensed with an unsubstituted or at least mono-substituted mono- or bicyclic ring system and/or which may be bonded via a linear or branched, unsubstituted or at least mono-substituted alkylene, alkenylene or alkinylene group;
R^{4d}, R^{5d} and R^{6d}, independently of one another, each represent a hydrogen atom or a halogen atom;
or
R^{4d} and R^{5d} together with the bridging carbon atoms form an unsubstituted 5- or 6-membered heterocyclic ring which contains 1, 2 or 3 heteroatom(s) independently selected from the group consisting of nitrogen, oxygen and sulfur as ring member(s) and which together with the phenyl ring which it is fused with forms a 9- or 10-membered bicyclic aromatic ring system;
R^{7d} and R^{8d}, independently of one another, each represent a hydrogen atom or a linear or branched, saturated or unsaturated, unsubstituted or at least mono-substituted aliphatic radical;
R^{9d} and R^{10d}, independently of one another, each represent a hydrogen atom or a linear or branched, saturated or unsaturated, unsubstituted or at least mono-substituted aliphatic radical;
R^{11d} represents a hydrogen atom; or a linear or branched, saturated or unsaturated, unsubstituted or at least mono-substituted aliphatic radical, which may contain 1, 2 or 3 heteroatom(s) independently selected from the group consisting of nitrogen, oxygen and sulfur as chain member(s);
or an unsubstituted or at least mono-substituted aryl or heteroaryl radical, which may be condensed with an unsubstituted or at least mono-substituted mono- or bicyclic ring system and/or which may be bonded via a linear or branched, unsubstituted or at least mono-substituted alkylene, alkenylene or alkinylene group;
-a -C(=O)-R^{13d} moiety or a -S(=O)₂-R^{14d} moiety;
R^{12d} represents a linear or branched, saturated or unsaturated, unsubstituted or at least mono-substituted aliphatic radical;
or an unsubstituted or at least mono-substituted aryl or heteroaryl radical, which may be condensed with an unsubstituted or at least mono-substituted mono- or bicyclic ring system and/or which may be bonded via a linear or branched, unsubstituted or at least mono-substituted alkylene, alkenylene or alkinylene group;
and
R^{13d} and R^{14d}, independently of one another, each represent a linear or branched, saturated or unsaturated, unsubstituted or at least mono-substituted aliphatic radical; or an unsubstituted or at least mono-substituted aryl or heteroaryl radical, which may be condensed with an unsubstituted or at least mono-substituted mono- or bicyclic ring system and/or which may be bonded via a linear or branched, unsubstituted or at least mono-substituted alkylene, alkenylene or alkinylene group which may contain 1, 2 or 3 heteroatom(s) independently selected from the group consisting of nitrogen, oxygen and sulfur as chain member(s);
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof, respectively.

Preferred compounds of general formula (Id) are those, wherein
X^{d} represents a -NR^{1d}R^{2d} moiety or a -OR^{3d} moiety;
R^{1d} represents an alkyl radical selected from the group consisting of -CH₂-CH₂-OH and -CH₂-CH₂-CH₂-OH;
an unsubstituted adamantyl radical;
an unsubstituted phenyl or pyrrolyl radical;
an unsubstituted napthyl radical which is bonded via an alkylene group selected form the group consisting of -CH₂-, -CH(CH₃)-, -CH₂-CH₂-, -CH₂-CH₂-CH₂- and -CH₂-CH₂-O-;
a phenyl radical which may be substituted with 1, 2 or 3 substituent(s) independently selected from the group consisting of methyl, ethyl, tert-butyl, methoxy, F and Cl and said phenyl radical is bonded via an alkylene group selected form the group consisting of -CH₂-, -CH(CH₃)-, -CH(Phenyl)-, -CH₂-CH₂-, -CH₂-CH₂-CH₂- and -CH₂-CH₂-O-;
a heteroaryl radical selected from the group consisting of pyridinyl, furanyl and pyrrolyl, whereby said pyridinyl, furanyl or pyrrolyl radical may be substituted with 1, 2 or 3 substituent(s) independently selected from the group consisting of methyl, ethyl, tert-butyl, methoxy, F and CI and said pyridinyl, furanyl or pyrrolyl radical is bonded via an alkylene group selected form the group consisting of -CH₂-, -CH(CH₃)-, -CH₂-CH₂, -CH₂-CH₂-CH₂- and -CH₂-CH₂-O-;
or a -C(=O)-R^{12d} moiety;
R^{2d} represents a hydrogen atom or a methyl radical;
or
R^{1d} and R^{2d} together with the bridging nitrogen atom form a moiety selected from the group consisting of 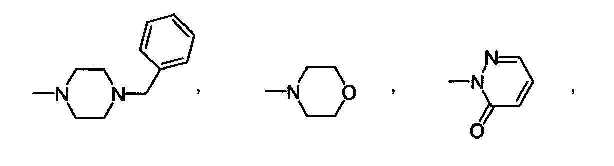 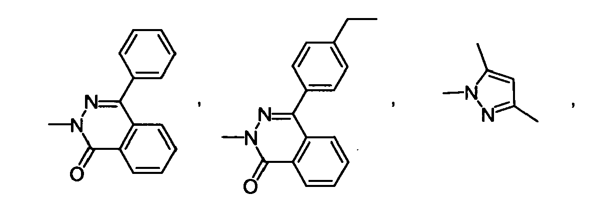 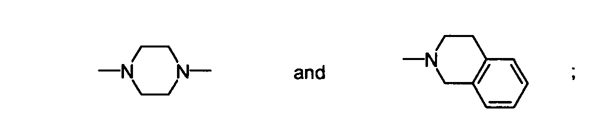 R^{3d} represents an unsubstituted phenyl radical;
R^{4d}, R^{5d} and R^{6d}, identical or different, each represent a hydrogen atom or a fluorine atom;
or
R^{4d} and R^{5d} together with the bridging carbon atoms form the following moiety, 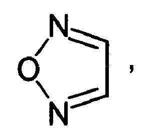 which together with the phenyl ring which it is fused with forms the following substituted bicyclic aromatic ring system 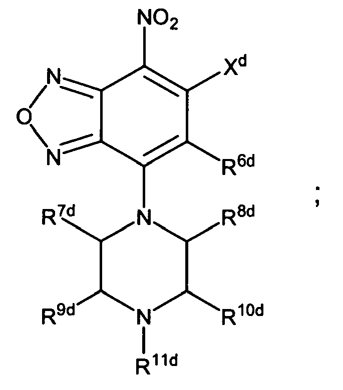 R^{7d} and R^{8d} each represent a hydrogen atom;
R^{9d} and R^{10d}, identical or different, each represent a hydrogen atom or a methyl radical;
R^{11d} represents a hydrogen atom;
an alkyl radical selected from the group consisting of methyl, n-butyl and -CH₂-CH₂-OH;
an unsubstituted phenyl or pyridinyl radical whereby said phenyl or pyridinyl radical may be bonded via a -(CH₂)- group;
a -C(=O)-R^{12d} moiety or a -S(=O)₂-R^{13d} moiety;
R^{12d} represents a phenyl or a thiophenyl radical whereby said phenyl or thiophenyl radical may be substituted with 1, 2 or 3 substituent(s) selected from the group consisting of methyl and chlorine;
R^{13d} represents a methyl radical or a phenyl or a thiophenyl radical whereby said phenyl or thiophenyl radical may be substituted with 1, 2 or 3 substituent(s) selected from the group consisting of methyl and chlorine
and
R^{14d} represents a methyl radical or a phenyl radical which may be substituted with 1, 2 or 3 methyl radical(s);
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Particularly preferred compounds of general formula (Id) are those selected from the group consisting of
[1022] [5-(4-Methyl-piperazin-1-yl)-2-nitro-phenyl]-(2-pyrrol-1-yl-ethyl)-amine,
[1023] (4-Fluoro-benzyl)-[5-(4-methyl-piperazin-1-yl)-2-nitro-phenyl]-amine,
[1024] N-[5-(4-Methyl-piperazin-1-yl)-2-nitro-phenyl]-benzamide,
[1025] N-Methyl-N-[5-(4-methyl-piperazin-1-yl)-2-nitro-phenyl]-benzamide,
[1026] [5-(4-Methyl-piperazin-1-yl)-2-nitro-phenyl]-pyrrol-1-yl-amine,
[1027] 2-[5-(4-Methyl-piperazin-1-yl)-2-nitro-phenyl]-2H-pyridazin-3-one,
[1028] 1-Methyl-4-(4-nitro-3-phenoxy-phenyl)-piperazine,
[1029] Benzyl-[5-(4-butyl-piperazin-1-yl)-2-nitro-phenyl]-amine,
[1030] Benzyl-[2-nitro-5-(4-pyridin-2-yl-piperazin-1-yl)-phenyl]-amine and
[1031] Benzyl-[2-nitro-5-(4-phenyl-2-yl-piperazin-1-yl)-phenyl]-amine;
[1032] Furan-2-ylmethyl-[5-(4-methyl-piperazin-1-yl)-2-nitro-phenyl]-amine,
[1033] 2-[4-(4-Nitro-3-phenethylamino-phenyl)-piperazin-1-yl]-ethanol,
[1034] (2-Nitro-5-piperazin-1-yl-phenyl)-(2-o-tolyloxy-ethyl)-amine
[1035] 2-[4-(3-Benzylamino-4-nitro-phenyl)-piperazin-1-yl]-ethanol,
[1036] 4-[5-(4-Methyl-piperazin-1-yl)-2-nitro-phenyl]-morpholine,
[1037] 2-[5-(4-Methyl-piperazin-1-yl)-2-nitro-phenyl]-4-phenyl-2H-phthalazin-1-one,
[1038] [2-(4-Chloro-phenoxy)-ethyl]-[5-(3,5-dimethyl-piperazin-1-yl)-2-nitro-phenyl]-amine,
[1039] 2-[4-[3-(Benzhydryl-amino)-4-nitro-phenyl]-piperazin-1-yl]-ethanol,
[1040] 4-[4-Fluoro-5-(4-methyl-piperazin-1-yl)-2-nitro-phenyl]-morpholine,
[1041] 2-[2-Nitro-5-[4-(toluene-4-sulfonyl)-piperazin-1-yl]-phenyl]-1,2,3,4-tetrahydro-isoquinoline,
[1042] 1-[3-(3,5-Dimethyl-pyrazol-1-yl)-4-nitro-phenyl]-4-methyl-piperazine,
[1043] Benzyl-(2-nitro-5-piperazin-1-yl-phenyl)-amine,
[1044] [5-(4-Methyl-piperazin-1-yl)-2-nitro-phenyl]-phenethyl-amine,
[1045] [5-(4-Methyl-piperazin-1-yl)-2-nitro-phenyl]-pyridin-3-ylmethyl-amine,
[1046] (3-Chloro-phenyl)-[4-[3-[(furanyl-2-ylmethyl)-amino]-4-nitro-phenyl]-piperazin-1-yl]-methanone,
[1047] (2-Nitro-5-piperazin-1-yl-phenyl)-pyridin-3-ylmethyl-amine,
[1048] 1-Benzyl-4-(2-nitro-5-piperazin-1-yl-phenyl)-piperazine,
[1049] Furan-2-ylmethyl-[5-( 4-methanesulfonyl-piperazin-1-yl)-2-nitro-phenyl]-amine,
[1050] Benzhydryl-[5-(4-methyl-piperazin-1-yl)-2-nitro-phenyl]-amine,
[1051] (2-Nitro-5-piperazin-1 -yl-phenyl)-(2-phenoxy-ethyl)-amine,
[1052] 2-[5-(4-Methyl-piperazin-1-yl)-2-nitro-phenyl]-4-phenyl-2H-phthalazin-1-one,
[1053] 1-[3-(3,5-Dimethyl-pyrazol-1-yl)-4-nitro-phenyl]-piperazine,
[1054] (2-Nitro-5-piperazin-1-yl-phenyl)-phenethyl-amine,
[1055] [5-(4-Benzenesu)fonyl-piperazin-1-yl)-2-nitro-phenyl]-furan-2-ylmethyl-amine,
[1056] [2-(3,4-Dimethoxy-phenyl)-ethyl]-(2-nitro-5-piperazin-1-yl-phenyl)-amine,
[1057] [4-[3-[(Furan-2-ylmethyl)-amino]-4-nitro-phenyl]-piperazin-1-yl]-m-tolyl-methanone,
[1058] [4-[3-[(Furan-2-ylmethyl)-amino]-4-nitro-phenyl]-piperazin-1-yl]-phenyl-methanone,
[1059] [4-[3-(3,5-Dimethyl-pyrazol-1-yl)-4-nitro-phenyl]-piperazin-1-yl]-thiophen-2-yl-methanone,
[1060] 4-(4-Ethyl-phenyl)-2-[5-[4-methyl-piperazin-1-yl)-2-nitro-phenyl]-2H-phthalalazin-1-one,
[1061] 3-[7-(4-Methyl-piperazin-1-yl)-4-nitro-benzo[1,2,5]oxadiazol-5-ylamino]-propan-1-ol,
[1062] 3-[4-Nitro-7-(4-phenyl-piperazin-1-yl)-benzo[1,2,5]oxadiazol-5-ylamino]-ethan-1-ol,
[1063] 3-[4-Nitro-7-(4-pyridin-piperazin-1-yl)-benzo[1,2,5]oxadiazol-5-ylamino]-propan-1-ol,
[1064] [2-(3,4-Dimethoxy-phenyl)-ethyl]-[5-(4-methyl-piperazin-1-yl)-2-nitro-phenyl]-amine,
[1065] [2-(3,4-Dimethyl-phenyl)-ethyl]-[5-(4-methyl-piperazin-1-yl)-2-nitro-phenyl]-amine,
[1066] [2-(4-tert-Butyl-phenoxyl-ethyl]-[5-(4-methyl-piperazin-1-yl)-2-nitro-phenyl]-amine,
[1067][2-(4-Methoxy-phenoxy)-ethyl]-[5-(4-methyl-piperazin-1-yl)-2-nitro-phenyl]-amine,
[1068] [5-(4-Methyl-piperazin-1-yl)-2-nitro-phenyl]-(2-m-tolyloxy-ethyl)-amine,
[1069] [2-(4-Chloro-phenoxy)-ethyl]-[5-(4-methyl-piperazin-1-yl)-2-nitro-phenyl]-amine,
[1070](2-Nitro-5-piperazin-1-yl-phenyl)-(1-phenyl-ethyl)-amine,
[1071] [2-(3-Methoxy-phenoxy)-ethyl]-[5-(4-methyl-piperazin-1-yl)-2-nitro-phenyl]-amine,
[1072] [2-(2-Methoxy-phenoxy)-ethyl]-[5-(4-methyl-piperazin-1-yl)-2-nitro-phenyl]-amine,
[1073] (4-Chloro-benzyl)-(2-nitro-5-piperazin-1-yl-phenyl)-amine,
[1074] Benzyl-[5-(4-benzyl-piperazin-1-yl)-2-nitro-phenyl]-amine,
[1075] Benzyl-[5-(4-methyl-piperazin-1-yl)-2-nitro-phenyl]-amine,
[1076] [5-(4-Methyl-piperazin-1-yl)-2-nitro-phenyl]-(2-o-tolyloxy-ethyl)-amine,
[1077] (4-Chloro-benzyl)-[5-(4-methyl-piperazin-1-yl)-2-nitro-phenyl]-amine,
[1078] Furan-2-ylmethyl-(2-nitro-5-piperazin-1-yl-phenyl)-amine,
[1079] [2-(4-Chloro-phenoxy)-ethyl]-(2-nitro-5-piperazin-1-yl-phenyl)-amine,
[1080] [5-(4-Methyl-piperazin-1-yl)-2-nitro-phenyl]-(1-phenyl-ethyl)-amine
[1081] 1-[4-(3-Benzylamino-4-nitro-phenyl)-piperazin-1-yl]-ethanone,
[1082] 2-[4-[3-(4-Methylpiperazin-1-yl)-4-nitrophenyl]piperazin-1-yl]ethanol,
[1083] 2-[4-[3-[2-(Naphthalen-2-yloxy)ethylamino]-4-nitrophenyl]piperazin-1-yl]ethanol,
[1084]2-[4-(3-{[1-(1-Adamantyl)ethyl]amino}-4-nitrophenyl)piperazin-1-yl]ethanol and
[1085] 2-[4-[3-(3,4-Dimethoxyphenethylamino)-4-nitrophenyl]piperazin-1-yl]ethanol;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

The compounds of general formula ld are prepared by a process, wherein at least one nitrobenzene compound of general formula II, 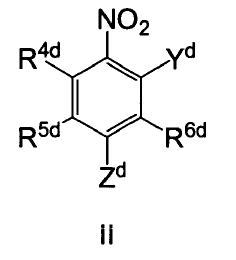 wherein R^{4d} to R^{6d} have any of the above given meanings, Y^{d} represents a chlorine atom, and Z^{d} represents a bromine or iodine atom; is reacted with at least one compound of general formula III, 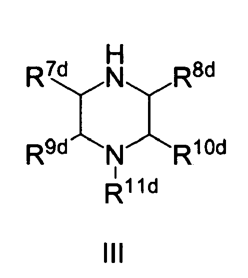 wherein R^{7d} to R^{11d} have any of the above given meaningsin a suitable reaction medium, preferably in at least an organic solvent, more preferably in at least an organic solvent selected from the group consisting of tetrahydrofuran, toluene and dioxane, preferably in the presence of at least one catalyst, more preferably in the presence of at least a palladium source, even more preferably in the presence of PdCl₂(dppf) wherein dppf is 1,1-bis(diphenylphosphino)-ferrocene, and/or at least one auxiliary agent, preferably 1,1-bis(diphenylphosphino)-ferrocene, and/or at least one base, preferably sodium *tert*-pentoxide, to yield a compound of general formula IV, 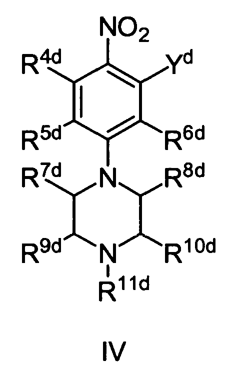 wherein R^{4d} to R^{11d} have any of the above given meanings and Y^{d} represents a chlorine atom; which is optionally purified and/or isolated, and the compound of general formula IV is reacted with at least one compound of general formula V, 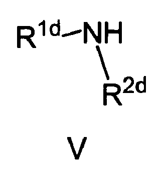 wherein R^{1d} and R^{2d} have any of the above given meanings, in a suitable reaction medium, preferably in at least an organic solvent, more preferably in at least an organic solvent selected from the group consisting of toluene or dimethoxyethane, preferably in the presence of at least one catalyst, more preferably in the presence of at least a palladium source, even more preferably in the presence of at least a palladium source selected from the group consisting of Pd(OAc)₂, wherein OAc is acetate, and Pd₂dba₃, wherein dba is dibenzylidene acetone, and/or at least one auxiliary agent, preferably (biph)P(*t*Bu)₂, wherein biph is biphenyl and *t*Bu is *tert-*butyl, and/or at least one base, preferably at least one base selected from the group consisting of K₃PO₄ and sodium *tert*-pentoxide to yield a compound of general formula VI, 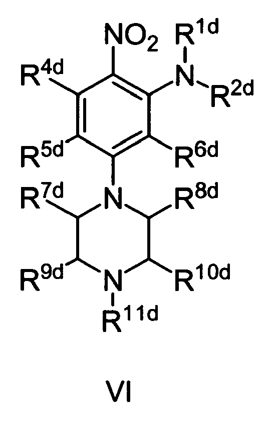 wherein R^{1d}, R^{2d} and R^{4d} to R^{11d} have any of the above given meanings which is optionally purified and/or isolated.

The compounds of general formula Id are prepared by a process, wherein at least one nitrobenzene compound of general formula VII, 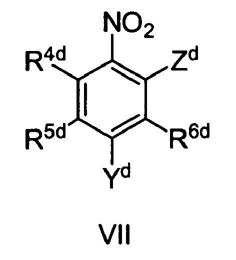 wherein R^{4d} to R^{6d} have any of the above given meanings, Z^{d} represents a bromine or iodine atom, and Y^{d} represents a chlorine atom, is reacted with at least one compound of general formula V, 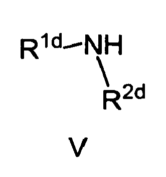 wherein R^{1d} and R^{2d} have any of the above given meaningsin a suitable reaction medium, preferably in at least an organic solvent, more preferably in at least an organic solvent selected from the group consisting of toluene and dioxane, preferably in the presence of at least one catalyst, more preferably in the presence of at least a palladium and/or copper source, even more preferably in the presence of at least a palladium and/or copper source selected from the group consisting of Pd(OAc)₂, wherein OAc is acetate, Pd₂dba₃, wherein dba is dibenzylidene acetone, and copper(I)iodide, and/or at least one auxiliary agent, preferably at least one auxiliary agent selected from the group consisting of 4,5-bis(diphenylphosphino)-9,9-dimethylxanthene (Xantphos), 1,1-bis(diphenylphosphino-ferrocene and P(*t*Bu)₃, wherein *t*Bu is tert-Butyl, and/or at least one base, preferably at least one base selected from the group consisting of K₃PO₄, Cs₂CO₃ and trans-1,2-diamino-methylcyclohexane, to yield a compound of general formula VIII, 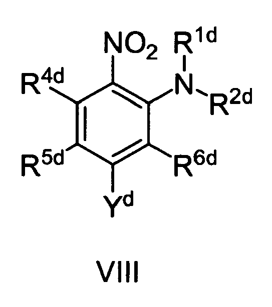 wherein R^{1d}, R^{2d} and R^{4d} to R^{6d} have any of the above given meanings and Y^{d} represents a chlorine atom; which is optionally purified and/or isolated, and the compound of general formula VIII is reacted with at least one compound of general formula III, 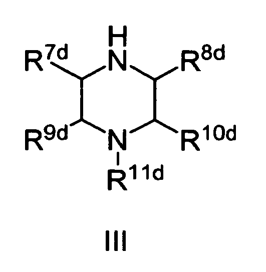 wherein R^{7d} to R^{11d} have any of the above given meanings, in a suitable reaction medium, preferably in at least an organic solvent, more preferably in at least an organic solvent selected from the group consisting of toluene, tetrahydrofuran and dimethoxyethane, preferably in the presence of at least one catalyst, more preferably in the presence of at least a palladium source, even more preferably in the presence of at least a palladium source selected from the group consisting of Pd(OAc)₂, wherein OAc is acetate, and Pd₂dba₃, wherein dba is dibenzylidene acetone, and/or at least one auxiliary agent, preferably (biph)P(tBu)₂, wherein biph is biphenyl and tBu is tert-butyl, and/or at least one base, preferably at least one base selected from the group consisting of K3PO₄ or sodium *tert*-pentoxide, to yield a compound of general formula VI, 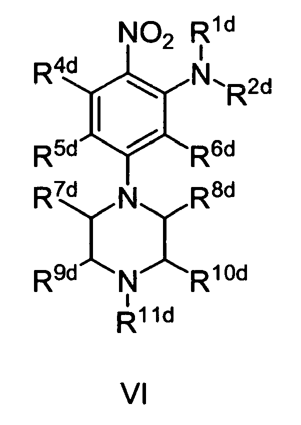 wherein R^{1d}, R^{2d} and R^{4d} to R^{11d} have any of the above given meanings, which is optionally purified and/or isolated.

The compounds of general formula ld are prepared by a process, wherein at least one nitrobenzene compound of general formula II, 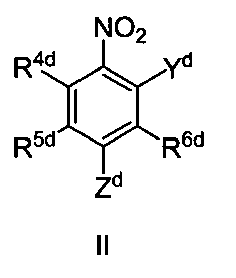 wherein R^{4d} to R^{6d} have any of the above given meanings, Y^{d} represents a chlorine atom, and Z^{d} represents a bromine or iodine atom; is reacted with at least one compound of general formula III, 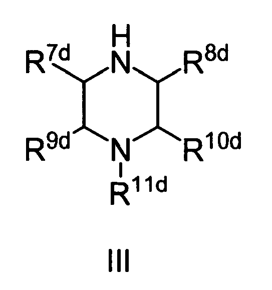 wherein R^{7d} to R^{11d} have any of the above given meanings, in a suitable reaction medium, preferably in at least an organic solvent, more preferably in at least an organic solvent selected from the group consisting of tetrahydrofuran or dioxane, preferably in the presence of at least one catalyst, more preferably in the presence of at least a palladium source, even more preferably in the presence of PdCl₂(dppf), wherein dppf is 1,1-bis(diphenylphosphino)-ferrocene, and/or at least one auxiliary agent, preferably 1,1-bis(diphenylphosphino)-ferrocene, and/or at least one base, preferably sodium *tert-pentoxide,* to yield a compound of general formula IV, 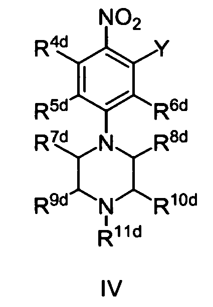 wherein R^{4d} to R^{11d} have any of the above given meanings and Y^{d} represents a chlorine atom; which is optionally purified and/or isolated, and the compound of general formula IV is reacted with at least one compound of general formula IX, 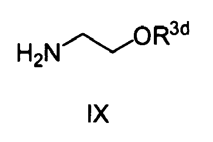 wherein R^{3d} has any of the above given meanings, in a suitable reaction medium, preferably in at least an organic solvent, more preferably in at least an organic solvent selected from the group consisting of toluene or dimethoxyethane, preferably in the presence of at least one catalyst, more preferably in the presence of at least a palladium source, even more preferably in the presence of at least a palladium source selected from the group consisting of Pd(OAc)₂, wherein OAc is acetate, and Pd₂dba₃, wherein dba is dibenzylidene acetone, and/or at least one auxiliary agent, preferably (biph)P(*t*Bu)₂, wherein biph is biphenyl and *t*Bu is *tert-butyl,* and/or at least one base, preferably at least one base selected from the group consisting of K₃PO₄ and sodium *tert*-pentoxideto yield a compound of general formula X, 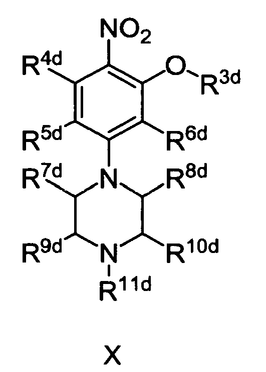 wherein R^{3d} to R^{11d} have any of the above given meanings, which is optionally purified and/or isolated.

Suitable reaction media include organic solvents, such as dialkyl ether, preferably diethyl ether and dimethoxyethane, or a cyclic ether, preferably tetrahydrofuran or dioxane; or a halogenated hydrocarbon, preferably dichloromethane or chloroform; an alcohol, preferably methanol or ethanol; an aprotic solvent, preferably acetonitrile, toluene, pyridine or dimethylformamide, or any other suitable reaction medium. Of course, mixtures of at least two classes of solvents or of at least two solvents of one class may also be used.

All of above mentioned reactions are preferably carried out in an oven-dried vial. The catalyst, the auxiliary agent, the base and the compound of general formula II, IV, VII or VIII are added in each case and the vial is subsequently evacuated and purged with argon. The organic solvent and the compound of general formula III, V or IX are added and the reaction is carried out in a sealed vial at a temperature between 100 °C and 110 °C, preferably at 100 °C in case of tetrahydrofurane or toluene as the organic solvent and at 110 °C in case of dimethoxyethane and dioxane as the organic solvent.

Suitable reaction conditions for carrying out the reaction between compounds of general formula II, IV, VII or VIII and compounds of general formula III, V or IX are described in the references of J.F. Hartwig et al., J. Am. Chem. Soc. 1996, 118, 7217-7218; S.L. Buchwald et al., J. Am. Chem. Soc. 2002, 124, 6043-6048; S. L. Buchwald et al. J. Am. Chem. Soc. 2002, 124, 7241-7424 and S.L. Buchwald et al., J. Am. Chem. Soc. 2002, 124, 11684-11688. The respective part of the description is hereby incorporated by reference and forms part of the present disclosure.

The compounds of general formulas IV, VI, VIII and X given above may be purified and/or isolated according to methods well known to those skilled in the art.

The compounds of general formulas IV, VI, VIII and X may be isolated by evaporating the reaction medium, addition of water and adjusting the pH value to obtain the compound in form of a solid that can be isolated by filtration, or by extraction with a solvent that is not miscible with water such as chloroform and purification by chromatography or recrystallisation from a suitable solvent.

Preferably, the compounds of general formula IV, VI, VIII and X may be obtained by filtration of the reaction mixture and subsequent separation of the reaction mixture on a TLC plate. Alternatively, the compounds of general formula I may be isolated by addition of water and methanol to the reaction mixture, evaporating the reaction mixture and purifying the residue by preparative HPLC.

The compounds of general formula II and VII are commercially available or may be prepared according to methods well known in the art, for example, analogous to the methods described in the bibliography of A. McKillop et al., Tetrahedron 1987, 43, 1753. The respective part of the literature description cited above is hereby incorporated by reference and forms part of the disclosure.

The compounds of general formula III, V and IX are commercially available or may be prepared according to methods well known in the art.

During some synthetic reactions described above or while preparing the compounds of general formulas III, V, VI, IX or X the protection of sensitive or reactive groups may be necessary and/or desirable. This can be performed by using conventional protective groups like those described in Protective groups in Organic Chemistry, ed. J. F. W. McOmie, Plenum Press, 1973; T.W. Greene & P.G.M. Wuts and Protective Groups in Organic Chemistry, John Wiley & sons, 1991. The respective parts of the description is hereby incorporated by reference and forms part of the disclosure. The protective groups may be eliminated when convenient by means well-known to those skilled in the art.

If the nitro-substituted phenyl-piperazine compounds of general formula Id are obtained in form of a mixture of stereoisomers, particularly enantiomers or diastereomers, said mixtures may be separated by standard procedures known to those skilled in the art, e.g. chromatographic methods or crystallization with chiral reagents.

The nitro-substituted phenyl-piperazine compounds of general formula ld and in each case stereoisomers thereof may be obtained in form of a corresponding salt according to methods well known to those skilled in the art, e.g. by reacting said compound with at least one inorganic and/or organic acid, preferably in a suitable reaction medium. Suitable reaction media include, for example, any of the ones given above.

Preferably as component (A) at least one compound is present which is selected from the group consisting of phenyl-piperazine-derived compounds of general formula (Ie) 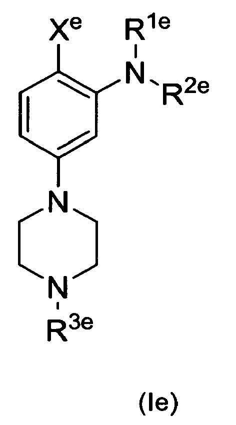 wherein
X^{e} represents -CN, -C(=O)-OH, -C(=O)-OR^{4e}, -O-R^{5e}, -NH₂, -NR^{6e}-C(=O)-R7^{e,} -NH-S(=O)₂^{-R8e} or -NH-R^{9e}_{;}
R^{1e} represents a hydrogen atom;
a saturated or unsaturated, unsubstituted or at least mono-substituted, optionally at least one heteroatom as a ring member containing cycloaliphatic radical, which may be bonded via a linear or branched, unsubstituted or at least mono-substituted alkylene, alkenylene or alkinylene group and/or which may be condensed with an unsubstituted or at least mono-substituted mono- or bicyclic ring system; or an unsubstituted or at least mono-substituted aryl or heteroaryl radical, which may be condensed with an unsubstituted or at least mono-substituted mono- or bicyclic ring system and/or which may be bonded via a linear or branched, unsubstituted or at least mono-substituted alkylene, alkenylene or alkinylene group which may contain 1, 2 or 3 heteroatom(s) independently selected from the group consisting of nitrogen, oxygen and sulfur as chain member(s);
R^{2e} represents a hydrogen atom or a -C(=O)-R^{10e} moiety;
or
R^{1e} and R^{2e} together with the bridging nitrogen form a nitro (NO₂)-group or
an unsubstituted or at least mono-substituted 5- or 6-membered heteroaryl radical which may be condensed with an unsubstituted or at least mono-substituted mono-or bicyclic ring system;
R^{3e} represents a linear or branched, saturated or unsaturated, unsubstituted or at least mono-substituted aliphatic radical;
R^{4e} represents a linear or branched, saturated or unsaturated, unsubstituted or at least mono-substituted aliphatic radical;
R^{5e} represents a linear or branched, saturated or unsaturated, unsubstituted or at least mono-substituted aliphatic radical;or
or an unsubstituted or at least mono-substituted aryl or heteroaryl radical, which may be condensed with an unsubstituted or at least mono-substituted mono- or bicyclic ring system and/or which may be bonded via a linear or branched, unsubstituted or at least mono-substituted alkylene, alkenylene or alkinylene group;
R^{6e} represents a hydrogen atom or
an unsubstituted or at least mono-substituted aryl or heteroaryl radical, which may be condensed with an unsubstituted or at least mono-substituted mono- or bicyclic ring system and/or which may be bonded via a linear or branched, unsubstituted or at least mono-substituted alkylene, alkenylene or alkinylene group;
R^{7e} represents a linear or branched, saturated or unsaturated, unsubstituted or at least mono-substituted aliphatic radical;
R^{8e} represents a linear or branched, saturated or unsaturated, unsubstituted or at least mono-substituted aliphatic radical;
or an unsubstituted or at least mono-substituted aryl or heteroaryl radical, which may be condensed with an unsubstituted or at least mono-substituted mono- or bicyclic ring system and/or which may be bonded via a linear or branched, unsubstituted or at least mono-substituted alkylene, alkenylene or alkinylene group;
R^{9e} represents a linear or branched, saturated or unsaturated, unsubstituted or at least mono-substituted aliphatic radical;
or an unsubstituted or at least mono-substituted aryl or heteroaryl radical, which may be condensed with an unsubstituted or at least mono-substituted mono- or bicyclic ring system and/or which may be bonded via a linear or branched, unsubstituted or at least mono-substituted alkylene, alkenylene or alkinylene group;
and
R^{10e} represents a linear or branched, saturated or unsaturated, unsubstituted or at least mono-substituted aliphatic radical;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof, respectively.

Preferred compounds of general formula (Ie) are those, wherein
X^{e} represents -CN, -C(=O)-OH, -C(=O)-OR^{4e}, -O-R^{5e}, -NH₂, -NR^{6e}-C(=O)-R^{7e}, -NH-S(=O)₂-R^{8e} or -NH-R^{9e};
R^{1e} represents
a hydrogen atom; or
an aryl or heteroaryl radical selected from the group consisting of phenyl, naphthyl, furyl (furanyl) and thiophenyl (thiophenyl), whereby said aryl or heteroaryl radical is bonded via a -(CH₂)-, -(CH₂)-(CH₂)-, -(CH₂)-(CH₂)-(CH₂)-, -O-(CH₂)-(CH₂)-, or -(CH₂)-(CH₂)-O-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂-CH₂-CH₃, -O-CH(CH₃)₂, -O-C(CH₃)₃, F, Cl, Br, -CN, -CF₃, -OCF₃, -OH and -SH. R² represents a hydrogen atom or a -C(=O)-R¹⁰ moiety;
R^{1e} and R^{2e} together with the bridging nitrogen atom form a nitro group or moiety selected from the group consisting of 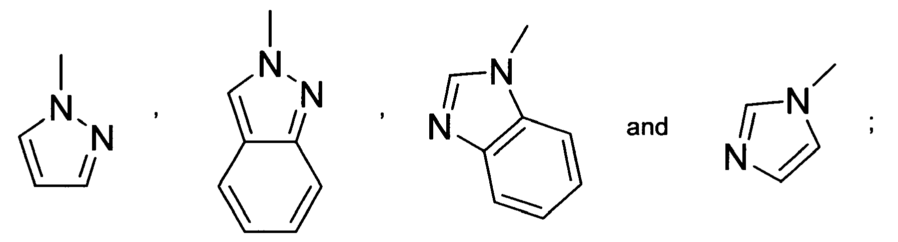 whereby each of these afore mentioned cyclic moieties may be unsubstituted or substituted with 1, 2 or 3 substituent(s) independently selected from the group consisting of methyl, ethyl, n-propyl, isopropyl, n-butyl, tert-butyl, sec-butyl, isobutyl, F, Cl, Br, I, -CN and -CF₃,
R^{3e} represents a methyl or ethyl radical;
R^{4e} represents a methyl or ethyl radical;
R^{5e} represents
an alkyl radical selected from the group consisting of methyl, ethyl, n-propyl, isopropyl, n-butyl, sec-butyl, isobutyl and tert-butyl;
or an aryl or heteroaryl radical selected from the group consisting of phenyl, naphthyl, furyl (furanyl) and thiophenyl (thiophenyl), whereby said aryl or heteroaryl radical is bonded via a -(CH₂)-, -(CH₂)-(CH₂)- or -(CH₂)-(CH₂)-(CH₂)- group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂-CH₂-CH₃, -O-CH(CH₃)₂, -O-C(CH₃)₃, F, Cl, Br,-CN, -CF₃, -OCF₃, -OH and -SH;
R^{6e} represents a hydrogen atom, or
a phenyl radical, whereby said phenyl radical may be bonded via a -(CH₂)-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, F, Cl, Br,-CF₃, -OCF₃, -OH and -SH;
R^{7e} represents a methyl or ethyl radical;
R^{8e} represents
an alkyl radical selected from the group consisting of methyl, ethyl, n-propyl, isopropyl, n-butyl, sec-butyl, isobutyl and tert-butyl; or
an aryl radical selected from the group consisting of phenyl and naphthyl, whereby said aryl radical may be bonded via a -(CH₂)-, or -(CH₂)-(CH₂)-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂-CH₂-CH₃, -O-CH(CH₃)₂, -O-C(CH₃)₃, F, Cl, Br,-CN, -CF₃, -OCF₃, -OH and -SH,
R^{9e} represents
an alkyl radical selected from the group consisting of methyl, ethyl, n-propyl, isopropyl, n-butyl, sec-butyl, isobutyl and tert-butyl;
or an aryl radical selected from the group consisting of phenyl and naphthyl, whereby said aryl radical may be bonded via a -(CH₂)-, -(CH₂)-(CH₂)- or -(CH₂)-(CH₂)-(CH₂)-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂-CH₂-CH₃, -O-CH(CH₃)₂, -O-C(CH₃)₃, F, Cl, Br, -CN, -CF₃, -OCF₃, -OH and -SH;
R^{10e} represents a methyl or ethyl radical;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof, respectively.

Particularly preferred compounds of general formula (Ie) are those selected from the group consisting of
[1086]4-(4-Methyl-piperazin-1-yl)-2-phenethylamino-benzoic acid,
[1087] 2-[(Furan-2-ylmethyl)-amino]-4-(4-methyl-piperazin-1-yl)-benzonitrile,
[1088] 2-[(Furan-2-ylmethyl)-amino]-4-(4-methyl-piperazin-1-yl)-benzoic acid,
[1089] 2-Benzylamino-4-(4-methyl-piperazin-1-yl)-benzoic acid methyl ester,
[1090] 2-Benzylamino-4-(4-methyl-piperazin-1-yl)-benzonitrile,
[1091] 4-(4-Methyl-piperazin-1-yl)-2-phenethylamino-benzoic acid methyl ester,
[1092]2-[(Furan-2-ylmethyl)-amino]-4-(4-methyl-piperazin-1-yl)-benzoic acid methyl ester,
[1093]2-Benzylamino-4-(4-methyl-piperazin-1-yl)-benzoic acid,
[1094] [2-Benzyloxy-5-(4-methyl-piperazin-1-yl)-phenyl]-phenethyl-amine,
[1095] [2-Benzyloxy-5-(4-methyl-piperazin-1-yl)-phenyl]-furan-2-yl-methyl amine,
[1096] Benzyl-[2-methoxy-5-(4-methyl-piperazin-1-yl)-phenyl]-amine,
[1097] [2-Methoxy-5-(4-methyl-piperazin-1-yl)-phenyl]-phenethyl-amine,
[1098] Furan-2-ylmethyl-[2-methoxy-5-(4-methyl-piperazin-1-yl)-phenyl]-amine,
[1099] Benzyl-[2-benzyloxy-5-(4-methyl-piperazin-1-yl)-phenyl]-amine,
[1100] N-[2-Acetyl-(2-phenoxyethyl)-amino]-4-(4-methyl-piperazin-1-yl)-phenyl]-acetamide,
[1101] N-[4-(4-Methyl-piperazin-1-yl)-2-(2-phenoxy-ethylamino)-phenyl]-acetamide,
[1102] N-[2-(Acetyl-amino)-4-(4-methyl-piperazin-1-yl)-phenyl]-N-benzyl-acetamide,
[1103] N-[2-(3,5-Dimethyl-pyrazol-1-yl)-4-(4-methyl-piperazin-1-yl)-phenyl]-acetamide,
[1104] N-[2-(Acetyl-furan-2-ylmethyl-amino)-4-(4-methyl-piperazin-1-yl)-phenyl]-acetamide,
[1105] N-[2-Benzylamino-4-(4-methyl-piperazin-1-yl)-phenyl]-acetamide,
[1106] N-[2-[Furan-2-ylmethyl)-amino]-4-(4-methyl-piperazin-1-yl)-phenyl]-acetamide,
[1107] N-[2-Amino-5-(4-methyl-piperazin-1-yl)phenyl]-N-furan-2-ylmethyl-acetamide,
[1108] N-[2-Amino-4-(4-methyl-piperazin-1-yl)-phenyl]-N-benzyl-acetamide,
[1109] N-[2-Benzylamino-4-(4-methyl-piperazin-1-yl)-phenyl]-benzenesulfonamide,
[1110] N-[2-Benzylamino-4-(4-methyl-piperazin-1-yl)-phenyl]-methansulfonamide,
[1111] 2-Benzyloxy-5-(4-methyl-piperazin-1-yl)-phenylamine,
[1112] Benzyl-[4-(4-methyl-piperazin-1-yl)-2-nitro-phenyl]-amine and
[1113] 2-Cyano-(5-piperazin-1-yl-methyl)-2-phenoxy-ethylamine

optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

The compounds of general formula le are prepared by a process, wherein at least one substituted benzene compound of general formula II, 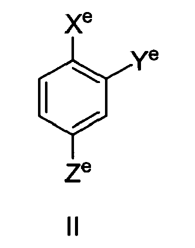 wherein X^{e} represents -CN, -C(=O)-OR^{4e}, -O-R^{5e} or -NO₂, R^{4e} and R^{5e} have any of the above given meanings, Y^{e} represents a chlorine atom, and Z^{e} represents a bromine or iodine atom; is reacted with at least one piperazine compound of general formula III, 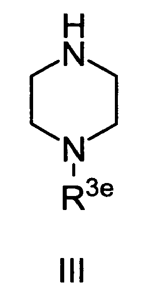 wherein R^{3e} has any of the above given meanings, in a suitable reaction medium, preferably in at least one organic solvent, more preferably in at least one organic solvent selected from the group consisting of tetrahydrofuran, toluene or dioxane, preferably in the presence of at least one catalyst, more preferably in the presence of at least a palladium source, even more preferably in the presence of at least one palladium source selected from the group consisting of Pd(OAc)₂, wherein OAc is acetate, and PdCl₂(dppf), wherein dppf is 1,1-bis(diphenylphosphino)-ferrocene, and/or at least one auxiliary agent, preferably at least one auxiliary agent selected from the group consisting of 1,1-bis(diphenylphosphino)-ferrocene and 2,2'-bis(diphenylphosphino)-1'1-binaphthyl(BINAP), optionally in form of its enantiomers or a racemate, and/or at least one base, preferably at least one base selected from the group consisting of sodium *tert*-pentoxide and Cs₂CO₃ to yield a compound of general formula IV, 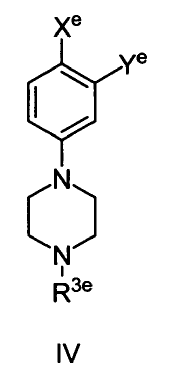 wherein X^{e} represents -CN, -C(=O)-OR^{4e}, -O-R^{5e} or -NO₂, R^{3e}, R^{4e} and R^{5e} have any of the above given meanings and Y^{e} represents a chlorine atom; which is optionally purified and/or isolated, and the compound of general formula IV is reacted with at least one compound of general formula V, 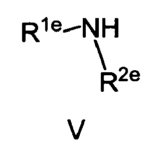 wherein R^{1e} and R^{2e} have any of the above given meanings or one of them represents a protecting group, preferably a -C(=O)-O-C(CH₃)₃ group, in a suitable reaction medium, preferably in at least one organic solvent, more preferably in at least one organic solvent selected from the group consisting of toluene, dioxane and dimethoxyethane, preferably in the presence of at least one catalyst, more preferably in the presence of at least a palladium source, even more preferably in the presence of at least a palladium source selected from the group consisting of Pd(OAc)₂, wherein OAc is acetate, and Pd₂dba₃, wherein dba is dibenzylidene acetone, and/or at least one auxiliary agent, preferably at least one auxiliary agent selected from the group consisiting of (biph)P(tBu)₂, wherein biph is biphenyl and *t*Bu is *tert*-butyl, and 4,5-bis(diphenylphosphino)-9,9-dimethylxanthene (xantphos), and and/or at least one base, preferably at least one base selected from the group consisting of K3PO₄, Cs₂CO₃ and sodium *tert*-pentoxide to yield a compound of general formula VI, 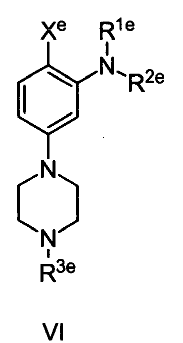 wherein X^{e} represents -CN, -C(=O)-OR^{4e}, -O-R^{5e} or -NO₂, R^{1e}, R^{2e} have any of the above given meanings or one of them represents a protecting group, preferably-C(=O)-O-C(CH₃)₃ and R^{3e}, R^{4e} and R^{5e} have any of the above given meanings, said compound of general formula VI is being optionally purified and/or isolated,
or at least one substituted benzene compound of general formula IIa, 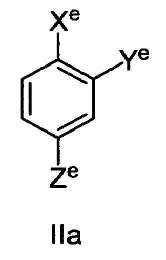 wherein X^{e} represents -CN, -C(=O)-OR^{4e}, -O-R^{5e} or -NO₂, R^{4e} and R^{5e} have any of the above given meanings, Z^{e} represents a chlorine atom, Y^{e} represents a bromine or iodine atom, is reacted with at least one compound of general formula V, 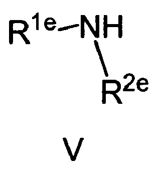 wherein R^{1e} and R²⁸ have any of the above given meanings or one of them represents a protecting group, preferably a -C(=O)-O-C(CH₃)₃ group in a suitable reaction medium, preferably in at least one organic solvent, more preferably in at least one organic solvent selected from the group consisting of toluene, dimethoxyethane and dioxane, preferably in the presence of at least one catalyst, more preferably in the presence of at least a palladium and/or copper source, even more preferably in the presence of at least a palladium and/or copper source selected from the group consisting of Pd(OAc)₂, wherein OAc is acetate, Pd₂dba₃, wherein dba is dibenzylidene acetone, and copper(I)iodide, and/or at least one auxiliary agent, preferably at least an auxiliary agent selected from the group consisting of 4,5-bis(diphenylphosphino)-9,9-dimethylxanthene (Xantphos), 1,1-bis(diphenylphosphino-ferrocene and P(*t*Bu)₃ wherein *t*Bu is tert-Butyl, and/or at least one base, preferably at least one base selected from the group consisting of K₃PO₄, Cs₂CO₃ and trans-1,2-diamino-methylcyclohexane to yield a compound of general formula VII, 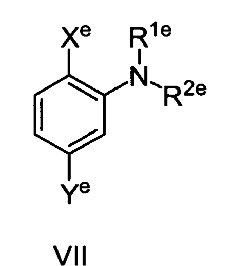 wherein X^{e} represents -CN, -C(=O)-OR^{4e}, -O-R^{5e} or -NO₂, R^{1e} and R^{2e} have any of the above given meanings or one of them represents a protecting group, preferably a
- C(=O)-O-C(CH₃)₃ group, R^{4e} and R^{5e} have any of the above given meanings, and Y represents a chlorine atom; said compound of general formula being optionally purified and/or isolated, and the compound of general formula VII is reacted with at least one compound of general formula III, 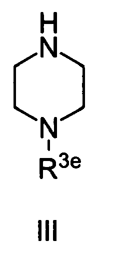
wherein R^{3e} has any of the above given meanings, in a suitable reaction medium, preferably in at least one organic solvent, more preferably in at least one organic solvent selected from the group consisting of tetrahydrofuran, toluene or dioxane, preferably in the presence of at least one catalyst, more preferably in the presence of at least a palladium source, even more preferably in the presence of at least one palladium source selected from the group consisting of Pd(OAc)₂, wherein OAc is acetate, and PdCl₂(dppf), wherein dppf is 1,1-bis(diphenylphosphino)-ferrocene, and/or at least one auxiliary agent, preferably at least one auxiliary agent selected from the group consisting of 1,1-bis(diphenylphosphino)-ferrocene and 2,2'-bis(diphenylphosphino)-1'1-binaphthyl (BINAP), optionally in form of its enantiomers or a racemate, and/or at least one base, preferably at least one base selected from the group consisting of sodium *tert*-pentoxide and Cs₂CO₃, to yield a compound of general formula VI, 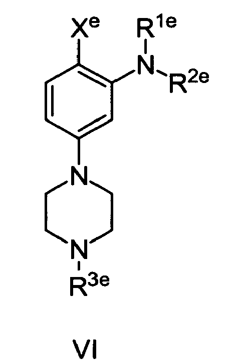 wherein X^{e} represents -CN, -C(=O)-OR^{4e}, -O-R^{5e} or -NO₂, R^{1e} and R^{2e} have any of the above given meanings or one of them represents a protecting group, preferably a -C(=O)-O-C(CH₃)₃ group, and R^{3e}, R^{4e} and R^{5e} have any of the above given meanings, and said compound of general formula VI is optionally purified and/or isolated,
or
at least one substituted benzene compound of general formula VIII, 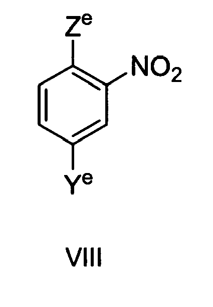 wherein Z^{e} represents bromine or iodine and Y^{e} represents chlorine, is reacted with at least one compound of general formula IX, 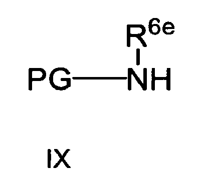 wherein R^{6e} has any of the above given meanings and PG represents a protecting group, preferably a-C(=O)-O-C(CH₃)₃ group, in a suitable reaction medium, preferably in at least one organic solvent, more preferably in at least one organic solvent selected from the group consisting of toluene and dioxane, preferably in the presence of at least one catalyst, more preferably in the presence of at least a palladium and/or copper source, even more preferably in the presence of at least a palladium and/or copper source selected from the group consisting of Pd(OAc)₂ wherein OAc is acetate, Pd₂dba₃ wherein dba is dibenzylidene acetone and copper(I)iodide, and/or at least one auxiliary agent, preferably at least an auxiliary agent selected from the group consisting of 4,5-bis(diphenylphosphino)-9,9-dimethylxanthene (Xantphos), 1,1-bis(diphenylphosphino-ferrocene and P(*t*Bu)₃ wherein *t*Bu is tert-Butyl, and/or at least one base, preferably at least one base selected from the group consisting of K₃PO₄, Cs₂CO₃ and trans-1,2-diamino-methylcyclohexane to yield a compound of general formula XI, 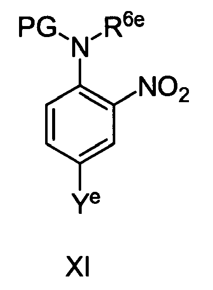 wherein R^{6e} has any of the above given meanings, PG represents a protecting group, preferably a-C(=O)-O-C(CH₃)₃ group and Y^{e} represents chlorine; which is optionally purified and/or isolated, and the compound of general formula XI reacted with at least one compound of general formula III, 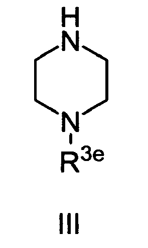 wherein R^{3e} has any of the above given meanings, in a suitable reaction medium, preferably in at least one organic solvent, more preferably in at least one organic solvent selected from the group consisting of tetrahydrofuran, toluene or dioxane, preferably in the presence of at least one catalyst, more preferably in the presence of at least a palladium source, even more preferably in the presence of at least one palladium source selected from the group consisting of Pd(OAc)₂, wherein OAc is acetate, and PdCl₂(dppf), wherein dppf is 1,1-bis(diphenylphosphino)-ferrocene, and/or at least one auxiliary agent, preferably at least one auxiliary agent selected from the group consisting of 1,1-bis(dipheny)phosphino)-ferrocene and 2,2'-bis(diphenylphosphino)-1'1-binaphthyl (BINAP), optionally in form of its enantiomers or a racemate, and/or at least one base, preferably at least one base selected from the group consisting of sodium *tert*-pentoxide and Cs₂CO₃, to yield a compound of general formula XII, 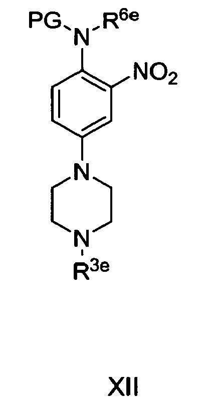 wherein R^{3e} and R^{6e} have any of the above given meanings and PG represents a protecting group, preferably a-C(=O)-O-C(CH₃)₃ group, which is optionally purified and/or isolated, and the compound of general formula XII is reacted with at least one acid in a suitable reaction medium to yield a compound of general formula XIII, 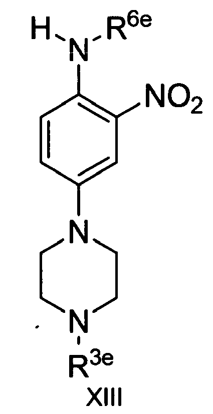 wherein R^{3e} and R^{6e} have any of the above given meanings, which is optionally purified and/or isolated, and the compound of general formula is reacted with hydrogen in the presence of at least one catalyst, preferably in the presence of at least one palladium source, more preferably in the presence of palladium on charcoal, in a suitable reaction medium, preferably in at least one organic solvent, more preferably in an organic solvent selected from the group consisting of dioxane, tetrahydrofuran and diethyl ether, to yield a compound of general formula XIV, 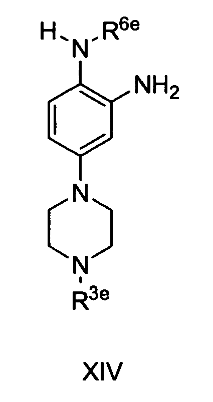 wherein R^{3e} and R^{6e} have any of the above given meanings, which is optionally purified and/or isolated, and the compound of general formula XIV is reacted with at least one compound of general formula R^{7e}-C(=O)-O-C(=O)-R^{7e}, wherein R^{7e} any of the above given meanings, and/or at least one compound of general formula R^{10e}-C(=O)-O-C(=O)-R^{10e}, wherein R^{10e} has any of the above given meanings, optionally in the presence of at least one base, preferably in the presence of at least one organic base, more preferably in the presence of at least an organic base selected from the group consisting of pyridine, triethylamine and diisopropylethylamine, in a suitable reaction medium, preferably in at least one organic solvent, more preferably in at least one organic solvent selected from the group consisting of dioxane, tetrahydrofuran and diethyl ether, to yield a compound of general formula I, wherein X^{e} represents -NR^{6e}-C(=O)R^{7e}, R^{1e} represents a hydrogen atom, R^{2e} represents a hydrogen atom or a -C(=O)-R^{10e}-moiety and R^{3e}, R^{6e}, R^{7e} and R^{10e} have any of the above given meanings, which is optionally purified and/or isolated,
and/or at least one compound compound of general formula VI, wherein X^{e} represents -CN, -C(=O)-OR^{4e} or -O-R^{5e}, R^{1e} and R^{2e} have any of the above given meanings or one of them represents a protecting group, preferably a -C(=O)-O-C(CH₃)₃-group, R^{3e}, R^{4e} and R^{5e} have any of the above given meanings, is reacted with at least one acid, preferably at least one acid selected from the group consisting of sulfuric acid, hydrochloric acid and acetic acid, in a suitable reaction medium, preferably in at least one organic solvent, more preferably in at least one organic solvent selected from the group consisting of dioxane and tetrahydrofuran, to yield a compound of general formula I, wherein X^{e} represents -CN, -C(=O)-OR^{4e} or -O-R^{5e}, R^{1e}and R^{3e} to R^{5e} have any of the above given meanings and R^{2e} represents hydrogen, which is optionally purified and/or isolated,
and optionally at least one compound of general formula I, wherein X^{e} represents-CN, -C(=O)-OR^{4e} or -O-R^{5e}, R^{1e} and R^{3e} to R^{5e} have any of the above given meanings and R^{2e} represents hydrogen, is reacted with hydrogen in the presence of at least one catalyst, preferably in the presence of at least one palladium source, more preferably in the presence of palladium on charcoal, in a suitable reaction medium, preferably in at least one organic solvent, more preferably in an organic solvent selected from the group consisting of dioxane, tetrahydrofuran and diethyl ether, to yield a compound of general formula I, wherein X^{e} represents -CN, -C(=O)-OR^{4e} or -O-R^{5e}, R^{3e} to R^{5e} have any of the above given meanings and R^{1e} and R^{2e} each represent hydrogen,
and/or
at least one compound of general formula VI, wherein X^{e} represents -C(=O)-OR^{4e}, R^{1e} and R^{2e} have any of the above given meanings or one of them represents a protecting group, preferably a -C(=O)-O-C(CH₃)₃-group, R^{3e} and R^{4e} have any of the above given meanings, is reacted with at least one base, preferably at least one metal hydroxide, more preferably at least one metal hydroxide selected from the group consisting of lithium hydroxide and potassium hydroxide, in a suitable reaction medium, preferably in a mixture of at least one organic solvent and water, more preferably in a mixture of at least one organic solvent selected from the group consisting of dioxane, ethanol and methanol and water, to yield a compound of general formula XV, wherein X^{e} represents -C(=O)-OH, R^{1e} and R^{2e} have any of the above given meanings or one of them represents a protecting group, preferably a-C(=O)-O-C(CH₃)₃-group, R^{3e} has any of the above given meanings, which is optionally purified and/or isolated and at least one compound of general formula XV is reacted with at least one acid, preferably at least one acid selected from the group consisting of sulfuric acid, hydrochloric acid and acetic acid, in a suitable reaction medium, preferably in at least one organic solvent, more preferably in at least one organic solvent selected from the group consisting of dioxane and tetrahydrofuran, to yield a compound of general formula I, wherein X^{e} represents -C(=O)-OH, R^{1e} and R^{3e} have any of the above given meanings and R^{2e} represents hydrogen, which is optionally purified and/or isolated,
and/or
at least one compound of general formula VI, wherein X^{e} represents -NO₂, R^{1e} and R^{2e} have any of the above given meanings or one of them represents a protecting group, preferably a -C(=O)-O-C(CH₃)₃-group and R^{3e} has any of the above given meanings, is reacted with hydrogen in the presence of at least one catalyst, preferably in the presence of at least one palladium source, more preferably in the presence of palladium on charcoal, in a suitable reaction medium, preferably in at least one organic solvent, more preferably in an organic solvent selected from the group consisting of dioxane, tetrahydrofuran and diethyl ether, to yield a compound of general formula XVI, wherein X^{e} represents -NH₂, R^{1e} and R^{2e} have any of the above given meanings or one of them represents a protecting group, preferably a-C(=O)-O-C(CH₃)₃-group, and R^{3e} has any of the above given meanings, which is optionally purified and/or isolated, and at least one compound of general formula XVI, is reacted with at least one acid, preferably at least one acid selected from the group consisting of sulfuric acid, hydrochloric acid and acetic acid, in a suitable reaction medium, preferably in at least one organic solvent, more preferably in at least one organic solvent selected from the group consisting of dioxane and tetrahyrofuran, to yield a compound of general formula I, wherein X^{e} represents -NH₂, R^{1e} and R^{3e} have any of the above given meanings and R^{2e} represents hydrogen, which is optionally purified and/or isolated,
and optionally at least one compound of general formula I, wherein X^{e} represents-NH₂, R^{1e} and R^{3e} have any of the above given meanings and R^{2e} represents hydrogen, is reacted with at least one compound of general formula R^{7e}-C(=O)-O-C(=O)-R^{7e} and/or at least one compound of general formula R^{10e}-C(=O)-O-C(=O)-R^{10e}, wherein R^{7e} and R^{10e} have any of the above given meanings optionally in the presence of at least one base, preferably in the presence of at least one organic base, more preferably in the presence of at least an organic base selected from the group consisting of pyridine, triethylamine and diisopropylethylamine, in a suitable reaction medium, preferably in at least one organic solvent, more preferably in at least one organic solvent selected from the group consisting of dioxane, tetrahydrofuran and diethyl ether, to yield a compound of general formula I, wherein X^{e} represents -NH-C(=O)-R^{7e} and R^{1e} to R^{3e} have any of the above given meanings, which is optionally purified and/or isolated,
and/or optionally at least one compound of general formula I, wherein X^{e} represents -NH₂ and R^{1e} and R^{3e} have any of the above given meanings and R^{2e} represents hydrogen, is reacted with at least one compound of general formula R^{8e}-S(=O)-W, wherein R^{8e} has any of the above given meanings and W represents a halogen atom, preferably a chlorine atom, optionally in the presence of at least one base, preferably in the presence of at least one organic base, more preferably in the presence of an organic base selected from the group consisting of pyridine, triethylamine and diisopropylethylamine, in a suitable reaction medium, preferably in at least one organic solvent, more preferably in at least one organic solvent selected from the group consisting of dioxane, tetrahydrofuran and diethyl ether, to yield a compound of general formula I, wherein X^{e} represents -NH-S(=O)₂-R^{8e} and R^{1e}, R^{3e} and R^{8e} have any of the above given meanings and R^{2e} represents hydrogen, which is optionally purified and/or isolated.

Suitable reaction media include organic solvents, such as dialkyl ether, preferably diethyl ether and dimethoxyethane, or a cyclic ether, preferably tetrahydrofuran or dioxane; or a halogenated hydrocarbon, preferably dichloromethane or chloroform; an alcohol, preferably methanol or ethanol; an aprotic solvent, preferably acetonitrile, pyridine, toluene or dimethylformamide, or any other suitable reaction medium. Of course, mixtures of at least two classes of solvents or of at least two solvents of one class may also be used.

If the above mentioned reactions are carried out in an oven-dried vial, the catalyst, the auxiliary agent, the base and the compound of general formula II, IIa, IV, VII, VIII or XI are added in each case and the vial is subsequently evacuated and purged with argon. The organic solvent and the compound of general formula III, V and IX are added and the reaction is carried out in a sealed vial at a temperature between 100 °C and 110 °C, preferably at 100 °C in case of tetrahydrofurane or toluene as the organic solvent and at 110 °C in case of dimethoxyethane and dioxane as the organic solvent.

Suitable reaction conditions for carrying out the reaction between compounds of general formula II, IIa, IV, VII, VIII or XI and compounds of general formula III, V and IX are described in the references of J.F. Hartwig et al., J. Am. Chem. Soc. 1996, 118, 7217-7218; S.L. Buchwald et al., J. Org. Chem. 2000, 65, 1144-1157; S.L. Buchwald et al., J. Am. Chem. Soc. 2002, 124, 6043-6048; S. L. Buchwald et al. J. Am. Chem. Soc. 2002, 124, 7241-7424 and S.L. Buchwald et al., J. Am. Chem. Soc. 2002, 124, 11684-11688. The respective part of the description is hereby incorporated by reference and forms part of the present disclosure.

The compounds of general formula I, IV, VI, VII, XI, XII; XIII, XIV, XV and XVI may be isolated by evaporating the reaction medium, addition of water and adjusting the pH value to obtain the compound in form of a solid that can be isolated by filtration, or by extraction with a solvent that is not miscible with water such as chloroform and purification by chromatography or recrystallisation from a suitable solvent.

Preferably, the compounds of general formula I, IV, VI, VII, XI, XII, XIII, XIV, XV and XVI may be obtained by filtration of the reaction mixture and subsequent separation of the reaction mixture on a TLC plate. Alternatively, the compounds of general formula I, IV, VI, VII, XI, XII, XIII, XIV, XV and XVI may be isolated by addition of water and methanol to the reaction mixture, evaporating the reaction mixture and purifying the residue by preparative HPLC.

The compounds of general formula II, IIa, VIII and IX are commercially available or may be prepared according to methods well known in the art, for example, analogous to the methods described in the bibliography of A. McKillop et al., Tetrahedron 1987, 43, 1753. The respective part of the literature description cited above is hereby incorporated by reference and forms part of the disclosure.

During some synthetic reactions described above or while preparing the compounds of general formulas II, IIa, VIII and IX the protection of sensitive or reactive groups may be necessary and/or desirable. This can be performed by using conventional protective groups like those described in Protective groups in Organic Chemistry, ed. J. F. W. McOmie, Plenum Press, 1973; T.W. Greene & P.G.M. Wuts and Protective Groups in Organic Chemistry, John Wiley & sons, 1991. The respective parts of the description is hereby incorporated by reference and forms part of the disclosure. The protective groups may be eliminated when convenient by means well-known to those skilled in the art.

If the substituted phenyl-piperazine compounds of general formula Ie are obtained in form of a mixture of stereoisomers, particularly enantiomers or diastereomers, said mixtures may be separated by standard procedures known to those skilled in the art, e.g. chromatographic methods or crystallization with chiral reagents.

The substituted phenyl-piperazine compounds of general formula le and in each case stereoisomers thereof may be obtained in form of a corresponding salt according to methods well known to those skilled in the art, e.g. by reacting said compound with at least one inorganic and/or organic acid, preferably in a suitable reaction medium. Suitable reaction media include, for example, any of the ones given above.

In another embodiment of the present invention as component (A) at least one compound is present which is selected from the group consisting of tetrahydroisoquinoline-derived sulfonamide compounds of general formula (If) 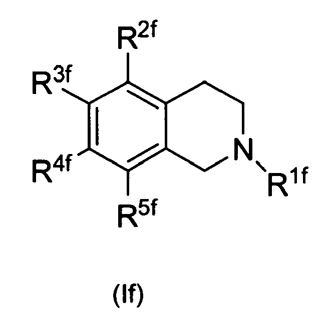 wherein
R^{1f} represents a hydrogen atom; a -C(=O)-OR^{37f} moiety;
a linear or branched, saturated or unsaturated, unsubstituted or at least mono-substituted aliphatic radical; or a saturated or unsaturated, unsubstituted or at least mono-substituted, optionally at least one heteroatom as a ring member containing cycloaliphatic radical, which may be bonded via a linear or branched, unsubstituted or at least mono-substituted alkylene, alkenylene or alkinylene group and/or which may be condensed with an unsubstituted or at least mono-substituted mono- or bicyclic ring system;
R^{2f}, R^{3f}, R^{4f} and R^{5f}, independently of one another, each represent a hydrogen atom; F, Cl, Br, I, -NO₂; -NH₂; -SH; -OH; -CN; -C(=O)-OH; -C(=O)-H; -S(=O)₂OH;-C(=O)-NH₂; -S(=O)₂-NH₂; -C(=O)-R^{6f}; -S(=O)-R^{7f}; -S(=O)₂-R^{7f}; -OR^{8f}; -SR^{9f}; -C(=O)-OR^{10f}; -N(R^{11f})-S(=O)₂-R^{12f}; -NR^{13f}R^{14f}; -NH-R^{15f}; -C(=O)-NR^{16f}R^{17f}; C(=O)-NHR^{18f};
a linear or branched, saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono- or bicyclic ring system;
with the proviso that at least one of the substituents R^{2f}, R^{3f}, R^{4f} and R^{5f} represents a -N(R^{11f})-S(=O)₂-R^{12f} moiety;
R^{6f}, R^{7f} R^{8f}, R^{9f}, R^{10f} R^{13f}, R^{14f}, R^{15f}, R^{16f}, R^{17f} and R^{18f},
independently of one another, each represent a linear or branched, saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono- or bicyclic ring system;
R^{11f} represents a hydrogen atom or a linear or branched, saturated or unsaturated, unsubstituted or at least mono-substituted aliphatic radical;
R^{12f} represents a phenyl radical of general formula (Af), 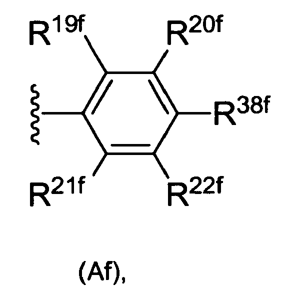 wherein
R^{19f}, R^{20f}, R^{21f}, R^{22f} and R^{38f}, independently of one another, each represent a hydrogen atom; F, Cl, Br, I, -NO₂; -NH₂; -SH; -OH; -CN; -C(=O)-OH; -C(=O)-H;-S(=O)₂-OH; -C(=O)-NH₂; -S(=O)₂-NH₂; -C(=O)-R^{23f}; -S(=O)-R^{24f}; -S(=O)_{2-R}^{24f}_{;} -OR^{25f}; -SR^{26f}; -C(=O)-OR^{27f}; -N(R^{28f})-S(=O)₂₋R^{29f}; -NH-S(=O)₂-R^{30f}; -NR^{31f}R^{32f}; -NH-R^{33f};-C(=O)-NHR^{34f}; -C(=O)NR^{35f}R ^{36f}; a linear or branched, saturated or unsaturated, unsubstituted or at least mono-substituted aliphatic radical; or a saturated or unsaturated, unsubstituted or at least mono-substituted, optionally at least one heteroatom as a ring member containing cycloaliphatic radical, which may be bonded via a linear or branched, unsubstituted or at least mono-substituted alkylene, alkenylene or alkinylene group and/or which may be condensed with an unsubstituted or at least mono-substituted mono- or bicyclic ring system;
an unsubstituted or at least mono-substituted aryl or heteroaryl radical, which may be condensed with an unsubstituted or at least mono-substituted mono- or bicyclic ring system and/or which may be bonded via a linear or branched, unsubstituted or at least mono-substituted alkylene, alkenylene or alkinylene group;
or a saturated or unsaturated, unsubstituted or at least mono-substituted, optionally at least one heteroatom as a ring member containing cycloaliphatic radical, which may be condensed with an unsubstituted or at least mono-substituted saturated, unsaturated or aromatic mono- or bicyclic ring system;
R^{23f}, R^{27f}, R^{28f}, R^{29f} and R^{30f}, independently of one another, each represent a linear or branched, saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono- or bicyclic ring system;
R^{24f}, R^{26f}, R^{31f}, R^{32f} and R^{33f}, each represent a linear or branched, saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be condensed with an unsubstituted or at least mono-substituted saturated or unsaturated, but not aromatic, mono- or bicyclic ring system;
R^{25f}, R^{34f}, R^{35f} and R^{36f}, represents a linear or branched, saturated or unsaturated, unsubstituted or at least mono-substituted aliphatic radical;
and R^{37f} represents a linear or branched, saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono- or bicyclic ring system;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

Preferred compounds of general formula (If) are those, wherein
R^{1f} represents a hydrogen atom; a -C(=O)-OR^{37f} moiety; a radical selected from the group consisting of methyl, ethyl, n-propyl, isopropyl, n-butyl, sec-butyl, isobutyl, tert-butyl, n-pentyl, n-hexyl, -CH₂-NH₂, -CH₂-NH-CH₃, -CH₂-N(CH₃)₂, -CH₂-N(C₂H₅)₂, -CH₂-NH-C₂H₅, -CH₂-CH₂-NH₂, -CH₂-CH₂-NH-CH₃, -CH₂-CH₂-N(CH₃)₂,-CH₂-CH₂-N(C₂H₅)₂, -CH₂-CH₂-NH-C₂H₅, -CH₂-CH₂-CH₂-NH-CH₃, -CH₂-CH₂-CH₂-N(CH₃)₂, -CH₂-CH₂-CH₂-N(C₂H₅)₂ and -CH₂-CH₂-CH₂-NH-C₂H₅; or a (hetero)cycloaliphatic radical selected from the group consisting of imidazolidinyl, aziridinyl, azetidinyl, pyrrolidinyl, piperidinyl, morpholinyl, thiomorpholinyl, piperazinyl, pyrazolidinyl and azepanyl, which may be bonded via a -(CH₂)_{1, 2 or 3}- group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl and isobutyl;
R^{2f}, R^{3f}, R^{4f} and R^{5f}, independently of one another, each represent a hydrogen atom; F, Cl, Br, I, -NO₂; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃; -N(R^{11f})-S(=O)₂-R^{12f}; or a radical selected from the group consisting of methyl, ethyl, n-propyl, isopropyl, tert-butyl, -CF₃, -CFH₂, -CF₂H, -CH₂-CF₃ and -CF₂-CF₃;
with the proviso that at least one of the substituents R^{2f}, R^{3f}, R^{4f} and R^{5f} represents a -N(R^{11f})-S(=O)₂-R^{12f} moiety;
R^{11f} represents a hydrogen atom, -S(=O)₂-R^{12f} or an alkyl radical selected from the group consisting of methyl, ethyl, n-propyl, isopropyl, n-butyl, sec-butyl, isobutyl and tert-butyl;
R^{12f} represents a phenyl radical of general formula (Af), 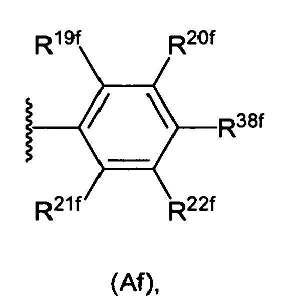 wherein
R^{19f}, R^{20f}, R^{21f}, R^{22f} and R^{38f}, independently of one another, each represent a hydrogen atom; F, Cl, Br, l, -NO₂; -NH₂; -SH; -OH; -CN; -C(=O)-OH; -C(=O)-H;-C(=O)-CH₃; -C(=O)-C₂H₅; -O-CH₃; -O-C₂H₅; -O-CF₃; -O-CFH₂; -O-CF₂H; -O-CH₂-CF₃; -O-CF₂-CF₃; -S-CH₃; -S-C₂H₅; -S-CF₃; -S-CFH₂; -S-CF₂H; -S-CH₂-CF₃; -S-CF₂-CF₃;-C(=O)-OCH₃; -C(=O)-OC₂H₅; methyl, ethyl, n-propyl, isopropyl, n-butyl, isobutyl, sec-butyl, tert-butyl, -CF₃, -CF₂H, -CFH₂, -CH₂-CF₃ and -CF₂-CF₃;
a radical selected from the group consisting of naphthyl, [1,3]-benzodioxolyl, [1,4]-benzodioxanyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl and imidazo[2,1-b]thiazolyl, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, F, Cl, Br, I, -CN,-CF₃, -OCF₃, -SCF₃, -CF₂H and -CFH₂;
or a radical selected from the group consisting of pyridinyl, pyrrolyl, imidazolyl, pyrazolyl, triazolyl, pyridazinyl, pyrimidinyl and pyrazinyl, which may be unsubstituted or optionally substituted with 1, 2, 3, 4 or 5 substituent(s) independently selected from the group consisting of F, Cl, Br, I, -NO₂; methyl, ethyl, n-propyl, isopropyl, n-butyl, isobutyl, sec-butyl, tert-butyl, -CF₃, -CF₂H, -CFH₂, -CH₂-CF₃ and -CF₂-CF₃;
and R^{37f} represents a radical selected from the group consisting of methyl, ethyl, n-propyl, isopropyl, n-butyl, sec-butyl, isobutyl, tert-butyl, n-pentyl, n-hexyl, fluorenyl, fluorenylmethyl, phenyl, benzyl and naphthyl;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

Particularly preferred compounds of general formula (if) are those selected from the group consisting of
[1114] N-(1,2,3,4-tetrahydroisoquinolin-6-yl)naphthalene-1-sulfonamide hydrochloride,
[1115] 2,2-dimethyl-6-(N-methylnaphthalene-1-sulfonamido)-1,2,3,4-tetrahydroisoquinolinium iodide,
[1116] N-(2-methyl-1,2,3,4-tetrahydroisoquinolin-6-yl)naphthalene-1-sulfonamide hydrochloride,
[1117] 5-chloro-3-methyl-N-(1,2,3,4-tetrahydroisoquinolin-6-yl)benzo[b]thiophene-2-sulfonamide hydrochloride,
[1118] 5-chloro-3-methyl-N-(2-methyl-1,2,3,4-tetrahydroisoquinolin-6-yl)benzo[b]thiophene-2-sulfonamide hydrochloride,
[1119]4-methyl-N-(1,2,3,4-tetrahydroisoquinolin-6-yl)naphthalene-1-sulfonamide hydrochloride,
[1120] 4-methyl-N-(2-methyl-1,2,3,4-tetrahydroisoquinolin-6-yl)naphthalene-1-sulfonamide hydrochloride,
[1121] N-(1,2,3,4-tetrahydroisoquinolin-6-yl)naphthalene-2-sulfonamide hydrochloride,
[1122] N-(2-methyl-1,2,3,4-tetrahydroisoquinolin-6-yl)naphthalene-2-sulfonamide hydrochloride,
[1123] 6-chloro-N-(1,2,3,4-tetrahydroisoquinolin-6-yl)imidazo[2,1-b]thiazole-5-sulfonamide hydrochloride,
[1124] 2-methoxy-5-methyl-N-(1,2,3,4-tetrahydroisoquinolin-6-yl)benzenesulfonamide hydrochloride,
[1125] N-(1,2,3,4-tetrahydroisoquinolin-6-yl)pyridine-3-sulfonamide dihydrochloride,
[1126] 6-(naphthalene-1-sulfonylamino)-3,4-dihydro-1H-isoquinoline-2-carboxylic acid tert-butyl ester,
[1127] 6-(5-chloro-3-methyl-benzo[b]thiophene-2-sulfonylamino)-3,4-dihydro-1H-isoquinoline-2-carboxylic acid tert-butyl ester,
[1128] 6-(4-methyl-naphthalene-1-sulfonylamino)-3,4 -dihydro-1H-isoquinoline-2-carboxylic acid tert-butyl ester,
[1129] 6-(naphthalene-2-sulfonylamino)-3,4-dihydro-1H-isoquinoline-2-carboxylic acid tert-butyl ester,
[1130] 6-(2-methoxy-5-methyl-benzenesulfonylamino)-3,4-dihydro-1H-isoquinoline-2-carboxylic acid tert-butyl ester and
[1131] 6-(pyridine-3-sulfonylamino)-3,4-dihydro-1H-isoquinoline-2carboxylic acid tert-butyl ester;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

The compounds of general formula If are prepared by a process, wherein at least one compound of general formula IV, 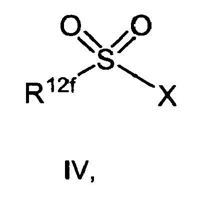 wherein R^{12f} has the meaning given above and X represents a leaving group, preferably a halogen atom, particularly preferably a chlorine atom, is reacted with at least one compound of general formula V, 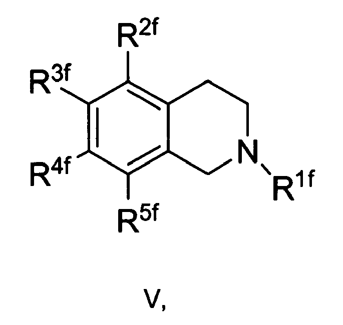 wherein R^{1f} to R^{5f} have the meaning given above, with the proviso that at least one substituent of the group consisting of R^{2f}, R^{3f}, R^{4f} and R^{5f} represents a -N(H)(R^{11f}) moiety, wherein R^{11f} has the meaning given above, or a protected derivative thereof, in a reaction medium, preferably in a reaction medium selected from the group consisting of pyridine, chloroform, dichloromethane, tetrahydrofurane and mixtures thereof, preferably in the presence of at least one base, more preferably in the presence of at least one base selected from the group consisting of triethylamine, diisopropylethylamine and diethylisopropylamine, preferably at a temperature between 0 °C and 30 °C.

If the substituted tetrahydroisoquinoline compounds of general formula If are obtained in form of a mixture of stereoisomers, particularly enantiomers or diastereomers, said mixtures may be separated by standard procedures known to those skilled in the art, e.g. chromatographic methods or crystallization with chiral reagents.

The substituted tetrahydroisoquinoline compounds of general formula If and in each case stereoisomers thereof may be obtained in form of a corresponding salt according to methods well known to those skilled in the art, e.g. by reacting said compound with at least one inorganic and/or organic acid, preferably in a suitable reaction medium. Suitable reaction media include, for example, any of the ones given above.

Compounds of general formula IV are in most cases commercially available or may be prepared by processes known to those skilled in the art.

Compounds of general formula V are in most cases commercially available or may be prepared by processes known to those skilled in the art.

In particular, 1,2,3,4-tetrahydroisoquinoline compounds with an amino group in position 5 can be prepared starting from 5-nitro-1,2,3,4-tetrahydroisoquinoline compounds. A process for the preparation of the latter compounds is described in K. V. Rao et al., Journal of Heterocyclic Chemistry, 1973, 10, 213 to 215.

In particular, 1,2,3,4-tetrahydroisoquinoline compounds with an amino group in position 6 are commercially available or can be prepared starting from 6-nitro-1,2,3,4-tetrahydroisoquinoline compounds. A process for the preparation of the latter compounds is described in G. J. Quallich, Journal of Organic Chemistry, 1998, 63, 4116 to 4119.

1,2,3,4-tetrahydroisoquinoline compounds with a nitro group in position 6 or 8 may be prepared by established procedures described in M. Tercel, Journal of Medicinal Chemistry, 1996, 39, 1084 to 1094.

In particular, 1,2,3,4-tetrahydroisoquinoline compounds with an amino group in position 7 are commercially available or can be prepared starting from 7-nitro-1,2,3,4-tetrahydroisoquinoline compounds. A process for the preparation of the latter compounds is described in J. F. Ajao et al., Journal of Heterocyclic Chemistry, 1985, 22, 329 to 331.

The N-methyl-8-amino-substituted 1,2,3,4-tetrahydroisoquinoline compounds were prepared by bromination and nitration of the corresponding 1,2,3,4-tetrahydroisoquinolines followed by two-step standard reduction conditions as described in M. Rey, Helvetica Chimica Acta, 1985, 66, 1828 to 1834 .

If any of the substituents in any of the above defined formulae represents or comprises a (hetero)cycloaliphatic radical, preferably a C₃₋₉ cycloalkyl radical or a C₄₋₉ cycloalkenyl radical, or a heterocyclic ring, preferably a 3- to 8-membered heterocyclic ring, said (hetero)cycloaliphatic radical, heterocyclic ring, C₃₋₉ cycloalkyl radical or C₄₋₉ cycloalkenyl radical may - if not defined otherwise - be unsubstituted or substituted by one or more substituents, preferably unsubstituted or optionally substituted with 1, 2, 3, 4 or 5 substituent(s). Said substituent(s) may preferably be selected independently from the group consisting of oxo (=O), thioxo (=S), C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl; whereby in each occurrence C₁₋₅-alkyl may be linear or branched and whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH,-NH₂ and -NO₂.

More preferably said substituents may be selected independently from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₃-CH₃-CH₃, -C(=O)-O-CH(CH₃)₂,-C(=O)-O-C(CH₃)₃, -C(=O)-CH₃, -C(=O)-C₂H₅, -C(=O)-CH₃-CH₃-CH₃, -C(=O)-CH(CH₃)₂, -C(=O)-C(CH₃)₃, F, Cl, Br, I, -CN, -CF₃, -OCF₃, -SCF₃, -OH, -SH, -NH₂,-NH-CH₃, -NH-C₂H₅, -NH-CH₂-CH₂-CH₃, -NH-CH(CH₃)₂, -NH-C(CH₃)₃, -N(CH₃)₂,-N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenoxy and benzyl; whereby in each occurrence said cyclic substituents may be unsubstituted or substituted by 1, 2 or 3 substituent(s) independently selected from the group consisting of methyl, ethyl, n-propyl, isopropyl, methoxy, ethoxy, F, Cl, Br,-CN, -CF₃, -OCF₃, -SCF₃, -OH, -SH, -NH₂ and -NO₂.

If any of the substituents in any of the above defined formulae represents or comprises a cycloaliphatic radical, C₃₋₉ cycloalkyl radical or C₄₋₉ cycloalkenyl radical which contains one or more, preferably 1, 2 or 3 heteroatom(s) as ring member(s), unless defined otherwise, each of these heteroatom(s) may preferably be selected independently from the group consisting of N, O and S.

If any of the substituents in any of the above defined formulae represents or comprises a heterocyclic ring which contains at least one further, preferably 1 or 2 further heteroatom(s) as ring member(s), unless defined otherwise, each of these heteroatom(s) may preferably be selected independently from the group consisting of N, O and S.

Suitable saturated or unsaturated, optionally at least one heteroatom as ring member containing cycloaliphatic radicals, C₃₋₉ cycloalkyl radicals or C₄₋₉ cycloalkenyl radicals may preferably be selected from the group consisting of cyclopropyl, cyclobutyl, cyclopentyl, cyclohexyl, cycloheptyl, cyclooctyl, cyclononyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1H-pyrrolyl, (2,3)-dihydro-1H-pyrrolyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, oxazolidinyl, (1,3)-dioxanyl, (1,4)-dioxanyl and (1,3)-dioxolanyl.

Suitable saturated or unsaturated heterocyclic rings which may be condensed with an unsubstituted or at least mono-substituted mono- or bicyclic ring system may preferably be selected from the group consisting of pyrrolidinyl, piperidinyl, piperazinyl, homopiperazinyl, morpholinyl, aziridinyl, azetidinyl, imidazolidinyl, thiomorpholinyl, pyrazolidinyl, azepanyl, diazepanyl, azocanyl, (1,2,3,6)-tetrahydropyridinyl, (1,2,3,4)-tetrahydropyridinyl, (1,2,5,6)-tetrahydropyridinyl, hexahydropyrimidinyl, (5,6)-dihydro-4H-pyrimidinyl, pyridazin-3(2H)-on-yl, phthalazin-1(2H)-on-yl, indolinyl, isoindolinyl, decahydronaphthyl, (1,2,3,4)-tetrahydroquinolinyl, (1,2,3,4)-tetrahydroisoquinolinyl, (1,2,3,4)-tetrahydronaphthyl, octahydro-cyclopenta[c]pyrrolyl, (1,3,4,7,9a)-hexahydro-2H-quinolizinyl, (1,2,3,5,6,8a)-hexahydro-indolizinyl, decahydroquinolinyl, octahydropyrrolo[1,2-a]pyrazinyl, octahydro-1H-pyrido[1,2-a]pyrazinyl, dodecahydrocarbazolyl, 9H-carbazolyl, decahydroisoquinolinyl and (2,3)-dihydro-1H-benzo[de]isoquinolinyl.

Suitable aromatic heterocyclic rings which may be condensed with an unsubstituted or at least mono-substituted mono- or bicyclic ring system may preferably be selected from the group consisting of imidazolyl, pyrazolyl, triazolyl, (4,5,6,7)-tetrahydro-2H-indazolyl, indazolyl and benzimidazolyl.

Suitable saturated or unsaturated, optionally at least one heteroatom as ring member containing cycloaliphatic radicals, C₃₋₉ cycloalkyl radicals or C₄₋₉ cycloalkenyl radicals which are condensed with an unsubstituted or at least mono-substituted mono- or bicyclic ring system may preferably be selected from the group consisting of indolinyl, isoindolinyl, decahydronaphthyl, (1,2,3,4)-tetrahydroquinolinyl, (1,2,3,4)-tetrahydroisoquinolinyl, (1,2,3,4)-tetrahydronaphthyl, octahydro-cyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydrocarbazolyl, 9H-carbazolyl, decahydroisoquinolinyl, (6,7)-dihydro-4H-thieno[3,2-c]pyridinyl, (2,3)-dihydro-1H-benzo[de]isoquinolinyl, octahydropyrrolo[1,2-a]pyrazinyl, octahydro-1H-pyrido[1,2-a]pyrazinyl, (1,2,3,7,8,8a)-hexahydroindolizinyl, (2,6,7,8,9,9a)-hexahydro-1H-quinolizinyl, octahydroindolizinyl, octahydro-1H-quinolizinyl, (1,2,3,5,8,8a)-hexahydroindolizinyl, (4,6,7,8,9,9a)-hexahydro-1H-quinolizinyl, fluorenyl and (1,2,3,4)-tetrahydroquinoxalinyl.

If any of the substituents in any of the above defined formulae represents an alkylene group, preferably an C₁₋₆ alkylene group, an alkenylene group, preferably an C₂₋₆ alkenylene group or an alkinylene group, preferably an C₂₋₆ alkinylene group, which may be substituted, said alkylene group, C₂₋₆ alkylene group, alkenylene group, C₂₋₆ alkenylene group, alkinylene group or C₂₋₆ alkinylene group may be unsubstituted or substituted by one or more substituents, preferably unsubstituted or optionally substituted with 1, 2 or 3 substituent(s). Said substituent(s) may preferably be selected independently from the group consisting of -O-C₁₋₅-alkyl, -S-C₁₋₅-alkyl, -F, Cl, Br, I, -CN, -CF₃, -OCF₃, -SCF₃, -OH, -SH, -NH₂, -NH(C₁₋₅-alkyl) and -N(C₁₋₅-alkyl)₂, whereby in each occurrence C₁₋₅-alkyl may be linear or branched. An alkenylene group comprises at least one carbon-carbon double bond, an alkinylene group comprises at least one carbon-carbon triple bond.

Suitable alkylene groups include -(CH₂)-, -CH(CH₃)-, -CH(phenyl), -(CH₂)₂-, -(CH₂)₃-, -(CH₂)₄-,-(CH₂)₅ and -(CH₂)₆-, suitable alkenylene groups include -CH=CH-, -CH₂-CH=CH- and -CH=CH-CH₂- and suitable alkinylene groups include -C≡C-, -CH₂-C≡C- and -C≡C-CH₂-.

If any of the substituents in any of the above defined formulae represents or comprises an aryl radical, including a 6-membered aryl radical such as phenyl or a 10-membered aryl radical such as naphthyl or a 14-membered aryl radical such as anthracenyl, said aryl radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of C₁₋₅-alkyl, -O-C₁₋₅-alkyl, -S-C₁₋₅-alkyl,-C(=O)-OH, -C(=O)-C₁₋₅-alkyl, -C(=O)-O-C₁₋₅-alkyl, -O-C(=O)-C₁₋₅-alkyl, F, Cl, Br, I,-CN, -CF₃, -OCF₃, -SCF₃, -SH, -NH₂, -NH(C₁₋₅-alkyl), -N(C₁₋₅-alkyl)₂, -NO₂, -CHO,-CF₂H, -CFH₂, -C(=O)-NH₂, -C(=O)-NH(C₁₋₅-alkyl), -C(=O)N(C₁₋₅-alkyl)₂, -S(=O)₂-C₁₋₅-alkyl, -S(=O)₂-phenyl, -C₁₋₅-alkylene-C(=O)-OH, -C₁₋₅-alkylene-C(=O)-O-C₁₋₅-alkyl,-NH-C(=O)-C₁₋₅-alkyl, -NH-S(=O)₂-C₁₋₅-alkyl, pyrrolidinyl, piperdinyl, morpholinyl, oxazolyl, isoxazolyl, pyridinyl, cyclopropyl, cyclobutyl, cyclopentyl, cyclohexyl, thiophenyl, furanyl, pyrrolidin-2,5-dionyl, phenyl, phenoxy and benzyl; whereby in each occurrence C₁₋₅-alkyl may be linear or branched and whereby said cyclic substituent(s) may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂.

More preferably said substituents may be selected independently from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃,-C(=O)-O-C_{2H5}, -C(=O)-O-CH₃-CH₃-CH₃, -C(=O)-O-CH(CH₃)₂, -C(=O)-O-C(CH₃)₃,-C(=O)-CH₃, -C(=O)-C₂H₅, -C(=O)-CH₃-CH₃-CH₃, -C(=O)-CH(CH₃)₂, -C(=O)-C(CH₃)₃, F, Cl, Br, l, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H,-CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)rCH₃.

Preferred aryl radicals, which may optionally be at least mono-substituted, are phenyl and naphthyl.

Suitable aryl radicals, which are condensed with an unsubstituted or at least mono-substituted saturated or unsaturated mono- or bicyclic ring system, may preferably be selected from the group consisting of indolinyl, isoindolinyl, (1,2,3,4)-tetrahydroquinolinyl, (1,2,3,4)-tetrahydroisoquinolinyl, (1,2,3,4)-tetrahydronaphthyl, (1,2,3,4)-tetrahydroquinoxalinyl, benzo[d]oxazol-2(3H)-onyl, benzo[d]thiazol-2(3H)-onyl, 2,3-dihydrobenzo[b][1,4]dioxinyl, benzo[d][1,3]dioxolyl, 3,4-dihydro-2H-benzo[b][1,4]oxazinyl, isochromanyl, chromanyl, 2,3-dihydrobenzofuranyl and 1H-benzo[b][1,4]diazepine-2,4(3H,5H)-dionyl.

If any of the substituents in any of the above defined formulae represents or comprises a heteroaryl radical, including a monocyclic 5- or 6-membered heteroaryl radical or a bi- or tricyclic 8-, 9-, 10-, 11-, 12-, 13- or 14 membered heteroaryl radical, said heteroaryl radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of C₁₋₅-alkyl, -O-C₁₋₅-alkyl, -S-C₁₋₅-alkyl,-C(=O)-OH, -C(=O)-C₁₋₅-alkyl, -C(=O)-O-C₁₋₅-alkyl, -O-C(=O)-C₁₋₅-alkyl, F, Cl, Br, I,-CN, -CF₃, -OCF₃, -SCF₃, -SH, -NH₂, -NH(C₁₋₅-alkyl), -N(C₁₋₅-alkyl)₂, -NO₂, -CHO,-CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, -C₁₋₅-alkylene-C(=O)-OH, -C₁₋₅-alkylene-C(=O)-O-C₁₋₅-alkyl,-NH-C(=O)-C₁₋₅-alkyl, -NH-S(=O)₂-C₁₋₅-alkyl, pyrrolidinyl, piperdinyl, morpholinyl, oxazolyl, isoxazolyl, pyridinyl, cyclopropyl, cyclobutyl, cyclopentyl, cyclohexyl, thiophenyl, furanyl, pyrrolidin-2,5-dionyl, phenyl, phenoxy and benzyl; whereby in each occurrence C₁₋₅-alkyl may be linear or branched and whereby said cyclic substituent(s) may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂.

More preferably said substituents may be selected independently from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂ -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃,-C(=O)-O-C₂H₅, -C(=O)-O-CH₃-CH₃-CH₃, -C(=O)-O-CH(CH₃)₂, -C(=O)-O-C(CH₃)₃,-C(=O)-CH₃, -C(=O)-C₂H₅, -C(=O)-CH₃-CH₃-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H,-CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃.

The heteroatom(s), which are present as ring member(s) in the heteroaryl radical, may, unless defined otherwise, independently be selected from the group consisting of nitrogen, oxygen and sulphur. Preferably the heteroaryl radical comprises 1, 2, 3 or 4 heteroatom(s).

Suitable bi- or tricyclic heteroaryl radicals, which may optionally be at least mono-substituted, may preferably be selected from the group consisting of indolyl, isoindolyl, quinolinyl, isoquinolinyl, benzo[b]furanyl, benzo[b]thiophenyl, benzimidazolyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzothiazolyl, benzisoxazolyl, benzisothiazolyl, imidazo[2,1-b]thiazolyl, 2H-chromenyl, indazolyl and quinazolinyl.

Suitable mono-, bi- or tricyclic heteroaryl radicals, which are condensed with an unsubstituted or at least mono-substituted saturated or unsaturated mono- or bicyclic ring system, may preferably be selected from the group consisting of [1,3]-benzodioxolyl, [1,4]-benzodioxanyl, [1,2,3,4]-tetrahydronaphthyl, (2,3)-dihydro-1H-cyclopenta[b]indolyl, [1,2,3,4]-tetrahydroquinolinyl, [1,2,3,4]-tetrahydroisoquinolinyl, [1,2,3,4]-tetrahydroquinazolinyl and [3,4]-dihydro-2H-benzo[1,4]oxazinyl.

Suitable monocyclic heteroaryl radicals, which may optionally be at least mono-substituted, may preferably be selected from the group consisting of pyridinyl, furyl (furanyl), thiophenyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, pyrimidinyl, pyrazinyl and pyranyl.

A mono- or bicyclic ring system according to the present invention - if not defined otherwise - means a mono- or bicyclic hydrocarbon ring system that may be saturated, unsaturated or aromatic. Each of its different rings may show a different degree of saturation, i.e. it may be saturated, unsaturated or aromatic. Optionally each of the rings of the mono- or bicyclic ring system may contain one or more, preferably 1, 2 or 3, heteroatom(s) as ring member(s), which may be identical or different and which can preferably be selected from the group consisting of N, O and S. The rings of the mono- or bicyclic ring system are preferably 5-, 6- or 7-membered.

Preferably a mono-or bicyclic ring system according to the present invention is a phenyl or naphthyl ring system.

The term "condensed" according to the present invention means that a ring or ring system is attached to another ring or ring system, whereby the terms "annulated" or "annelated" are also used by those skilled in the art to designate this kind of attachment.

Such a mono- or bicyclic ring system may - if not defined otherwise - be unsubstituted or substituted by one or more substituents, preferably unsubstituted or substituted with 1, 2, 3, 4 or 5 substituent(s). Said substituents may preferably be selected independently from the group consisting of C₁₋₅-alkyl, -O-C₁₋₅-alkyl, -S-C₁₋₅-alkyl, -C(=O)-OH, oxo (=O), thioxo (=S), -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂,-NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl; whereby in each occurrence C₁₋₅-alkyl may be linear or branched and whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂,

More preferably said substituents may be selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂ -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₃-CH₃-CH₃, -C(=O)-O-CH(CH₃)₂, -C(=O)-O-C(CH₃)₃, -C(=O)-CH₃, -C(=O)-C₂H₅, -C(=O)-CH₃-CH₃-CH₃, -C(=O)-CH(CH₃)₂,-C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH -S(=O)₂-phenyl, phenyl, phenoxy and benzyl; whereby in each occurrence said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and -NO₂.

If any of the substituents in any of the above defined formulae represents a saturated or unsaturated aliphatic radical, i.e. an alkyl radical, preferably an C₁₋₁₀ alkyl radical; an alkenyl radical, preferably an C₂₋₁₀ alkenyl radical or an alkinyl radical, preferably an C₂₋₁₀ alkinyl radical; said aliphatic radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of -O-C₁₋₅-alkyl, -S-C₁₋₅-alkyl F, Cl, Br, I, -CN, -CF₃, -OCF₃, -SCF₃, -OH, -SH, -NH₂, -NH(C₁₋₅-alkyl) and -N(C₁₋₅-alkyl)₂, whereby in each occurrence C₁₋₅-alkyl may be linear or branched. More preferably said substituent(s) may preferably be selected independently from the group consisting of -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, F, Cl, Br, I, -CN, -CF₃, -OCF₃,-SCF₃, -OH, -SH, -NH₂, NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂.

An alkenyl radical comprises at least one carbon-carbon double bond, an alkinyl radical comprises at least one carbon-carbon triple bond.

Suitable alkyl radicals, which may be substituted by one or more substituents, may preferably be selected from the group consisting of methyl, ethyl, n-propyl, isopropyl, n-butyl, isobutyl, sec-butyl, tert-butyl, n-pentyl, n-hexyl, n-heptyl, n-octyl, n-nonyl and n-decyl.

Suitable alkenyl radicals, which may be substituted by one or more substituents, may preferably be selected from the group consisting of vinyl, 1-propenyl, 2-propenyl, 1-butenyl, 2-butenyl and 3-butenyl.

Suitable alkinyl radicals, which may be substituted by one or more substituents, may preferably be selected from the group consisting of ethinyl, 1-propinyl, 2-propinyl, 1-butinyl, 2-butinyl and 3-butinyl.

The NMDA receptor is a cell-surface protein complex, widely distributed in the mammalian central nervous system that belongs to the class of ionotropic-glutamate receptors. It is involved in excitatory-synaptic transmission and the regulation of neuronal growth. The structure comprises a ligand-gated/voltage-sensitive ion channel. The NMDA receptor is highly complex and is believed to contain at least five distinct binding (activation) sites: a glycine-binding site, a glutamate-binding site (NMDA-binding site); a PCP-binding site, a polyamine-binding site, and a zinc-binding site. In general, a receptor antagonist is a molecule that blocks or reduces the ability of an agonist to activate the receptor. As used herein, an "NMDA-receptor antagonist" means any compound or composition, known or to be discovered, that when contacted with an NMDA receptor in vivo or in vitro, inhibits the flow of ions through the NMDA-receptor ion channel.

According to the present invention the meaning of the phrase "NMDA-receptor antagonist" encompasses any compound or composition that antagonizes the NMDA receptor by binding at the glycine site. Glycine-site NMDA-receptor antagonists can be identified by standard in vitro and in vivo assays. See, for example, the assays described in U.S. Pat. No. 6,251,903 (issued Jun. 26, 2001); U.S. Pat. No. 6,191,165 (issued Feb. 20, 2001); Grimwood et al. MOLECULAR PHARMACOLOGY 923 (1992); Yoneda et al. J. NEUROCHEM. 102 (1994); and Mayer et al. J. NEUROPHYSIOL. 645 (1988).

According to the present invention the meaning of the phrase "NMDA-receptor antagonist" encompasses any compound or composition that antagonizes the NMDA receptor by binding at the glutamate site. References that disclose NMDA-receptor antagonists as well as assays for identifying competitive NMDA-receptor antagonists include Jia-He Li, et al., J. MED. CHEM. 1955 (1995); Steinberg et al., NEUROSCI. LETT. 225 (1991); Meldrum et al., TRENDS PHARMACOL. SCI., 379 (1990); Willetts et al., TRENDS PHARMACOL. SCI. 423 (1990); Faden et al., TRENDS PHARMACOL. SCI. 29 (1992); Rogawski TRENDS PHARMACOL. SCI. 325 (1993); Albers et al., CLINICAL NEUROPHARM. 509 (1992); Wolfe et al., AM. J EMERG. MED., 174 (1995); and Bigge, BIOCHEM. PHARMACOL. 1547 (1993).

According to the present invention the meaning of the phrase "NMDA-receptor antagonist" encompasses any compound or composition that antagonizes the NMDA receptor by binding at the PCP (phencyclidine) site. Non-competitive NMDA-receptor antagonists can be identified using routine assays, for example, those described in U.S. Pat. Nos. 6,251,948 (issued Jun. 26, 2001); 5,985,586 (issued Nov. 16, 1999), and 6,025,369 (issued Feb. 15, 2000); Jacobson et al., J. PHARMACOL. EXP. THER. 243 (1987); and Thurkauf et al., J. MED. CHEM. 2257 (1988).

According to the present invention the meaning of "NMDA-receptor antagonist" encompasses compounds that block the NMDA receptor at the polyamine binding site, the zinc-binding site, and other NMDA-receptor antagonists that are either not classified herein according to a particular binding site or that block the NMDA receptor by another mechanism. Examples of NMDA-receptor antagonists that bind at the polyamine site include, but are not limited to, sperrine, spermidine, putrescine, and arcaine. Examples of assays useful to identify NMDA-receptor antagonists that act at the zinc or polyamine binding site are disclosed in U.S. Pat. No. 5,834,465 (issued Nov. 10, 1998).

NMDA-receptor antagonists for unse in the present invention include 3-((-)-2-carboxypiperazin-4-ylpropyl-1-phosphate (CPP); 3-(2-carboxypiperazin-4-yl)-propenyl-1-phosphonate (CPP-ene); 1-(cis-2-carboxypiperidine-4-yl)methyl-1-phosphonic acid (CGS 19755); D-2-Amino-5-phosphonopentanoic acid (AP5); 2-amino-phosphonoheptanoate (AP7); D,L-(E)-2-amino-4-methyl-5-phosphono-3-pentenoic acid carboxyethyl ester (CGP39551); 2-amino-4-methyl-5-phosphono-pent-3-enoic acid (CGP 40116); (4-phosphono-but-2-enylamino)-acetic acid (PD 132477); 2-amino-4-oxo-5-phosphono-pentanoic acid (MDL 100,453); 3-((phosphonylmethyl)-sulfinyl)-D,L-alanine; amino-(4-phosphonomethyl-phenyl)-acetic acid (PD 129635); 2-amino-3-(5-chloro-1-phosphonomethyl-1H-benzoimidazol-2-yl)-propionic acid; 2-amino-3-(3-phosphonomethyl-quinoxalin-2-yl)-propionic acid; 2-amino-3-(5-phosphonomethyl-biphenyl-3-yl)-propionic acid (SDZ EAB 515); 2-amino-3-[2-(2-phosphono-ethyl)-cyclohexyl]-propionic acid (NPC 17742); 4-(3-phosphono-propyl)-piperazine-2-carboxylic acid (D-CPP); 4-(3-phosphono-allyl)-piperazine-2-carboxylic acid (D-CPP-ene); 4-phosphonomethyl-piperidine-2-carboxylic acid (CGS 19755); 3-(2-phosphono-acetyl)-piperidine-2-carboxylic acid (MDL 100,925); 5-phosphono-1,2,3,4-tetrahydro-isoquinoline-3-carboxylic acid (SC 48981); 5-(2-phosphono-ethyl)-1,2,3,4-tetrahydro-isoquinoline-3-carboxylic acid (PD 145950); 6-phosphonomethyl-decahydro-isoquinoline-3-carboxylic acid (LY 274614); 4-(1H-tetrazol-5-ylmethyl)-piperidine-2-carboxylic acid (LY 233053 and 235723); 6-(1H-Tetrazol-5-ylmethyl)-decahydro-isoquinoline-3-carboxylic acid (LY 233536); ketamine; phencyclidine; dextromethorphan; dextrorphan; dexoxadrol; dizocilpine (MK-801); remacemide; thienylcyclohexylpiperidine (TCP) ; N-allylnometazocine (SKF 10,047); cyclazocine; etoxadrol; (1,2,3,4,9,9a-hexahydro-fluoren-4a-yl)-methyl-amine (PD 137889); (1,3,4,9,10,10a-hexahydro-2H-phenanthren-4a-yl)-methylamine (PD 138289); PD 138558; tiletamine; kynurenic acid; 7-chloro-kynurenic acid; memantine; nitromemantine; quinoxalinediones such as 6-cyano-7-nitroquinoxaline-2,3-dione (CNQX) and 6,7-dinitro-quinoxaline-2-,3-dione (DNQX); amantadine; eliprodil; iamotrigine; riluzole; aptiganel; flupirtine; celfotel; levemopamil; 1-(4-hydroxy-phenyl)-2-(4-phenylsulfanyl-piperidin-1-yl)-propan-1-one; 2-[4-(4-fluorobenzoyl)-piperidin-1-yl]-1-naphthalen-2-yl-ethanone (E 2001); 3-(1, 1-dimethyl-heptyl)-9-hydroxymethyl-6,6-dimethyl-6a,7,8,10a-tetrahydro-6H-benzo[c]chromen-1-ol (HU-211); 1-{4-[1-(4-chloro-phenyl)-1-methyl-ethyl]-2-methoxy-phenyl}-1H-[1,2,4]triazole-3-carboxylic acid amide (CGP 31358); acetic acid 10-hydroxy-7,9,7',9'-tetramethoxy-3,3'-dimethyl-3,4,3',4'-tetrahydro-1H,1'H-[5,5']bi[benzo[g]isochromenyl]-4-yl ester (ES 242-1); 14-hydroxy-11-isopropyl-10-methyl-5-octyl-10,13-diaza-tricyclo[6.6.1.04,1-5]pentadeca-1,4,6,8(15)-tetraen-12-one; and 4,5-dioxo-4,5-dihydro-1H-benzo-[g]indole-2,7,9-tricarboxylic acid (PQQ).

Particularly preferably memantine is present as component (B). Memantine (CAS Registry No. 41100-52-1), is an uncompetitive N-methyl-D-aspartate antagonist currently used for the treatment of dementia syndrome, spinal spasticity and Parkinson's disease. Chemically, memantine is 1-amino-3,5-dimethyladamantane of the adamantine class.

Further derivatives of memantine and nitromemantine can be prepared as outlined in U.S. patent application 2004/0122090.

The term "cognitive disorder" indicates disruptions in performance including one or more of the following signs:
1) memory deficits (impaired ability to learn new information or recall previously learned information;
2) one (or more) of the following disturbances:
   a) aphasia (language disturbance)
   b) apraxia (impaired ability to carry out motor activities despite intact motor function)
   c) agnosia (failure to recognize or identify objects despite in tact sensory function)
   d) disturbance in executive functioning (i.e. planning, organizing, sequencing, abstracting);
3) memory disturbances causing significant impairment in social or occupational functioning, and representing a significant decline from a previous level of functioning; and
4) impairment in cognitive functioning as evidenced by neuropsychological testing or quantified clinical assessment, accompanied by objective evidence of a systemic general medical condition or central nervous system dysfunction.

Cognitive disorders (or memory disorders) may include Alzheimer's disease, senile dementia process, learning disabilities caused by degenerative disorders, learning disabilities caused by non-degenerative disorders, memory or cognitive dysfunction such as mild cognitive impairment, age-related cognitive decline, cerebral senility, vascular dementia, AIDS-associated dementia, electric shock induced amnesia, memory impairment associated with depression or anxiety, cognitive defects in Parkinson's disease, Down's syndrome, stroke, traumatic brain injury, Huntington's disease, and attention deficit disorder; especially ADHD (attention deficit / hyperactivity disorder).

"Treatment" (e.g. of cognitive disorders, e.g. depression or e.g. of obesity and obesity-related disorders) refers to the administration of the compounds or combinations of the present invention to treat (in the case of cognitive disorders or dpression) the disorder or - more often - the symptoms of these disorders; or (for obesity) reduce or maintain the body weight of an obese subject. One outcome of treatment may be ameliorating the symptoms of e.g. Alzheimer's disease or the clinical depression or may be reducing the body weight of an obese subject relative to that subject's body weight immediately before the administration of the compounds or combinations of the present invention. A preferred aspect of the treatment, especially for cognitive disorders or depression, also involves the prophylaxis against the disorder, thus preventing the occurrence of its symptoms. Another outcome of treatment may be preventing body weight regain of body weight previously lost as a result of diet, exercise, or pharmacotherapy.

Another outcome of treatment may be decreasing the occurrence of and/or the severity of obesity-related diseases. Another outcome of treatment may be to maintain weight loss. The treatment may suitably result in a reduction in food or calorie intake by the subject, including a reduction in total food intake, or a reduction of intake of specific components of the diet such as carbohydrates or fats; and/or the inhibition of nutrient absorption; and/or the inhibition of the reduction of metabolic rate; and in weight reduction in patients in need thereof. The treatment may also result in an alteration of metabolic rate, such as an increase in metabolic rate, rather than or in addition to an inhibition of the reduction of metabolic rate; and/or in minimization of the metabolic resistance that normally results from weight loss.

The term "depression" or especially "clinical depression" is referring to a state of sadness, melancholia or despair that has advanced to the point of being disruptive to an individual's social functioning and/or activities of daily living.

"Obesity" is a condition in which there is an excess of body fat. The operational definition of obesity is based on the Body Mass Index(BMI), which is calculated as body weight per height in meters squared (kg/m²)."Obesity" refers to a condition whereby an otherwise healthy subject has a Body Mass Index (BMI) greater than or equal to 30kg/m², or a condition whereby a subject with at least one co-morbidity has a BMI greater than or equal to 27kg/m². An "obese subject" is an otherwise healthy subject with a Body Mass Index (BMI) greater than or equal to 30 kg/m² or a subject with at least one co-morbidity with a BMI greater than or equal to 27 kg/m². A "subject at risk of obesity" is an otherwise healthy subject with a BMI of 25 kg/m² to less than 30 kg/m² or a subject with at least one co-morbidity with a BMI of 25 kg/m² to less than 27 kg/m².

The increased risks associated with obesity occur at a lower Body Mass Index(BMI) in Asians. In Asian countries, including Japan, "obesity" refers to a condition whereby a subject with at least one obesity-induced or obesity-related co-morbidity, that requires weight reduction or that would be improved by weight reduction, has a BMI greater than or equal to 25 kg/m². In Asian countries, including Japan, an "obese subject" refers to a subject with at least one obesity-induced or obesity-related co-morbidity that requires weight reduction or that would be improved by weight reduction, with a BMI greater than or equal to 25kg/m². In Asia-Pacific, a "subject at risk of obesity" is a subject with a BMI of greater than 23 kg/m² to less than 25 kg/m².

As used herein, the term "obesity" is meant to encompass all of the above definitions of obesity.

Obesity-induced or obesity-related co-morbidities include, but are not limited to, diabetes, non-insulin dependent diabetes mellitus-type II (2), impaired glucose tolerance, impaired fasting glucose, insulin resistance syndrome, dyslipidemia, hypertension, hyperuricacidemia, gout, coronary artery disease, myocardial infarction, angina pectoris, sleep apnea syndrome, Pickwickian syndrome, fatty liver; cerebral infarction, cerebral thrombosis, transient ischemic attack, orthopedic disorders, arthritis deformans, lumbodynia, emmeniopathy, and infertility.

In particular, co-morbidities include: hypertension, hyperlipidemia, dyslipidemia, glucose intolerance, cardiovascular disease, sleep apnea, diabetes mellitus, and other obesity-related conditions.

The term "Metabolic syndrome", also known as syndrome X, is defined in the Third Report of the National Cholesterol Education Program Expert Panel on Detection, Evaluation and Treatment of High Blood Cholesterol in Adults (ATP-E). E. S. Ford et al., JAMA, Vol. 287 (3), Jan. 16, 2002, pp 356-359. Briefly, a person is defined as having Metabolic syndrome if the person has three or more of the following symptoms: abdominal obesity, hypertriglyceridemia, low HDL cholesterol, high blood pressure, and high fasting plasma glucose.

The terms "administration of" and or "administering a" compound should be understood to mean providing a compound of the invention or a prodrug of a compound of the invention to a subject in need of treatment.

The instant pharmaceutical composition includes administration of a single pharmaceutical dosage formulation which contains both the compound with 5-HT₆ receptor affinity, and at least one NMDA-receptor ligand, as well as administration of each active agent in its own separate pharmaceutical dosage formulation. Where separate dosage formulations are used, the individual components of the composition can be administered at essentially the same time, i. e., concurrently, or at separately staggered times, i. e. sequentially prior to or subsequent to the administration of the other component of the composition. The instant pharmaceutical composition is therefore to be understood to include all such regimes of simultaneous or alternating treatment, and the terms "administration" and "administering" are to be interpreted accordingly.

Administration in these various ways are suitable for the present compositions as long as the beneficial pharmaceutical effect of the combination of the compound with 5-HT₆ receptor affinity, and at least one NMDA-receptor ligand, is realised by the patient at substantially the same time.

Such beneficial effect is preferably achieved when the target blood level concentrations of each active drug are maintained at substantially the same time. It is preferred that the combination of the compound with 5-HT₆ receptor affinity, and at least one NMDA-receptor ligand, be co-administered concurrently on a once-a-day dosing schedule; however, varying dosing schedules, such as the compound with 5-HT₆ receptor affinity once a day and the NMDA-receptor ligand once, twice or more times per day, is also encompassed herein. A single oral dosage formulation comprised of both a compound with 5-HT₆ receptor affinity and a NMDA-receptor ligand is preferred. A single dosage formulation will provide convenience for the patient, which is an important consideration especially for patients with diabetes or obese patients who may be in need of multiple medications.

The term "subject" as used herein refers to an animal, preferably a mammal, most preferably a human, who has been the object of treatment, observation or experiment.

The administration of the composition of the present invention in order to practice the present methods of therapy is carried out by administering a therapeutically effective amount of the compounds in the composition to a subject in need of such treatment or prophylaxis. The need for a prophylactic administration according to the methods of the present invention is determined via the use of well known risk factors. The effective amount of an individual compound is determined, in the final analysis, by the physician in charge of the case, but depends on factors such as the exact disease to be treated, the severity of the disease and other diseases or conditions from which the patient suffers, the chosen route of administration, other drugs and treatments which the patient may concomitantly require, and other factors in the physician's judgement.

The term "therapeutically effective amount" as used herein means the amount of the active compounds in the composition that will elicit the biological or medical response in a tissue, system, subject, or human that is being sought by the researcher, veterinarian, medical doctor or other clinician, which includes alleviation of the symptoms of the disorder being treated. The novel methods of treatment of this invention are for disorders known to those skilled in the art.

The term "salt" as used herein is to be understood as meaning any form of the compounds in which they assume an ionic form or are charged and are coupled with a counter-ion (a cation or anion) or are in solution. By this are also to be understood complexes of the active compound with other molecules and ions, in particular complexes which are complexed via ionic interactions.

The term "physiologically acceptable salt" is understood in particular, in the context of this invention, as salt (as defined above) formed either with a physiologically tolerated acid, that is to say salts of the particular active compound with inorganic or organic acids which are physiologically tolerated - especially if used on humans and/or mammals - or with at least one, preferably inorganic, cation which are physiologically tolerated - especially if used on humans and/or mammals. Examples of physiologically tolerated salts of particular acids are salts of: hydrochloric acid, hydrobromic acid, sulfuric acid, hydrobromide, monohydrobromide, monohydrochloride or hydrochloride, methiodide, methanesulfonic acid, formic acid, acetic acid, oxalic acid, succinic acid, malic acid, tartaric acid, mandelic acid, fumaric acid, lactic acid, citric acid, glutamic acid, hippuric acid, picric acid and/or aspartic acid. Examples of physiologically tolerated salts of particular bases are salts of alkali metals and alkaline earth metals and with NH₄.

Solvates, preferably hydrates, of the compounds of the present invention and in each case of corresponding stereoisomers may also be obtained by standard procedures known to those skilled in the art.

The term "solvate" according to this invention is to be understood as meaning any form of the compounds in which they have attached to it via non-covalent binding another molecule (most likely a polar solvent) especially including hydrates and alcoholates, e.g. methanolate.

The active substance combination according to this invention comprises preferably 1-99% by weight of the component (A) and 99-1 % by weight of the component (B), more preferably 10-80% by weight of the component (A) and 80-20% by weight of the component (B), these percentages referring to the total weight of both components (A) and (B).

Another aspect of the present invention is a medicament, which comprises an inventive active substance combination and optionally one or more pharmacologically acceptable adjuvants.

Said medicament is suitable for simultaneous NMDA-receptor inhibition and 5-HT₆-receptor regulation.

Said medicament is particularly preferably suitable for the prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Metabolic Syndrome, Peripheral Nervous System Disorders, Central Nervous System Disorders, arthritis, epilepsy, anxiety, panic, depression, cognitive disorders, memory disorders, cardiovascular diseases, senile dementia processes, such as Alzheimer's, Parkinson's and/or Huntington's Disease, schizophrenia, psychosis, infantile hyperkinesia (ADHD, attention deficit / hyperactivity disorder), pain, hypertensive syndrome, inflammatory diseases, immunologic diseases or for improvement of cognition.

Said medicament is more particularly preferably suitable for the prophylaxis and/or treatment of cognitive disorders or memory disorders like Alzheimer's disease, senile dementia process, learning disabilities caused by degenerative disorders, learning disabilities caused by non-degenerative disorders, memory or cognitive dysfunction such as mild cognitive impairment, age-related cognitive decline, cerebral senility, vascular dementia, AIDS-associated dementia, electric shock induced amnesia, memory impairment associated with depression or anxiety, cognitive defects in Parkinson's disease, Down's syndrome, stroke, traumatic brain injury, Huntington's disease, and attention deficit disorder; especially ADHD (attention deficit / hyperactivity disorder).

Said medicament is more particularly preferably also suitable for the prophylaxis and/or treatment of depression as well as anxiety and panic.

Said medicament is more particularly preferably also suitable for the regulation of appetite, for maintenance, increase or reduction of body weight, for prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome.

Said medicament is even more particularly preferably suitable for the the prophylaxis and/or treatment of obesity.

Said medicament is also particularly preferably suitable for the prophylaxis and/or treatment of obesity-related disorders such as elevated plasma insulin concentrations and insulin resistance, dyslipidemias, hyperlipidemia, endometrial, breast, prostate and colon cancer, osteoarthritis, obstructive sleep apnea, cholelithiasis, gallstones, heart disease, abnormal heart rhythms and arrythmias, myocardial infarction, congestive heart failure, coronary heart disease, sudden death, stroke, polycystic ovary disease, craniopharyngioma, the Prader-Willi Syndrome and Frohlich's syndrome. Further examples of obesity-related disorders are reproductive hormone abnormalities, sexual and reproductive dysfunction, such as impaired fertility, infertility, hypogonadism in males and hirsutism in females, fetal defects associated with maternal obesity, gastrointestinal motility disorders, such as obesity-related gastro-esophageal reflux, respiratory disorders, such as obesity-related hypoventilation syndrome (Pickwickian syndrome), breathlessness, cardiovascular disorders, inflammation, such as systemic inflammation of the vasculature, arteriosclerosis, hypercholesterolemia, hyperuricaemia, lower back pain, gallbladder disease, gout, kidney cancer, and increased anesthetic risk.

Another aspect of the present invention is the use of an inventive active substance combination for the manufacture of a medicament for simultaneous NMDA-receptor inhibition and 5-HT₆-receptor regulation.

Another aspect of the present invention is the use of an inventive active substance combination for the manufacture of a medicament for the regulation of appetite, for maintenance, increase or reduction of body weight, for prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Peripheral Nervous System Disorders, Central Nervous System Disorders, arthritis, epilepsy, anxiety, panic, depression, preferably bipolar disorders, cognitive disorders, memory disorders, cardiovascular diseases, senile dementia processes, neurodegenerative disorders, preferably Alzheimer's disease, Parkinson's diesease, Huntington's Disease and/or multiple sclerosis, schizophrenia, psychosis, infantile hyperkinesia (ADHD, attention deficit / hyperactivity disorder), pain, hypertensive syndrome, inflammatoric diseases, immunologic diseases or for improvement of cognition.

Particularly preferred is the use of an inventive active substance combination for the manufacture of a medicament for the prophylaxis and/or treatment of cognitive disorders or memory disorders like Alzheimer's disease, senile dementia process, learning disabilities caused by degenerative disorders, learning disabilities caused by non-degenerative disorders, memory or cognitive dysfunction such as mild cognitive impairment, age-related cognitive decline, cerebral senility, vascular dementia, AIDS-associated dementia, electric shock induced amnesia, memory impairment associated with depression or anxiety, cognitive defects in Parkinson's disease, Down's syndrome, stroke, traumatic brain injury, Huntington's disease, and attention deficit disorder; especially ADHD (attention deficit / hyperactivity disorder).

Also particularly preferred is the use of an inventive active substance combination for the manufacture of a medicament for the prophylaxis and/or treatment of depression as well as anxiety and panic.

Also particularly preferred is the use of an inventive active substance combination for the manufacture of a medicament for the regulation of appetite, for maintenance, increase or reduction of body weight, for prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome.

Those skilled in the art understand that the components (A) and (B) of the active substance combination according to the present invention may be administered simultaneously or sequentially to one another, whereby in each case components (A) and (B) may be administered via the same or different administration pathways, e.g. orally or parentally. preferably both components (A) and (B) are administered simultaneously in one and the same administration form.

Yet another aspect of the present invention are pharmaceutical formulations in different pharmaceutical forms comprising an inventive active substance combination and optionally one or more pharmacologically acceptable adjuvants.

As well known to somebody skilled in the art the pharmaceutical formulations may - depending on their route of administration, also contain one or more auxiliary substances known to those skilled in the art.

The pharmaceutical formulations according to the present invention may be produced according to standard procedures known to those skilled in the art, e.g. from the tables of contents from "Pharmaceutics: the Science of Dosage Forms", Second Edition, Aulton, M.E. (Ed.) Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 and "The Theory and Practice of Industrial Pharmacy", Lachman L., Lieberman H. and Kanig J. (Eds.), Lea & Febiger, Philadelphia (1986). The respective descriptions are incorporated by reference and are part of the disclosure.

Preferred pharmaceutical formulations are solid pharmaceutical forms, preferably tablets, chewing tablets, chewing gums, dragées, capsules, suppositories, powder preparations, transdermal therapeutic systems, transmucosal therapeutic systems, preferably tablets or capsules.

Preferred pharmaceutical formulations are also liquid and semi-liquid pharmaceutical forms such as drops or such as juice, sirup, solution, emulsion, suspension, preferably drops or solutions.

In an additional preferred embodiment, the pharmaceutical formulations are in the form of multiparticulates, preferably microtablets, microcapsules, microspheroids, granules, crystals or pellets, optionally compacted in a tablet, filled in a capsule or suspended in a suitable liquid.

The pharmaceutical formulations according to the present invention are particularly preferably suitable for oral, intravenous, intramuscular, subcutaneous, intrathecal, epidural, buccal, sublingual, pulmonal, rectal, transdermal, nasal or intracerebroventricular application, more particularly for oral, intravenous or intraperitoneal application.

In one embodiment of the present invention the pharmaceutical formulation comprises at least one of the components (A) and (B) of the active substance combination at least partially in a sustained-release form.

By incorporating one or both of these components (A) and (B) at least partially or completely in a sustained-release form it is possible to extend the duration of their effect, allowing for the beneficial effects of such a sustained-release form, e.g. the maintenance of even concentrations in the blood.

Suitable sustained-release forms as well as materials and methods for their preparation are known to those skilled in the art, e.g. from the tables of contents from "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000);"Controlled Drug Delivery", Vol. I, Basic Concepts, Bruck, S.D. (Ed.), CRC Press Inc., Boca Raton (1983) and from Takada, K. and Yoshikawa, H., "Oral Drug delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encylopedia of Controlled Drug Delivery, Mathiowitz, E. (Ed.), John Wiley & Sons, Inc., New York (1999), Vol. 2, 698-728. The respective descriptions are incorporated by reference and are part of the disclosure.

If the pharmaceutical formulation according to the present invention comprises at least one of the components (A) and (B) at least partially in a sustained-release form, said sustained release may preferably be achieved by the application of at least one coating or provision of a matrix comprising at least one sustained-release material.

The sustained-release material is preferably based on an optionally modified, water-insoluble, natural, semisynthetic or synthetic polymer, or a natural, semisynthetic or synthetic wax or fat or fatty alcohol or fatty acid, or on a mixture of at least two of these afore mentioned components.

The water-insoluble polymers used to produce a sustained-release material are preferably based on an acrylic resin, which is preferably selected from the group of poly(meth)acrylates, particularly preferably poly(C₁-₄)alkyl (meth)acrylates, poly(C₁₋₄)dialkylamino(C₁₋₄)alkyl (meth)acrylates and/or copolymers or mixtures thereof, and very particularly preferably copolymers of ethyl acrylate and methyl methacrylate with a monomer molar ratio of 2:1 (Eudragit NE30D^{®}), copolymers of ethyl acrylate, methyl methacrylate and trimethylammonium ethyl methacrylate-chloride with a monomer molar ratio of 1:2:0.1 (Eudragit RS^{®}), copolymers of ethyl acrylate, methyl methacrylate and trimethylammonium ethyl methacrylate-chloride with a monomer molar ratio of 1:2:0.2 (Eudragit RL^{®}), or a mixture of at least two of the above-mentioned copolymers. These coating materials are commercially available as 30 wt.% aqueous latex dispersions, i.e. as Eudragit RS30D^{®}, Eudragit NE30D^{®} or Eudragit RL30D^{®}, and may also be used as such for coating purposes.

In another embodiment, the sustained-release material is based on water-insoluble cellulose derivatives, preferably alkyl celluloses, particularly preferably ethyl cellulose, or cellulose esters, e.g. cellulose acetate. Aqueous ethyl cellulose dispersions are commercially available, for example, under the trademarks Aquacoat^{®} or Surelease^{®}.

As natural, semisynthetic or synthetic waxes, fats or fatty alcohols, the sustained-release material may be based on carnauba wax, beeswax, glycerol monostearate, glycerol monobehenate, glycerol ditripalmitostearate, microcrystalline wax, cetyl alcohol, cetylstearyl alcohol or a mixture of at least two of these components.

The afore mentioned polymers of the sustained-release material may also comprise a conventional, physiologically acceptable plasticizer in amounts known to those skilled in the art.

Examples of suitable plasticizers are lipophilic diesters of a C₆-C₄₀ aliphatic or aromatic dicarboxylic acid and a C₁-C₈ aliphatic alcohol, e.g. dibutyl phthalate, diethyl phthalate, dibutyl sebacate or diethyl sebacate, hydrophilic or lipophilic citric acid esters, e.g. triethyl citrate, tributyl citrate, acetyltributyl citrate or acetyltriethyl citrate, polyethylene glycols, propylene glycol, glycerol esters, e.g. triacetin, Myvacet^{®} (acetylated mono- and diglycerides, C₂₃H₄₄O₅ to C₂₅H₄₇O₇), medium-chain triglycerides (Miglyol^{®}), oleic acid or mixtures of at least two of said plasticizers.

Aqueous dispersions of Eudragit RS^{®} and optionally Eudragit RL^{®} preferably contain triethyl citrate. The sustained-release material may comprise one or more plasticisers in amounts of, for example, 5 to 50 wt.% based on the amount of polymer(s) used.

The sustained-release material may also contain other conventional auxiliary substances known to those skilled in the art, e.g. lubricants, coloured pigments or surfactants.

The pharmaceutical formulation of the present invention may also comprise at least one of the components (A) and (B) covered by an enteric coating form which dissolves as a function of pH. Because of this coating, part or all of the pharmaceutical formulation can pass through the stomach undissolved and the components (A) and/or (B) are only released in the intestinal tract. The enteric coating preferably dissolves at a pH of between 5 and 7.5.

The enteric coating may be based on any enteric material known to those skilled in the art, e.g. on methacrylic acid/methyl methacrylate copolymers with a monomer molar ratio of 1:1 (Eudragit L^{®})_{;} methacrylic acid/methyl methacrylate copolymers with a monomer molar ratio of 1:2 (Eudragit S^{®}), methacrylic acid/ethyl acrylate copolymers with a monomer molar ratio of 1:1 (Eudragit L30D-55^{®}), methacrylic acid/methyl acrylate/methyl methacrylate copolymers with a monomer molar ratio of 7:3:1 (Eudragit FS^{®}), shellac, hydroxypropyl methyl cellulose acetate-succinates, cellulose acetate-phthalates or a mixture of at least two of these components, which can optionally also be used in combination with the above-mentioned water-insoluble poly(meth)acrylates, preferably in combination with Eudragit NE30D^{®} and/or Eudragit RL^{®} and/or Eudragit RS^{®}.

The coatings of the pharmaceutical formulations of the present invention may be applied by the conventional processes known to those skilled in the art, e.g. from Johnson, J.L., "Pharmaceutical tablet coating", Coatings Technology Handbook (Second Edition), Satas, D. and Tracton, A.A. (Eds), Marcel Dekker, Inc. New York, (2001), 863-866; Carstensen, T., "Coating Tablets in Advanced Pharmaceutical Solids", Swarbrick, J. (Ed.), Marcel Dekker, Inc. New York (2001), 455-468; Leopold, C.S., "Coated dosage forms for colon-specific drug delivery", Pharmaceutical Science & Technology Today, 2(5), 197-204 (1999), Rhodes, C.T. and Porter, S.C., Coatings, in Encyclopedia of Controlled Drug Delivery. Mathiowitz, E. (Ed.), John Wiley & Sons, Inc., New York (1999), Voi. 1, 299-311. The respective descriptions are incorporated by reference and are part of the disclosure.

In another embodiment, the pharmaceutical formulation of the present invention contains one or both of components (A) and (B) not only in sustained-release form, but also in non-sustained-release form. By combination with the immediately released form, a high initial dose can be achieved for the rapid onset of the beneficial effect. The slow release from the sustained-release form then prevents the beneficial effect from diminishing. Such a pharmaceutical formulation is particularly useful for the treatment of acute health problems.

This may be achieved, for example, by a pharmaceutical formulation having at least one immediate-release coating comprising at least one of the components (A) and (B) to provide for rapid onset of the beneficial effect after administration to the patient.

The present invention also relates to the treatment the aforementioned disorders and/or diseases with a combination of at least one compound with 5-HT₆ receptor affinity and at least one NMDA-receptor ligand which may be administered separately, therefore the invention also relates to combining separate pharmaceutical compositions into a kit form. The kit, according to this invention, comprises two separate pharmaceutical compositions: a first unit dosage form comprising a prophylactically or therapeutically effective amount of at least one NMDA-receptor ligand, or a pharmaceutically acceptable salt or ester thereof, and a pharmaceutically acceptable carrier or diluent in a first unit dosage form, and a second unit dosage form comprising a prophylactically or therapeutically effective amount of at least one compound with 5-HT₆ receptor affinity, or a pharmaceutically acceptable salt or ester thereof, and a pharmaceutically acceptable carrier or diluent in a second unit dosage form. The kit further comprises a container. Such kits are especially suited for the delivery of solid oral forms such as tablets or capsules. Such a kit preferably includes a number of unit dosages. Such kits can include a card having the dosages oriented in the order of their intended use. An example of such a kit is a "blister pack". Blister packs are well known in the packaging industry and are widely used for packaging pharmaceutical unit dosage forms. If desired, a memory aid can be provided, for example in the form of numbers, letters, or other markings or with a calendar insert, designating the days or time in the treatment schedule in which the dosages can be administered.

## Claims

1. An active substance combination that comprises
(A) at least one compound with 5-HT₆ receptor affinity,
and
(B) at least one NMDA-receptor ligand.

2. The combination according to claim 1, **characterized in that** the binding of compounds present as component (A) to the 5-HT₆-receptor is determined by a Kᵢ value of less than 7000 nM, preferably of less than 200 nM, more preferably of less than 100 nM.

3. The combination according to claim 1 or 2, **characterized in that** the compound present as component (B) acts as NMDA-receptor antagonist.

4. The combination according to any of claim 1 to 3, **characterized in that** the binding of compounds present as component (B) to the NMDA-receptor is determined by an EC₅₀ or IC₅₀ value of less than 300 µM, preferably less than 100 µM.

5. The combination according to one or more of claims 1 to 4, **characterized in that** as component (A) at least one compound is present which is selected from the group consisting of the benzoxazinone-derived sulfonamide compounds of general formula (Ia) 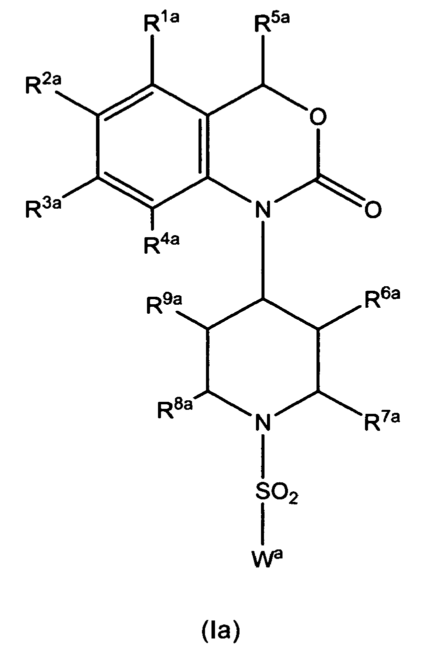 wherein
R^{1a}, R^{2a}, R^{3a} and R^{4a}, independently of one another, each represent a hydrogen atom; halogen; an unbranched or branched, saturated or unsaturated, unsubstituted or at least mono-substituted aliphatic radical; a saturated or unsaturated, unsubstituted or at least mono-substituted, optionally at least one heteroatom as ring member containing cycloaliphatic radical, which may be bonded via an unsubstituted or at least mono-substituted alkylene group and/or which may be condensed with an unsubstituted or at least mono-substituted mono- or bicyclic ring system; an unsubstituted or at least mono-substituted aryl- or heteroaryl radical, which may be bonded via an unsubstituted or at least mono-substituted alkylene group and/or which may be condensed with an unsubstituted or at least mono-substituted mono- or bicyclic ringsystem; nitro; cyano; -O-R^{10a}; -O-(C=O)-R^{11a}; -(C=O)-OR^{11a};-SR^{12a}; -SOR^{12a}; -SO₂R^{12a}; -NH-SO₂R^{12a}; -SO₂NH₂ or -NR^{13a}R^{14a};
R^{5a} represents a hydrogen atom; an unbranched or branched, saturated or unsaturated, unsubstituted or at least mono-substituted aliphatic radical or a saturated or unsaturated, unsubstituted or at least mono-substituted, optionally at least one heteroatom as ring member containing cycloaliphatic radical;
R^{6a}, R^{7a}, R^{8a}, R^{9a}, independently of one another, each represent a hydrogen atom; an unbranched or branched, saturated or unsaturated, unsubstituted or at least mono-substituted aliphatic radical; a saturated or unsaturated, unsubstituted or at least mono-substituted, optionally at least one heteroatom as ring member containing cycloaliphatic radical; a cyano group or a -C(=O)-OR^{15a} moiety;
W^{a} represents an unbranched or branched, saturated or unsaturated, unsubstituted or at least mono-substituted aliphatic radical;
a saturated or unsaturated, unsubstituted or at least mono-substituted, optionally at least one heteroatom as ring member containing cycloaliphatic radical, which may be bonded via an optionally mono-substituted alkylene group and/or which may be condensed with an unsubstituted or at least mono-substituted mono- or bicyclic ring system;
an unsubstituted or at least mono-substituted aryl or heteroaryl radical, which may be bonded via an unsubstituted or at least mono-substituted alkylene or alkenylene group and/or which may be condensed with an unsubstituted or at least mono-substituted mono- or bicyclic ring system;
a -NR^{16a}R^{17a} moiety, or
a -C(=O)-R^{18a} moiety;
R^{10a} represents a hydrogen atom; an unbranched or branched, saturated or unsaturated, unsubstituted or at least mono-substituted aliphatic radical; a saturated or unsaturated, unsubstituted or at least mono-substituted, optionally at least one heteroatom as ring member containing cycloaliphatic radical, which may be bonded via an unsubstituted or at least mono-substituted alkylene group and/or which may be condensed with an unsubstituted or at least mono-substituted mono- or bicyclic ring system; or an unsubstituted or at least mono-substituted aryl or heteroaryl radical, which may be bonded via an unsubstituted or at least mono-substituted alkylene group and/or which may be condensed with an unsubstituted or at least mono-substituted mono- or bicyclic ring system;
R^{11a} represents a hydrogen atom; an unbranched or branched, saturated or unsaturated, unsubstituted or at least mono-substituted aliphatic radical; a saturated or unsaturated, unsubstituted or at least mono-substituted, optionally at least one heteroatom as ring member containing cycloaliphatic radical, which may be bonded via an unsubstituted or at least mono-substituted alkylene group and/or which may be condensed with an unsubstituted or at least mono-substituted mono- or bicyclic ring system; or an unsubstituted or at least mono-substituted aryl or heteroaryl radical, which may be bonded via an unsubstituted or at least mono-substituted alkylene group and/or which may be condensed with an unsubstituted or at least mono-substituted mono- or bicyclic ring system;
R^{12a} represents an unbranched or branched, saturated or unsaturated, unsubstituted or at least mono-substituted aliphatic radical; a saturated or unsaturated, unsubstituted or at least mono-substituted, optionally at least one heteroatom as ring member containing cycloaliphatic radical, which may be bonded via an unsubstituted or at least mono-substituted alkylene group and/or which may be condensed with an unsubstituted or at least mono-substituted mono- or bicyclic ring system; or an unsubstituted or at least mono-substituted aryl or heteroaryl radical, which may be bonded via an unsubstituted or at least mono-substituted alkylene group and/or which may be condensed with an unsubstituted or at least mono-substituted mono- or bicyclic ring system;
R^{13a} and R^{14a}, independently of one another, each represent a hydrogen atom; an unbranched or branched, saturated or unsaturated, unsubstituted or at least mono-substituted aliphatic radical; a saturated or unsaturated, unsubstituted or at least mono-substituted, optionally at least one heteroatom as ring member containing cycloaliphatic radical, which may be bonded via an unsubstituted or at least mono-substituted alkylene group and/or which may be condensed with an unsubstituted or at least mono-substituted mono- or bicyclic ring system; or an unsubstituted or at least mono-substituted aryl or heteroaryl radical, which may be bonded via an unsubstituted or at least mono-substituted alkylene group and/or which may be condensed with an unsubstituted or at least mono-substituted mono- or bicyclic ring system;
or R^{13a} and R^{14a} together with the bridging nitrogen atom form a saturated, unsaturated or aromatic heterocyclic ring, which is unsubstituted or at least mono-substituted and/or which may contain at least one further heteroatom as a ring member;
R^{15a} represents a hydrogen atom; an unbranched or branched, saturated or unsaturated, unsubstituted or at least mono-substituted aliphatic radical; a saturated or unsaturated, unsubstituted or at least mono-substituted, optionally at least one heteroatom as ring member containing cycloaliphatic radical or an unsubstituted or at least mono-substituted aryl or heteroaryl radical, which may be bonded via an unsubstituted or at least mono-substituted alkylene group and/or which may be condensed with an unsubstituted or at least mono-substituted mono- or bicyclic ring system;
R^{16a} represents an unbranched or branched, saturated or unsaturated, unsubstituted or at least mono-substituted aliphatic radical;
R^{17a} represents an unbranched or branched, saturated or unsaturated, unsubstituted or at least mono-substituted aliphatic radical, and
R^{18a} represents an unsubstituted or at least mono-substituted aryl radical;
optionally in form of one of its stereoisomers, preferably enantiomers or diastereomers, its racemate or in form of a mixture of at least two of its stereoisomers, preferably enantiomers or diastereomers, in any mixing ratio, or a physiologically acceptable salt thereof, or a solvate, respectively:
and indole-derived sulfonamide compounds of general formula (Ib) 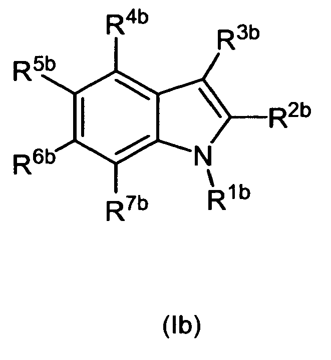 wherein
R^{1b} represents a hydrogen atom; a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via a linear or branched alkylene group and/or which may be condensed with an unsubstituted or at least mono-substituted mono-or bicyclic ring system; an unsubstituted or at least mono-substituted aryl or heteroaryl radical, which may be bonded via a linear or branched alkylene group and/or which may be condensed with an unsubstituted or at least mono-substituted mono- or bicyclic ring system; a -(CH₂)_{mb}-NR^{13b}R^{14b} moiety with mb = 0, 1, 2, 3, 4 or 5; a -C(=O)-R^{8b} moiety; a -S(=O)₂-R^{9b} moiety; or a -S(=O)₂-C(H)A^{b}B^{b} moiety;
R^{2b} represents a hydrogen atom; -F; -Cl; -Br; -I; -NO₂; -NH₂; -SH; -OH; -CN;-C(=O)-OH; -O-R^{10b}; -S-R^{11b}; -C(=O)-OR^{12b}; a linear or branched, saturated or unsaturated, unsubstituted or at least mono-substituted, optionally at least one heteroatom as a chain member containing aliphatic radical; a saturated or unsaturated, unsubstituted or at least mono-substituted, optionally at least one heteroatom as a ring member containing cycloaliphatic radical, which may be bonded via a linear or branched alkylene group and/or which may be condensed with an unsubstituted or at least mono-substituted mono- or bicyclic ring system; or an unsubstituted or at least mono-substituted aryl or heteroaryl radical, which may be bonded via a linear or branched alkylene group and/or which may be condensed with an unsubstituted or at least mono-substituted mono- or bicyclic ring system;
R^{3b} represents a hydrogen atom; -F; -Cl; -Br; -I; -NO₂; -CN; -O-R^{10b}; -S-R^{11b}; a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via an unsubstituted or at least mono-substituted alkylene group and/or which may be condensed with an unsubstituted or at least mono-substituted mono- or bicyclic ring system; an unsubstituted or at least mono-substituted aryl or heteroaryl radical, which may be bonded via an unsubstituted or at least mono-substituted alkylene group and/or which may be condensed with an unsubstituted or at least mono-substituted mono- or bicyclic ring system; a -CH(OC₂H₅)-CH₂-NR^{13b}R^{14b} moiety or a -(CH₂)_{nb}-NR^{13b}R^{14b} moiety with nb = 0, 1, 2, 3, 4 or 5; a -S(=O)₂-R^{9b} moiety; a -S(=O)₂-C(H)A^{b}B^{b} moiety; or a -C(=O)-(CH₂)_{pb}-C(=O)-N-D^{b} E^{b} moiety with pb = 0, 1, 2, 3, 4 or 5;
R^{4b}, R^{5b}, R^{6b} and R^{7b}, independently of one another, each represent a hydrogen atom; -NO₂; -NH₂; -SH; -OH; -CN; -C(=O)-OH; -C(=O)-H; -S(=O)₂-OH; -C(=O)-NH₂; -S(=O)₂-NH₂; -C(=O)-R^{8b}; -S(=O)₂-R^{9b}; -O-R^{10b}; -S-R^{11b};-C(=O)-OR^{12b}; -N(R^{15b})-S(=O)₂-R^{16b}; -NH-R^{17b}; -NR^{18b}R^{19b}; -C(=O)-NHR^{20b},-C(=O)-NR^{21b}R^{22b}; -S(=O)₂-NHR^{23b}; -S(=O)₂-NR^{24b}R^{25b}; -O-C(=O)-R^{26b}; -NH-C(=O)-R^{27b}; -NR^{28b}-C(=O)-R^{29b}; NH-C(=O)-O-R^{30b}; NR^{31b}-C(=O)-O-R^{32b};-S(=O)₂-O-R^{33b} ; a halogen atom; a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via a linea or branched alkylene group; or an unsubstituted or at least mono-substituted aryl or heteroaryl radical, which may be bonded via a linear or branched alkylene group;
with the proviso that at least one of the substituents R^{4b}, R^{5b}, R^{6b} and R^{7b} represents a -N(R^{15b})-S(=O)-R^{16b} moiety;
R^{8b}, R^{12b}, R^{17b}, R^{18b}, R^{19b}, R^{20b}, R^{21b}, R^{22b}, R^{23b}, R²⁴b, R^{25b}, R^{26b}, R^{27b}, R^{28b}, R^{29b}, R^{30b}, R^{31b}, R^{32b} and R^{33b}, independently of one another, each represent a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via a linear or branched alkylene group; or an unsubstituted or at least mono-substituted aryl or heteroaryl radical, which may be bonded via a linear or branched alkylene, alkenylene or alkinylene group;
R^{9b} represents a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via a linear or branched alkylene group and/or which may be condensed with an unsubstituted or at least mono-substituted mono- or bicyclic ring system; or an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
R¹⁰b and R^{11b}, independently of one another, each represent a linear or branched, saturated or unsaturated, unsubstituted or at least mono-substituted aliphatic radical; or an unsubstituted or at least mono-substituted aryl or heteroaryl radical, which may be bonded via a linear or branched alkylene group;
R^{13b} and R^{14b}, independently of one another, each represent a hydrogen atom; or a linear or branched, saturated or unsaturated, unsubstituted or at least mono-substituted aliphatic radical;
or
R^{13b} and R^{14b} together with the bridging nitrogen form an unsubstituted or at least mono-substituted, saturated, unsaturated or aromatic heterocyclic ring which may contain at least one further heteroatom as a ring member and/or which may be condensed with an unsubstituted or at least mono-substituted mono- or bicyclic ring system;
R^{15b} represents a hydrogen atom; a linear or branched, saturated or unsaturated, unsubstituted or at least mono-substituted aliphatic radical or a-S(=O)₂-R^{16b} moiety;
R^{16b} represents a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via a linear or branched, unsubstituted or at least mono-substituted alkylene, alkenylene or alkinylene group and/or which may be condensed with an unsubstituted or at least mono-substituted mono- or bicyclic ring system; or an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
A^{b} and B^{b} together with the bridging carbon form an unsubstituted or at least mono-substituted, saturated or unsaturated cycloaliphatic ring which may contain at least one further heteroatom as a ring member and/or which may be condensed with an unsubstituted or at least mono-substituted mono- or bicyclic ring system;
D^{b} and E^{b} together with the bridging nitrogen form an unsubstituted or at least mono-substituted, saturated, unsaturated or aromatic heterocyclic ring which may contain at least one further heteroatom as a ring member and/or which may be condensed with an unsubstituted or at least mono-substituted mono-or bicyclic ring system;
or
D^{b} and E^{b}, independently of one another, each represent a hydrogen atom; a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via a linear or branched alkylene group and/or which may be condensed with an unsubstituted or at least mono-substituted mono- or bicyclic ring system; or an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof, respectively:
and indazolyl- and (2,3)-dihydro-indolyl-derived sulfonamide compounds of general formula (Ic) 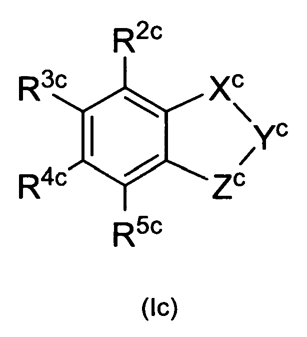 wherein
X^{c}-Y^{c} from left to right represents CR^{1c}=N and Z^{c} is N[(CH_{2c})_{nc}R^{6c}]
or
X^{c}-Y^{c} from left to right represents CR^{7c}=N, Z^{c} is NH, R^{7c} represents the following moiety 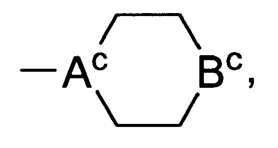 A^{c} represents CH or N and B^{c} represents NR^{8c}, O or S;
X^{c}-Y^{c} from left to right represents C[(CH_{2c})_{nc}R^{9c}]=N and Z^{c} is NR^{10c}
or
X^{c}-Y^{c} represents CH₂-CH₂ and Z^{c} is N[(CH_{2c})_{nc}R^{11c}];
nc is 0, 1, 2, 3 or 4;
R^{1c} represents a hydrogen atom; NO₂; -NH₂; -SH; -OH; -CN; -C(=O)-R^{12c};-OR^{13c}; -SR^{14c}; -F; -Cl, -Br; -I; a linear or branched, saturated or unsaturated, unsubstituted or at least mono-substituted aliphatic radical; or an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
R^{2c}, R^{3c}, R^{4c} and R^{5c}, independently of one another, each represent a hydrogen atom; -NO₂; -NH₂; -SH; -OH; -CN; -C(=O)-H; -C(=O)-R^{12c}; -OR^{13c};-SR^{14c}; -N(R^{15c})-S(=O)₂-R^{16c}; -NH-R^{17c}; -NR^{18c}R^{19c}; -F; -Cl, -Br; -I; a linear or branched, saturated or unsaturated, unsubstituted or at least mono-substituted aliphatic radical; or an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
with the proviso that at least one of the substituents R^{2c}, R^{3c}, R^{4c} and R^{5c} represents a -N(R^{15c})-S(=O)₂-R^{16c} moiety;
R ^{6c}, R^{9c} and R^{11c}, independently of one another, each represent a -NR^{20c}R^{21c} radical
or
a saturated or unsaturated, unsubstituted or at least mono-substituted, optionally at least one heteroatom as a ring member containing cycloaliphatic radical, which may be bonded via a linear or branched, unsubstituted or at least mono-substituted alkylene, alkenylene or alkinylene group and/or which may be condensed with an unsubstituted or at least mono-substituted mono-or bicyclic ring system;
R^{8c} represents -C(=O)-R^{22c}; a linear or branched, saturated or unsaturated, unsubstituted or at least mono-substituted aliphatic radical; or an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
R^{10c} represents a linear or branched, saturated or unsaturated, unsubstituted or at least mono-substituted aliphatic radical-; or a -S(=O)₂-R^{23c} moiety;
R^{12C}, R^{13c} R^{14c}, R^{17c}, R^{18c} and R^{19c}, independently of one another, each represent a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via a linear or branched, unsubstituted or at least mono-substituted alkylene, alkenylene or alkinylene group and/or which may be condensed with an unsubstituted or at least mono-substituted mono- or bicyclic ring system; or an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
R^{15c} represents a hydrogen atom; a linear or branched, saturated or unsaturated, unsubstituted or at least mono-substituted aliphatic radical; or a-S(=O)₂-R^{24c} moiety;
R^{16c} and R^{24c}, independently of one another, each represent an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
R^{20c} and R^{21c}, independently of one another, each represent a hydrogen atom; or a linear or branched, saturated or unsaturated, unsubstituted or at least mono-substituted aliphatic radical;
or R^{20c} and R^{21c} together with the bridging nitrogen form an unsubstituted or at least mono-substituted, saturated, unsaturated or aromatic heterocyclic ring which may contain at least one further heteroatom as a ring member and/or which may be condensed with an unsubstituted or at least mono-substituted mono- or bicyclic ring system;
R^{22c} represents a linear or branched, saturated or unsaturated, unsubstituted or at least mono-substituted aliphatic radical; or an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
and
R^{23c} represents a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via a linear or branched, unsubstituted or at least mono-substituted alkylene, alkenylene or alkinylene group and/or which may be condensed with an unsubstituted or at least mono-substituted mono- or bicyclic ring system; or an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof, respectively;
and phenyl-piperazine-derived compounds of general formula (Id) 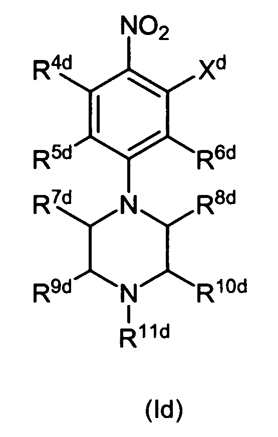 wherein
X^{d} represents a -NR1^{d}R^{2d} moiety or a -OR^{3d} moiety;
R^{1d} represents a linear or branched, saturated or unsaturated, unsubstituted or at least mono-substituted aliphatic radical;
an unsubstituted or at least mono-substituted radical selected from the group consisting of adamantyl, bicyclo[2.2.1]heptyl and bicyclo[3.1.1]heptyl, which may be bonded via a linear or branched, unsubstituted or at least mono-substituted alkylene, alkenylene or alkinylene group which may contain 1, 2 or 3 heteroatom(s) independently selected from the group consisting of nitrogen, oxygen and sulfur as chain member(s);
a saturated or unsaturated, unsubstituted or at least mono-substituted, optionally at least one heteroatom as a ring member containing cycloaliphatic radical, which may be bonded via a linear or branched, unsubstituted or at least mono-substituted alkylene, alkenylene or alkinylene group and/or which may be condensed with an unsubstituted or at least mono-substituted mono- or bicyclic ring system;
or an unsubstituted or at least mono-substituted aryl or heteroaryl radical, which may be condensed with an unsubstituted or at least mono-substituted mono- or bicyclic ring system and/or which may be bonded via a linear or branched, unsubstituted or at least mono-substituted alkylene, alkenylene or alkinylene group which may contain 1, 2 or 3 heteroatom(s) independently selected from the group consisting of nitrogen, oxygen and sulfur as chain member(s); or a -C(=O)-R^{12d} moiety;
R^{2d} represents a hydrogen atom or a linear or branched, saturated or unsaturated, unsubstituted or at least mono-substituted aliphatic radical;
or
R^{1d} and R^{2d} together with the bridging nitrogen form an optionally at least mono-substituted, saturated, unsaturated or aromatic heterocyclic ring which may contain at least one further heteroatom as a ring member and/or which may be condensed with an unsubstituted or at least mono-substituted mono- or bicyclic ring system;
R^{3d} represents or an unsubstituted or at least mono-substituted aryl or heteroaryl radical, which may be condensed with an unsubstituted or at least mono-substituted mono- or bicyclic ring system and/or which may be bonded via a linear or branched, unsubstituted or at least mono-substituted alkylene, alkenylene or alkinylene group;
R^{4d}, R^{5d} and R^{6d}, independently of one another, each represent a hydrogen atom or a halogen atom;
or
R^{4d} and R^{5d} together with the bridging carbon atoms form an unsubstituted 5- or 6-membered heterocyclic ring which contains 1, 2 or 3 heteroatom(s) independently selected from the group consisting of nitrogen, oxygen and sulfur as ring member(s) and which together with the phenyl ring which it is fused with forms a 9- or 10-membered bicyclic aromatic ring system;
R^{7d} and R^{8d}, independently of one another, each represent a hydrogen atom or a linear or branched, saturated or unsaturated, unsubstituted or at least mono-substituted aliphatic radical;
R^{9d} and R^{10d}, independently of one another, each represent a hydrogen atom or a linear or branched, saturated or unsaturated, unsubstituted or at least mono-substituted aliphatic radical;
R^{11d} represents a hydrogen atom; or a linear or branched, saturated or unsaturated, unsubstituted or at least mono-substituted aliphatic radical, which may contain 1, 2 or 3 heteroatom(s) independently selected from the group consisting of nitrogen, oxygen and sulfur as chain member(s);
or an unsubstituted or at least mono-substituted aryl or heteroaryl radical, which may be condensed with an unsubstituted or at least mono-substituted mono- or bicyclic ring system and/or which may be bonded via a linear or branched, unsubstituted or at least mono-substituted alkylene, alkenylene or alkinylene group; -a -C(=O)-R^{13d} moiety or a -S(=O)₂-R^{14d} moiety;
R^{12d} represents a linear or branched, saturated or unsaturated, unsubstituted or at least mono-substituted aliphatic radical;
or an unsubstituted or at least mono-substituted aryl or heteroaryl radical, which may be condensed with an unsubstituted or at least mono-substituted mono- or bicyclic ring system and/or which may be bonded via a linear or branched, unsubstituted or at least mono-substituted alkylene, alkenylene or alkinylene group; and
R^{13d} and R^{14d}, independently of one another, each represent a linear or branched, saturated or unsaturated, unsubstituted or at least mono-substituted aliphatic radical; or an unsubstituted or at least mono-substituted aryl or heteroaryl radical, which may be condensed with an unsubstituted or at least mono-substituted mono- or bicyclic ring system and/or which may be bonded via a linear or branched, unsubstituted or at least mono-substituted alkylene, alkenylene or alkinylene group which may contain 1, 2 or 3 heteroatom(s) independently selected from the group consisting of nitrogen, oxygen and sulfur as chain member(s);
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof, respectively;
and phenyl-piperazine-derived compounds of general formula (Ie) 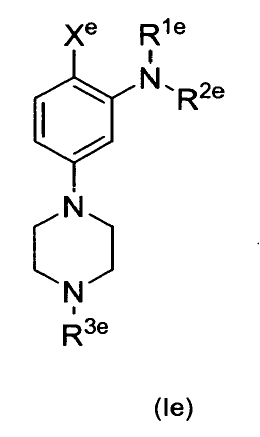 wherein
X^{e} represents -CN, -C(=O)-OH, -C(=O)-OR ^{4e}, -O-R^{5e}, -NH₂, -NR^{6e}-C(=O)-R^{7e}, -NH-S(=O)₂-R^{8e} or -NH-R^{9e};
R^{1e} represents a hydrogen atom;
a saturated or unsaturated, unsubstituted or at least mono-substituted, optionally at least one heteroatom as a ring member containing cycloaliphatic radical, which may be bonded via a linear or branched, unsubstituted or at least mono-substituted alkylene, alkenylene or alkinylene group and/or which may be condensed with an unsubstituted or at least mono-substituted mono- or bicyclic ring system;
or an unsubstituted or at least mono-substituted aryl or heteroaryl radical, which may be condensed with an unsubstituted or at least mono-substituted mono- or bicyclic ring system and/or which may be bonded via a linear or branched, unsubstituted or at least mono-substituted alkylene, alkenylene or alkinylene group which may contain 1, 2 or 3 heteroatom(s) independently selected from the group consisting of nitrogen, oxygen and sulfur as chain member(s);
R^{2e} represents a hydrogen atom or a -C(=O)-R^{10e} moiety;
or
R^{1e} and R^{2e} together with the bridging nitrogen form a nitro (NO₂)-group or
an unsubstituted or at least mono-substituted 5- or 6-membered heteroaryl radical which may be condensed with an unsubstituted or at least mono-substituted mono- or bicyclic ring system;
R^{3e} represents a linear or branched, saturated or unsaturated, unsubstituted or at least mono-substituted aliphatic radical;
R^{4e} represents a linear or branched, saturated or unsaturated, unsubstituted or at least mono-substituted aliphatic radical;
R^{5e} represents a linear or branched, saturated or unsaturated, unsubstituted or at least mono-substituted aliphatic radical; or
or an unsubstituted or at least mono-substituted aryl or heteroaryl radical, which may be condensed with an unsubstituted or at least mono-substituted mono- or bicyclic ring system and/or which may be bonded via a linear or branched, unsubstituted or at least mono-substituted alkylene, alkenylene or alkinylene group;
R^{6e} represents a hydrogen atom or
an unsubstituted or at least mono-substituted aryl or heteroaryl radical, which may be condensed with an unsubstituted or at least mono-substituted mono- or bicyclic ring system and/or which may be bonded via a linear or branched, unsubstituted or at least mono-substituted alkylene, alkenylene or alkinylene group;
R^{7e} represents a linear or branched, saturated or unsaturated, unsubstituted or at least mono-substituted aliphatic radical;
R^{8e} represents a linear or branched, saturated or unsaturated, unsubstituted or at least mono-substituted aliphatic radical;
or an unsubstituted or at least mono-substituted aryl or heteroaryl radical, which may be condensed with an unsubstituted or at least mono-substituted mono- or bicyclic ring system and/or which may be bonded via a linear or branched, unsubstituted or at least mono-substituted alkylene, alkenylene or alkinylene group;
R^{9e} represents a linear or branched, saturated or unsaturated, unsubstituted or at least mono-substituted aliphatic radical;
or an unsubstituted or at least mono-substituted aryl or heteroaryl radical, which may be condensed with an unsubstituted or at least mono-substituted mono- or bicyclic ring system and/or which may be bonded via a linear or branched, unsubstituted or at least mono-substituted alkylene, alkenylene or alkinylene group;
and
R^{10e} represents a linear or branched, saturated or unsaturated, unsubstituted or at least mono-substituted aliphatic radical;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof, respectively;
and tetrahydroisoquinoline-derived sulfonamide compounds of general formula (If) 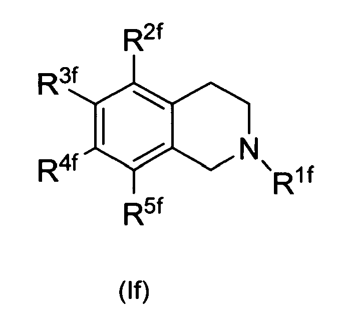 wherein
R^{1f} represents a hydrogen atom; a -C(=O)-OR^{37f} moiety;
a linear or branched, saturated or unsaturated, unsubstituted or at least mono-substituted aliphatic radical; or a saturated or unsaturated, unsubstituted or at least mono-substituted, optionally at least one heteroatom as a ring member containing cycloaliphatic radical, which may be bonded via a linear or branched, unsubstituted or at least mono-substituted alkylene, alkenylene or alkinylene group and/or which may be condensed with an unsubstituted or at least mono-substituted mono- or bicyclic ring system;
R^{2f}, R^{3f} R^{4f} and R^{5f}, independently of one another, each represent a hydrogen atom; F, Cl, Br, 1, -NO₂; -NH₂; -SH; -OH; -CN; -C(=O)-OH; -C(=O)-H;-S(=O)₂-OH; -C(=O)-NH₂; -S(=O)₂-NH₂; -C(=O)-R^{6f}; -S(=O)-R^{7f}; -S(=O)₂-R^{7f}; -OR^{8f}; -SR^{9f}; -C(=O)-OR^{10f}; -N(R^{11f})-S(=O)₂-R^{12f}; -NR^{13f}R^{14f}; -NH-R^{15f}; -C(=O)-NR^{16f}R^{17f}; C(=O)-NHR^{18f};
a linear or branched, saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono- or bicyclic ring system;
with the proviso that at least one of the substituents R^{2f}, R^{3f}, R^{4f} and R^{5f} represents a -N(R^{11f})-S(=O)₂-R^{12f} moiety;
R^{6f}, R^{7f}, R^{8f}, R^{9f}, R^{10f}, R^{13f}, R^{14f}, R^{15f}, R^{16f}, R^{17f} and R^{18f} independently of one another, each represent a linear or branched, saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono- or bicyclic ring system;
R^{11f} represents a hydrogen atom or a linear or branched, saturated or unsaturated, unsubstituted or at least mono-substituted aliphatic radical;
R^{12f} represents a phenyl radical of general formula (Af), 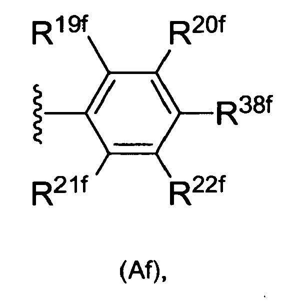 wherein
R^{19f}, R^{20f}, R^{21f}, R^{22f} and R^{38f}, independently of one another, each represent a hydrogen atom; F, Cl, Br, I, -NO₂; -NH₂; -SH; -OH;-CN; -C(=O)-OH; -C(=O)-H; -S(=O)₂-OH; -C(=O)-NH₂; -S(=O)₂-NH₂; -C(=O)-R^{23f}; -S(=O)-R^{24f}; -S(=O)₂-R^{24f}; -OR^{25f}; -SR^{26f}; -C(=O)-OR^{27f}; -N(R^{28f})-S(=O)₂-R^{29f}; -NH-S(=O)₂-R^{30f}; -NR^{31f}R^{32f};-NH-R^{33f}; -C(=O)-NHR^{34f}; -C(=O)-NR^{35f}R^{36f}; a linear or branched, saturated or unsaturated, unsubstituted or at least mono-substituted aliphatic radical; or a saturated or unsaturated, unsubstituted or at least mono-substituted, optionally at least one heteroatom as a ring member containing cycloaliphatic radical, which may be bonded via a linear or branched, unsubstituted or at least mono-substituted alkylene, alkenylene or alkinylene group and/or which may be condensed with an unsubstituted or at least mono-substituted mono- or bicyclic ring system;
an unsubstituted or at least mono-substituted aryl or heteroaryl radical, which may be condensed with an unsubstituted or at least mono-substituted mono- or bicyclic ring system and/or which may be bonded via a linear or branched, unsubstituted or at least mono-substituted alkylene, alkenylene or alkinylene group;
or a saturated or unsaturated, unsubstituted or at least mono-substituted, optionally at least one heteroatom as a ring member containing cycloaliphatic radical, which may be condensed with an unsubstituted or at least mono-substituted saturated, unsaturated or aromatic mono- or bicyclic ring system;
R^{23f}, R^{27f}, R^{28f}, R^{29f} and R^{30f}, independently of one another, each represent
a linear or branched, saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono- or bicyclic ring system;
R^{24f}, R^{26f}, R^{31f} R^{32f} and R^{33f}, each represent a linear or branched, saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be condensed with an unsubstituted or at least mono-substituted saturated or unsaturated, but not aromatic, mono- or bicyclic ring system;
R^{25f}, R^{34f}, R^{35f} and R^{36f}, represents a linear or branched, saturated or unsaturated, unsubstituted or at least mono-substituted aliphatic radical;
and R^{37f} represents a linear or branched, saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono- or bicyclic ring system;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

6. The combination according to claim 5, **characterized in that** the indole-derived sulfonamide is selected from the group consisting of compounds of general formula (Ih) 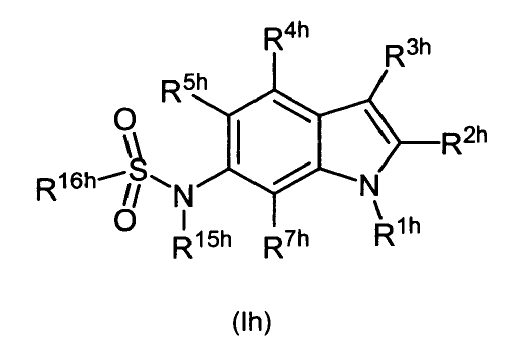 wherein
R^{1h} represents a saturated or unsaturated, unsubstituted or at least mono-substituted, optionally at least one heteroatom as a ring member containing cycloaliphatic radical, which may be bonded via a linear or branched alkylene group and/or which may be condensed with an unsubstituted or at least mono-substituted mono- or bicyclic ring system; or a -(CH₂)ₘₕ-NR^{13h}R^{14h} moiety with mh = 0, 1, 2, 3, 4 or 5;
R^{2th} represents a hydrogen atom; -F; -Cl; -Br; -l; -NO₂; -CN; -O-R^{10h}; a linear or branched, saturated or unsaturated, unsubstituted or at least mono-substituted aliphatic radical; or an unsubstituted or at least mono-substituted aryl or heteroaryl radical, which may be bonded via a linear or branched alkylene group and/or which may be condensed with an unsubstituted or at least mono-substituted mono- or bicyclic ring system;
R³ⁿ represents a hydrogen atom; -F; -Cl; -Br; -l; -NO₂; -CN; -O-R^{10h}; a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via an unsubstituted or at least mono-substituted alkylene group and/or which may be condensed with an unsubstituted or at least mono-substituted mono- or bicyclic ring system; an unsubstituted or at least mono-substituted aryl or heteroaryl radical, which may be bonded via an unsubstituted or at least mono-substituted alkylene group and/or which may be condensed with an unsubstituted or at least mono-substituted mono- or bicyclic ring system; or a -(CH2)ₙₕ-NR^{13h}R^{14h} moiety with nh = 0, 1, 2, 3, 4 or 5;
R^{4h}, R^{5h} and R^{7h}, independently of one another, each represent a hydrogen atom; -NO₂; -CN; -O-R^{10h}; -C(=O)-OR^{12h}; a halogen atom; a linear or branched, saturated or unsaturated, unsubstituted or at least mono-substituted aliphatic radical; or an unsubstituted or at least mono-substituted aryl or heteroaryl radical, which may be bonded via a linear or branched alkylene group;
R^{10h} represents a linear or branched, saturated or unsaturated, unsubstituted or at least mono-substituted aliphatic radical; or an unsubstituted or at least mono-substituted aryl or heteroaryl radical, which may be bonded via a linear or branched alkylene group;
R^{13h} and R^{14h}, independently of one another, each represent a hydrogen atom; or a linear or branched, saturated or unsaturated, unsubstituted or at least mono-substituted aliphatic radical;
or
R^{13h} and R^{14h} together with the bridging nitrogen form an unsubstituted or at least mono-substituted, saturated, unsaturated or aromatic heterocyclic ring which may contain at least one further heteroatom as a ring member and/or which may be condensed with an unsubstituted or at least mono-substituted mono- or bicyclic ring system;
R^{15h} represents a hydrogen atom; a linear or branched, saturated or unsaturated, unsubstituted or at least mono-substituted aliphatic radical or a-S(=O)₂-R^{16h} moiety;
and R^{16h} represents an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof, respectively;
and compounds of general formula (Ik) 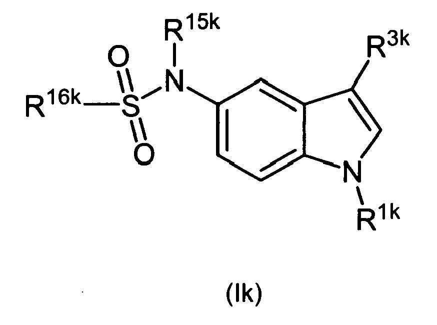 wherein
R^{1k} represents a hydrogen atom; a linear or branched, saturated or unsaturated, unsubstituted or at least mono-substituted aliphatic radical; an unsubstituted or at least mono-substituted phenyl radical or an unsubstituted or at least mono-substituted benzyl radical;
R^{3k} represents a saturated or unsaturated, unsubstituted or at least mono-substituted, optionally at least one heteroatom as a ring member containing cycloaliphatic radical, which may be bonded via an unsubstituted or at least mono-substituted alkylene group and/or which may be condensed with an unsubstituted or at least mono-substituted mono- or bicyclic ring system; or a-(CH₂)ₙₖ-NR^{13k}R^{14k} moiety with nk = 0, 1, 2, 3, 4 or 5;
R^{13k} and R^{14k}, independently of one another, each represent a hydrogen atom; or a linear or branched, saturated or unsaturated, unsubstituted or at least mono-substituted aliphatic radical;
or
R^{13k} and R^{14k} together with the bridging nitrogen form an unsubstituted or at least mono-substituted, saturated, unsaturated or aromatic heterocyclic ring which may contain at least one further heteroatom as a ring member and/or which may be condensed with an unsubstituted or at least mono-substituted mono- or bicyclic ring system;
R^{15k} represents a hydrogen atom; or a linear or branched, saturated or unsaturated, unsubstituted or at least mono-substituted aliphatic radical;
and R^{16k} represents an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof, respectively;
and compounds of general formula (Im) 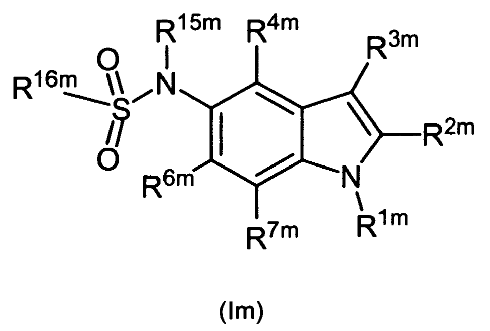 wherein
R^{1m} represents a saturated or unsaturated, unsubstituted or at least mono-substituted, optionally at least one heteroatom as a ring member containing cycloaliphatic radical, which may be bonded via a linear or branched alkylene group and/or which may be condensed with an unsubstituted or at least mono-substituted mono- or bicyclic ring system; or a -(CH₂)ₘₘ-NR^{13m}R^{14m} moiety with mm = 0, 1, 2, 3, 4 or 5;
R^{2m} represents a hydrogen atom; -F; -Cl; -Br; -I; -NO₂; -CN; -O-R^{10m}; a linear or branched, saturated or unsaturated, unsubstituted or at least mono-substituted aliphatic radical; or an unsubstituted or at least mono-substituted aryl or heteroaryl radical, which may be bonded via a linear or branched alkylene group and/or which may be condensed with an unsubstituted or at least mono-substituted mono- or bicyclic ring system;
R^{3m} represents a hydrogen atom; -F; -Cl; -Br; -I; -NO₂; -CN; -O-R^{10m}; a linear or branched, saturated or unsaturated, unsubstituted or at least mono-substituted aliphatic radical; or an unsubstituted or at least mono-substituted aryl or heteroaryl radical, which may be bonded via an unsubstituted or at least mono-substituted alkylene group and/or which may be condensed with an unsubstituted or at least mono-substituted mono- or bicyclic ring system;
R^{4m}, R^{6m} and R^{7m}, independently of one another, each represent a hydrogen atom; -NO₂; -CN; -O-R^{10m}; a halogen atom; a linear or branched, saturated or unsaturated, unsubstituted or at least mono-substituted aliphatic radical; or an unsubstituted or at least mono-substituted aryl or heteroaryl radical, which may be bonded via a linear or branched alkylene group;
R^{10m} represents a linear or branched, saturated or unsaturated, unsubstituted or at least mono-substituted aliphatic radical; or an unsubstituted or at least mono-substituted aryl or heteroaryl radical, which may be bonded via a linear or branched alkylene group;
R^{13m} and R^{14m}, independently of one another, each represent a hydrogen atom; or a linear or branched, saturated or unsaturated, unsubstituted or at least mono-substituted aliphatic radical;
or
R^{13m} and R^{14m} together with the bridging nitrogen form an unsubstituted or at least mono-substituted, saturated, unsaturated or aromatic heterocyclic ring which may contain at least one further heteroatom as a ring member and/or which may be condensed with an unsubstituted or at least mono-substituted mono- or bicyclic ring system;
R^{15m} represents a hydrogen atom; a linear or branched, saturated or unsaturated, unsubstituted or at least mono-substituted aliphatic radical or a-S(=O)₂-R^{16m} moiety;
and R^{16m} represents an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof, respectively;
and compounds of general formula (In) 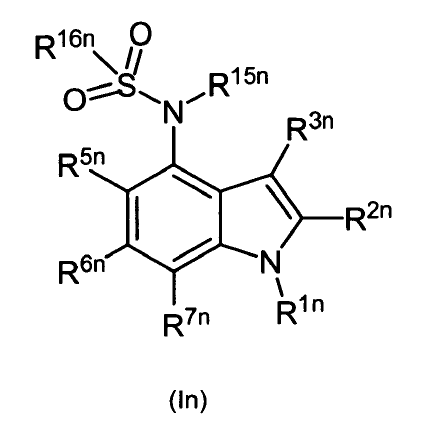 wherein
R¹ⁿ represents a saturated or unsaturated, unsubstituted or at least mono-substituted, optionally at least one heteroatom as a ring member containing cycloaliphatic radical, which may be bonded via a linear or branched alkylene group and/or which may be condensed with an unsubstituted or at least mono-substituted mono- or bicyclic ring system; or a -(CH₂)ₘₙ-NR¹³ⁿR¹⁴ⁿ moiety with mn = 0, 1, 2, 3, 4 or 5;
R²ⁿ represents a hydrogen atom; -F; -Cl; -Br; -1; -NO₂; -CN; -O-R¹⁰ⁿ; a linear or branched, saturated or unsaturated, unsubstituted or at least mono-substituted aliphatic radical; or an unsubstituted or at least mono-substituted aryl or heteroaryl radical, which may be bonded via a linear or branched alkylene group and/or which may be condensed with an unsubstituted or at least mono-substituted mono- or bicyclic ring system;
R³ⁿ represents a hydrogen atom; -F; -Cl; -Br; -I; -NO₂; -CN; -O-R¹⁰ⁿ; a linear or branched, saturated or unsaturated, unsubstituted or at least mono-substituted aliphatic radical; an unsubstituted or at least mono-substituted aryl or heteroaryl radical, which may be bonded via an unsubstituted or at least mono-substituted alkylene group and/or which may be condensed with an unsubstituted or at least mono-substituted mono- or bicyclic ring system; or a-(CH₂)ₙₙ-NR¹³ⁿR¹⁴ⁿ moiety with nn = 0, 1, 2, 3, 4 or 5;
R⁵ⁿ, R⁶ⁿ and R⁷ⁿ, independently of one another, each represent a hydrogen atom; -NO₂; -CN; -O-R¹⁰ⁿ; a halogen atom; a linear or branched, saturated or unsaturated, unsubstituted or at least mono-substituted aliphatic radical; or an unsubstituted or at least mono-substituted aryl or heteroaryl radical, which may be bonded via a linear or branched alkylene group;
R¹⁰ⁿ represents a linear or branched, saturated or unsaturated, unsubstituted or at least mono-substituted aliphatic radical; or an unsubstituted or at least mono-substituted aryl or heteroaryl radical, which may be bonded via a linear or branched alkylene group;
R¹³ⁿ and R¹⁴ⁿ, independently of one another, each represent a hydrogen atom; or a linear or branched, saturated or unsaturated, unsubstituted or at least mono-substituted aliphatic radical;
or
R¹³ⁿ and R¹⁴ⁿ together with the bridging nitrogen form an unsubstituted or at least mono-substituted, saturated, unsaturated or aromatic heterocyclic ring which may contain at least one further heteroatom as a ring member and/or which may be condensed with an unsubstituted or at least mono-substituted mono- or bicyclic ring system;
R¹⁵ⁿ represents a hydrogen atom; a linear or branched, saturated or unsaturated, unsubstituted or at least mono-substituted aliphatic radical or a-S(=O)₂₋R¹⁶ⁿ moiety;
R¹⁶ⁿ represents an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono-or bicyclic ring system;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof, respectively;
and compounds of general formula (Io) 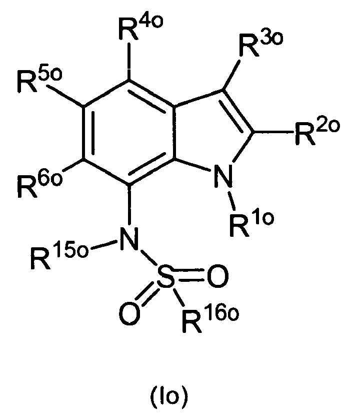 wherein
R^{1o} represents a saturated or unsaturated, unsubstituted or at least mono-substituted, optionally at least one heteroatom as a ring member containing cycloaliphatic radical, which may be bonded via a linear or branched alkylene group and/or which may be condensed with an unsubstituted or at least mono-substituted mono- or bicyclic ring system; or a -(CH₂)ₘₒ-NR^{13o}R^{14o} moiety with mo = 0, 1, 2, 3, 4 or 5;
R^{2o} represents a hydrogen atom; -F; -Cl; -Br; -I; -NO₂; -CN; -O-R^{10o}; a linear or branched, saturated or unsaturated, unsubstituted or at least mono-substituted aliphatic radical; or an unsubstituted or at least mono-substituted aryl or heteroaryl radical, which may be bonded via a linear or branched alkylene group and/or which may be condensed with an unsubstituted or at least mono-substituted mono- or bicyclic ring system;
R³⁰ represents a hydrogen atom; -F; -Cl; -Br; -1; -NO₂; -CN; -O-R^{10o}; a linear or branched, saturated or unsaturated, unsubstituted or at least mono-substituted aliphatic radical; an unsubstituted or at least mono-substituted aryl or heteroaryl radical, which may be bonded via an unsubstituted or at least mono-substituted alkylene group and/or which may be condensed with an unsubstituted or at least mono-substituted mono- or bicyclic ring system; a-CH(OC₂H₅)-CH₂-NR^{13o}R^{14o} moiety or a -(CH₂)ₙₒ-NR^{13o}R^{14o} moiety with no = 0, 1,2, 3, 4 or 5;
R^{4o}, R^{5o} and R^{6o}, independently of one another, each represent a hydrogen atom; -NO₂; -CN; -O-R^{10o}; a halogen atom; a linear or branched, saturated or unsaturated, unsubstituted or at least mono-substituted aliphatic radical; or an unsubstituted or at least mono-substituted aryl or heteroaryl radical, which may be bonded via a linear or branched alkylene group;
R^{10o} represents a linear or branched, saturated or unsaturated, unsubstituted or at least mono-substituted aliphatic radical; or an unsubstituted or at least mono-substituted aryl or heteroaryl radical, which may be bonded via a linear or branched alkylene group;
R^{13o} and R^{14o}, independently of one another, each represent a hydrogen atom; or a linear or branched, saturated or unsaturated, unsubstituted or at least mono-substituted aliphatic radical;
or
R^{13o} and R^{14o} together with the bridging nitrogen form an unsubstituted or at least mono-substituted, saturated, unsaturated or aromatic heterocyclic ring which may contain at least one further heteroatom as a ring member and/or which may be condensed with an unsubstituted or at least mono-substituted mono- or bicyclic ring system;
R^{15o} represents a hydrogen atom; a linear or branched, saturated or unsaturated, unsubstituted or at least mono-substituted aliphatic radical or a-S(=O)₂-R^{16o} moiety;
and R^{16o} represents an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof, respectively;
and compounds of general formula (1p) 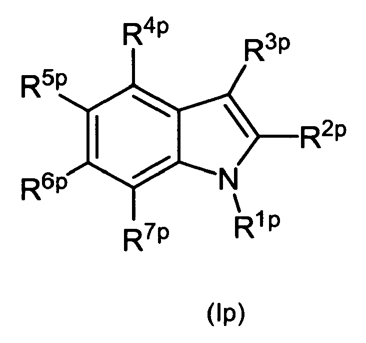 wherein
R^{1p}represents a -S(=O)₂R^{9p} moiety or a -S(=O)₂-C(H)A^{p}B^{p} moiety;
R^{2p} represents a hydrogen atom; -F; -Cl; -Br; -l; -NO₂; -OH; -CN; -O-R^{10p}; -S-R^{11p}; a linear or branched, saturated or unsaturated, unsubstituted or at least mono-substituted aliphatic radical; or a saturated or unsaturated, unsubstituted or at least mono-substituted, optionally at least one heteroatom as a ring member containing cycloaliphatic radical, which may be bonded via a linear or branched alkylene group and/or which may be condensed with an unsubstituted or at least mono-substituted mono- or bicyclic ring system;
R^{3p} represents a saturated or unsaturated, unsubstituted or at least mono-substituted, optionally at least one heteroatom as a ring member containing cycloaliphatic radical, which may be bonded via an unsubstituted or at least mono-substituted alkylene group and/or which may be condensed with an unsubstituted or at least mono-substituted mono- or bicyclic ring system; or a-(CH₂)ₙₚ-NR^{13p}R^{14p} moiety with np = 0, 1, 2, 3, 4 or 5;
R^{4p}, R^{5p}, R^{6p} and R^{7p}, independently of one another, each represent a hydrogen atom; -NO₂; -NH₂; -OH; -CN; -C(=O)-R^{8p-}; -O-R^{10p}; -S-R^{11p}; -NH-R^{17p}; -NR^{18p}R^{19p}; a halogen atom; a linear or branched, saturated or unsaturated, unsubstituted or at least mono-substituted aliphatic radical; or a saturated or unsaturated, unsubstituted or at least mono-substituted, optionally at least one heteroatom as a ring member containing cycloaliphatic radical, which may be bonded via a linear or branched alkylene group;
R^{8p} represents a hydrogen atom or a linear or branched, saturated or unsaturated, unsubstituted or at least mono-substituted aliphatic radical;
R^{8p}, R^{17p}, R^{18p} and R^{19p}, independently of one another, each represent a linear or branched, saturated or unsaturated, unsubstituted or at least mono-substituted aliphatic radical; or an unsubstituted or at least mono-substituted aryl or heteroaryl radical, which may be bonded via a linear or branched alkylene, alkenylene or alkinylene group;
R^{9p} represents a linear or branched, saturated or unsaturated, unsubstituted or at least mono-substituted aliphatic radical;
R^{10p} and R^{11p}, independently of one another, each represent a linear or branched, saturated or unsaturated, unsubstituted or at least mono-substituted aliphatic radical; or an unsubstituted or at least mono-substituted aryl or heteroaryl radical, which may be bonded via a linear or branched alkylene group;
R^{13p} and R^{14p}, independently of one another, each represent a hydrogen atom; or a linear or branched, saturated or unsaturated, unsubstituted or at least mono-substituted aliphatic radical;
or
R^{13p} and R^{14p} together with the bridging nitrogen form an unsubstituted or at least mono-substituted, saturated, unsaturated or aromatic heterocyclic ring which may contain at least one further heteroatom as a ring member and/or which may be condensed with an unsubstituted or at least mono-substituted mono- or bicyclic ring system;
A^{p} and B^{p} together with the bridging carbon form an unsubstituted or at least mono-substituted, saturated or unsaturated cycloaliphatic ring which may contain at least one further heteroatom as a ring member and/or which may be condensed with an unsubstituted or at least mono-substituted mono- or bicyclic ring system;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof, respectively;
and compounds of general formula (Iq) 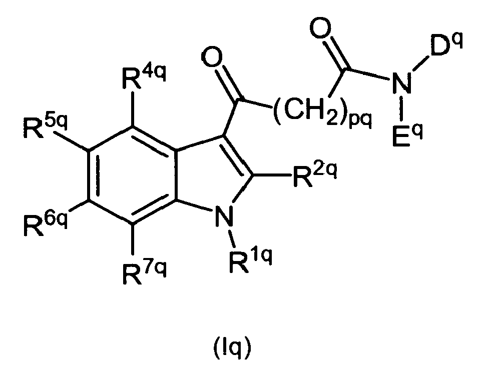 wherein
R^{1q} represents a hydrogen atom; a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via a linear or branched alkylene group and/or which may be condensed with an unsubstituted or at least mono-substituted mono-or bicyclic ring system; an unsubstituted or at least mono-substituted aryl or heteroaryl radical, which may be bonded via a linear or branched alkylene group and/or which may be condensed with an unsubstituted or at least mono-substituted mono- or bicyclic ring system; a -C(=O)-R^{8q} moiety; a -S(=O)₂-R^{9q} moiety;
R^{2q} represents a hydrogen atom; -F; -Cl; -Br; -I; -NO₂; -NH₂; -SH; -OH; -CN; a linear or branched, saturated or unsaturated, unsubstituted or at least mono-substituted, optionally at least one heteroatom as a chain member containing aliphatic radical; a saturated or unsaturated, unsubstituted or at least mono-substituted, optionally at least one heteroatom as a ring member containing cycloaliphatic radical, which may be bonded via a linear or branched alkylene group and/or which may be condensed with an unsubstituted or at least mono-substituted mono- or bicyclic ring system; or an unsubstituted or at least mono-substituted aryl or heteroaryl radical, which may be bonded via a linear or branched alkylene group and/or which may be condensed with an unsubstituted or at least mono-substituted mono- or bicyclic ring system;
R^{4q}, R^{5q}, R^{6q} and R^{7q}, independently of one another, each represent a hydrogen atom; -NO₂; -NH₂; -SH; -OH; -CN; -C(=O)-OH; -C(=O)-H; -S(=O)₂-OH; -C(=O)-NH₂; -S(=O)₂-NH₂; -C(=O)-R^{8q}; -S(=O)₂-R^{9q}; -O-R^{10q}; -S-R^{11q};-C(=O)-OR^{12q}; -N(R^{15q})-S(=O)₂-_{R}^{16q}; -NH-R^{17q}; -NR^{18q}R^{19q}; -C(=O)-NHR^{20q},-C(=O)-NR^{21q}R^{22q}; -S(=O)₂-NHR^{23q}; -S(=O)₂-NR^{24q}R^{25q}; -O-C(=O)-R^{26q}; -NH-C(=O)-R^{27q}; -NR^{28q}-C(=O)-R^{29q}; NH-C(=O)-O-R^{30q}; NR^{31q}-C(=O)-O-R^{32q};-S(=O)₂-O-R^{33q}; a halogen atom; a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via a linear or branched alkylene group; or an unsubstituted or at least mono-substituted aryl or heteroaryl radical, which may be bonded via a linear or branched alkylene group;
with the proviso that at least one of the substituents R^{4q}, R^{5q}, R^{6q} and R^{7q} represents a -N(R^{15q})-S(=O)₂-R^{16q} moiety;
R^{8q}, R^{12q}, R^{17q}, R^{18q}, R^{19q}, R^{20q}, R^{21q}, R^{22q}, R^{23q}, R^{24q}, R^{25q}, R^{26q}, R^{27q}, R^{28q}, R^{29q}, R^{30q}, R^{31q}, R^{32q} and R^{33q}, independently of one another, each represent a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via a linear or branched alkylene group; or an unsubstituted or at least mono-substituted aryl or heteroaryl radical, which may be bonded via a linear or branched alkylene, alkenylene or alkinylene group;
R^{9q} represents a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via a linear or branched alkylene group and/or which may be condensed with an unsubstituted or at least mono-substituted mono- or bicyclic ring system; or an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
R^{10q} and R^{11q}, independently of one another, each represent a linear or branched, saturated or unsaturated, unsubstituted or at least mono-substituted aliphatic radical; or an unsubstituted or at least mono-substituted aryl or heteroaryl radical, which may be bonded via a linear or branched alkylene group;
R^{15q} represents a hydrogen atom; a linear or branched, saturated or unsaturated, unsubstituted or at least mono-substituted aliphatic radical or a-S(=O)₂-R^{16q} moiety;
R^{16q} represents a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via a linear or branched, unsubstituted or at least mono-substituted alkylene, alkenylene or alkinylene group and/or which may be condensed with an unsubstituted or at least mono-substituted mono- or bicyclic ring system; or an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
D^{q} and E^{q} together with the bridging nitrogen form an unsubstituted or at least mono-substituted, saturated, unsaturated or aromatic heterocyclic ring which may contain at least one further heteroatom as a ring member and/or which may be condensed with an unsubstituted or at least mono-substituted mono-or bicyclic ring system;
or
D^{q} and E^{q} independently of one another, each represent a hydrogen atom; a linear or branched, saturated or unsaturated, unsubstituted or at least mono-substituted aliphatic radical; a saturated or unsaturated, unsubstituted or at least mono-substituted, optionally at least one heteroatom as a ring member containing cycloaliphatic radical, which may be bonded via a linear or branched alkylene group and/or which may be condensed with an unsubstituted or at least mono-substituted mono- or bicyclic ring system; or an unsubstituted or at least mono-substituted aryl or heteroaryl radical, which may be bonded via a linear or branched, unsubstituted or at least mono-substituted alkylene, alkenylene or alkinylene group and/or which may be condensed with an unsubstituted or at least mono-substituted mono- or bicyclic ring system;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof, respectively.

7. The combination according to one or more of claims 1 to 6, **characterized in that** as component (B) at least one compound is present which is selected from the group consisting of
3-((-)-2-carboxypiperazin-4-ylpropyl-1-phosphate (CPP); 3-(2-carboxypiperazin-4-yl)-propenyl-1-phosphonate (CPP-ene); 1-(cis-2-carboxypiperidine-4-yl)methyl-1-phosphonic acid (CGS 19755); D-2-Amino-5-phosphonopentanoic acid (AP5); 2-amino-phosphonoheptanoate (AP7); D,L-(E)-2-amino-4-methyl-5-phosphono-3-pentenoic acid carboxyethyl ester (CGP39551); 2-amino-4-methyl-5-phosphono-pent-3-enoic acid (CGP 40116); (4-phosphono-but-2-enylamino)-acetic acid (PD 132477); 2-amino-4-oxo-5-phosphono-pentanoic acid (MDL 100,453); 3-((phosphonylmethyl)-sulfinyl)-D,L-alanine; amino-(4-phosphonomethyl-phenyl)-acetic acid (PD 129635); 2-amino-3-(5-chloro-1-phosphonomethyl-1H-benzoimidazol-2-yl)-propionic acid; 2-amino-3-(3-phosphonomethyl-quinoxalin-2-yl)-propionic acid; 2-amino-3-(5-phosphonomethyl-biphenyl-3-yl)-propionic acid (SDZ EAB 515); 2-amino-3-[2-(2-phosphono-ethyl)-cyclohexyl]-propionic acid (NPC 17742); 4-(3-phosphono-propyl)-piperazine-2-carboxylic acid (D-CPP); 4-(3-phosphono-allyl)-piperazine-2-carboxylic acid (D-CPP-ene); 4-phosphonomethyl-piperidine-2-carboxylic acid (CGS 19755); 3-(2-phosphono-acetyl)-piperidine-2-carboxylic acid (MDL 100,925); 5-phosphono-1,2,3,4-tetrahydro-isoquinoline-3-carboxylic acid (SC 48981); 5-(2-phosphono-ethyl)-1,2,3,4-tetrahydro-isoquinoline-3-carboxylic acid (PD 145950); 6-phosphonomethyl-decahydro-isoquinoline-3-carboxylic acid (LY 274614); 4-(1H-tetrazol-5-ylmethyl)-piperidine-2-carboxylic acid (LY 233053 and 235723); 6-(1H-Tetrazol-5-ylmethyl)-decahydro-isoquinoline-3-carboxylic acid (LY 233536); ketamine; phencyclidine; dextromethorphan; dextrorphan; dexoxadrol; dizocilpine (MK-801); remacemide; thienylcyclohexylpiperidine (TCP) ; N-allylnometazocine (SKF 10,047); cyclazocine; etoxadrol; (1,2,3,4,9,9a-hexahydro-fluoren-4a-yl)-methyl-amine (PD 137889); (1,3,4,9,10,10a-hexahydro-2H-phenanthren-4a-yl)-methyl-amine (PD 138289); PD 138558; tiletamine; kynurenic acid; 7-chloro-kynurenic acid; memantine; quinoxalinediones such as 6-cyano-7-nitroquinoxaline-2,3-dione (CNQX) and 6,7-dinitro-quinoxaline-2-,3-dione (DNQX); amantadine; eliprodil; iamotrigine; riluzole; aptiganel; flupirtine; celfotel; levemopamil; 1-(4-hydroxy-phenyl)-2-(4-phenylsulfanyl-piperidin-1-yl)-propan-1-one; 2-[4-(4-fluoro-benzoyl)-piperidin-1-yl]-1-naphthalen-2-yl-ethanone (E 2001); 3-(1,1-dimethyl-heptyl)-9-hydroxymethyl-6,6-dimethyl-6a,7,8,10a-tetrahydro-6H-benzo[c]chromen-1-ol (HU-211); 1-{4-[1-(4-chloro-phenyl)-1-methyl-ethyl]-2-methoxy-phenyl}-1H-[1,2,4]triazole-3-carboxylic acid amide (CGP 31358); acetic acid 10-hydroxy-7,9,7',9'-tetramethoxy-3,3'-dimethyl-3,4,3',4'-tetrahydro-1H,1'H-[5,5']bi[benzo[g]isochromenyl]-4-yl ester (ES 242-1); 14-hydroxy-11-isopropyl-10-methyl-5-octyl-10,13-diaza-tricyclo[6.6.1.04,1-5]pentadeca-1,4,6,8(15)-tetraen-12-one; and 4,5-dioxo-4,5-dihydro-1H-benzo-[g]indole-2,7,9-tricarboxylic acid (PQQ); preferably memantine is present as component (B).

8. The combination according to one or more of claims 1 to 7, **characterized in that** it comprises 1 - 99% by weight of component (A) and 99 - 1 % by weight of component (B), more preferably 10 - 80% by weight of component (A) and 80 - 20% by weight of component (B), in each case referring to the total weight of both components (A) and (B).

9. A medicament comprising an active substance combination according to one or more of claims 1 to 8 and optionally one or more pharmacologically acceptable adjuvants.

10. A medicament according to claim 9, for simultaneous NMDA-receptor inhibitionand 5-HT₆-receptor regulation.

11. A medicament according to claim 9 or 10 for regulation of appetite, for maintenance, increase or reduction of body weight, for prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Metabolic Syndrome, Peripheral Nervous System Disorders, Central Nervous System Disorders, arthritis, epilepsy, anxiety, panic, depression, cognitive disorders, memory disorders, cardiovascular diseases, senile dementia processes, such as Alzheimer's, Parkinson's and/or Huntington's Disease, schizophrenia, psychosis, infantile hyperkinesia (ADHD, attention deficit / hyperactivity disorder), pain, hypertensive syndrome, inflammatory diseases, immunologic diseases or for improvement of cognition.

12. Use of the combination according to one or more of claims 1 to 8 for the manufacture of a medicament for simultaneous NMDA-receptor inhibition and 5-HT₆-receptor regulation.

13. Use of the combination according to one or more of to one or more of claims 1 to 8, for the manufacture of a medicament for the prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Metabolic Syndrome, Peripheral Nervous System Disorders, Central Nervous System Disorders, arthritis, epilepsy, anxiety, panic, depression, cognitive disorders, memory disorders, cardiovascular diseases, senile dementia processes, such as Alzheimer's, Parkinson's and/or Huntington's Disease, schizophrenia, psychosis, infantile hyperkinesia (ADHD, attention deficit / hyperactivity disorder), pain, hypertensive syndrome, inflammatory diseases, immunologic diseases or for improvement of cognition.

14. A pharmaceutical formulation, **characterized in that** it comprises an active substance combination according to one or more of claims 1 to 8 and optionally one or more pharmacologically acceptable adjuvants.

15. The pharmaceutical formulation according to claim 14, **characterized in that** it is present in solid pharmaceutical forms such as tablets, tablets, chewing tablets, chewing gums, dragées, capsules, suppositories, powder preparations, transdermal therapeutic systems, transmucosal therapeutic systems, or in liquid and semi-liquid pharmaceutical forms such as drops or such as juice, sirup, solution, emulsion, suspension, preferably in form of tablets, capsules, drops or solution.

16. The pharmaceutical formulation according to claim 14, **characterized in that** it is present in form of of multiple particles, preferably microtablets, microcapsules, microspheroids, granules, crystals or pellets, optionally compacted in a tablet, filled in a capsule or suspended in a suitable liquid.

17. The pharmaceutical formulation according to one or more of claims 14 to 16, **characterized in that** it is for oral, intravenous, intramuscular, subcutaneous, intrathecal, epidural, buccal, sublingual, pulmonal, rectal, transdermal, nasal or intracerebroventricular application, preferably oral or intravenous.

18. The pharmaceutical formulation according to one or more of claims 14 to 16, **characterized in that** at least one of the components of the active substance combination (A) or (B) is present at least partially in sustained-release form.

19. The pharmaceutical formulation according to claim 18, **characterized in that** the medicament has at least one coating or at least one matrix comprising at least one material, which sustains active substance release.

20. The pharmaceutical formulation according to claim 19, **characterized in that** the sustained-release material is based on optionally modified, water-insoluble, natural, semisynthetic or synthetic polymer, or a natural wax or fat or fatty alcohol or semisynthetic or synthetic fatty acid, or on a mixture of at least two of these afore mentioned components.

21. The pharmaceutical formulation according to claim 20, **characterized in that** the water-insoluble polymer is based on an acrylic resin, which is preferably selected from the group of poly(meth)acrylates, poly(C₁₋₄)dialkylamino(C₁₋₄₎alkyl (meth)acrylates and/or copolymers thereof or a mixture of at least two of the afore-mentioned polymers.

22. The pharmaceutical formulation according to claim 20, **characterized in that** the water-insoluble polymers are cellulose derivatives, preferably alkyl cellulose and even more preferably ethyl cellulose, or cellulose esters.

23. The pharmaceutical formulation according to claim 20, **characterized in that** the wax is carnauba wax, beeswax, glycerol monostearate, glycerol monobehenate, glycerol ditripalmitostearate, microcrystalline wax or a mixture of at least two of these components.

24. The pharmaceutical formulation according to one or more of claims 20 to 23, **characterized in that** polymers have been used in combination with one or more plasticizers.

25. The pharmaceutical formulation according to one or more of claims 18 to 24, **characterized in that** besides the sustained-release form, at least one of the active substance components (A) or (B) is present in a non-sustained-release form.
